# EUROPEAN PATENT APPLICATION

(11) **EP 2 450 358 A1**
(43) Date of publication of application: **09.05.2012**
(21) Application number: 11181803.5
(22) Date of filing: 01.06.2007
(51) Int. Cl.: C07D 471/04, C07D 471/14, C07D 513/14, A61K 31/519, A61K 31/4745, A61K 31/437, A61K 31/4375, A61P 25/00

(54) **Fused, tricyclic sulfonamide inhibitors of gamma secretase**

(30) Priority: 02.06.2006 US 810431 P; 22.12.2006 US 876932 P; 10.04.2007 US 922771 P
(62) Divisional of application: 07797980.5
(71) Applicant: Elan Pharmaceuticals Inc., South San Francisco, CA 94080 (US)
(72) Inventor: Konradi, Andrei W., Burlingame, CA California 94010 (US); Probst, Gary, San Francisco, CA California 94102 (US); Aubele, Danielle L., Burlingame, CA California 94010 (US); Garofalo, Albert W., South San Francisco, CA California 94080 (US); Hom, Roy, San Francisco, CA California 94102 (US); Neitzel, Martin L., Pacifica, CA California 94044 (US); Semko, Christopher M., Fremont, CA California 94536 (US); Truong, Anh P., Burlingame, CA California 94010 (US)
(74) Representative: Cabinet Plasseraud

(57) **Abstract**

The invention provides compounds of formula I: 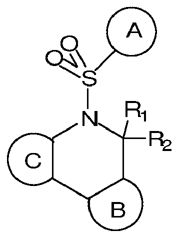 or pharmaceutically salts thereof, where R₁, R₂ and the A, B, and C-rings are as defined herein. Compounds of formula I are useful in treating or preventing cognitive disorders, such as Alzheimer's disease. The invention also encompasses pharmaceutical compositions comprising compounds or salts of formula I, methods of preparing the desired compunds, and methods of treating cognitive disorders, such as Alzheimer's disease, using the compounds or salts of formula I.

## Description

This application claims priority from U.S. Provisional Application No. 60/810,431, filed June 2, 2006; U.S. Provisional Application No. 60/876,932, filed December 22, 2006; and U.S. Provisional Application No. 60/922,771, filed April 10, 2007. Each reference is incorporated by reference in its entirety.

### Background of the Invention

### Field of the Invention

The invention relates to fused, tricyclic sulfonamido compounds, which inhibit gamma secretase, ß-amyloid peptide release and/or its synthesis. Therefore, the fused, tricyclic sulfonamido compounds are useful in the prevention of cognitive disorders, such as Alzheimer's disease, in patients susceptible to cognitive disorders and/or in the treatment of patients with cognitive disorders in order to inhibit further deterioration in their condition. The compounds of the invention are also useful for initiating or increasing angiogenesis.

### State of the Art

Alzheimer's Disease (AD) is a degenerative brain disorder characterized clinically by progressive loss of memory, cognition, reasoning, judgment and emotional stability that gradually leads to profound mental deterioration and ultimately death. AD is a very common cause of progressive mental failure (dementia) in aged humans and is believed to represent the fourth most common medical cause of death in the United States. AD has been observed in races and ethnic groups worldwide and presents a major present and future public health problem. The disease is currently estimated to affect about two to three million individuals in the United States alone. AD is at present incurable. No treatment that effectively prevents AD or reverses its symptoms and course is currently known.

The brains of individuals with AD exhibit characteristic lesions termed senile (or amyloid) plaques, amyloid angiopathy (amyloid deposits in blood vessels) and neurofibrillary tangles. Large numbers of these lesions, particularly amyloid plaques and neurofibrillary tangles, are generally found in several areas of the human brain important for memory and cognitive function in patients with AD. Smaller numbers of these lesions in a more restrictive anatomical distribution are also found in the brains of most aged humans who do not have clinical AD. Amyloid plaques and amyloid angiopathy also characterize the brains of individuals with Trisomy 21 (Down's Syndrome) and Hereditary Cerebral Hemorrhage with Amyloidosis of the Dutch Type (HCHWA-D). At present, a definitive diagnosis of AD usually requires observing the aforementioned lesions in the brain tissue of patients who have died with the disease or, rarely, in small biopsied samples of brain tissue taken during an invasive neurosurgical procedure.

The principal chemical constituent of the amyloid plaques and vascular amyloid deposits (amyloid angiopathy) characteristic of AD and the other disorders mentioned above is an approximately 4.2 kilodalton (kD) protein of about 39-43 amino acids designated the ß-amyloid peptide (ßAP) or sometimes Aß, AßP or ß/A4. ß-Amyloid peptide was first purified and a partial amino acid sequence was provided by Glenner et al., Biochem. Biophys. Res. Commun., 120:885-890 (1984) The isolation procedure and the sequence data for the first 28 amino acids are described in U.S. Patent No. 4,666,829.

Molecular biological and protein chemical analyses have shown that the ß-amyloid peptide is a small fragment of a much larger precursor protein termed the amyloid precursor protein (APP), that is normally produced by cells in many tissues of various animals, including humans. Knowledge of the structure of the gene encoding APP has demonstrated that ß-amyloid peptide arises as a peptide fragment that is cleaved from APP by protease enzyme(s). Sequential processing of the precursor protein by the enzymes referred to generically as beta- and gamma-secretases, give rise to the ß-amyloid peptide fragment. Both enzymes have now been molecularly cloned, and characterized to differing levels.

Several lines of evidence indicate that progressive cerebral deposition of ß-amyloid peptide plays a seminal role in the pathogenesis of AD and can precede cognitive symptoms by years or decades. See, for example, Selkoe, Neuron, 6:487-498 (1991). The most important line of evidence is the discovery that missense DNA mutations at amino acid 717 of the 770-amino acid isoform of APP can be found in affected members but not unaffected members of several families with a genetically determined (familial) form of AD (A. Goate, et al. Nature, 349:704-706 (1991); M-C. Chartier-Harlin, Nature, 353:844-846, (1991); and Murrell et al., Science, 254:97-99 (1991.) Another such mutation, known as the Swedish variant, is comprised of a double mutation changing lysine⁵⁹⁵-methionine⁵⁹⁶ to asparagine⁵⁹⁵-leucine⁵⁹⁶ (with reference to the 695 isoform was found in a Swedish family) was reported in 1992 (Mullan et al., Nature Genet., 1:345-347 (1992). Genetic linkage analyses have demonstrated that these mutations, as well as certain other mutations in the APP gene, are the specific molecular cause of AD in the affected members of such families. In addition, a mutation at amino acid 693 of the 770-amino acid isoform of APP has been identified as the cause of the ß-amyloid peptide deposition disease, HCHWA-D, and a change from alanine to glycine at amino acid 692 appears to cause a phenotype that resembles AD is some patients but HCHWA-D in others. The discovery of these and other mutations in APP in genetically based cases of AD prove that alteration of APP metabolism, and subsequent deposition of its ß-amyloid peptide fragment, can cause AD.

Despite the progress which has been made in understanding the underlying mechanisms of AD and other ß-amyloid peptide related diseases, there remains a need to develop methods and compositions for treatment of the disease(s). Ideally, the treatment methods would advantageously be based on drugs, which are capable of inhibiting ß-amyloid peptide release and/or its synthesis *in vivo.*

One approach toward inhibiting amyloid peptide synthesis *in vivo* is by inhibiting gamma secretase, the enzyme responsible for the carboxy-terminal cleavage resulting in production of ß-amyloid peptide fragments of 40 or 42 residues in length. The immediate substrates for gamma secretase are β-cleaved, as well as α-cleaved carboxy-terminal fragments (CTF) of APP. The gamma-secretase cleavage site on β- and α-CTF fragments occurs in the predicted transmembrane domain of APP. Inhibitors of gamma-secretase have been demonstrated to effect amyloid pathology in transgenic mouse models (Dovey, H. F., V. John, J. P. Anderson, L. Z. Chen, P. de Saint Andrieu, L. Y. Fang, S. B. Freedman, B. Folmer, E. Goldbach, E. J. Holsztynska et al. (2001). "Functional gamma-secretase inhibitors reduce beta-amyloid peptide levels in brain." J Neurochem 76(1): 173-81.)

Gamma secretase is recognized to be a multi-subunit complex comprised of the presenilins (PS1 or PS2), Nicastrin, Aph-1, and Pen 2 (De Strooper, B. (2003). "Aph-1, Pen-2, and Nicastrin with Presenilin generate an active gamma-Secretase complex." Neuron 38(1): 9-12; Edbauer, D., E. Winkler, J. T. Regula, B. Pesold, H. Steiner and C. Haass (2003). "Reconstitution of gamma-secretase activity." Nat Cell Biol 5(5): 486-8; Kimberly, W. T., M. J. LaVoie, B. L. Ostaszewski, W. Ye, M. S. Wolfe and D. J. Selkoe (2003). "Gamma-secretase is a membrane protein complex comprised of presenilin, nicastrin, Aph-1, and Pen-2." Proc Natl Acad Sci U S A 100(11): 6382-7). Much evidence indicates that PS comprises the catalytic moiety of the complex, while the other identified subunits are necessary for proper maturation and sub-cellular localization of the active enzyme complex (reviewed in De Strooper, B. (2003). "Aph-1, Pen-2, and Nicastrin with Presenilin generate an active gamma-Secretase complex." Neuron 38(1): 9-12.) Consistent with this hypothesis: PS knock-out mice exhibit significant reductions in β-amyloid production (De Strooper, B., P. Saftig, K. Craessaerts, H. Vanderstichele, G. Guhde, W. Annaert, K. Von Figura and F. Van Leuven (1998). "Deficiency of presenilin-1 inhibits the normal cleavage of amyloid precursor protein." Nature 391(6665): 387-90; Haass, C. and D. J. Selkoe (1998). "Alzheimer's disease. A technical KO of amyloid-beta peptide." Nature 391(6665): 339-40; Herreman, A., L. Serneels, W. Annaert, D. Collen, L. Schoonjans and B. De Strooper (2000). "Total inactivation of gamma-secretase activity in presenilin-deficient embryonic stem cells." Nat Cell Biol 2(7): 461-2); point mutations of putative active site aspartate residues in PS transmembrane domains inhibit β-amyloid production in cells in a dominant negative fashion (Wolfe, M. S., W. Xia, B. L. Ostaszewski, T. S. Diehl, W. T. Kimberly and D. J. Selkoe (1999). "Two transmembrane aspartates in presenilin-1 required for presenilin endoproteolysis and gamma-secretase activity." Nature 398(6727): 513-7; Kimberly, W. T., W. Xia, T. Rahmati, M. S. Wolfe and D. J. Selkoe (2000). "The transmembrane aspartates in presenilin 1 and 2 are obligatory for gamma-secretase activity and amyloid beta-protein generation." J Biol Chem 275(5): 3173-8); active site directed substrate-based transition state isosteres designed to inhibit gamma secretase directly conjugate to PS (Esler, W. P., W. T. Kimberly, B. L. Ostaszewski, T. S. Diehl, C. L. Moore, J. Y. Tsai, T. Rahmati, W. Xia, D. J. Selkoe and M. S. Wolfe (2000). "Transition-state analogue inhibitors of gamma-secretase bind directly to presenilin-1." Nat Cell Biol 2(7): 428-34; Li, Y. M., M. Xu, M. T. Lai, Q. Huang, J. L. Castro, J. DiMuzio-Mower, T. Harrison, C. Lellis, A. Nadin, J. G. Neduvelil et al. (2000). "Photoactivated gamma-secretase inhibitors directed to the active site covalently label presenilin 1." Nature 405(6787): 689-94); finally, allosteric gamma secretase inhibitors have likewise been demonstrated to bind directly to PS (Seiffert, D., J. D. Bradley, C. M. Rominger, D. H. Rominger, F. Yang, J. E. Meredith, Jr., Q. Wang, A. H. Roach, L. A. Thompson, S. M. Spitz et al. (2000). "Presenilin-1 and -2 are molecular targets for gamma-secretase inhibitors." J Biol Chem 275(44): 34086-91.)

Current evidence indicates that in addition to APP processing leading to β-amyloid synthesis, gamma-secretase also mediates the intra-membrane cleavage of other type I transmembrane proteins (reviewed in Fortini, M. E. (2002). "Gamma-secretase-mediated proteolysis in cell-surface-receptor signaling." Nat Rev Mol Cell Biol 3(9): 673-84, see also Struhl, G. and A. Adachi (2000). "Requirements for presenilin-dependent cleavage of notch and other transmembrane proteins." Mol Cell 6(3): 625-36.) Noteworthy among the known substrates of gamma-secretase is mammalian Notch 1. The Notch 1 protein is important for cell fate determination during development, and tissue homeostasis in the adult. Upon ligand engagement via the Notch ecto-domain, Notch undergoes sequential extra-cellular and intra-membrane processing analogous to APP. The intra-membrane processing of Notch mediated by gamma secretase leads to release of the Notch intracellular domain (NICD). The NICD fragment mediates Notch signaling via translocation to the nucleus, where it regulates expression of genes mediating cellular differentiation in many tissues during development, as well as in the adult.

Disruption of Notch signaling via genetic knock-out (KO) results in embryonic lethal phenotype in mice (Swiatek, P. J., C. E. Lindsell, F. F. del Amo, G. Weinmaster and T. Gridley (1994). "Notch1 is essential for postimplantation development in mice." Genes Dev 8(6): 707-19; Conlon, R. A., A. G. Reaume and J. Rossant (1995). "Notch1 is required for the coordinate segmentation of somites." Development 121(5): 1533-45.) The Notch KO phenotype is very similar to the phenotype observed PS 1 KO mice, and precisely reproduced by PS1/PS2 double KO mice (De Strooper et al. (1998). "Deficiency of presenilin-1 inhibits the normal cleavage of amyloid precursor protein." Nature 391(6665): 387-90; Donoviel, D. B., A. K. Hadjantonakis, M. Ikeda, H. Zheng, P. S. Hyslop and A. Bernstein (1999). "Mice lacking both presenilin genes exhibit early embryonic patterning defects." Genes Dev 13(21): 2801-10; Herreman, A., L. Serneels, W. Annaert, D. Collen, L. Schoonjans and B. De Strooper (2000). "Total inactivation of gamma-secretase activity in presenilin-deficient embryonic stem cells." Nat Cell Biol 2(7): 461-2.) This convergence of phenotypes observed in knock-out mice of either the substrate (Notch) or the enzyme (PS) suggests that inhibitors of gamma secretase that also inhibit Notch function may be limited as therapeutic agents owing to the importance of Notch function in adult tissues (Fortini, M. E. (2002). "Gamma-secretase-mediated proteolysis in cell-surface-receptor signaling." Nat Rev Mol Cell Biol 3(9): 673-84.) As APP knock-out mice develop normally and without an overt phenotype Zheng, H., M. Jiang, M. E. Trumbauer, R. Hopkins, D. J. Sirinathsinghji, K. A. Stevens, M. W. Conner, H. H. Slunt, S. S. Sisodia, H. Y. Chen et al. (1996). "Mice deficient for the amyloid precursor protein gene." Ann N Y Acad Sci 777: 421-6; Zheng, H., M. Jiang, M. E. Trumbauer, D. J. Sirinathsinghji, R. Hopkins, D. W. Smith, R. P. Heavens, G. R. Dawson, S. Boyce, M. W. Conner et al. (1995). "beta-Amyloid precursor protein-deficient mice show reactive gliosis and decreased locomotor activity." Cell 81(4): 525-31, the cumulative evidence, therefore, suggests that preferred gamma secretase inhibitors would have selectivity for inhibiting gamma secretase processing of APP over gamma secretase processing of Notch.

Acute inhibition of gamma secretase by DAPT in-vivo was demonstrated to lead to reduction of Aβ in the brain of PDAPP mice. Dovey, H. F., John, V., et al. (2001). "Functional gamma-secretase inhibitors reduce beta-amyloid peptide levels in brain." J Neurochem 76(1): 173-81.

Gamma-secretase inhibitors have also been shown to increase angiogenesis. See US 2006/0264380. As such, the gamma secretase inhibitors of the invention are useful in promoting angiogenesis.

### Summary of the Invention

In a broad aspect, the invention provides compounds of Formula I: 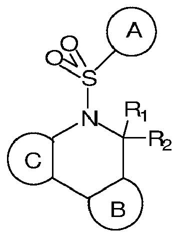 stereoisomers, tautomers, hydrates, mixtures of stereoisomers and/or tautomers, or pharmaceutically acceptable salts thereof, wherein
the C-ring is cycloalkyl, aryl, heterocycloalkyl, or heteroaryl, each of which is optionally substituted with 1, 2, 3, or 4 groups that are independently halogen, C₁-C₆ alkyl, C₁-C₆ alkoxy, aryloxy, aryl-(C₁-C₆alkoxy), C₁-C₆ haloalkyl (such as CF₃), C₁-C₆ haloalkoxy (such as OCF₃), hydroxyl, hydroxy-(C₁-C₆ alkyl), -NR'R", -(C₁-C₆ alkyl)-NR'R", -NR'C(O)OR', -COR', -C(O)OR', -C(O)NR'R", -OC(O)NR'R", -NR'C(O)NR'R"; - (C₁-C₆ alkyl)-C(O)OR', -(C₁-C₆ alkyl)-C(O)NR'R", -NR'C(O)R", NO₂, CN, oxo, aryl-(C₁-C₆ alkyl), aryl, heteroaryl, C₂-C₆ alkenyl, C₂-C₆ alkynyl, -NR'SO₂R", -SO₂-NR'R", -S(O)_{z} aryl (where the aryl group is preferably naphthyl or phenyl, still more preferably, phenyl), or -S(O)_{z} (C₁-C₆ alkyl), where the alkyl, alkenyl and alkynyl portions of the above are unsubstituted or substituted with 1, 2 or 3 groups that are independently halogen, hydroxyl, -NR'R" or C₁-C₆ alkoxy, and where the aryl or heteroaryl portions of the above are optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R"; or where two adjacent substituents form -O-(CH₂)₁₋₃-O-;
the A-ring is aryl, cycloalkyl, heteroaryl or heterocycloalkyl, where each ring is optionally substituted at a substitutable position with halogen, C₁-C₆ alkyl, C₂-C₆ alkenyl, C₂-C₆ alkynyl, C₁-C₆ alkoxy, C₁-C₆ haloalkyl, C₁-C₆ haloalkoxy, hydroxyl, hydroxy-(C₁-C₆ alkyl), CN, NO₂, aryloxy, aryl-(C₁-C₆ alkoxy)-, -SO₂-(C₁-C₆ alkyl), -NR'R", C₁-C₆ alkanoyl, -C(O)OR', -(C₁-C₆ alkyl)-C(O)OR', heteroaryl, aryl, aryl-(C₁-C₆ alkyl)-, - SO₂-NR'R", -C(O)NR'R", -OC(O)NR'R", -NR'C(O)R", -NR'C(O)NR'R", or -NR'C(O)OR';
the B-ring is a heteroaryl or heterocycloalkyl ring, each of which is optionally substituted at a substitutable position with a group that is independently C₁-C₆ alkyl, C₁-C₆ alkoxy, -NR'R", -SO₂-NR'R", -C(O)NR'R", -NR'SO₂R -NR'SO₂NR", -NR'SO₂-(C₁-C₆ alkyl), -NR'SO₂-phenyl, -NR'C(O)R", -NR'C(O)NR'R", -NR'C(O)OR', -(C₁-C₆ alkyl)-NR'R", -(C₁-C₆ alkyl)-C(O)OR', -(C₁-C₆ alkyl)-C(O)NR'R", NO₂, CN, -C(O)OR', hydroxyl, hydroxy-(C₁-C₆ alkyl), -S(O)_{z} (C₁-C₆ alkyl), -S(O)_{z} aryl, halogen, C₁-C₆ haloalkyl, C₁-C₆ haloalkoxy, phenyl, C₁-C₆ alkanoyl, aryl-(C₁-C₆ alkanoyl) (where the aryl group is preferably naphthyl or phenyl, more preferably phenyl), aryl-(C₁-C₆ alkyl) (preferably phenethyl or benzyl, more preferably, benzyl), arylcarbonyl, heteroarylcarbonyl, or heteroaryl-(C₁-C₆ alkanoyl), where the heteroaryl group is preferably pyridyl, pyrimidyl, thienyl or furanyl; where the aryl or heteroaryl groups are optionally substituted with 1 to 5 groups that are independently halogen, hydroxyl, C₁-C₆ alkyl, C₁-C₆ alkoxy, C₁-C₆ alkanoyl, C₁-C₆ haloalkyl, C₁-C₆ haloalkoxy, CN or NO₂; and
   where each z is independently 0, 1, or 2;
R₁ is hydrogen or C₁-C₆ alkyl;
R₂ is hydrogen, C₁-C₆ alkyl, C₁-C₄ haloalkyl, hydroxyl, hydroxy-(C₁-C₄ alkyl), -NO₂, -CN, C₂-C₆ alkenyl, C₂-C₆ alkynyl, C₁-C₆ alkanoyl, -C(O)OR', -NR'R", -X(CO)Y, - (C(R₃₀)₂)₁₋₄X(CO)Y, unsubstituted C₃-C₆ cycloalkyl, C₃-C₆ cycloalkyl substituted with one or more (e.g., 1-4) R₅₀ groups, unsubstituted aryl, aryl substituted with one or more (e.g., 1-4) R₅₀ groups, unsubstituted heteroaryl; or heteroaryl substituted with one or more (e.g., 1-4) R₅₀ groups, unsubstituted heterocycloalkyl; or heterocycloalkyl substituted with one or more (e.g., 1-4) R₅₀ groups; where
   each R₃₀ is independently hydrogen or C₁-C₆ alkyl;
   each R₅₀ is independently selected from: halogen, C₁-C₆ alkyl, C₁-C₆ haloalkyl, -OH, -O-(C₁-C₆ alkyl), -OCF₃, -CN, -NR₆₀R₇₀, -C(O)O-(C₁-C₆ alkyl), -CONR₆₀R₇₀, -(C₁-C₆) alkyl-NR₆₀R₇₀, -NR₆₀COalkyl, -NR₆₀COaryl, -NR₆₀COheteroaryl, -NR₆₀CONR₆₀R₇₀,
   X is: -O-, -NH-, or -N(alkyl)-;
   Y is selected from -O-(C₁-C₆ alkyl), -O-phenyl, -NR₆₀R₇₀, or -N(R₃₀)(CH₂)₂-₆NR₆₀R₇₀;
   R₆₀ and R₇₀ are independently selected from: hydrogen, C₁-C₆ alkyl, cycloalkyl, arylalkyl; heteroarylalkyl; 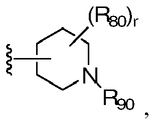 and 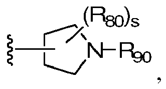 or
   R₆₀ and R₇₀ taken together with the nitrogen atom to which they are bound form a heterocycloalkyl group selected from: 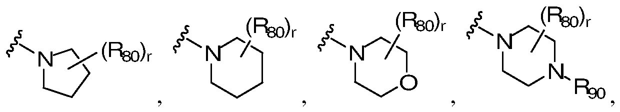 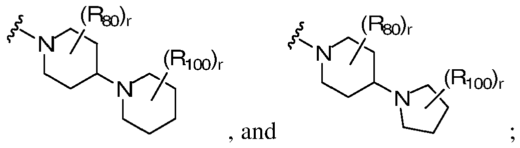 where
   each R₈₀ is independently unsubstituted C₁-C₆ alkyl or C₁-C₆ alkyl substituted with hydroxyl or halogen;
   each R₉₀ is independently H; unsubstituted C₁-C₆ alkyl; C₁-C₆ alkyl substituted with hydroxyl or halogen, unsubstituted cycloalkyl; cycloalkyl substituted with one or more (e.g., 1-4) R₅₀ groups; aryl-(C₁-C₆alkyl); heteroarylalkyl; -C(O)O-(C₁-C₆alkyl), -C(O)O-aryl; -SO_{Z}-(C₁-C₆alkyl); -SO_{z}-aryl; unsubstituted aryl; aryl substituted with one or more (e.g., 1-4) R₅₀ groups; heteroaryl; or heteroaryl substituted with one or more (e.g., 1-4) R₅₀ groups;
   each R₁₀₀ is independently hydrogen or C₁-C₆ alkyl;
   each r is 0 to 4;
   each s is 0 to 3; or
R₁ and R₂ combined are oxo or =N-OR where R is hydrogen, C₁-C₆ alkyl, aryl or arylalkyl; or
R₁ and R₂ together with the carbon to which they are attached form a C₃-C₆ cycloalkyl group wherein one of the carbons might be replaced with a heteroatom selected from N, O or S and wherein said C₃-C₆ cycloalkyl ring may be optionally substituted with C₁-C₆ alkyl or halogen; and
R' and R" are independently hydrogen, C₁-C₆ alkyl, or phenyl, where the phenyl is optionally substituted with 1 to 5 groups that are independently halogen, hydroxyl, C₁-C₆ alkyl, C₁-C₆ alkoxy, C₁-C₆ alkanoyl, C₁-C₄ haloalkyl, C₁-C₄ haloalkoxy, CN or NO₂, or
R' and R" taken together with the nitrogen atom to which they are bound form a 3 to 7 membered heterocycloalkyl group that may have an additional heteroatom selected from N, O or S and that may be optionally substituted with 1 to 3 R₈₀ or R₉₀ groups;
provided that 5-tosyl-4,5-dihydro-2H-pyrazolo[4,3-c]quinoline, 2-phenyl-5-tosyl-4,5-dihydro-2H-pyrazolo[4,3-c]quinoline, 7-methoxy-2-phenyl-5-tosyl-4,5-dihydro-2H-pyrazolo[4,3-c]quinoline, 8-methoxy-5-tosyl-4,5-dihydro-2H-pyrazolo[4,3-c]quinoline, 8-methoxy-2-phenyl-5-tosyl-4,5-dihydro-2H-pyrazolo[4,3-c]quinoline, 3-(methylthio)-5-tosyl-4,5-dihydro-2H-pyrazolo[4,3-c]quinoline, 7-methoxy-5-tosyl-4,5-dihydro-2H-pyrazolo[4,3-c]quinoline, 5-tosyl-4,5-dihydro-2H-pyrazolo[4,3-c]quinoline, ethyl 1-(4-sulfamoylphenyl)-5-tosyl-4,5-dihydro-1H-pyrazolo[4,3-c]quinoline-3-carboxylate, 1-(4-sulfamoylphenyl)-5-tosyl-4,5-dihydro-1H-pyrazolo[4,3-c]quinoline-3-carboxamide;and ethyl 1-methyl-5-tosyl-4,5-dihydro-1H-pyrazolo[4,3-c]quinoline-3-carboxylate, are not encompassed by formula I.

The compounds of Formula I inhibit β-amyloid peptide release and/or its synthesis and, therefore, are useful in the prevention of Alzheimer's Disease (AD) in patients susceptible to AD and/or in the treatment of patients with AD in order to inhibit further deterioration in their condition. The invention also, encompasses pharmaceutical compositions containing the compounds of Formula I, and methods employing such compounds or compositions in the treatment of cognitive diseases, including Alzheimer's disease.

The invention also provides a method of treating a patient who has, or in preventing a patient from getting, a disease or condition selected from the group consisting of Alzheimer's disease, for helping prevent or delay the onset of Alzheimer's disease, for treating patients with mild cognitive impairment (MCI) and preventing or delaying the onset of Alzheimer's disease in those who would progress from MCI to AD, for treating Down's syndrome, for treating humans who have Hereditary Cerebral Hemorrhage with Amyloidosis of the Dutch-Type, for treating cerebral amyloid angiopathy and preventing its potential consequences, i.e. single and recurrent lobar hemorrhages, for treating other degenerative dementias, including dementias of mixed vascular and degenerative origin, dementia associated with Parkinson's disease, dementia associated with progressive supranuclear palsy, dementia associated with cortical basal degeneration, age related macular degeneration or diffuse Lewy body type of Alzheimer's disease and who is in need of such treatment which comprises administration of a therapeutically effective amount of a compound of formula I.

The invention further provides a method of influencing a disease state in a cell, a group of cells, or an organism, comprising: administering at least one gamma-secretase inhibitor or a gamma-secretase pathway inhibitor of formula I, or a pharmaceutically acceptable salt thereof, to the cell, group of cells, or organism, wherein the disease is selected from the group consisting of atherosclerosis, hemangioma, hemangioendothelioma, vascular malformations, warts, pyogenic granulomas, hair growth, Kaposi's sarcoma, scar keloids, allergic edema, neoplasms, psoriasis, decubitus or stasis ulcers, gastrointestinal ulcers, dysfunctional uterine bleeding, follicular cysts, ovarian hyperstimulation, endometriosis, neoplasms, preeclampsia, placental insufficiency, respiratory distress, ascites, peritoneal sclerosis, adhesion formation, metastatic spreading, coronary artery disease, ischemic heart disease, ischemic limb disease, obesity, rheumatoid arthritis, synovitis, bone destruction, cartilage destruction, osteomyelitis, pannus growth, osterphyte formation, cancer, aseptic necrosis, impaired fracture healing, hepatitis, pneumonia, glomerulonephritis, asthma, nasal polyps, liver regeneration, pulmonary hypertension, systemic hypertension, diabetes, retinopathy of prematurity, diabetic retinopathy, choroidal disorders, intraocular disorders (e.g. age related macular degeneration), leukomafacia, stroke, vascular dementia, disease, thyroiditis, thyroid enlargement, thyroid pseudocyst, tumor metastasis, lymphoproliferative disorders, lympgoedema, AIDS, and hematologic malignancies.

Still further, the invention provides a method of increasing the angiogenic process in a cell, a group of cells, or an organism, comprising administering a pharmaceutical composition comprising a pharmaceutically effective amount of at least one gamma-secretase inhibitor or gamma-secretase pathway inhibitor of formula I, or a pharmaceutically acceptable salt thereof, to the cell, group of cells, or organism.

Further still, the invention provides a method of increasing the angiogenic process in a cell, a group of cells, or an organism, comprising administering a pharmaceutical composition comprising a pharmaceutically effective amount of at least one gamma-secretase inhibitor or gamma-secretase pathway inhibitor of formula I, or a pharmaceutically acceptable salt thereof, to the cell, group of cells, or organism, wherein the pharmaceutical composition is administered to prevent, treat, or cure a condition selected from the group consisting of atherosclerosis, hemangioma, hemangioendothelioma, vascular malformations, warts, pyogenic granulomas, hair growth, Kaposi's sarcoma, scar keloids, allergic edema, neoplasms, psoriasis, decubitus or stasis ulcers, gastrointestinal ulcers, dysfunctional uterine bleeding, follicular cysts, ovarian hyperstimulation, endometriosis, neoplasms, preeclampsia, placental insufficiency, respiratory distress, ascites, peritoneal sclerosis, adhesion formation, metastatic spreading, coronary artery disease, ischemic heart disease, eschemic limb disease, obesity, rheumatoid arthritis, synovitis, bone destruction, cartilage destruction, osteomyelitis, pannus growth, osterphyte formation, cancer, aseptic necrosis, impaired fracture healing, hepatitis, pneumonia, glomerulonephritis, asthma, nasal polyps, liver regeneration, pulmonary hypertension, systemic hypertension, diabetes, retinopathy of prematurity, diabetic retinopathy, choroidal disorders, intraocular disorders (e.g. age related macular degeneration), leukomafacia, stroke, vascular dementia, disease, thyroiditis, thyroid enlargement, thyroid pseudocyst, tumor metastasis, lymphoproliferative disorders, lympgoedema, AIDS, and hematologic malignancies.

The invention also provides a method for screening for a substance which initiates or increases angiogenesis, comprising: measuring an activity of a gamma-secretase pathway in the presence of a candidate compound in a suitable model; measuring an activity of a gamma-secretase pathway in the absence of a candidate compound; and comparing said activity in the presence of a candidate compound with said activity in the absence of the candidate compound, wherein a change in activity indicates that said candidate initiates or increases angiogenesis.

In another aspect, the invention provides methods of preparing the compounds of interest, as well as intermediates useful in preparing the compounds of interest.

The invention further provides a method of treating an A beta-related disease comprising administering a therapeutically effective amount of a compound or salt of Formula I to a patient in need of such treatment.

### Detailed Description of the Invention

As described above, the invention provides for compounds according to Formula I.

In another aspect, the invention provides compounds of formula 2, i.e., compounds of formula I wherein the A-ring is phenyl or naphthyl, each which is optionally substituted at a substitutable position with halogen, C₁-C₆ alkyl, C₂-C₆ alkenyl, C₂-C₆ alkynyl, C₁-C₆ alkoxy, C₁-C₆ haloalkyl, C₁-C₆ haloalkoxy, hydroxyl, hydroxy-(C₁-C₄ alkyl), CN, aryloxy, aryl-(C₁-C₄ alkoxy), -SO₂-(C₁-C₆ alkyl), -NR'R", C₁-C₆ alkanoyl, -C(O)OR', -(C₁-C₄alkyl)-C(O)OR', pyridyl, phenyl, phenyl-(C₁-C₄ alkyl), -SO₂-NR'R", -C(O)NR'R", -OC(O)NR'R", - NR'C(O)R", -NR'C(O)NR'R", or -NR'C(O)OR', where each R' and R" is independently hydrogen, C₁-C₆ alkyl or phenyl.

In still another aspect, the invention provides compounds of formula 3, i.e., compounds of formula I wherein the B-ring is pyrazolyl, imidazolyl, pyrrolyl, triazolyl, pyrrolidinyl, pyrazolidinyl, imidazolidinyl, pyridyl, pyrimidyl, or isoxazolyl, each of which is optionally substituted at a substitutable position with a group that is independently C₁-C₆ alkyl, C₁-C₆ alkoxy, -NR'R", -C(O)OR', -SO₂NR'R", -C(O)NR'R", -NR'SOR", -NR'SO₂NR'R", -NR'SO₂-(C₁-C₆ alkyl), -NR'SO₂-phenyl, -NR'C(O)R", -NR'C(O)NR'R", -NR'C(O)O-(C₁-C₆ alkyl), -NR'C(O)O-phenyl, -(C₁-C₄ alkyl)-NR'R", -(C₁-C₄ alkyl)-C(O)OR', -(C₁-C₄ alkyl)-C(O)NR'R", NO₂, CN, hydroxyl, hydroxy-(C₁-C₆ alkyl), -S(O)_{z} (C₁-C₆ alkyl), -S(O)_{z} phenyl, halogen, C₁-C₄ haloalkyl, C₁-C₄ haloalkoxy, phenyl, C₁-C₆ alkanoyl, phenyl-(C₁-C₄ alkanoyl), phenethyl, benzyl, phenylcarbonyl, heteroarylcarbonyl, or heteroaryl-(C₁-C₄ alkanoyl), where the heteroaryl group is pyridyl, pyrimidyl, thienyl or furanyl; where the phenyl or heteroaryl groups are optionally substituted with 1 to 5 groups that are independently halogen, hydroxyl, C₁-C₆ alkyl, C₁-C₆ alkoxy, C₁-C₆ alkanoyl, C₁-C₄ haloalkyl, C₁-C₄ haloalkoxy, CN or NO₂.

In yet another aspect, the invention provides compounds of formula 4, i.e., compounds of formula I wherein the A-ring is phenyl or naphthyl, each of which is optionally substituted at a substitutable position with halogen, C₁-C₆ alkyl, C₂-C₆ alkenyl, C₂-C₆ alkynyl, C₁-C₆ alkoxy, C₁-C₄ haloalkyl, C₁-C₄ haloalkoxy, hydroxyl, hydroxy-(C₁-C₄ alkyl), CN, phenyloxy, benzyloxy, -SO₂-(C₁-C₆ alkyl), -NR'R", C₁-C₆ alkanoyl, -C(O)OR', -(C₁-C₄alkyl)-C(O)OR', pyridyl, phenyl, phenyl-(C₁-C₄ alkyl), -SO₂-NR'R", -C(O)NR'R", -OC(O)NR'R", - NR'C(O)R", -NR'C(O)NR'R", or -NR'C(O)OR', where each R' and R" is independently H, C₁-C₆ alkyl or phenyl; and the B-ring is pyrazolyl, imidazolyl, pyrrolyl, triazolyl, pyrrolidinyl, pyrazolidinyl, imidazolidinyl, pyridyl, pyrimidyl, or isoxazolyl, each of which is optionally substituted at a substitutable position with a group that is independently C₁-C₆ alkyl, C₁-C₆ alkoxy, -NR'R", -SO₂-NR'R", -C(O)NR'R", -NR'C(O)R", -NR'C(O)NR'R", -NR'C(O)O-(C₁-C₆ alkyl), -NR'C(O)O-phenyl, NO₂, CN, -C(O)OR', hydroxyl, hydroxy-(C₁-C₆ alkyl), halogen, -S(O)_{z} (C₁-C₆ alkyl), -S(O)_{z} aryl, halogen, C₁-C₂ haloalkyl, C₁-C₂ haloalkoxy, benzyl, or phenyl.

In yet another aspect, the invention provides compounds of formula 4-1, i.e., compounds of formula I wherein the C-ring is cyclohexyl, cyclopentyl, phenyl, naphthyl, thienyl, imidazolyl, pyrimidyl, pyrazinyl, furanyl, thiazolyl, or pyridyl, each of which is optionally substituted at each substitutable position with groups that are independently halogen, C₁-C₆ alkyl, C₁-C₆ alkoxy, aryloxy, aryl-(C₁-C₄alkoxy), C₁-C₆ haloalkyl, (such as CF₃), C₁-C₆ haloalkoxy (such as OCF₃), hydroxyl, hydroxy-(C₁-C₆ alkyl), -NR'R", -(C₁-C₄ alkyl)-NR'R", -NR'C(O)O-(C₁-C₆ alkyl), -NR'C(O)O-phenyl, -COR', -C(O)OR', -C(O)NR'R", -OC(O)NR'R", -NR'C(O)NR'R", -(C₁-C₆ alkyl)-C(O)OR', -(C₁-C₆ alkyl)-C(O)NR'R", -NR'C(O)R", NO₂, CN, oxo, benzyl, phenyl, oxazolyl, pyrazolyl, thiazolyl, pyridyl, furanyl, thienyl, C₂-C₆ alkenyl, C₂-C₆ alkynyl, -NR'SO₂R", -SO₂-NR'R", -S(O)z (C₁-C₆ alkyl), or -S(O)_{z} aryl, where the alkyl, alkenyl and alkynyl portions of the above are unsubstituted or substituted with 1, 2 or 3 groups that are independently halogen, hydroxyl, - NR'R" or C₁-C₆ alkoxy, and where the aryl or heteroaryl portions of the above are optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R"; or
where two adjacent substituents of the C-ring C -O-(CH₂)₁-₃-O-.

In still another aspect, the invention provides compounds of formula 4-2, i.e., compounds of formula I, 4, or 4-1, wherein the B-ring is pyrazolyl, imidazolyl, pyrrolyl, triazolyl, pyrrolidinyl, pyrazolidinyl, imidazolidinyl, pyridyl, pyrimidyl, or isoxazolyl, each of which is optionally substituted at a substitutable position with a group that is independently C₁-C₆ alkyl, C₁-C₆ alkoxy, -NR'R", -SO₂-NR'R", -C(O)NR'R", -NR'C(O)R", -NR'C(O)NR'R", -NR'C(O)O-(C₁-C₆ alkyl), -NR'C(O)O-phenyl, NO₂, CN, -C(O)OR', hydroxyl, hydroxy-(C₁-C₆ alkyl), halogen, -S(O)_{z} (C₁-C₆ alkyl), -S(O)_{z} aryl, halogen, C₁-C₂ haloalkyl, C₁-C₂ haloalkoxy, benzyl, or phenyl.

In still another aspect, the invention provides compounds of formula 4-3, i.e., compounds of formula I, 4, or 4-1, wherein the B-ring has the formula: 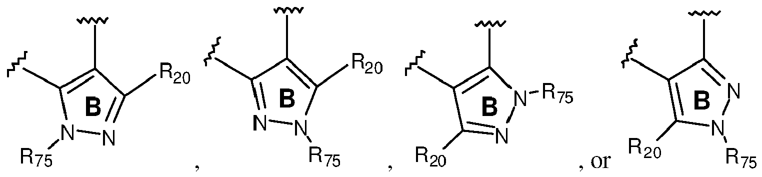 wherein
R₂₀ is hydrogen, C₁-C₆ alkyl, C₁-C₆ alkoxy, -NR'R", -(C₁-C₄ alkyl)-NR'R", -S(O)_{z} (C₁-C₆ alkyl), hydroxyl, halogen, CN, NO₂, CH₂F, CHF₂, CF₃, -C(O)OR', -C(O)NR'R", -(C₁-C₆ alkyl)-C(O)OR', -(C₁-C₆ alkyl)-C(O)NR'R", or phenyl, and
R₇₅ is hydrogen, C₁-C₆ alkyl, C₁-C₆ alkanoyl, phenyl-(C₁-C₆ alkanoyl)-, -C(O)NR'R", -S(O)_{z} (C₁-C₆ alkyl), -S(O)z-aryl, -SO₂NR'R", phenyl, phenyl-(C₁-C₆alkyl) (preferably phenethyl or benzyl, more preferably, benzyl), phenylcarbonyl, benzylcarbonyl, or heteroarylcarbonyl, where the heteroaryl group is pyridyl, pyrimidyl, thienyl or furanyl; where z is 0, 1, or 2.

In still another aspect, the invention provides compounds of formula 5, i.e., compounds of according to any one of formulas I, 4, 4-1, or 4-2, having the formula: 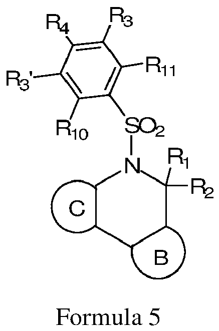 stereoisomers, tautomers, mixtures of stereoisomers and/or tautomers or pharmaceutically acceptable salts thereof, wherein
the C-ring is an optionally substituted cyclohexyl, phenyl, thienyl, imidazolyl, thiazolyl, isothiazolyl, oxazolyl, isoxazolyl, pyrrolyl, pyrazolyl, pyrimidyl, pyrazinyl, thiazolyl, or pyridyl;
R₃, R_{3'}, R₄, R₁₀ or R₁₁ are independently hydrogen, halogen, C₁-C₆ alkyl, C₂-C₆ alkenyl, C₂-C₆ alkynyl, C₁-C₆ alkoxy, C₁-C₆ haloalkyl, C₁-C₆ haloalkoxy, hydroxyl, hydroxy-(C₁-C₄ alkyl), CN, NO₂, aryloxy (such as phenyloxy), aryl-(C₁-C₄alkoxy) such as benzyloxy, -SO₂-(C₁-C₆ alkyl), -NR'R", C₁-C₆ alkanoyl, -C(O)OR', -(C₁-C₄ alkyl)-C(O)OR', pyridyl, phenyl, phenyl-(C₁-C₄ alkyl)-, -SO₂-NR'R", -C(O)NR'R", -OC(O)NR'R", -NR'C(O)R", -NR'C(O)NR'R", or -NR'C(O)O-R', where each R' and R" is independently hydrogen, C₁-C₆ alkyl, or phenyl, where the phenyl is optionally substituted with 1 to 5 groups that are independently halogen, hydroxyl, C₁-C₆ alkyl, C₁-C₆ alkoxy, C₁-C₆ alkanoyl, C₁-C₄ haloalkyl, C₁-C₄ haloalkoxy, CN or NO₂; or
R₄ and R_{3'}, or R₁₀ and R₃, and the carbons to which they are attached form a benzo ring.

In another aspect, the invention provides compounds of formula 6, i.e., compounds of formula 5, wherein the C-ring is cyclohexyl, phenyl, thienyl, imidazolyl, pyrimidyl, pyrazinyl, thiazolyl, or pyridyl, each of which is optionally substituted at each substitutable position with groups that are independently halogen, C₁-C₆ alkyl, C₁-C₆ alkoxy, aryloxy, aryl-(C₁-C₄alkoxy), C₁-C₆ haloalkyl, (such as CF₃), C₁-C₆ haloalkoxy (such as OCF₃), hydroxyl, hydroxy-(C₁-C₆ alkyl), -NR'R", -(C₁-C₄ alkyl)-NR'R", -NR'C(O)O-(C₁-C₆ alkyl), -NR'C(O)O-phenyl, -COR', -C(O)OR', -C(O)NR'R", - OC(O)NR'R", -NR'C(O)NR'R", -(C₁-C₆ alkyl)-C(O)OR', -(C₁-C₆ alkyl)-C(O)NR'R", -NR'C(O)R", NO₂, CN, oxo, benzyl, phenyl, oxazolyl, pyrazolyl, thiazolyl, pyridyl, furanyl, thienyl, C₂-C₆ alkenyl, C₂-C₆ alkynyl, -NR'SO₂R", -SO₂-NR'R" -S(O)_{z} (C₁-C₆ alkyl), or -S(O)_{z} aryl, where the alkyl, alkenyl and alkynyl portions of the above are unsubstituted or substituted with 1, 2 or 3 groups that are independently halogen, hydroxyl, -NR'R" or C₁-C₆ alkoxy, and where the aryl or heteroaryl portions of the above are optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R"; or
where two adjacent substituents of the C-ring C -O-(CH₂)₁₋₃-O-.

In still another aspect, the invention provides compounds of formula 6-1, i.e., compounds of formula 6 wherein the B-ring is pyrazolyl, imidazolyl, pyrrolyl, triazolyl, pyrrolidinyl, pyrazolidinyl, imidazolidinyl, or isoxazolyl, each of which is unsubstituted.

In still another aspect, the invention provides compounds of formula 6-2, i.e., compounds of formula 6 wherein the B-ring has the formula: 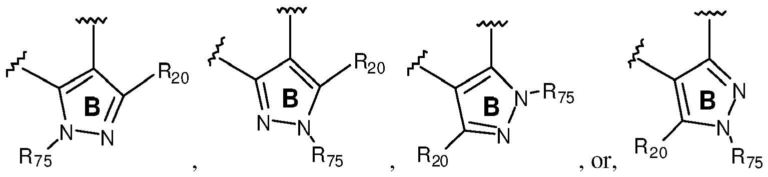 wherein
R₂₀ is hydrogen, C₁-C₆ alkyl, C₁-C₆ alkoxy, -NR'R", -(C₁-C₄ alkyl)-NR'R", -S(O)_{z} (C₁-C₆ alkyl), hydroxyl, halogen, CN, NO₂, CH₂F, CHF₂, CF₃, -C(O)OR', -C(O)NR'R", -(C₁-C₆ alkyl)-C(O)OR', -(C₁-C₆ alkyl)-C(O)NR'R", or phenyl, and
R₇₅ is hydrogen, C₁-C₆ alkyl, C₁-C₆ alkanoyl, phenyl-(C₁-C₆ alkanoyl)-, -C(O)NR'R", -S(O)_{z} (C₁-C₆ alkyl), -S(O)z -aryl, -SO₂NR'R", phenyl, phenyl-(C₁-C₆alkyl) (preferably phenethyl or benzyl, more preferably, benzyl), phenylcarbonyl, benzylcarbonyl, or heteroarylcarbonyl, where the heteroaryl group is pyridyl, pyrimidyl, thienyl or furanyl.

In yet another aspect, the invention provides compounds of formula 6-3, i.e., compounds of formula 6-2 wherein the B-ring has the formula:

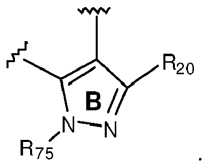

In yet another aspect, the invention provides compounds of formula 6-4, i.e., compounds of formula 6-2 wherein the B-ring has the formula:

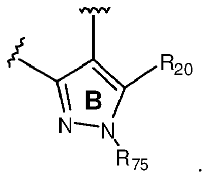

In yet another aspect, the invention provides compounds of formula 6-5, i.e., compounds of formula 6-2 wherein the B-ring has the formula:

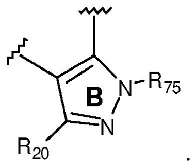

In yet another aspect, the invention provides compounds of formula 6-6, i.e., compounds of formula 6-2 wherein the B-ring has the formula:

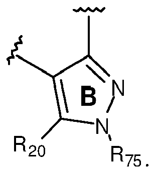

In a further aspect, the invention provides compounds of formula 6-6a, i.e., compounds according to any one of formulas 6-2, 6-3, 6-4, 6-5, or 6-6, wherein R₂₀ is hydrogen, C₁-C₄ alkyl, CH₂F, CHF₂, CF₃, -C(O)OR', -S(O)_{z} (C₁-C₄ alkyl), or hydroxyl; and R₇₅ is hydrogen, C₁-C₄ alkyl, or -S(O)_{z} -phenyl.

In another aspect, the invention provides compounds of formula 6-6b, i.e., compounds of formula 6-6a, wherein R₂₀ is hydrogen or methyl, and R₇₅ is hydrogen or methyl.

In yet another aspect, the invention provides compounds of formula 6-6c, i.e., compounds of formula 6-6a, wherein R₂₀ is hydrogen or methyl, and R₇₅ is hydrogen.

In still yet another aspect, the invention provides compounds of formula 6-6d, i.e., compounds of formula 6-6a, wherein R₂₀ is hydrogen and R₇₅ is hydrogen.

In yet another aspect, the invention provides compounds of formula 6-7, i.e., compounds of formula 6 wherein the B-ring has the formula: 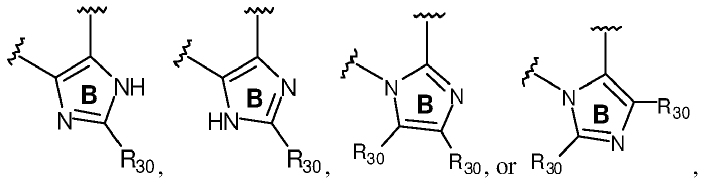 wherein each R₃₀ is independently hydrogen, C₁-C₄ alkyl, C₁-C₄ alkoxy, -NR'R", C₁-C₄ alkythio, hydroxyl, halogen, CH₂F, CHF₂, CF₃, or phenyl.

In still yet another aspect, the invention provides compounds of formula 6-8, i.e., compounds of formula 6-7 wherein the B-ring has the formula:

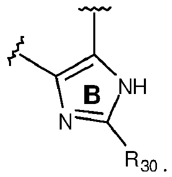

In yet another aspect, the invention provides compounds of formula 6-9, i.e., compounds of formula 6-7 wherein the B-ring has the formula:

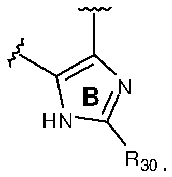

In yet another aspect, the invention provides compounds of formula 6-10, i.e., compounds of formula 6-7 wherein the B-ring has the formula:

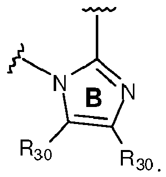

In yet another aspect, the invention provides compounds of formula 6-11, i.e., compounds of formula 6-7 wherein the B-ring has the formula:

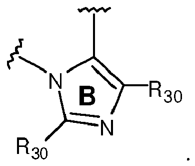

In a further aspect, the invention provides compounds of formula 6-11a, i.e., compounds according to any one of formulas 6-8, 6-9, 6-10, or 6-11, wherein each R₃₀ is independently hydrogen, C₁-C₄ alkyl, CH₂F, CHF₂, or CF₃.

In another aspect, the invention provides compounds of formula 6-11b, i.e., compounds of formula 6-11a, wherein each R₃₀ is independently hydrogen or methyl.

In still another aspect, the invention provides compounds of formula 6-11c, i.e., compounds of formula 6-11a, wherein each R₃₀ is hydrogen.

In yet still another aspect, the invention provides compounds of formula 6-12, i.e., compounds of formula 6 wherein the B-ring has the formula:

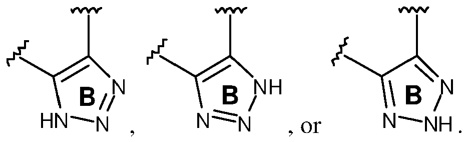

In yet another aspect, the invention provides compounds of formula 6-13, i.e., compounds of formula 6-12 wherein the B-ring has the formula:

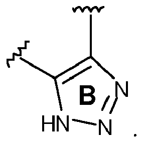

In yet another aspect, the invention provides compounds of formula 6-14, i.e., compounds of formula 6-12 wherein the B-ring has the formula:

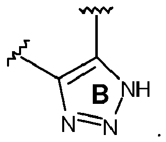

In yet another aspect, the invention provides compounds of formula 6-15, i.e., compounds of formula 6-12 wherein the B-ring has the formula:

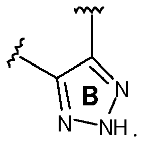

In still another aspect, the invention provides compounds of formula 7, i.e., compounds of formulas 4, 4-1, 4-2, 4-3, 5, or any one of formulas 6, 6-1, 6-2, 6-3, 6-4, 6-5, 6-6, 6-6a, 6-6b, 6-6c, 6-6d, 6-7, 6-8, 6-9, 6-10, 6-11, 6-11a, 6-11b, 6-11c, 6-12, 6-13, 6-14, or 6-15 wherein the C-ring is phenyl or cyclohexyl, which is optionally substituted with 1, 2, 3, or 4 groups that are independently halogen, C₁-C₄ alkyl, C₁-C₄ alkoxy, aryloxy, aryl-(C₁-C₄ alkoxy), C₁-C₄ haloalkyl (in another aspect, CF₃), C₁-C₄ haloalkoxy (in another aspect, OCF₃), hydroxyl, hydroxy-(C₁-C₄ alkyl), -NR'R", -(C₁-C₄ alkyl)-NR'R", -NR'C(O)O-(C₁-C₆ alkyl), -NR'C(O)O-phenyl, -C(O)OR', -C(O)NR'R", -OC(O)NR'R", -NR'C(O)NR'R"; -(C₁-C₆ alkyl)-C(O)OR', -(C₁-C₆ alkyl)-C(O)NR'R", -NR'C(O)R", NO₂, CN, oxo, benzyl, phenyl, oxazolyl, pyrazolyl, thiazolyl, pyridyl, furanyl, thienyl, C₂-C₆ alkenyl, C₂-C₆ alkynyl, -S(O)_{z} phenyl, or -S(O)_{z} (C₁-C₆ alkyl), where the alkyl, alkenyl and alkynyl portions of the above are unsubstituted or substituted with 1, 2 or 3 groups that are independently halogen, hydroxy, -NR'R" or C₁-C₆ alkoxy, and where the aryl or heteroaryl portions of the above are optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R" or
where two adjacent substituents from the C-ring form -O-(CH₂)₁-₃-O-. In one embodiment, the C-ring is an optionally substituted phenyl.

In another aspect, the invention provides compounds of formula 7-1, i.e., compounds of formula 7 wherein the C-ring is unsubstituted phenyl.

In another aspect, the invention provides compounds of formula 7-1a, i.e., compounds of formula 7 wherein the C-ring is unsubstituted cyclohexyl.

In another aspect, the invention provides compounds of formula 7-2, i.e., compounds of formula 7, wherein the C-ring is phenyl substituted with 1 or 2 groups that are independently Cl, F, methyl, ethyl, isopropyl, methoxy, ethoxy, CF₃, OCF₃, OH, CH₂OH, NH₂, NH(CH₃), N(CH₃)₂, or -OC(O)N(CH₃)₂. In one embodiment, the C-ring is bis-substituted with at least one halogen. In another embodiment, the halogen is F. In still another embodiment, the halogen is Cl. In another embodiment, the C-ring is bis-substituted with two halogens, which are the same or different. In still another embodiment, the C-ring is substituted at least at position 7. In another embodiment, the C-ring is substituted at positions 7 and 8. In another embodiment, the C-ring is monosubstituted with a halogen. In another embodiment, the halogen is F. In still another embodiment, the halogen is Cl. In still another embodiment, the C-ring is substituted at position 7. In yet another embodiment, the C-ring is substituted at position 8.

In another aspect, the invention provides compounds of formula 7-2a, i.e., compounds of formula 7, wherein the C-ring is cyclohexyl substituted with 1 or 2 groups that are independently Cl, F, methyl, ethyl, isopropyl, methoxy, ethoxy, CF₃, OCF₃, OH, CH₂OH, NH₂, NH(CH₃), N(CH₃)₂, or -OC(O)N(CH₃)₂.

In yet another aspect, the invention provides compounds of formula 7-3, i.e., compounds of formula 7, wherein the C-ring is phenyl substituted with 1 or 2 groups that are Cl, F, methyl, ethyl, isopropyl, methoxy, CF₃, OCF₃, OH, -OC(O)N(CH₃)₂ or with two adjacent substituents forming -O-(CH₂)₁-₃-O-. In one embodiment, the C-ring is monosubstituted with a halogen. In another embodiment, the halogen is Cl. In still another embodiment, the halogen is F. In another embodiment, the C-ring is substituted at the 7-position. In yet another embodiment, the C-ring is substituted at the 8-position. In another embodiment, the C-ring is bis-substituted at the 7- and 8-positions. In still another embodiment, the C-ring is substituted at the 7- and 8-positions, with a group that is halogen, methyl, or methoxy. In yet another embodiment, the two groups are the same. In a further embodiment, the two groups are the same, and are a halogen (such as F or Cl).

In yet another aspect, the invention provides compounds of formula 7-3a, i.e., compounds of formula 7, wherein the C-ring is cyclohexyl substituted with 1 or 2 groups that are Cl, F, methyl, ethyl, isopropyl, methoxy, CF₃, OCF₃, OH, or -OC(O)N(CH₃)₂.

In still yet another aspect, the invention provides compounds of formula 7-3b, i.e., compounds of formula 7, wherein the C-ring is phenyl substituted with 1 or 2 groups that are independently -NR'C(O)O-(C₁-C₆ alkyl), -NR'C(O)O-phenyl, -C(O)OR', -C(O)NR'R", -(C₁-C₆ alkyl)-C(O)OR', -(C₁-C₆ alkyl)-C(O)NR'R", -NR'C(O)R", -OC(O)NR'R", -NR'C(O)NR'R"; NO₂, CN, phenyl, -S(O)_{z} phenyl, or -S(O)_{z} (C₁-C₆ alkyl), where the phenyl portions of the above are optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl) or -NR'R". In one embodiment, the C-ring is substituted at least at the 7-position. In another embodiment, the C-ring is substituted at least at the 8-position.

In still yet another aspect, the invention provides compounds of formula 7-3c, i.e., compounds of formula 7, wherein the C-ring is cyclohexyl substituted with 1 or 2 groups that are independently -NR'C(O)O-(C₁-C₆ alkyl), -NR'C(O)O-phenyl, -C(O)OR', -C(O)NR'R", -(C₁-C₆ alkyl)-C(O)OR', -(C₁-C₄ alkyl)-C(O)NR'R", -NR'C(O)R", -OC(O)NR'R", -NR'C(O)NR'R"; NO₂, CN, phenyl, -S(O)_{z} phenyl, or -S(O)_{z} (C₁-C₆ alkyl), where the phenyl portions of the above are optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R".

In still another aspect, the invention provides compounds of formula 7-3d, i.e., compounds of formula 7, wherein the C-ring is phenyl substituted with 1 or 2 groups that are independently C₁-C₄ alkyl, C₂-C₆ alkenyl, C₂-C₆ alkynyl, where the alkyl, alkenyl and alkynyl portions of the above are unsubstituted or substituted with 1 or 2 groups that are independently halogen, hydroxy, -NR'R" or C₁-C₄ alkoxy. In one embodiment, the C-ring is substituted at least at the 7-position. In another embodiment, the C-ring is substituted at least at the 8-position.

In still another aspect, the invention provides compounds of formula 7-3e, i.e., compounds of formula 7, wherein the C-ring is cyclohexyl substituted with 1 or 2 groups that are independently C₁-C₄ alkyl, C₂-C₆ alkenyl, C₂-C₆ alkynyl, where the alkyl, alkenyl and alkynyl portions of the above are unsubstituted or substituted with 1 or 2 groups that are independently halogen, hydroxy, -NR'R" or C₁-C₄ alkoxy.

In still another aspect, the invention provides compounds of formula 7-3f, i.e., compounds of formula 7, wherein the C-ring is phenyl substituted with 1 or 2 groups that are independently -C(O)OH, -C(O)O-(C₁-C₆ alkyl), NO₂, CN, or phenyl, where the phenyl group is optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R". In one embodiment, the C-ring is substituted at least at the 7-position. In another embodiment, the C-ring is substituted at least at the 8-position.

In still another aspect, the invention provides compounds of formula 7-3g, i.e., compounds of formula 7, wherein the C-ring is cyclohexyl substituted with 1 or 2 groups that are independently -C(O)OH, -C(O)O-(C₁-C₆ alkyl), NO₂, CN, or phenyl, where the phenyl group is optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R".

In yet another aspect, the invention provides compounds of formula 7-3h, i.e., compounds of formula 7, wherein the C-ring is phenyl substituted with 1 or 2 groups that are independently C₁-C₄ alkyl, C₁-C₄ alkoxy, halogen, -C(O)NR'R", -NR'C(O)R", -OC(O)NR'R", -NR'C(O)NR'R", -NR'C(O)O-(C₁-C₆ alkyl), or -NR'C(O)O-phenyl, where the phenyl group is optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R". In one embodiment, the C-ring is substituted at least at the 7-position. In another embodiment, the C-ring is substituted at least at the 8-position.

In yet another aspect, the invention provides compounds of formula 7-3i, i.e., compounds of formula 7, wherein the C-ring is cyclohexyl substituted with 1 or 2 groups that are independently C₁-C₄ alkyl, C₁-C₄ alkoxy, halogen, -C(O)NR'R", -NR'C(O)R", -OC(O)NR'R", -NR'C(O)NR'R", -NR'C(O)O-(C₁-C₆ alkyl) or -NR'C(O)O-phenyl, where the phenyl group is optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R".

In another aspect, the invention provides compounds of formula 7-4, i.e., compounds of any one of formulas 7, 7-1, 7-1a, 7-2, 7-2a, 7-3, 7-3a, 7-3b, 7-3c, 7-3d, 7-3e, 7-3f, 7-3g, 7-3h, 7-3i, wherein the B-ring has the formula: 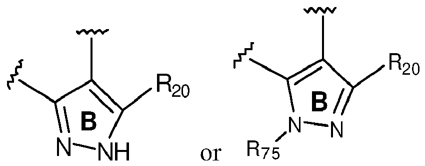 wherein R₂₀ is hydrogen, C₁-C₆ alkyl, C₁-C₆ alkoxy, -NR'R", -(C₁-C₄ alkyl)-NR'R", -S(O)_{z} (C₁-C₆ alkyl), hydroxyl, halogen, CN, NO₂, CH₂F, CHF₂, CF₃, -C(O)OR', -C(O)NR'R", -(C₁-C₆ alkyl)-C(O)OR', -(C₁-C₆ alkyl)-C(O)NR'R", or phenyl; and R₇₅ is H or C₁-C₄ alkyl (such as methyl or ethyl). In one embodiment, R₂₀ is hydrogen or methyl. In another embodiment, R₂₀ is hydrogen. In still another embodiment, R₂₀ and R₇₅ are both H.

In yet still another aspect, the invention provides compounds of formula 7-4a, i.e., compounds of formula 7-4, wherein R₄ is halogen, C₁-C₆ haloalkyl, or C₁-C₆ haloalkoxy; R₃, R₃, R₁₀ and R₁₁ are independently halogen, methyl or hydrogen; and R₂ is hydrogen, C₁-C₃ alkyl, or C₃-C₆ cycloalkyl. In one embodiment, R₄ is Cl, while R₃, R_{3'}, R₁₀ and R₁₁ are H.

In yet still another aspect, the invention provides compounds of formula 7-4aa, i.e., compounds of formula 7-4a, wherein R₄ is Cl, F, CH₂F, CHF₂, CF₃, OCH₂F, OCHF₂, or OCF₃; R₃, R_{3'}, R₁₀ and R₁₁ are independently Cl, F, methyl or hydrogen; and R₂ is independently hydrogen, methyl, ethyl, propyl, isopropyl, or cyclopropyl. In one embodiment, R₁ is hydrogen or methyl. In another embodiment, R₁ is hydrogen.

In yet still another aspect, the invention provides compounds of formula 7-4a1, i.e., compounds of formula 7-4, wherein R₄ is halogen (in one aspect, chloro or fluoro) C₁-C₆ haloalkyl or C₁-C₆ haloalkoxy; R₃, R_{3'}, R₁₀ and R₁₁ are independently halogen, methyl or hydrogen; and R₂ is NO₂ or CN.

In yet still another aspect, the invention provides compounds of formula 7-4a2, i.e., compounds of formula 7-4, wherein R₄ is halogen (in one aspect, chloro or fluoro) C₁-C₆ haloalkyl or C₁-C₆ haloalkoxy; R₃, R_{3'}, R₁₀ and R₁₁ are independently halogen, methyl or hydrogen; and R₂ is C₂-C₆ alkenyl or C₂-C₆ alkynyl.

In yet still another aspect, the invention provides compounds of formula 7-4a3, i.e., compounds of formula 7-4, wherein R₄ is halogen (in one aspect, chloro or fluoro) C₁-C₆ haloalkyl or C₁-C₆ haloalkoxy; R₃, R_{3'}, R₁₀ and R₁₁ are independently halogen, methyl or hydrogen; and R₂ is C₁-C₄ haloalkyl. In one embodiment, R₄ is Cl, while R₃, R_{3'}, R₁₀ and R₁₁ are H.

In still another aspect, the invention provides compounds of formula 7-4a4, i.e., compounds of formula 7-4, 7-4a, 7-4a1, 7-4a2, or 7-4a3 wherein R₁ is hydrogen.

In still yet another aspect, the invention provides compounds of formula 7-4a5, i.e., compounds of formula 7-4, wherein R₁ and R₂ combined are oxo.

In still yet another aspect, the invention provides compounds of formula 7-4a6, i.e., compounds of formula 7-4, wherein R₁ and R₂ combined are =N-OR where R is hydrogen, C₁-C₆ alkyl, aryl (such as phenyl) or arylalkyl (such as benzyl or phenethyl).

In yet another aspect, the invention provides compounds of formula 7-4a7, i.e., compounds of formula 7-4, wherein R₁ and R₂ together with the carbon to which they are attached form a C₃-C₆ cycloalkyl group.

In yet another aspect, the invention provides compounds of formula 7-4a8, i.e., compounds of formula 7-4, wherein R₁ and R₂ together with the carbon to which they are attached form a cyclopropyl group.

In yet another aspect, the invention provides compounds of formula 7-4a9, i.e., compounds of formula 7-4 or 7-4a, wherein R₁ is hydrogen and R₂ is cyclopropyl. In one embodiment, the compound is racemic. In another embodiment, the compound is enantiomerically enriched.

In yet another aspect, the invention provides compounds of formula 7-4a10, i.e., compounds of formula 7-4 or 7-4a, wherein R₄ is CF₃; R₁ is H; R₂ is H, or C₃-C₆ cycloalkyl; R₂₀ is H or methyl; R₇₅ is H or methyl; and the C-ring is phenyl substituted with two halogens.

In yet still another aspect, the invention provides compounds of formula 7-4b, i.e., compounds according to any one of formulas 7, 7-1, 7-1a, 7-2, 7-2a, 7-3, 7-3a, 7-3b, 7-3c, 7-3d, 7-3e, 7-3f, 7-3g, 7-3h, 7-3i, 7-4, wherein R₂ is -X(CO)Y or -(C(R₃)₂)₁₋₄X(CO)Y.

In yet still another aspect, the invention provides compounds of formula 7-4c, i.e., compounds of formula 7-4b, wherein X is O; and Y is -NR₆₀R₇₀ or -N(R₃₀)(CH₂)₂₋₆NR₆₀R₇₀; where
R₆₀ and R₇₀ are independently selected from: hydrogen, methyl, ethyl, (C₃ -C₈)cycloalkyl, aryl-(C₁-C₆ alkyl) (such as benzyl or phenethyl), 4-pyridylmethyl, 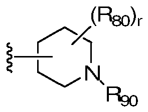 or 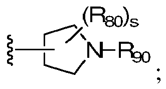 or
R₆₀ and R₇₀ taken together with the nitrogen atom to which they are bound form a heterocycloalkyl group selected from: 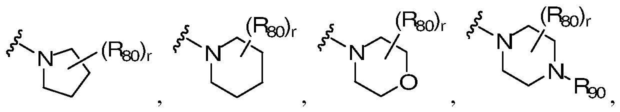 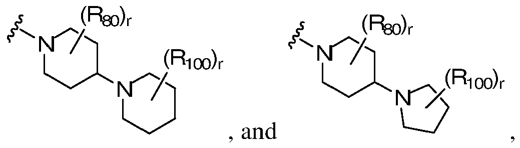 where
   each R₈₀ is independently unsubstituted C₁-C₄ alkyl or C₁-C₄ alkyl substituted with hydroxyl or halogen;
   each R₉₀ is independently hydrogen, unsubstituted C₁-C₄ alkyl, C₁-C₄ alkyl substituted with hydroxyl or halogen, unsubstituted C₃-C₈ cycloalkyl, C₃-C₈ cycloalkyl substituted with one or more (e.g., 1-4) R₅₀groups, phenyl-(C₁-C₄ alkyl)-, pyridyl-(C₁-C₄ alkyl)-, thienyl-(C₁-C₄ alkyl), -C(O)O-(C₁-C₄ alkyl), -C(O)O-phenyl, -S0₂-(C₁-C₆ alkyl), -SO₂-phenyl, unsubstituted phenyl, phenyl substituted with one or more (e.g., 1-4) R₅₀ groups, pyridyl, thienyl, pyridyl substituted with one or more (e.g., 1-4) R₅₀ groups, thienyl substituted with one or more (e.g., 1-4) R₅₀ groups, where
   each R₅₀ is independently selected from: halogen, C₁-C₆ alkyl, C₁-C₆ haloalkyl, -OH, -O-(C₁-C₄ alkyl), OCF₃, -CN, -NR₆₀R₇₀, -C(O)O-(C₁-C₄ alkyl), -CONR₆₀R₇₀, -(C₁-C₆ alkyl)-NR₆₀R₇₀, -NR₆₀CO-(C₁-C₄ alkyl), -NR₆₀CO-phenyl, -NR₆₀CO-pyridyl, -NR₆₀CO-thienyl, and -NR₆₀CONR₆₀R₇₀;
   each R₁₀₀ is independently hydrogen or C₁-C₄ alkyl;
   each r is 0 to 4; and
   each s is 0 to 3.

In yet still another aspect, the invention provides compounds of formula 7-4c1, i.e., compounds of formula 7-4b, wherein Y is -O-(C₁-C₆ alkyl). In one embodiment, Y is -O-(C₁-C₄ alkyl).

In yet still another aspect, the invention provides compounds of formula 7-4c2, i.e., compounds of formula 7-4b, wherein Y is -O-phenyl.

In yet still another aspect, the invention provides compounds of formula 7-4d, i.e., compounds of formula 7-4c, wherein said group 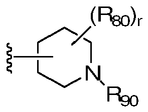 is a group of the formula: 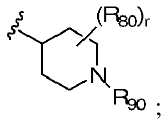 and said group 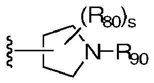 is a group of the formula:

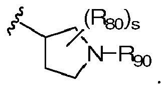

In a further aspect, the invention provides compounds of formula 7-4d1, i.e., compounds according to any one of formulas, 7-4b, 7-4c, 7-4c1, 7-4c2, or 7-4d, wherein R₁ is H or C₁-C₄ alkyl. In one embodiment, R₁ is H or methyl. In another embodiment, R₁ is H.

In still another aspect, the invention provides compounds of formula 7-5, i.e., compounds of formulas 4, 4-1, 4-2, 4-3, 5, or any one of formulas 6, 6-1, 6-2, 6-3, 6-4, 6-5, 6-6, 6-6a, 6-6b, 6-6c, 6-6d, 6-7, 6-8, 6-9, 6-10, 6-11, 6-11a, 6-11b, 6-11c, 6-12, 6-13, 6-14, or 6-15 wherein the C-ring is 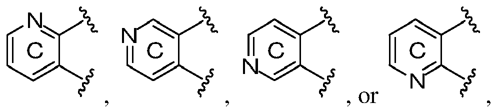 or N-oxide derivatives thereof,
each of which is optionally substituted with 1, 2, or 3 groups that are independently halogen, C₁-C₄ alkyl, C₁-C₄ alkoxy, aryloxy, aryl-(C₁-C₄ alkoxy), C₁-C₄ haloalkyl (in another aspect, CF₃), C₁-C₄ haloalkoxy (in another aspect, OCF₃), hydroxyl, hydroxy-(C₁-C₄ alkyl), -NR'R", -(C₁-C₆ alkyl)-NR'R", -NR'C(O)O-(C₁-C₆ alkyl), -NR'C(O)O-phenyl, -COR', -C(O)OR', -C(O)NR'R", -OC(O)NR'R", -NR'C(O)NR'R"; -(C₁-C₆ alkyl)-C(O)OR', -(C₁-C₆ alkyl)-C(O)NR'R", -NR'C(O)R", NO₂, CN, benzyl, phenyl, oxazolyl, pyrazolyl, thiazolyl, pyridyl, furanyl, thienyl, C₂-C₆ alkenyl, C₂-C₆ alkynyl, -NR'SO₂R", -SO₂-NR'R", -S(O)_{z} aryl (where the aryl group is preferably naphthyl or phenyl, still more preferably, phenyl), or -S(O)_{z} (C₁-C₆ alkyl), where the alkyl, alkenyl and alkynyl portions of the above are unsubstituted or substituted with 1, 2, or 3 groups that are independently halogen, hydroxyl, -NR'R" or C₁-C₆ alkoxy, and where the aryl or heteroaryl portions of the above are optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R". In one embodiment, the invention provides compounds of wherein the C-ring is pyridyl or pyridyl N-oxide substituted with 1 or 2 groups that are independently -NR'C(O)O-(C₁-C₆ alkyl), -NR'C(O)O-phenyl, -C(O)OR', -C(O)NR'R", -(C₁-C₆ alkyl)-C(O)OR', -(C₁-C₆ alkyl)-C(O)NR'R", -NR'C(O)R", -OC(O)NR'R", -NR'C(O)NR'R"; NO₂, CN, phenyl, -S(O)_{z} aryl (where the aryl group is preferably naphthyl or phenyl, still more preferably, phenyl) or -S(O)_{z} (C₁-C₆ alkyl) where the aryl portions of the above are optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R". In an alternative embodiment, the C-ring is pyridyl or pyridyl N-oxide substituted with 1 or 2 groups that are independently C₁-C₄ alkyl, C₂-C₆ alkenyl, C₂-C₆ alkynyl, where the alkyl, alkenyl and alkynyl portions of the above are unsubstituted or substituted with 1 or 2 groups that are independently halogen, hydroxy, -NR'R" or C₁-C₄ alkoxy. In another alternative embodiment, the C-ring is pyridyl or pyridyl N-oxide substituted with 1 or 2 groups that are independently -C(O)OH, -C(O)O-(C₁-C₄ alkyl), NO₂, CN, or phenyl, where the phenyl group is optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R". In still another alternative embodiment, the C-ring is pyridyl or pyridyl N-oxide substituted with 1 or 2 groups that are independently C₁-C₄ alkyl, C₁-C₄ alkoxy, halogen, -C(O)NR'R", -NR'C(O)R", -OC(O)N-(C₁-C₆ alkyl)₂, -NR'C(O)O-(C₁-C₆ alkyl), -NR'C(O)O-phenyl, where the phenyl group is optionally substituted with 1, 2, 3, or 4 groups that are independently C₁-C₆ alkyl, C₁-C₆ alkoxy, halogen, C₁-C₆ haloalkyl (such as CF3), C₁-C₆ haloalkoxy (such as OCF3), hydroxyl, hydroxy-(C₁-C₆ alkyl), or -NR'R".

In still another aspect, the invention provides compounds of formula 7-5a, i.e., compounds of formulas 4, 4-1, 4-2, 4-3, 5, or any one of formulas 6, 6-1, 6-2, 6-3, 6-4, 6-5, 6-6, 6-6a, 6-6b, 6-6c, 6-6d, 6-7, 6-8, 6-9, 6-10, 6-11, 6-11a, 6-11b, 6-11c, 6-12, 6-13, 6-14, or 6-15 wherein the C-ring is 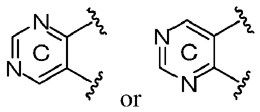 each of which is optionally substituted with 1 or 2, groups that are independently halogen, C₁-C₄ alkyl, C₁-C₄ alkoxy, aryloxy, aryl-(C₁-C₄ alkoxy), C₁-C₄ haloalkyl (in another aspect, CF₃), C₁-C₄ haloalkoxy (in another aspect, OCF₃), hydroxyl, hydroxy-(C₁-C₄ alkyl), -NR'R", -(C₁-C₄ alkyl)-NR'R", -NR'C(O)O-(C₁-C₆ alkyl), -NR'C(O)O-phenyl, -COR', -C(O)OR', -C(O)NR'R", -OC(O)NR'R", -NR'C(O)NR'R"; -(C₁-C₆ alkyl)-C(O)OR', -(C₁-C₆ alkyl)-C(O)NR'R", -NR'C(O)R", NO₂, CN, benzyl, phenyl, oxazolyl, pyrazolyl, thiazolyl, pyridyl, furanyl, thienyl, C₂-C₆ alkenyl, C₂-C₆ alkynyl, -NR'SO₂R", -SO₂-NR'R", -S(O)_{z} aryl (where the aryl group is preferably naphthyl or phenyl, still more preferably, phenyl), or -S(O)_{z} (C₁-C₆ alkyl), where the alkyl, alkenyl and alkynyl portions of the above are unsubstituted or substituted with 1, 2 or 3 groups that are independently halogen, hydroxyl, -NR'R" or C₁-C₆ alkoxy, and where the aryl or heteroaryl portions of the above are optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R". In one embodiment, the invention provides compounds wherein the C-ring is pyrimidinyl substituted with 1 or 2 groups that are independently -NR'C(O)O-(C₁-C₆ alkyl), -NR'C(O)O-phenyl, -C(O)OR', -C(O)NR'R", -(C₁-C₆ alkyl)-C(O)OR', -(C₁-C₆ alkyl)-C(O)NR'R", -NR'C(O)R", -OC(O)NR'R", NO₂, CN, phenyl, -S(O)_{z} aryl (where the aryl group is preferably naphthyl or phenyl, still more preferably, phenyl) or -S(O)_{z} (C₁-C₆ alkyl) where the aryl portions of the above are optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R". In an alternative, the C-ring is pyrimidinyl substituted with 1 or 2 groups that are independently C₁-C₄ alkyl, C₂-C₆ alkenyl, C₂-C₆ alkynyl, where the alkyl, alkenyl and alkynyl portions of the above are unsubstituted or substituted with 1 or 2 groups that are independently halogen, hydroxyl, -NR'R" or C₁-C₄ alkoxy. In a further alternative embodiment, the C-ring is pyrimidinyl substituted with 1 or 2 groups that are independently -COOH, -C(O)O-(C₁-C₄ alkyl), NO₂, CN, or phenyl, where the phenyl group is optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R". In still another alternative embodiment, the C-ring is pyrimidinyl substituted with 1 or 2 groups that are independently C₁-C₄ alkyl, C₁-C₄ alkoxy, halogen, -C(O)NR'R", -NR'C(O)R", -OC(O)N-(C₁-C₆ alkyl)₂, -NR'C(O)O-(C₁-C₆ alkyl), -NR'C(O)O-phenyl, where the phenyl group is optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R".

In still another aspect, the invention provides compounds of formula 7-5b, i.e., compounds of formulas 4, 4-1, 4-2, 4-3, 5, or any one of formulas 6, 6-1, 6-2, 6-3, 6-4, 6-5, 6-6, 6-6a, 6-6b, 6-6c, 6-6d, 6-7, 6-8, 6-9, 6-10, 6-11, 6-1 1a, 6-11b, 6-11c, 6-12, 6-13, 6-14, or 6-15, wherein the C-ring is 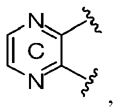 which is optionally substituted with 1 or 2, groups that are independently halogen, C₁-C₄ alkyl, C₁-C₄ alkoxy, aryloxy, aryl-(C₁-C₄alkoxy), C₁-C₄ haloalkyl (in another aspect, CF₃), C₁-C₄ haloalkoxy (in another aspect, OCF₃), hydroxyl, hydroxy-(C₁-C₄ alkyl), -NR'R", -(C₁-C₄ alkyl)-NR'R", -NR'C(O)O-(C₁-C₆ alkyl), -NR'C(O)Ophenyl, -COR', -C(O)OR', -C(O)NR'R", -OC(O)NR'R", -NR'C(O)NR'R"; -(C₁-C₆ alkyl)-C(O)OR', -(C₁-C₆ alkyl)-C(O)NR'R", -NR'C(O)R", NO₂, CN, benzyl, phenyl, oxazolyl, pyrazolyl, thiazolyl, pyridyl, furanyl, thienyl, C₂-C₆ alkenyl, C₂-C₆ alkynyl, -NR'SO₂R", -SO₂-NR'R", -S(O)_{z} aryl (where the aryl group is preferably naphthyl or phenyl, still more preferably, phenyl), or -S(O)_{z} (C₁-C₆ alkyl), where the alkyl, alkenyl and alkynyl portions of the above are unsubstituted or substituted with 1, 2, or 3 groups that are independently halogen, hydroxyl, -NR'R" or C₁-C₆ alkoxy, and where the aryl or heteroaryl portions of the above are optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R". In one embodiment, the invention provides compounds wherein the C-ring is pyrazine substituted with 1 or 2 groups that are independently -NR'C(O)O-(C₁-C₆ alkyl), -NR'C(O)O-phenyl, -C(O)OR', -C(O)NR'R", -(C₁-C₆ alkyl)-C(O)OR', -(C₁-C₆ alkyl)-C(O)NR'R", - NR'C(O)R", -OC(O)N(C₁-C₆ alkyl)₂, NO₂, CN, phenyl, -S(O)_{z} aryl (where the aryl group is preferably naphthyl or phenyl, still more preferably, phenyl) or -S(O)_{z} (C₁-C₆ alkyl) where the aryl portions of the above are optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R". In an alternative embodiment, the C-ring is pyrazine substituted with 1 or 2 groups that are independently C₁-C₄ alkyl, C₂-C₆ alkenyl, C₂-C₆ alkynyl, where the alkyl, alkenyl and alkynyl portions of the above are unsubstituted or substituted with 1 or 2 groups that are independently halogen, hydroxyl, -NR'R" or C₁-C₄ alkoxy. In another alternative aspect, the C-ring is pyrazine substituted with 1 or 2 groups that are independently -C(O)OH, -C(O)O-(C₁-C₄ alkyl), NO₂, CN, or phenyl, where the phenyl group is optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R". In still another alternative embodiment, the C-ring is pyrazine substituted with 1 or 2 groups that are independently C₁-C₄ alkyl, C₁-C₄ alkoxy, halogen, -C(O)NR'R", -NR'C(O)R", -OC(O)N(C₁-C₆ alkyl)₂, -NR'C(O)O-(C₁-C₆ alkyl) or -NR'C(O)O-phenyl, where the phenyl group is optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R".

In still another aspect, the invention provides compounds of formula 7-5c, i.e., compounds of formulas 4, 4-1, 4-2, 4-3, 5, or any one of formulas 6, 6-1, 6-2, 6-3, 6-4, 6-5, 6-6, 6-6a, 6-6b, 6-6c, 6-6d, 6-7, 6-8, 6-9, 6-10, 6-11, 6-11a, 6-11b, 6-11c, 6-12, 6-13, 6-14, or 6-15 wherein the C-ring is: 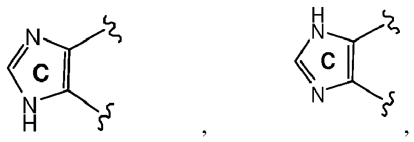 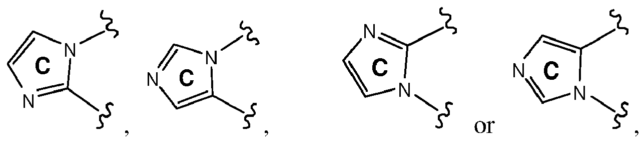 each of which is optionally substituted with 1 or 2 groups that are independently halogen, C₁-C₄ alkyl, C₁-C₄ alkoxy, aryloxy, aryl-(C₁-C₄ alkoxy), C₁-C₄ haloalkyl (in another aspect, CF₃), C₁-C₄ haloalkoxy (in another aspect, OCF₃), hydroxyl, hydroxy-(C₁-C₄ alkyl), -NR'R", -(C₁-C₆ alkyl)-NR'R", -NR'C(O)O-(C₁-C₆ alkyl), -NR'C(O)O-phenyl, -COR', -C(O)OR', -C(O)NR'R", -OC(O)NR'R", -NR'C(O)NR'R"; -(C₁-C₆ alkyl)-C(O)OR', -(C₁-C₆ alkyl)-C(O)NR'R", -NR'C(O)R", NO₂, CN, benzyl, phenyl, oxazolyl, pyrazolyl, thiazolyl, pyridyl, furanyl, thienyl, C₂-C₆ alkenyl, C₂-C₆ alkynyl, -NR'SO₂R", -SO₂-NR'R", -S(O)_{z} aryl (where the aryl group is preferably naphthyl or phenyl, still more preferably, phenyl), or -S(O)_{z} (C₁-C₆ alkyl), where the alkyl, alkenyl and alkynyl portions of the above are unsubstituted or substituted with 1, 2, or 3 groups that are independently halogen, hydroxyl, -NR'R" or C₁-C₆ alkoxy, and where the aryl or heteroaryl portions of the above are optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R".

In still another aspect, the invention provides compounds of formula 7-5c1, i.e., compounds of formula 7-5c wherein the C-ring is:

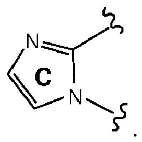

In still another aspect, the invention provides compounds of formula 7-5d, i.e., compounds of formula 4, 4-1, 4-2, 4-3, 5, or any one of formulas 6, 6-1, 6-2, 6-3, 6-4, 6-5, 6-6, 6-6a, 6-6b, 6-6c, 6-6d, 6-7, 6-8, 6-9, 6-10, 6-11, 6-11a, 6-11b, 6-11c, 6-12, 6-13, 6-14, or 6-15, wherein the C-ring is: 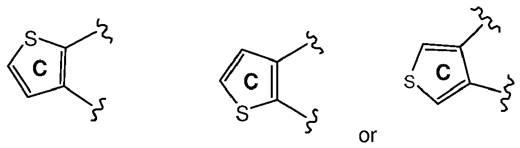 each of which is optionally substituted with 1 or 2 groups that are independently halogen, C₁-C₄ alkyl, C₁-C₄ alkoxy, aryloxy, aryl-(C₁-C₄ alkoxy), C₁-C₄ haloalkyl (in another aspect, CF₃), C₁-C₄ haloalkoxy (in another aspect, OCF₃), hydroxyl, hydroxy-(C₁-C₄ alkyl), -NR'R", -(C₁-C₆ alkyl)-NR'R", -NR'C(O)O-(C₁-C₆ alkyl), -NR'C(O)O-phenyl, -COR', -C(O)OR', -C(O)NR'R", -OC(O)NR'R", -NR'C(O)NR'R"; -(C₁-C₆ alkyl)-C(O)OR', -(C₁-C₆ alkyl)-C(O)NR'R", -NR'C(O)R", NO₂, CN, benzyl, phenyl, oxazolyl, pyrazolyl, thiazolyl, pyridyl, furanyl, thienyl, C₂-C₆ alkenyl, C₂-C₆ alkynyl, -NR'SO₂R" -SO₂-NR'R", -S(O)_{z} aryl (where the aryl group is preferably naphthyl or phenyl, still more preferably, phenyl), or -S(O)_{z} (C₁-C₆ alkyl), where the alkyl, alkenyl and alkynyl portions of the above are unsubstituted or substituted with 1, 2, or 3 groups that are independently halogen, hydroxyl, -NR'R" or C₁-C₆ alkoxy, and where the aryl or heteroaryl portions of the above are optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R".

In still another aspect, the invention provides compounds of formula 7-5d1, i.e., compounds of formula 7-5d wherein the C-ring is:

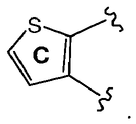

In still another aspect, the invention provides compounds of formula 7-5d2, i.e., compounds of formula 7-5d wherein the C-ring is:

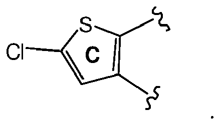

In another aspect, the invention provides compounds of formula 7-6, i.e., compounds of formula 7-5, 7-5a, 7-5b, 7-5c or 7-5d, wherein the C-ring is unsubstituted.

In another aspect, the invention provides compounds of formula 7-7, i.e., compounds of formula 7-5, 7-5a, 7-5b, 7-5c or 7-5d, wherein the C-ring is substituted with 1 or 2 groups that are independently Cl, F, methyl, ethyl, isopropyl, methoxy, ethoxy, CF₃, OCF₃, OH, or C(O)CH₃.

In yet another aspect, the invention provides compounds of formula 7-8, i.e., compounds of formula 7-5, 7-5a, 7-5b, 7-5c or 7-5d, wherein the C-ring is substituted with 1 or 2 groups that are Cl, F, or methyl.

In still yet another aspect, the invention provides compounds of formula 7-9, i.e., compounds according to any one of formulas 7-5, 7-6, 7-7, or 7-8, where the C-ring has the following structure:

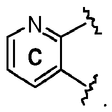

In still yet another aspect, the invention provides compounds of formula 7-10, i.e., compounds according to any one of formulas 7-5, 7-6, 7-7, or 7-8, where the C-ring has the following structure:

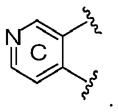

In still yet another aspect, the invention provides compounds of formula 7-10a, i.e., compounds according to any one of formulas 7-5, 7-6, 7-7, or 7-8, where the C-ring has the following structure:

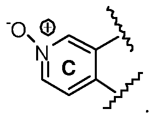

In still yet another aspect, the invention provides compounds of formula 7-11, i.e., compounds according to any one of formulas 7-5, 7-6, 7-7, or 7-8, where the C-ring has the following structure:

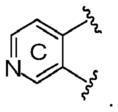

In still yet another aspect, the invention provides compounds of formula 7-11a, i.e., compounds according to formula 7-5, where the C-ring has the following structure:

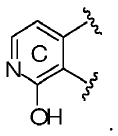

In still yet another aspect, the invention provides compounds of formula 7-12, i.e., compounds according to any one of formulas 7-5, 7-6, 7-7, or 7-8, where the C-ring has the following structure:

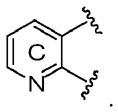

In a further aspect, the invention provides compounds of formula 7-12a, i.e., compounds of formula 4, 4-1, 4-2, 4-3, 5, or any one of formulas 6, 6-1, 6-2, 6-3, 6-4, 6-5, 6-6, 6-6a, 6-6b, 6-6c, 6-6d, 6-7, 6-8, 6-9, 6-10, 6-11, 6-11a, 6-11b, 6-11c, 6-12, 6-13, 6-14, or 6-15, wherein the C-ring is thiazolyl, which is optionally substituted with 1 or 2 groups that are independently halogen, C₁-C₄ alkyl, C₁-C₄ alkoxy, aryloxy, aryl-(C₁-C₄ alkoxy), C₁-C₄ haloalkyl (in another aspect, CF₃), C₁-C₄ haloalkoxy (in another aspect, OCF₃), hydroxyl, hydroxy-(C₁-C₄ alkyl), -NR'R", -(C₁-C₆ alkyl)-NR'R", -NR'C(O)O-(C₁-C₆ alkyl), -NR'C(O)O-phenyl, -COR', -C(O)OR', -C(O)NR'R", -OC(O)NR'R", -NR'C(O)NR'R"; -(C₁-C₆ alkyl)-C(O)OR', -(C₁-C₆ alkyl)-C(O)NR'R", -NR'C(O)R", NO₂, CN, benzyl, phenyl, oxazolyl, pyrazolyl, thiazolyl, pyridyl, furanyl, thienyl, C₂-C₆ alkenyl, C₂-C₆ alkynyl, -NR'SO₂R" -SO₂-NR'R", -S(O)_{z} aryl (where the aryl group is preferably naphthyl or phenyl, still more preferably, phenyl), or -S(O)_{z} (C₁-C₆ alkyl), where the alkyl, alkenyl and alkynyl portions of the above are unsubstituted or substituted with 1, 2, or 3 groups that are independently halogen, hydroxyl, -NR'R" or C₁-C₆ alkoxy, and where the aryl or heteroaryl portions of the above are optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R".

In another aspect, the invention provides compounds of formula 7-12b, i.e., compounds of formula 7-12a, wherein the thiazolyl ring is substituted with one group that is halogen, C₁-C₄ alkyl, C₁-C₄ alkoxy, or benzyl.

In still another aspect, the invention provides compounds of formula 7-12c, i.e., compounds of formula 7-12a, wherein the thiazolyl ring is substituted with C₁-C₄ alkyl.

In yet another aspect, the invention provides compounds of formula 7-12d, i.e., compounds of formula 7-12a, wherein the thiazolyl ring is substituted with C₁-C₄ haloalkyl (in another aspect, CF₃), C₁-C₄ haloalkoxy (in another aspect, OCF₃), hydroxyl, hydroxy-(C₁-C₄ alkyl), -NR'R", or -(C₁-C₆ alkyl)-NR'R".

In yet still another aspect, the invention provides compounds of formula 7-12e, i.e., compounds of formula 7-12a, wherein the thiazolyl ring is substituted with C₂-C₄ alkyl, -(C1-C₄ alkyl)-C(O)OR', or -(C₁-C₄ alkyl)-C(O)NR'R".

In another aspect, the invention provides compounds of formula 7-12f, i.e., compounds of formula 7-12a, wherein the thiazolyl ring is:

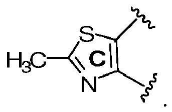

In yet still another aspect, the invention provides compounds of formula 7-13, i.e., compounds according to any one of formulas 7-5, 7-5a, 7-5b, 7-5c, 7-5c1, 7-5d, 7-5d1, 7-5d2, 7-6, 7-7, 7-8, 7-9, 7-10, 7-10a, 7-11, 7-11a, 7-12, 7-12a, 7-12b, 7-12c, 7-12d, 7-12e, or 7-12f, wherein R₂ is -X(CO)Y or -(C(R₃)₂)₁₋₄X(CO)Y.

In still another aspect, the invention provides compounds of formula 7-13a, i.e., compounds according to any one of formulas 7-5, 7-5a, 7-5b, 7-5c, 7-5c1, 7-5d, 7-5d1, 7-5d2, 7-6, 7-7, 7-8, 7-9, 7-10, 7-10a, 7-11, 7-11a, 7-12, 7-12a, 7-12b, 7-12c, 7-12d, 7-12e, or 7-12f, wherein R₂ is hydrogen, C₁-C₃ alkyl or C₃-C₆ cycloalkyl.

In still another aspect, the invention provides compounds of formula 7-13b, i.e., compounds according to any one of formulas 7-5, 7-5a, 7-5b, 7-5c, 7-5c1, 7-5d, 7-5d1, 7-5d2, 7-6, 7-7, 7-8, 7-9, 7-10, 7-10a, 7-11, 7-11a, 7-12, 7-12a, 7-12b, 7-12c, 7-12d, 7-12e, or 7-12f, wherein R₂ is NO₂ or CN.

In still another aspect, the invention provides compounds of formula 7-13c, i.e., compounds according to any one of formulas 7-5, 7-5a, 7-5b, 7-5c, 7-5c1, 7-5d, 7-5d1, 7-5d2, 7-6, 7-7, 7-8, 7-9, 7-10, 7-10a, 7-11, 7-11a, 7-12, 7-12a, 7-12b, 7-12c, 7-12d, 7-12e, or 7-12f, wherein R₂ is C₂-C₆ alkenyl or C₂-C₆ alkynyl.

In still another aspect, the invention provides compounds of formula 7-13d, i.e., compounds according to any one of formulas 7-5, 7-5a, 7-5b, 7-5c, 7-5c1, 7-5d, 7-5d1, 7-5d2, 7-6, 7-7, 7-8, 7-9, 7-10, 7-10a, 7-11, 7-11a, 7-12, 7-12a, 7-12b, 7-12c, 7-12d, 7-12e, or 7-12f, wherein R₂ is C₁-C₄ haloalkyl.

In still another aspect, the invention provides compounds of formula 7-13e, i.e., compounds according to any one of formulas 7-5, 7-5a, 7-5b, 7-5c, 7-5c1, 7-5d, 7-5d1, 7-5d2, 7-6, 7-7, 7-8, 7-9, 7-10, 7-10a, 7-11, 7-11a, 7-12, 7-12a, 7-12b, 7-12c, 7-12d, 7-12e, 7-12f, 7-13, 7-13a, 7-13b, 7-13c, or 7-13d, wherein R₁ is hydrogen.

In still yet another aspect, the invention provides compounds of formula 7-13f, i.e., compounds according to any one of formulas 7-5, 7-5a, 7-5b, 7-5c, 7-5c1, 7-5d, 7-5d1, 7-5d2, 7-6, 7-7, 7-8, 7-9, 7-10, 7-10a, 7-11, 7-11a, 7-12, 7-12a, 7-12b, 7-12c, 7-12d, 7-12e, or 7-12f, wherein R₁ and R₂ combined are oxo.

In still yet another aspect, the invention provides compounds of formula 7-13g, i.e., compounds according to any one of formulas 7-5, 7-5a, 7-5b, 7-5c, 7-5c1, 7-5d, 7-5d1, 7-5d2, 7-6, 7-7, 7-8, 7-9, 7-10, 7-10a, 7-11, 7-11a, 7-12, 7-12a, 7-12b, 7-12c, 7-12d, 7-12e, or 7-12f, wherein R₁ and R₂ combined are =N-OR, where R is hydrogen, C₁-C₆ alkyl, aryl (such as phenyl) or arylalkyl (such as benzyl or phenethyl).

In yet another aspect, the invention provides compounds of formula 7-13h, i.e., compounds according to any one of formulas 7-5, 7-5a, 7-5b, 7-5c, 7-5c1, 7-5d, 7-5d1, 7-5d2, 7-6, 7-7, 7-8, 7-9, 7-10, 7-10a, 7-11, 7-11a, 7-12, 7-12a, 7-12b, 7-12c, 7-12d, 7-12e, or 7-12f, wherein R₁ and R₂ together with the carbon to which they are attached form a C₃-C₆ cycloalkyl group.

In yet another aspect, the invention provides compounds of formula 7-131, i.e., compounds according to any one of formulas 7-5, 7-5a, 7-5b, 7-5c, 7-5c1, 7-5d, 7-5d1, 7-5d2, 7-6, 7-7, 7-8, 7-9, 7-10, 7-10a, 7-11, 7-11a, 7-12, 7-12a, 7-12b, 7-12c, 7-12d, 7-12e, or 7-12f, wherein R₁ and R₂ together with the carbon to which they are attached form a cyclopropyl group.

In yet another aspect, the invention provides compounds of formula 7-13j, i.e., compounds according to any one of formulas 7-5, 7-5a, 7-5b, 7-5c, 7-5c1, 7-5d, 7-5d1, 7-5d2, 7-6, 7-7, 7-8, 7-9, 7-10, 7-10a, 7-11, 7-11a, 7-12, 7-12a, 7-12b, 7-12c, 7-12d, 7-12e, or 7-12f, wherein R₁ is hydrogen and R₂ is cyclopropyl. In one embodiment, the compound is racemic. In another embodiment, the compound is enantiomerically enriched.

In yet still another aspect, the invention provides compounds of formula 7-14, i.e., compounds of formula 7-13, wherein,

X is O; and Y is -NR₆₀R₇₀ or -N(R₃₀)(CH₂)₂₋₆NR₆₀R₇₀; where R₆₀ and R₇₀ are independently selected from: hydrogen, methyl, ethyl, -(C₃-C₈)cycloalkyl, aryl-(C₁-C₆ alkyl)
(such as benzyl or phenethyl), 4-pyridylmethyl, 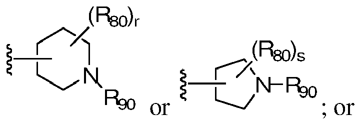 R₆₀ and R₇₀ taken together with the nitrogen atom to which they are bound form a heterocycloalkyl group selected from 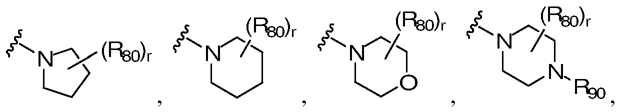 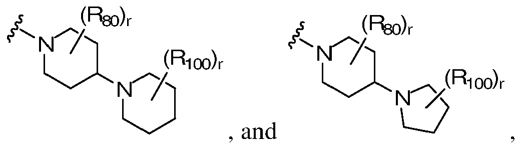 where
each R₈₀ is independently unsubstituted C₁-C₄ alkyl or C₁-C₄ alkyl substituted with hydroxyl or halogen;
each R₉₀ is independently hydrogen, unsubstituted C₁-C₄ alkyl, C₁-C₄ alkyl substituted with hydroxyl or halogen, unsubstituted C₃-C₈ cycloalkyl, C₃-C₈ cycloalkyl substituted with one or more (e.g., 1-4) R₅₀ groups, phenyl-(C₁-C₄ alkyl), pyridyl-(C₁-C₄ alkyl), thienyl-(C₁-C₄ alkyl), -C(O)O-(C₁-C₄ alkyl), -C(O)O-phenyl, -SO₂-(C₁-C₆alkyl), -SO₂-phenyl, unsubstituted phenyl, phenyl substituted with one or more (e.g., 1-4) R₅₀ groups, pyridyl, thienyl, pyridyl substituted with one or more (e.g., 1-4) R₅₀ groups, thienyl substituted with one or more (e.g., 1-4) R₅₀ groups, where
each R₅₀ is independently selected from: halogen, C₁-C₆ alkyl, C₁-C₆ haloalkyl, -OH, -O (C₁-C₄ alkyl), OCF₃, -CN, -NR₆₀R₇₀, -C(O)O-(C₁-C₄ alkyl), -CONR₆₀R₇₀, -(C₁-C₆alkyl)-NR₆₀R₇₀, -NR₆₀CO-(C₁-C₄ alkyl), -NR₆₀CO-phenyl, -NR₆₀CO-pyridyl, -NR₆₀CO-thienyl, and -NR₆₀CONR₆₀R₇₀,
each R₁₀₀ is independently hydrogen or C₁-C₄ alkyl;
each r is 0 to 4; and
each s is 0 to 3.

In yet still another aspect, the invention provides compounds of formula 7-15, i.e., compounds of formula 7-14, wherein said group 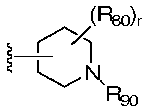 is a group of the formula: 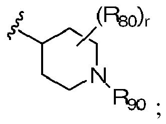 and said group 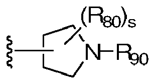 is a group of the formula:

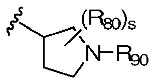

In yet still another aspect, the invention provides compounds of formula 7-16, i.e., compounds of formula 7-13, wherein Y is -O-(C₁-C₆ alkyl). In one embodiment, Y is -O-(C₁-C₄ alkyl).

In yet still another aspect, the invention provides compounds of formula 7-17, i.e., compounds of formula 7-13, wherein Y is -O-phenyl.

In a further aspect, the invention provides compounds of formula 7-18, i.e., compounds according to any one of formulas, 7-14, 7-15, 7-16, or 7-17, wherein R₁ is H or C₁-C₄ alkyl. In one embodiment, R₁ is H or methyl. In another embodiment, R₁ is H.

In still yet another aspect, the invention provides compounds of formula 8, i.e., compounds according to formula 6 or any one of formulas 7, 7-1, 7-1a, 7-2, 7-2a, 7-3, 7-3a, 7-3b, 7-3c, 7-3d, 7-3e, 7-3f, 7-3g, 7-3h, 7-3i, 7-4, 7-4a, 7-4aa, 7-4a1, 7-4a2, 7-4a3, 7-4a4, 7-4a5, 7-4a6, 7-4a7, 7-4a8, 7-4a9, 7-4a10, 7-4b, 7-4c, 7-4c1, 7-4c2, 7-4d, 7-4d1, 7-5, 7-5a, 7-5b, 7-5c, 7-5c1, 7-5d, 7-5d1, 7-5d2, 7-6, 7-7, 7-8, 7-9, 7-10, 7-10a, 7-11, 7-11a, 7-12, 7-12a, 7-12b, 7-12c, 7-12d, 7-12e, 7-12f, 7-13, 7-13a, 7-13b, 7-13c, 7-13d, 7-13e, 7-13f, 7-13g, 7-13h, 7-13i, 7-13j, 7-14, 7-15, 7-16, 7-17, or 7-18 wherein
R₃ and R₃' are hydrogen, halogen, C₁-C₆ alkyl, C₁-C₆ alkoxy, CF₃, CN;
R₄ is hydrogen, halogen, C₁-C₆ alkyl, C₁-C₆ alkoxy, C₁-C₆ haloalkyl, C₁-C₆ haloalkoxy, CN, -SO₂-(C₁-C₆ alkyl), or -NR'R",
R₁₀ and R₁₁ are independently hydrogen, or methyl.

In still yet another aspect, the invention provides compounds of formula 8-1, i.e., compounds of formula 8, wherein R₃ and R_{3'} are independently hydrogen, halogen, or methyl.

In still yet another aspect, the invention provides compounds of formula 8-2, i.e., compounds of formula 8, wherein R₄ is hydrogen, F, Cl, C₁-C₆ alkoxy, CH₂F, CHF₂, CF₃, OCF₃, OCHF₂, OCH₂F, or CN.

In still yet another aspect, the invention provides compounds of formula 8-3, i.e., compounds of formula 8 wherein R₄ is, -SO₂-(C₁-C₆ alkyl), or -NR'R".

In still yet another aspect, the invention provides compounds of formula 8-4, i.e., compounds according to any one of formulas 8, 8-1, 8-2,or 8-3, wherein R₃, R_{3'} R₁₀ and R₁₁ are hydrogen.

In still yet another aspect, the invention provides compounds of formula 8-5, i.e., compounds of formula 8, 8-1, 8-2, or 8-4, wherein R₄ is CF₃.

In still yet another aspect, the invention provides compounds of formula 8-6, i.e., compounds of formula 8, 8-1, 8-2, or 8-4, wherein R₄ is chloro. In one embodiment, R₄ is Cl, while R₃, R_{3'}, R₁₀ and R₁₁ are H.

In still yet another aspect, the invention provides compounds of formula 8-7, i.e., compounds of formula 8, 8-1, 8-2, or 8-4, wherein R₄ is fluoro.

In still yet another aspect, the invention provides compounds of formula 8-8, i.e., compounds of formula 8, 8-1, 8-2, or 8-4, wherein R₄ is OCF₃.

In still another aspect, the invention provides compounds of formula 8-9, i.e., compounds of formula 8-5. 8-6, 8-7 or 8-8, wherein the B-ring is a pyrazolyl ring, the C ring is a phenyl ring optionally substituted with halogen, C₁-C₆ alkyl, C₁-C₆ alkoxy, haloalkyl, haloalkoxy, OH or OC(O)N(C₁-C₆ alkyl)₂; R₁ is hydrogen or C₁-C₆ alkyl, and R₂ is hydrogen, C₁-C₆ alkyl, C₃-C₆ cycloalkyl, or haloalkyl. In one embodiment, the C-ring is substituted at least at the 7-position. In another embodiment, the C-ring is substituted at least at the 8-position. In still another embodiment, the C-ring is substituted at the 7- and 8-positions.

In still another aspect, the invention provides compounds of formula 8-10, i.e. compounds of formula 8-5, 8-6, 8-7, or 8-8, wherein the B-ring is a pyrazolyl ring, the C ring is a phenyl ring optionally substituted with one or more groups that are independently halogen (such as F or Cl), C₁-C₆ alkyl, C₁-C₆ alkoxy, haloalkyl (such as CF₃), haloalkoxy (such as OCF₃), OH or OC(O)N(C₁-C₆ alkyl)₂; and R₁ and R₂ together form a cycloalkyl, oxo or C=NOR group where R is hydrogen, alkyl or phenyl. In one embodiment, the C-ring is substituted at least at the 7-position. In another embodiment, the C-ring is substituted at least at the 8-position. In still another embodiment, the C-ring is substituted at both the 7- and 8-positions.

In still another aspect, the invention provides compounds of formula 8-11, i.e. compounds of formula 8-5. 8-6, 8-7 or 8-8, wherein the B-ring is a pyrazolyl ring, the C-ring is a pyridyl ring or an N-oxide-pyridyl ring optionally substituted with halogen, C₁-C₆ alkyl, C₁-C₆ alkoxy, haloalkyl, haloalkoxy, OH or OC(O)N(C₁-C₆ alkyl)₂; R₁ is hydrogen or C₁-C₆ alkyl, and R₂ is hydrogen, C₁-C₆ alkyl, C₃-C₆ cycloalkyl, or haloalkyl.

In still another aspect, the invention provides compounds of formula 8-11a, i.e. compounds of formula 8-11, where the B-ring and the C-ring together with the piperidine ring form an optionally substituted 4,5-dihydro-2H-pyrazolo[4,3-c][1,7]naphthyridine.

In still another aspect, the invention provides compounds of formula 8-11b, i.e. compounds of formula 8-11, where the B-ring and the C-ring together with the piperidine ring form an optionally substituted 4,5-dihydro-2H-pyrazolo[4,3-c][1,7]naphthyridine 7-oxide.

In still another aspect, the invention provides compounds of formula 8-11c, i.e. compounds of formula 8-11, where the B-ring and the C-ring together with the piperidine ring form an optionally substituted 4,5-dihydro-2H-pyrazolo[4,3-c][1,8]naphthyridine.

In still another aspect, the invention provides compounds of formula 8-11d, i.e. compounds of formula 8-11, where the B-ring and the C-ring together with the piperidine ring form an optionally substituted 4,5-dihydro-2H-pyrazolo[4,3-c][1,8]naphthyridine 8-oxide.

In still another aspect, the invention provides compounds of formula 8-11e, i.e. compounds of any of the formula 8-12, 8-12a, 8-12b, 8-12c, 8-12d, where R₁ is H and R₂ is cycloalkyl. In one embodiment, R₂ is cyclopropyl, cyclopentyl, or cyclohexyl. In still another embodiment, R₂ is cyclopropyl. In another embodiment, the compound is racemic. In yet another embodiment, the compound is enantiomerically enriched.

In still another aspect, the invention provides compounds of formula 8-12, i.e. compounds of formula 8-5. 8-6, 8-7 or 8-8, wherein the B-ring is a pyrazolyl ring, the C-ring is a pyridyl ring or an N-oxide-pyridyl ring optionally substituted with halogen, C₁-C₆ alkyl, C₁-C₆ alkoxy, haloalkyl, haloalkoxy, OH or OC(O)N(C₁-C₆ alkyl)₂; and R₁ and R₂ together form a cycloalkyl, oxo or C=N-OR group where R is hydrogen, alkyl or phenyl.

In still another aspect, the invention provides compounds of formula 8-12a, i.e. compounds of formula 8-12, where the B-ring and the C-ring together with the piperidine ring form an optionally substituted 4,5-dihydro-2H-pyrazolo[4,3-c][1,7]naphthyridine.

In still another aspect, the invention provides compounds of formula 8-12b, i.e. compounds of formula 8-12, where the B-ring and the C-ring together with the piperidine ring form an optionally substituted 4,5-dihydro-2H-pyrazolo[4,3-c][1,7]naphthyridine 7-oxide.

In still another aspect, the invention provides compounds of formula 8-12c, i.e. compounds of formula 8-12, where the B-ring and the C-ring together with the piperidine ring form an optionally substituted 4,5-dihydro-2H-pyrazolo[4,3-c][1,8]naphthyridine.

In still another aspect, the invention provides compounds of formula 8-12d, i.e. compounds of formula 8-12, where the B-ring and the C-ring together with the piperidine ring form an optionally substituted 4,5-dihydro-2H-pyrazolo[4,3-c][1,8]naphthyridine 8-oxide.

In still another aspect, the invention provides compounds of formula 8-13, i.e. compounds of any of the formula 8-12, 8-12a, 8-12b, 8-12c, 8-12d, where R₁ and R₂ together form a C₃-C₆ cycloalkyl ring.

In still another aspect, the invention provides compounds of formula 8-14, i.e. compounds of any of the formula 8-12, 8-12a, 8-12b, 8-12c, 8-12d, where R₁ and R₂ together are oxo.

In another aspect, the invention provides compounds of formula 9, i.e., compounds of formula I, wherein the A-ring is C₃-C₈ cycloalkyl, which is optionally substituted at a substitutable position with halogen, C₁-C₆ alkyl, C₂-C₆ alkenyl, C₂-C₆ alkynyl, C₁-C₆ alkoxy, C₁-C₆ haloalkyl, C₁-C₆ haloalkoxy, hydroxyl, hydroxy-(C₁-C₄ alkyl), CN, NO₂, aryloxy (such as phenyloxy), aryl-(C₁-C₄alkoxy) (such as benzyloxy), -SO₂-(C₁-C₆ alkyl), -NR'R", C₁-C₆ alkanoyl, -C(O)OR', -(C₁-C₄ alkyl)-C(O)OR', pyridyl, phenyl, phenyl-(C₁-C₄ alkyl), -SO₂-NR'R", -C(O)NR'R", -OC(O)NR'R", -NR'C(O)R", -NR'C(O)NR'R", -NR'C(O)O-(C₁-C₆ alkyl), or NR'C(O)O-phenyl where each R' and R" is independently hydrogen or C₁-C₆ alkyl; and

the B-ring is pyrazolyl, imidazolyl, pyrrolyl, triazolyl, pyrrolidinyl, pyrazolidinyl, or imidazolidinyl, each of which is optionally substituted at a substitutable position with a group that is independently C₁-C₆ alkyl, C₁-C₆ alkoxy, -NR'R", -SO₂-NR'R", -C(O)NR'R", -NR'C(O)R", -NR'C(O)NR'R", -NR'C(O)O-(C₁-C₆ alkyl), -NR'C(O)O-phenyl, NO₂, CN, -C(O)OR', hydroxyl, hydroxy-(C₁-C₆ alkyl), -S(O)_{z} (C₁-C₆ alkyl), -S(O)_{z} aryl, halogen, C₁-C₂ haloalkyl, C₁-C₂ haloalkoxy, benzyl or phenyl.

In still another aspect, the invention provides compounds of formula 10, i.e., compounds of formula 9 having the following formula: 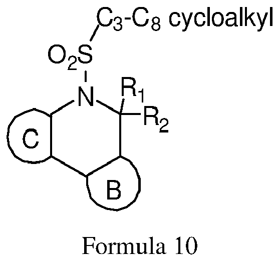 stereoisomers, tautomers, mixtures of stereoisomers and/or tautomers or pharmaceutically acceptable salts thereof, wherein
the cycloalkyl group is optionally substituted at a substitutable position with halogen, C₁-C₆ alkyl, C₂-C₆ alkenyl, C₂-C₆ alkynyl, C₁-C₆ alkoxy, C₁-C₆ haloalkyl, C₁-C₆ haloalkoxy, hydroxyl, CN, NO₂, aryloxy, aryl-(C₁-C₄alkoxy), -SO₂-(C₁-C₆ alkyl), -NR'R", C₁-C₆ alkanoyl, -C(O)OR', -(C₁-C₄alkyl)-C(O)OR', pyridyl, phenyl, phenyl-(C₁-C₄ alkyl), -SO₂-NR'R", -C(O)NR'R", -OC(O)NR'R", -NR'C(O)R", -NR'C(O)NR'R", or -NR'C(O)OR', where each R' and R" is independently hydrogen or C₁-C₆ alkyl; and
the C-ring is cyclohexyl, phenyl, thienyl, imidazolinyl, thiazolyl, isothiazolyl, oxazolyl, isoxazolyl, pyrrolyl, pyrazolyl, pyrimidyl, pyrazinyl or pyridyl, each of which is optionally substituted at each substitutable position with groups that are independently halogen, C₁-C₆ alkyl, C₁-C₆ alkoxy, aryloxy, aryl-(C₁-C₆ alkoxy), C₁-C₆ haloalkyl, C₁-C₆ haloalkoxy, hydroxyl, hydroxy-(C₁-C₆ alkyl), -NR'R", -(C₁-C₄ alkyl)-NR'R", -NR'C(O)O-(C₁-C₆ alkyl), -NR'C(O)O-phenyl, -COR', -C(O)OR', -C(O)NR'R", -OC(O)NR'R", -NR'C(O)NR'R"; -(C₁-C₆ alkyl)-C(O)OR', -(C₁-C₆ alkyl)-C(O)NR'R", -NR'C(O)R", NO₂, CN, oxo, benzyl, phenyl, oxazolyl, pyrazolyl, thiazolyl, pyridyl, furanyl, thienyl, C₂-C₆ alkenyl, C₂-C₆ alkynyl, -NR'SO₂R", -SO₂-NR'R", -S(O)_{z} aryl (where the aryl group is preferably naphthyl or phenyl, still more preferably, phenyl),or -S(O)_{z} (C₁-C₆ alkyl), where the alkyl, alkenyl and alkynyl portions of the above are unsubstituted or substituted with 1, 2 or 3 groups that are independently halogen, hydroxyl, -NR'R" or C₁-C₆ alkoxy, and where the aryl or heteroaryl portions of the above are optionally substituted with 1, or 2 groups that are independently C₁-C₄ alkyl, C₁-C₄ alkoxy, halogen, CF₃, OCF₃, hydroxy, hydroxy-(C₁-C₄ alkyl), or -NR'R".

In still another aspect, the invention provides compounds of formula 10-1, i.e., compounds of formula 10, wherein the B-ring is pyrazolyl, imidazolyl, pyrrolyl, triazolyl, pyrrolidinyl, pyrazolidinyl, imidazolidinyl, pyridyl, pyrimidyl, or isoxazolyl, each of which is unsubstituted.

In still another aspect, the invention provides compounds of formula 10-2, i.e., compounds of formula 10, wherein the B-ring has the formula: 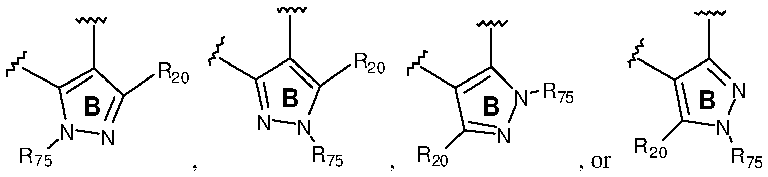 wherein
R₂₀ is hydrogen, C₁-C₆ alkyl, C₁-C₆ alkoxy, -NR'R", -(C₁-C₄ alkyl)-NR'R", -S(O)_{z} (C₁-C₆ alkyl), hydroxyl, halogen, CN, NO₂, CH₂F, CHF₂, CF₃, -C(O)OR', -C(O)NR'R", -(C₁-C₆ alkyl)-C(O)OR', -(C₁-C₆ alkyl)-C(O)NR'R", or phenyl, and
R₇₅ is hydrogen, C₁-C₆ alkyl, C₁-C₆ alkanoyl, phenyl-(C₁-C₆ alkanoyl), -C(O)NR'R", -S(O)₂(C₁-C₆ alkyl), -S(O)₂-aryl, -SO₂NR'R", phenyl, phenyl C₁-C₆ alkyl (preferably phenethyl or benzyl, more preferably, benzyl), phenylcarbonyl, benzylcarbonyl, or heteroarylcarbonyl, where the heteroaryl group is pyridyl, pyrimidyl, thienyl or furanyl.

In still another aspect, the invention provides compounds of formula 10-3, i.e., compounds of formula 10-2, wherein the B-ring has the formula:

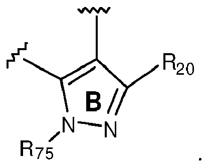

In still another aspect, the invention provides compounds of formula 10-4, i.e., compounds of formula 10-2, wherein the B-ring has the formula:

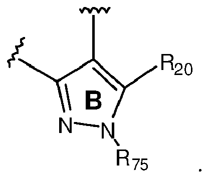

In still another aspect, the invention provides compounds of formula 10-5, i.e., compounds of formula 10-2, wherein the B-ring has the formula:

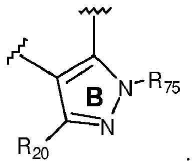

In still another aspect, the invention provides compounds of formula 10-6, i.e., compounds of formula 10-2, wherein the B-ring has the formula:

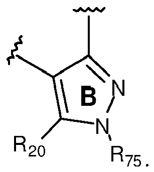

In a further aspect, the invention provides compounds of formula 10-6a, i.e., compounds according to any one of formulas 10-2, 10-3, 10-4, 10-5, or 10-6, wherein R₂₀ is hydrogen, C₁-C₄ alkyl, CH₂F, CHF₂, CF₃, -C(O)OR', -S(O)_{z} (C₁-C₄ alkyl), or hydroxyl; and R₇₅ is hydrogen, C₁-C₄ alkyl, or -S(O)_{z} -phenyl.

In another aspect, the invention provides compounds of formula 10-6b, i.e., compounds of formula 10-6a, wherein R₂₀ is hydrogen or methyl, and R₇₅ is hydrogen or methyl.

In yet another aspect, the invention provides compounds of formula 10-6c, i.e., compounds of formula 10-6a, wherein R₂₀ is hydrogen or methyl, and R₇₅ is hydrogen.

In still yet another aspect, the invention provides compounds of formula 10-6d, i.e., compounds of formula 10-6a, wherein R₂₀ is hydrogen and R₇₅ is hydrogen.

In still another aspect, the invention provides compounds of formula 10-7, i.e., compounds of formula 10, wherein the B-ring has the formula: 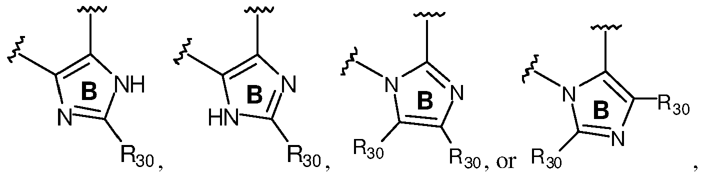 wherein R₃₀ is hydrogen, C₁-C₄ alkyl, C₁-C₄ alkoxy, -NR'R", C₁-C₄ alkythio, hydroxyl, halogen, CH₂F, CHF₂, CF₃, or phenyl.

In still another aspect, the invention provides compounds of formula 10-8, i.e., compounds of formula 10-7, wherein the B-ring has the formula:

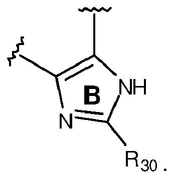

In still another aspect, the invention provides compounds of formula 10-9, i.e., compounds of formula 10-7, wherein the B-ring has the formula:

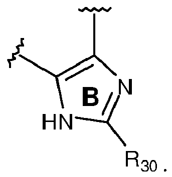

In still another aspect, the invention provides compounds of formula 10-10, i.e., compounds of formula 10-7, wherein the B-ring has the formula:

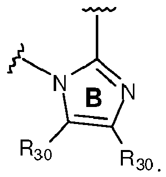

In still another aspect, the invention provides compounds of formula 10-11, i.e., compounds of formula 10-7, wherein the B-ring has the formula:

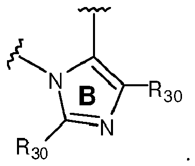

In a further aspect, the invention provides compounds of formula 10-11a, i.e., compounds according to any one of formulas 10-8, 10-9, 10-10, or 10-11, wherein each R₃₀ is independently hydrogen, C₁-C₄ alkyl, CH₂F, CHF₂, or CF₃.

In another aspect, the invention provides compounds of formula 10-11b, i.e., compounds of formula 10-11a, wherein each R₃₀ is independently hydrogen or methyl.

In still another aspect, the invention provides compounds of formula 10-11c, i.e., compounds of formula 10-11a, wherein each R₃₀ is hydrogen.

In still another aspect, the invention provides compounds of formula 10-12, i.e., compounds of formula 10, wherein the B-ring has the formula:

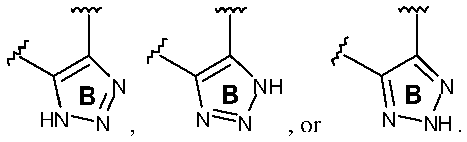

In still another aspect, the invention provides compounds of formula 10-13, i.e., compounds of formula 10-12, wherein the B-ring has the formula:

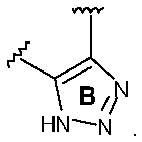

In still another aspect, the invention provides compounds of formula 10-14, i.e., compounds of formula 10-12, wherein the B-ring has the formula:

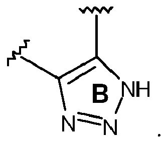

In still another aspect, the invention provides compounds of formula 10-15, i.e., compounds of formula 10-12, wherein the B-ring has the formula:

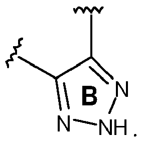

In yet another aspect, the invention provides compounds of formula 11, i.e., compounds of formula 9 or any one of formulas 10, 10-1, 10-2, 10-3, 10-4, 10-5, 10-6, 10-6a, 10-6b, 10-6c, 10-6d, 10-7, 10-8, 10-9, 10-10, 10-11, 10-11a, 10-11b, 10-11c, 10-12, 10-13, 10-14, or 10-15 wherein the C-ring is phenyl or cyclohexyl, which is optionally substituted with 1, 2, 3, or 4 groups that are independently halogen, C₁-C₄ alkyl, C₁-C₄ alkoxy, aryloxy, aryl-(C₁-C₆ alkoxy), C₁-C₄ haloalkyl (in another aspect, CF₃), C₁-C₄ haloalkoxy (in another aspect, OCF₃), hydroxyl, hydroxy-(C₁-C₄ alkyl), -NR'R", -(C₁-C₄ alkyl)-NR'R", -NR'C(O)O-(C₁-C₆ alkyl), -NR'C(O)O-phenyl, -COR', -C(O)OR', -C(O)NR'R", -OC(O)NR'R", -NR'C(O)NR'R"; -(C₁-C₆ alkyl)-C(O)OR', -(C₁-C₆ alkyl)-C(O)NR'R", -NR'C(O)R", NO₂, CN, oxo, benzyl, phenyl, oxazolyl, pyrazolyl, thiazolyl, pyridyl, furanyl, thienyl, C₂-C₆ alkenyl, C₂-C₆ alkynyl, -NR'SO₂R", -SO₂-NR'R", -S(O)_{z} aryl, or -S(O)_{z} (C₁-C₆ alkyl), where the alkyl, alkenyl and alkynyl portions of the above are unsubstituted or substituted with 1 or 2 groups that are independently halogen, hydroxyl, -NR'R" or C₁-C₆ alkoxy, and where the aryl or heteroaryl portions of the above are optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R". In one embodiment, the C-ring is an optionally substituted phenyl.

In yet another aspect, the invention provides compounds of formula 11-1, i.e., compounds of formula 11 wherein the C-ring is unsubstituted phenyl.

In yet another aspect, the invention provides compounds of formula 11-1a, i.e., compounds of formula 11 wherein the C-ring is unsubstituted cyclohexyl.

In yet another aspect, the invention provides compounds of formula 11-2, i.e., compounds of formula 11, wherein the C-ring is phenyl substituted with 1 or 2 groups that are independently Cl, F, methyl, ethyl, isopropyl, methoxy, ethoxy, CF₃, OCF₃, OH, CH₂OH, NH₂, NH(CH₃), N(CH₃)₂ or -OC(O)N(CH₃)₂. In one embodiment, the C-ring is bis-substituted with at least one halogen. In another embodiment, the halogen is F. In still another embodiment, the halogen is Cl. In another embodiment, the C-ring is bis-substituted with two halogens, which are the same or different. In still another embodiment, the C-ring is substituted at least at position 7. In another embodiment, the C-ring is substituted at positions 7 and 8. In another embodiment, the C-ring is monosubstituted with a halogen. In another embodiment, the halogen is F. In still another embodiment, the halogen is Cl. In still another embodiment, the C-ring is substituted at position 7. In yet another embodiment, the C-ring is substituted at position 8.

In still yet another aspect, the invention provides compounds of formula 11-2a, i.e., compounds of formula 11, wherein the C-ring is phenyl substituted with 1 or 2 groups that are independently -NR'C(O)O-(C₁-C₆ alkyl), -NR'C(O)O-phenyl, -C(O)OR', -C(O)NR'R", -OC(O)NR'R", -NR'C(O)NR'R"; -(C₁-C₆ alkyl)-C(O)OR', -(C₁-C₆ alkyl)-C(O)NR'R", -NR'C(O)R", NO₂, CN, phenyl, -S(O)_{z} phenyl, or -S(O)_{z} (C₁-C₆ alkyl), where the phenyl portions of the above are optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R". In one embodiment, the C-ring is substituted at least at the 7-position. In another embodiment, the C-ring is substituted at least at the 8-position.

In still another aspect, the invention provides compounds of formula 11-2b, i.e., compounds of formula 11, wherein the C-ring is phenyl substituted with 1 or 2 groups that are independently C₁-C₄ alkyl, C₂-C₆ alkenyl, C₂-C₆ alkynyl, where the alkyl, alkenyl and alkynyl portions of the above are unsubstituted or substituted with 1 or 2 groups that are independently halogen, hydroxyl, -NR'R" or C₁-C₄ alkoxy. In one embodiment, the C-ring is substituted at least at the 7-position. In another embodiment, the C-ring is substituted at least at the 8-position.

In still another aspect, the invention provides compounds of formula 11-2c, i.e., compounds of formula 11, wherein the C-ring is phenyl substituted with 1 or 2 groups that are independently -C(O)OH, -C(O)O-(C₁-C₄ alkyl), NO₂, CN, or phenyl, where the phenyl group is optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R". In one embodiment, the C-ring is substituted at least at the 7-position. In another embodiment, the C-ring is substituted at least at the 8-position.

In yet another aspect, the invention provides compounds of formula 11-2d, i.e., compounds of formula 11, wherein the C-ring is phenyl substituted with 1 or 2 groups that are independently C₁-C₄ alkyl, C₁-C₄ alkoxy, halogen, -C(O)NR'R", -NR'C(O)R", -OC(O)NR'R", -NR'C(O)NR'R", -NR'C(O)O-(C₁-C₆ alkyl), or -NR'C(O)O-phenyl, where the phenyl group is optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R". In one embodiment, the C-ring is substituted at least at the 7-position. In another embodiment, the C-ring is substituted at least at the 8-position.

In yet another aspect, the invention provides compounds of formula 11-2e, i.e., compounds of formula 11, wherein the C-ring is cyclohexyl substituted with 1 or 2 groups that are independently Cl, F, methyl, ethyl, isopropyl, methoxy, ethoxy, CF₃, OCF₃, OH, CH₂OH, NH₂, NH(CH₃), N(CH₃)₂, or -OC(O)N(CH₃)₂.

In still yet another aspect, the invention provides compounds of formula 11-2f, i.e., compounds of formula 11, wherein the C-ring is cyclohexyl substituted with 1 or 2 groups that are independently -NR'C(O)O-(C₁-C₆ alkyl), -NR'C(O)O-phenyl, -C(O)OR', -C(O)NR'R", -OC(O)NR'R", -NR'C(O)NR'R"; -(C₁-C₄ alkyl)-C(O)OR', -(C₁-C₄ alkyl)-C(O)NR'R", -NR'C(O)R", NO₂, CN, phenyl, -S(O)_{z} phenyl, or -S(O)_{z} (C₁-C₆ alkyl), where the phenyl portions of the above are optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R".

In still another aspect, the invention provides compounds of formula 11-2g, i.e., compounds of formula 11, wherein the C-ring cyclohexyl substituted with 1 or 2 groups that are independently C₁-C₄ alkyl, C₂-C₆ alkenyl, C₂-C₆ alkynyl, where the alkyl, alkenyl and alkynyl portions of the above are unsubstituted or substituted with 1 or 2 groups that are independently halogen, hydroxyl, -NR'R" or C₁-C₄ alkoxy.

In still another aspect, the invention provides compounds of formula 11-2h, i.e., compounds of formula 11, wherein the C-ring is cyclohexyl substituted with 1 or 2 groups that are independently -C(O)OH, -C(O)O-(C₁-C₄ alkyl), NO₂, CN, or phenyl, where the phenyl group is optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R".

In yet another aspect, the invention provides compounds of formula 11-2i, i.e., compounds of formula 11, wherein the C-ring is cyclohexyl substituted with 1 or 2 groups that are independently C₁-C₄ alkyl, C₁-C₄ alkoxy, halogen, -C(O)NR'R", -NR'C(O)R", -OC(O)NR'R", -NR'C(O)NR'R", -NR'C(O)O-(C₁-C₆ alkyl) or -NR'C(O)O-phenyl, where the phenyl group is optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R".

In yet another aspect, the invention provides compounds of formula 11-3, i.e., compounds of formula 11, wherein the C-ring is phenyl or cyclohexyl substituted with 1 or 2 groups that are Cl, F, methyl, ethyl, isopropyl, methoxy, CF₃, OCF₃, OH, NH₂, NH(CH₃), N(CH₃)₂ or -OC(O)N(CH₃)₂.

In yet still another aspect, the invention provides compounds of formula 11-3a, i.e., compounds according to any one of formulas 11, 11-1, 11-2, or 11-3, wherein R₂ is -X(CO)Y or -(C(R₃)₂)₁₋₄X(CO)Y.

In yet still another aspect, the invention provides compounds of formula 11-3a1, i.e., compounds according to any one of formulas 11, 11-1, 11-1a, 11-2, 11-2a, 11-2b, 11-2c, 11-2d, 11-2e, 11-2f, 11-2g, 11-2h, 11-2i, or 11-3, wherein R₂ is hydrogen, C₁-C₃ alkyl or C₃-C₆-cycloalkyl.

In yet still another aspect, the invention provides compounds of formula 11-3a2, i.e., compounds according to any one of formulas 11, 11-1, 11-1a, 11-2, 11-2a, 11-2b, 11-2c, 11-2d, 11-2e, 11-2f, 11-2g, 11-2h, 11-2i,or 11-3, wherein R₂ is NO₂ or CN.

In yet still another aspect, the invention provides compounds of formula 11-3a3, i.e., compounds according to any one of formulas 11, 11-1, 11-1a, 11-2, 11-2a, 11-2b, 11-2c, 11-2d, 11-2e, 11-2f, 11-2g, 11-2h, 11-2i, or 11-3, wherein R₂ is C₂-C₆ alkenyl or C₂-C₆ alkynyl.

In yet still another aspect, the invention provides compounds of formula 11-3a4, i.e., compounds according to any one of formulas 11, 11-1, 11-1a, 11-2, 11-2a, 11-2b, 11-2c, 11-2d, 11-2e, 11-2f, 11-2g, 11-2h, 11-2i, or 11-3, wherein R₂ is C₁-C₄ haloalkyl.

In still another aspect, the invention provides compounds of formula 11-3a5, i.e., compounds according to any one of formulas 11, 11-1, 11-1a, 11-2, 11-2a, 11-2b, 11-2c, 11-2d, 11-2e, 11-2f, 11-2g, 11-2h, 11-2i, 11-3, 11-3a, 11-3a1, 11-3a2, 11-3a3, or 11-3a4, wherein R₁ is hydrogen.

In still yet another aspect, the invention provides compounds of formula 11-3a6, i.e., compounds according to any one of formulas 11, 11-1, 11-1a, 11-2, 11-2a, 11-2b, 11-2c, 11-2d, 11-2e, 11-2f, 11-2g, 11-2h, 11-2i, or 11-3, wherein R₁ and R₂ combined are oxo.

In still yet another aspect, the invention provides compounds of formula 11-3a7, i.e., compounds according to any one of formulas 11, 11-1, 11-1a, 11-2, 11-2a, 11-2b, 11-2c, 11-2d, 11-2e, 11-2f, 11-2g, 11-2h, 11-2i, or 11-3, wherein R₁ and R₂ combined are =N-OR, where R is hydrogen, C₁-C₆ alkyl, aryl (such as phenyl) or arylalkyl (such as benzyl or phenethyl).

In yet another aspect, the invention provides compounds of formula 11-3a8, i.e., compounds according to any one of formulas 11, 11-1, 11-1a, 11-2, 11-2a, 11-2b, 11-2c, 11-2d, 11-2e, 11-2f, 11-2g, 11-2h, 11-2i, or 11-3, wherein R₁ and R₂ together with the carbon to which they are attached form a C₃-C₆ cycloalkyl group.

In yet another aspect, the invention provides compounds of formula 11-3a9, i.e., compounds according to any one of formulas 11, 11-1, 11-1a, 11-2, 11-2a, 11-2b, 11-2c, 11-2d, 11-2e, 11-2f, 11-2g, 11-2h, 11-2i, or 11-3, wherein R₁ and R₂ together with the carbon to which they are attached form a cyclopropyl group.

In yet another aspect, the invention provides compounds of formula 11-3a10, i.e., compounds according to any one of formulas 11, 11-1, 11-1a, 11-2, 11-2a, 11-2b, 11-2c, 11-2d, 11-2e, 11-2f, 11-2g, 11-2h, 11-2i, or 11-3, wherein R₁ is hydrogen and R₂ is cyclopropyl. In one embodiment, the compound is racemic. In another embodiment, the compound is enantiomerically enriched.

In yet still another aspect, the invention provides compounds of formula 11-3b, i.e., compounds of formula 11-3a, wherein,

X is O; and Y is -NR₆₀R₇₀ or -N(R₃₀)(CH₂)₂₋₆NR₆₀R₇₀; where R₆₀ and R₇₀ are independently selected from: hydrogen, methyl, ethyl, (C₃-C₈)cycloalkyl, aryl-(C₁-C₆alkyl) (such as benzyl or phenethyl), 4-pyridylmethyl, 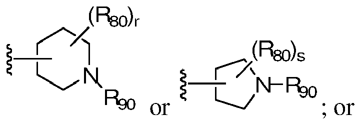
R₆₀ and R₇₀ taken together with the nitrogen atom to which they are bound form a heterocycloalkyl group selected from 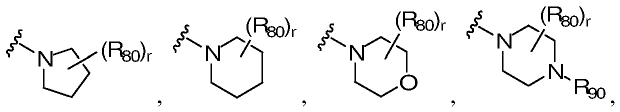 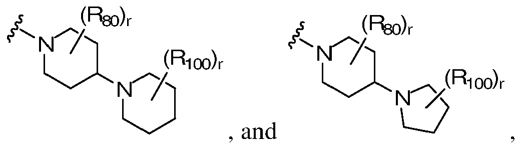 where
each R₈₀ is independently unsubstituted C₁-C₄ alkyl or C₁-C₄ alkyl substituted with hydroxy or halogen;
each R₉₀ is independently hydrogen, unsubstituted C₁-C₄ alkyl, C₁-C₄ alkyl substituted with hydroxy or halogen, unsubstituted C₃-C₈ cycloalkyl, C₃-C₈ cycloalkyl substituted with one or more (e.g., 1-4) R₅₀ groups, phenyl-(C₁-C₄ alkyl), pyridyl-(C₁-C₄ alkyl), thienyl-(C₁-C₄ alkyl), -C(O)O-(C₁-C₄ alkyl), -C(O)O-phenyl, -SO₂-(C₁-C₆alkyl), -SO₂-phenyl, unsubstituted phenyl, phenyl substituted with one or more (e.g., 1-4) R₅₀ groups, pyridyl, thienyl, pyridyl substituted with one or more (e.g., 1-4) R₅₀ groups, thienyl substituted with one or more (e.g., 1-4) R₅₀ groups, where
each R₅₀ is independently selected from: halogen, C₁-C₆ alkyl, - C₁-C₆ haloalkyl, -OH, -O (C₁-C₄ alkyl), -OCF₃, -CN, - NR₆₀R₇₀, -C(O)O-(C₁-C₄ alkyl), -CONR₆₀R₇₀, -(C₁-C₆ alkyl)-NR₆₀R₇₀, -NR₆₀CO-(C₁-C₄ alkyl), -NR₆₀CO-phenyl, -NR₆₀CO-pyridyl, -NR₆₀CO-thienyl, and -NR₆₀CONR₆₀R₇₀,
each R₁₀₀ is independently hydrogen or C₁-C₄ alkyl;
each r is 0 to 4; and
each s is 0 to 3.

In yet still another aspect, the invention provides compounds of formula 11-3b1, i.e., compounds of formula 11-3a, wherein Y is -O-(C₁-C₆ alkyl). In one embodiment, Y is -O-(C₁-C₄ alkyl).

In yet still another aspect, the invention provides compounds of formula 11-3b2, i.e., compounds of formula 11-3a, wherein Y is -O-phenyl.

In yet still another aspect, the invention provides compounds of formula 11-3c, i.e., compounds of formula 11-3b, wherein said group 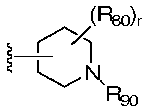 is a group of the formula: 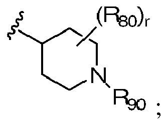 and, said group 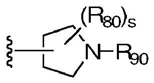 is a group of the formula:

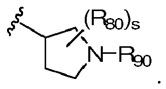

In a further aspect, the invention provides compounds of formula 11-3d, i.e., compounds according to any one of formulas, 11-3b, 11-3b1, 11-3b2, or 11-3c, wherein R₁ is H or C₁-C₄ alkyl. In one embodiment, R₁ is H or methyl. In another embodiment, R₁ is H.

In yet another aspect, the invention provides compounds of formula 11-4, i.e., compounds according to formula 9 or any one of formulas 10, 10-1, 10-2, 10-3, 10-4, 10-5, 10-6, 10-6a, 10-6b, 10-6c, 10-6d, 10-7, 10-8, 10-9, 10-10, 10-11, 10-11a, 10-11b, 10-11c, 10-12, 10-13, 10-14, or 10-15 wherein the C-ring is 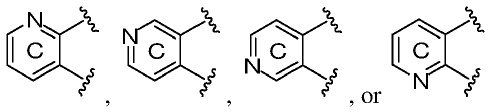 or N-oxide derivatives thereof,
each of which is optionally substituted with 1, 2, or 3 groups that are independently halogen, C₁-C₄ alkyl, C₁-C₄ alkoxy, aryloxy, aryl-(C₁-C₆ alkoxy), C₁-C₄ haloalkyl (in another aspect, CF₃), C₁-C₄ haloalkoxy (in another aspect, OCF₃), hydroxyl, hydroxy-(C₁-C₄ alkyl), -NR'R", -(C₁-C₄ alkyl)-NR'R", -NR'C(O)O-(C₁-C₆ alkyl), -NR'C(O)O-phenyl, -COR', -C(O)OR', -C(O)NR'R", -OC(O)NR'R", -NR'C(O)NR'R"; -(C₁-C₆ alkyl)-C(O)OR', -(C₁-C₆ alkyl)-C(O)NR'R", -NR'C(O)R", NO₂, CN, benzyl, phenyl, oxazolyl, pyrazolyl, thiazolyl, pyridyl, furanyl, thienyl, C₂-C₆ alkenyl, C₂-C₆ alkynyl, -NR'SO₂R", -SO₂-NR'R", -S(O)_{z} aryl (where the aryl group is preferably naphthyl or phenyl, still more preferably, phenyl), or -S(O)_{z} (C₁-C₆ alkyl), where the alkyl, alkenyl and alkynyl portions of the above are unsubstituted or substituted with 1, 2 or 3 groups that are independently halogen, hydroxyl, -NR'R" or C₁-C₆ alkoxy, and where the aryl or heteroaryl portions of the above are optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R". In another embodiment, the invention provides compounds of formula 11-4 wherein the C-ring is pyridyl or pyridyl N-oxide substituted with 1 or 2 groups that are independently -NR'C(O)O-(C₁-C₆ alkyl), -NR'C(O)O-phenyl, -C(O)OR', - C(O)NR'R", -OC(O)NR'R", -NR'C(O)NR'R"; -(C₁-C₆ alkyl)-C(O)OR', -(C₁-C₆ alkyl)-C(O)NR'R", -NR'C(O)R", NO₂, CN, phenyl, -S(O)_{z} aryl (where the aryl group is preferably naphthyl or phenyl, still more preferably, phenyl) or -S(O)_{z} (C₁-C₆ alkyl) where the aryl portions of the above are optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R". In an alternative embodiment, the C-ring is pyridyl or pyridyl N-oxide substituted with 1 or 2 groups that are independently C₁-C₄ alkyl, C₂-C₆ alkenyl, C₂-C₆ alkynyl, where the alkyl, alkenyl and alkynyl portions of the above are unsubstituted or substituted with 1 or 2 groups that are independently halogen, hydroxyl, -NR'R" or C₁-C₄ alkoxy. In another alternative embodiment, the C-ring is pyridyl or pyridyl N-oxide substituted with 1 or 2 groups that are independently -C(O)OH, -C(O)O-(C₁-C₄ alkyl), NO₂, CN, or phenyl, where the phenyl group is optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R". In still another alternative embodiment, the C-ring is pyridyl or pyridyl N-oxide substituted with 1 or 2 groups that are independently C₁-C₄ alkyl, C₁-C₄ alkoxy, halogen, -C(O)NR'R", -NR'C(O)R", -OC(O)NR'R", -NR'C(O)NR'R"; -NR'C(O)O-(C₁-C₆ alkyl), or -NR'C(O)O-phenyl, where the phenyl group is optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R".

In still another aspect, the invention provides compounds of formula 11-4a, i.e., compounds of formula 9 or any one of formulas 10, 10-1, 10-2, 10-3, 10-4, 10-5, 10-6, 10-7, 10-8, 10-9, 10-10, 10-11, 10-12, 10-13, 10-14, or 10-15 wherein the C-ring is 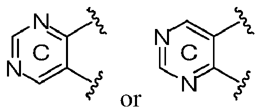 each of which is optionally substituted with 1 or 2 groups that are independently halogen, C₁-C₄ alkyl, C₁-C₄ alkoxy, aryloxy, aryl-(C₁-C₄ alkoxy), C₁-C₄ haloalkyl (in another aspect, CF₃), C₁-C₄ haloalkoxy (in another aspect, OCF₃), hydroxyl, hydroxy-(C₁-C₄ alkyl), -NR'R", -(C₁-C₄ alkyl)-NR'R",-NR'C(O)O-(C₁-C₆ alkyl), -NR'C(O)O-phenyl, -COR', -C(O)OR', -C(O)NR'R", -(C₁-C₆ alkyl)-C(O)OR', -(C₁-C₆ alkyl)-C(O)NR'R", -OC(O)NR'R", -NR'C(O)NR'R"; -NR'C(O)R", NO₂, CN, benzyl, phenyl, oxazolyl, pyrazolyl, thiazolyl, pyridyl, furanyl, thienyl, C₂-C₆ alkenyl, C₂-C₆ alkynyl, -NR'SO₂R", -SO₂-NR'R", -S(O)_{z} aryl (where the aryl group is preferably naphthyl or phenyl, still more preferably, phenyl), or -S(O)_{z} (C₁-C₆ alkyl), where the alkyl, alkenyl and alkynyl portions of the above are unsubstituted or substituted with 1, 2 or 3 groups that are independently halogen, hydroxyl, -NR'R" or C₁-C₆ alkoxy, and where the aryl or heteroaryl portions of the above are optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R". In one embodiment, the invention provides compounds of formula 11-4a wherein the C-ring is pyrimidinyl substituted with 1 or 2 groups that are independently -NR'C(O)O-(C₁-C₆ alkyl), -NR'C(O)O-phenyl, -C(O)OR', -C(O)NR'R", -OC(O)NR'R", -NR'C(O)NR'R"; -(C₁-C₆ alkyl)-C(O)OR', -(C₁-C₆ alkyl)-C(O)NR'R", -NR'C(O)R", NO₂, CN, phenyl, -S(O)_{z} aryl (where the aryl group is preferably naphthyl or phenyl, still more preferably, phenyl) or -S(O)_{z} (C₁-C₆ alkyl) where the aryl portions of the above are optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R". In an alternative embodiment, the C-ring is pyrimidinyl substituted with 1 or 2 groups that are independently C₁-C₄ alkyl, C₂-C₆ alkenyl, C₂-C₆ alkynyl, where the alkyl, alkenyl and alkynyl portions of the above are unsubstituted or substituted with 1 or 2 groups that are independently halogen, hydroxyl, - NR'R" or C₁-C₄ alkoxy. In another alternative embodiment, the C-ring is pyrimidinyl substituted with 1 or 2 groups that are independently -C(O)OH, -C(O)O-(C₁-C₄ alkyl), NO₂, CN, or phenyl, where the phenyl group is optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R". In still another alternative embodiment, the C-ring is pyrimidinyl substituted with 1 or 2 groups that are independently C₁-C₄ alkyl, C₁-C₄ alkoxy, halogen, -C(O)NR'R", -NR'C(O)R", -OC(O)NR'R", -NR'C(O)NR'R"; or -NR'C(O)O-(C₁-C₆ alkyl) or -NR'C(O)O-phenyl, where the phenyl group is optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R".

In still another aspect, the invention provides compounds of formula 11-4b, i.e., compounds of formula 9 or any one of formulas 10, 10-1, 10-2, 10-3, 10-4, 10-5, 10-6, 10-6a, 10-6b, 10-6c, 10-6d, 10-7, 10-8, 10-9, 10-10, 10-11, 10-11a, 10-11b, 10-11c, 10-12, 10-13, 10-14, or 10-15 wherein the C-ring is 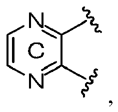 which is optionally substituted with 1 or 2 groups that are independently halogen, C₁-C₄ alkyl, C₁-C₄ alkoxy, aryloxy, aryl-(C₁-C₄ alkoxy), C₁-C₄ haloalkyl (in another aspect, CF₃), C₁-C₄ haloalkoxy (in another aspect, OCF₃), hydroxyl, hydroxy-(C₁-C₄ alkyl), -NR'R", -(C₁-C₆ alkyl)-NR'R", -NR'C(O)O-(C₁-C₆ alkyl), -NR'C(O)O-phenyl, -COR', -C(O)OR', -C(O)NR'R", -OC(O)NR'R", -NR'C(O)NR'R"; -(C₁-C₆ alkyl)-C(O)OR', -(C₁-C₆ alkyl)-C(O)NR'R", -NR'C(O)R", NO₂, CN, benzyl, phenyl, oxazolyl, pyrazolyl, thiazolyl, pyridyl, furanyl, thienyl, C₂-C₆ alkenyl, C₂-C₆ alkynyl, -NR'SO₂R", -SO₂-NR'R", -S(O)_{z} aryl (where the aryl group is preferably naphthyl or phenyl, still more preferably, phenyl), or -S(O)_{z} (C₁-C₆ alkyl), where the alkyl, alkenyl and alkynyl portions of the above are unsubstituted or substituted with 1, 2 or 3 groups that are independently halogen, hydroxyl, -NR'R" or C₁-C₆ alkoxy, and where the aryl or heteroaryl portions of the above are optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R". In one embodiment, the invention provides compounds of formula 11-4b wherein the C-ring is pyrazine substituted with 1 or 2 groups that are independently -NR'C(O)O-(C₁-C₆ alkyl), -NR'C(O)O-phenyl, -C(O)OR', -C(O)NR'R", -(C₁-C₆ alkyl)-C(O)OR', -(C₁-C₆ alkyl)-C(O)NR'R", -NR'C(O)R", NO₂, CN, phenyl, -S(O)_{z} aryl (where the aryl group is preferably naphthyl or phenyl, still more preferably, phenyl) or -S(O)_{z} (C₁-C₆ alkyl) where the aryl portions of the above are optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R". In an alternative embodiment, the C-ring is pyrazine substituted with 1 or 2 groups that are independently C₁-C₄ alkyl, C₂-C₆ alkenyl, C₂-C₆ alkynyl, where the alkyl, alkenyl and alkynyl portions of the above are unsubstituted or substituted with 1 or 2 groups that are independently halogen, hydroxyl, - NR'R" or C₁-C₄ alkoxy. In another alternative embodiment, the C-ring is pyrazine substituted with 1 or 2 groups that are independently -C(O)OH, -C(O)O-(C₁-C₄ alkyl), NO₂, CN, or phenyl, where the phenyl group is optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R". In still another alternative embodiment, the C-ring is pyrazine substituted with 1 or 2 groups that are independently C₁-C₄ alkyl, C₁-C₄ alkoxy, halogen, -C(O)NR'R", -NR'C(O)R", -OC(O)NR'R", -NR'C(O)NR'R"; -NR'C(O)O-(C₁-C₆ alkyl), or -NR'C(O)O-phenyl, where the phenyl group is optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R".

In still another aspect, the invention provides compounds of formula 11-4c, i.e., compounds of formula 9 or any one of formulas 10, 10-1, 10-2, 10-3, 10-4, 10-5, 10-6, 10-6a, 10-6b, 10-6c, 10-6d, 10-7, 10-8, 10-9, 10-10, 10-11, 10-11a, 10-11b, 10-11c, 10-12, 10-13, 10-14, or 10-15 wherein the C-ring is: 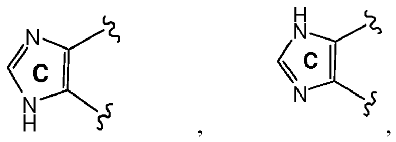 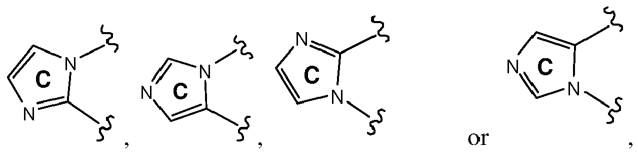 each of which is optionally substituted with 1 or 2 groups that are independently halogen, C₁-C₄ alkyl, C₁-C₄ alkoxy, aryloxy, aryl-(C₁-C₄ alkoxy), C₁-C₄ haloalkyl (in another aspect, CF₃), C₁-C₄ haloalkoxy (in another aspect, OCF₃), hydroxyl, hydroxy-(C₁-C₄ alkyl), -NR'R", -(C₁-C₆ alkyl)-NR'R", -NR'C(O)O-(C₁-C₆ alkyl), -NR'C(O)O-phenyl, -COR', -C(O)OR', -C(O)NR'R", -OC(O)NR'R", -NR'C(O)NR'R"; -(C₁-C₆ alkyl)-C(O)OR', -(C₁-C₆ alkyl)-C(O)NR'R", -NR'C(O)R", NO₂, CN, benzyl, phenyl, oxazolyl, pyrazolyl, thiazolyl, pyridyl, furanyl, thienyl, C₂-C₆ alkenyl, C₂-C₆ alkynyl, -NR'SO₂R", -SO₂-NR'R", -S(O)_{z} aryl (where the aryl group is preferably naphthyl or phenyl, still more preferably, phenyl), or -S(O)_{z} (C₁-C₆ alkyl), where the alkyl, alkenyl and alkynyl portions of the above are unsubstituted or substituted with 1, 2, or 3 groups that are independently halogen, hydroxyl, -NR'R" or C₁-C₆ alkoxy, and where the aryl or heteroaryl portions of the above are optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R".

In still another aspect, the invention provides compounds of formula 11-4c1, i.e., compounds of formula 11-4c wherein the C-ring is:

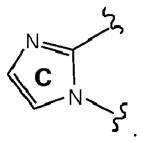

In still another aspect, the invention provides compounds of formula 11-4d, i.e., compounds of formula 9 or any one of formulas 10, 10-1, 10-2, 10-3, 10-4, 10-5, 10-6, 10-6a, 10-6b, 10-6c, 10-6d, 10-7, 10-8, 10-9, 10-10, 10-11, 10-11a, 10-11b, 10-11c, 10-12, 10-13, 10-14, or 10-15 wherein the C-ring is: 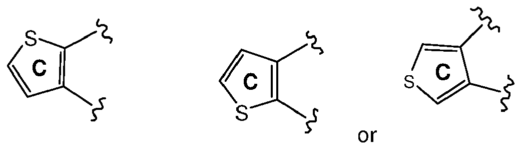 each of which is optionally substituted with 1 or 2 groups that are independently halogen, C₁-C₄ alkyl, C₁-C₄ alkoxy, aryloxy, aryl-(C₁-C₄ alkoxy), C₁-C₄ haloalkyl (in another aspect, CF₃), C₁-C₄ haloalkoxy (in another aspect, OCF₃), hydroxyl, hydroxy-(C₁-C₄ alkyl), -NR'R", -(C₁-C₆ alkyl)-NR'R", -NR'C(O)O-(C₁-C₆ alkyl), -NR'C(O)O-phenyl, -COR', -C(O)OR', -C(O)NR'R", -OC(O)NR'R", -NR'C(O)NR'R"; -(C₁-C₆ alkyl)-C(O)OR', -(C₁-C₆ alkyl)-C(O)NR'R", -NR'C(O)R", NO₂, CN, benzyl, phenyl, oxazolyl, pyrazolyl, thiazolyl, pyridyl, furanyl, thienyl, C₂-C₆ alkenyl, C₂-C₆ alkynyl, -NR'SO₂R", -SO₂-NR'R", -S(O)_{z} aryl (where the aryl group is preferably naphthyl or phenyl, still more preferably, phenyl), or -S(O)_{z} (C₁-C₆ alkyl), where the alkyl, alkenyl and alkynyl portions of the above are unsubstituted or substituted with 1, 2, or 3 groups that are independently halogen, hydroxyl, - NR'R" or C₁-C₆ alkoxy, and where the aryl or heteroaryl portions of the above are optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl) or -NR'R".

In still another aspect, the invention provides compounds of formula 11-4dl, i.e., compounds of formula 11-4d wherein the C-ring is:

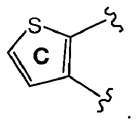

In still another aspect, the invention provides compounds of formula 11-4d2, i.e., compounds of formula 11-4d wherein the C-ring is:

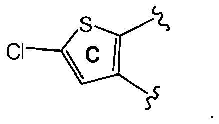

In another aspect, the invention provides compounds of formula 11-5, i.e., compounds of formula 11-4, 11-4a, 11-4b, 11-4c or 11-4d, wherein the C-ring is unsubstituted.

In another aspect, the invention provides compounds of formula 11-5a, i.e., compounds of formula 11-4, 11-4a, 11-4b, 11-4c or 11-4d wherein the C-ring is substituted with 1 or 2 groups that are independently Cl, F, methyl, ethyl, isopropyl, methoxy, ethoxy, CF₃, OCF₃, OH, or C(O)CH₃.

In yet another aspect, the invention provides compounds of formula 11-5b, i.e., compounds of formula 11-4, 11-4a, 11-4b, 11-4c or 11-4d, wherein the C-ring is substituted with 1 or 2 groups that are Cl, F, or methyl.

In still yet another aspect, the invention provides compounds of formula 11-5c, i.e., compounds according to any one of formulas 11-4, 11-5, 11-5a, or 11-5b, where the C-ring has the following structure:

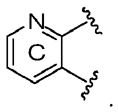

In still yet another aspect, the invention provides compounds of formula 11-5d, i.e., compounds according to any one of formulas 11-4, 11-5, 11-5a, or 11-5b, where the C-ring has the following structure:

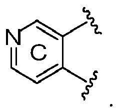

In still yet another aspect, the invention provides compounds of formula 11-5dl, i.e., compounds according to any one of formulas 11-4, where the C-ring has the following structure:

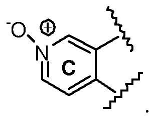

In still yet another aspect, the invention provides compounds of formula 11-5e, i.e., compounds according to any one of formulas 11-4, 11-5, 11-5a, or 11-5b, where the C-ring has the following structure:

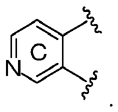

In still yet another aspect, the invention provides compounds of formula 11-5el, i.e., compounds according to formula 11-4, where the C-ring has the following structure:

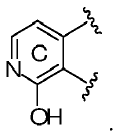

In still yet another aspect, the invention provides compounds of formula 11-5f, i.e., compounds according to any one of formulas 11-4, 11-5, 11-5a, or 11-5b, where the C-ring has the following structure:

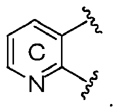

In a further aspect, the invention provides compounds of formula 11-5f1, i.e., compounds of formula 9 or any one of formulas 10, 10-1, 10-2, 10-3, 10-4, 10-5, 10-6, 10-6a, 10-6b, 10-6c, 10-6d, 10-7, 10-8, 10-9, 10-10, 10-11, 10-11a, 10-11b, 10-11c, 10-12, 10-13, 10-14, or 10-15 wherein the C-ring is thiazolyl, which is optionally substituted with 1 or 2 groups that are independently halogen, C₁-C₄ alkyl, C₁-C₄ alkoxy, aryloxy, aryl-(C₁-C₄ alkoxy), C₁-C₄ haloalkyl (in another aspect, CF₃), C₁-C₄ haloalkoxy (in another aspect, OCF₃), hydroxyl, hydroxy-(C₁-C₄ alkyl), -NR'R", -(C₁-C₆ alkyl)-NR'R", -NR'C(O)O-(C₁-C₆ alkyl), -NR'C(O)O-phenyl, -COR', -C(O)OR', -C(O)NR'R", -OC(O)NR'R", -NR'C(O)NR'R"; -(C₁-C₆ alkyl)-C(O)OR', -(C₁-C₆ alkyl)-C(O)NR'R", -NR'C(O)R", NO₂, CN, benzyl, phenyl, oxazolyl, pyrazolyl, thiazolyl, pyridyl, furanyl, thienyl, C₂-C₆ alkenyl, C₂-C₆ alkynyl, -NR'SO₂R" -SO₂-NR'R", -S(O)_{z} aryl (where the aryl group is preferably naphthyl or phenyl, still more preferably, phenyl), or -S(O)_{z} (C₁-C₆ alkyl), where the alkyl, alkenyl and alkynyl portions of the above are unsubstituted or substituted with 1, 2, or 3 groups that are independently halogen, hydroxyl, -NR'R" or C₁-C₆ alkoxy, and where the aryl or heteroaryl portions of the above are optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R".

In another aspect, the invention provides compounds of formula 11-5f2, i.e., compounds of formula 11-5f1, wherein the thiazolyl ring is substituted with one group that is halogen, C₁-C₄ alkyl, C₁-C₄ alkoxy, or benzyl.

In still another aspect, the invention provides compounds of formula 11-5f3, i.e., compounds of formula 11-5f1, wherein the thiazolyl ring is substituted with C₁-C₄ alkyl.

In yet another aspect, the invention provides compounds of formula 11-5f4, i.e., compounds of formula 11-5f1, wherein the thiazolyl ring is substituted with C₁-C₄ haloalkyl (in another aspect, CF₃), C₁-C₄ haloalkoxy (in another aspect, OCF₃), hydroxyl, hydroxy-(C₁-C₄ alkyl), -NR'R", or -(C₁-C₆ alkyl)-NR'R".

In yet still another aspect, the invention provides compounds of formula 11-5f5, i.e., compounds of formula 11-5f1, wherein the thiazolyl ring is substituted with C₂-C₄ alkyl, -(C₁-C₄ alkyl)-C(O)OR', or -(C₁-C₄ alkyl)-C(O)NR'R".

In another aspect, the invention provides compounds of formula 11-5f6, i.e., compounds of formula 11-5f1, wherein the thiazolyl ring is:

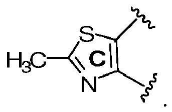

In yet still another aspect, the invention provides compounds of formula 11-5g, i.e., compounds according to any one of formulas 11-4, 11-4a, 11-4b, 11-4c, 11-4c1, 11-4d, 11-4d1, 11-4d2, 11-5, 11-5a, 11-5b, 11-5c, 11-5d, 11-5d1, 11-5e, 11-5e1, 11-5f, 11-5f1, 11-5f2, 11-5f3, 11-5f4,11-5f5, or 11-5f6, wherein R₂ is -X(CO)Y or -(C(R₃)₂)₁₋₄X(CO)Y.

In still another aspect, the invention provides compounds of formula 11-5g1, i.e., compounds according to any one of formulas 11-4, 11-4a, 11-4b, 11-4c, 11-4c1, 11-4d, 11-4d1, 11-4d2, 11-5, 11-5a, 11-5b, 11-5c, 11-5d, 11-5d1, 11-5e, 11-5e1, 11-5f, 11-5f1, 11-5f2, 11-5f3, 11-5f4, 11-5f5, or 11-5f6, wherein R₂ is H, C₁-C₃ alkyl or C₃-C₆ cyclopropyl.

In still another aspect, the invention provides compounds of formula 11-5g2, i.e., compounds according to any one of formulas 11-4, 11-4a, 11-4b, 11-4c, 11-4c1, 11-4d, 11-4d1, 11-4d2, 11-5, 11-5a, 11-5b, 11-5c, 11-5d, 11-5d1, 11-5e, 11-5e1, 11-5f, 11-5f1, 11-5f2, 11-5f3, 11-5f4, 11-5f5, or 11-5f6, wherein R₂ is NO₂ or CN.

In still another aspect, the invention provides compounds of formula 11-5g3, i.e., compounds according to any one of formulas 11-4, 11-4a, 11-4b, 11-4c, 11-4c1, 11-4d, 11-4d1, 11-4d2, 11-5, 11-5a, 11-5b, 11-5c, 11-5d, 11-5d1, 11-5e, 11-5e1, 11-5f, 11-5f1, 11-5f2, 11-5f3, 11-5f4, 11-5f5, or 11-5f6, wherein R₂ is C₂-C₆ alkenyl or C₂-C₆ alkynyl.

In still another aspect, the invention provides compounds of formula 11-5g4, i.e., compounds according to any one of formulas 11-4, 11-4a, 11-4b, 11-4c, 11-4c1, 11-4d, 11-4d1, 11-4d2, 11-5, 11-5a, 11-5b, 11-5c, 11-5d, 11-5d1, 11-5e, 11-5e1, 11-5f, 11-5f1, 11-5f2, 11-5f3, 11-5f4, 11-5f5, or 11-5f6, wherein R₂ is C₁-C₄ haloalkyl.

In still another aspect, the invention provides compounds of formula 11-5g5, i.e., compounds according to any one of formulas 11-4, 11-4a, 11-4b, 11-4c, 11-4c1, 11-4d, 11-4d1, 11-4d2, 11-5, 11-5a, 11-5b, 11-5c, 11-5d, 11-5d1, 11-5e, 11-5e1, 11-5f, 11-5f1, 11-5f2, 11-5f3, 11-5f4, 11-5f5, 11-5f6, 11-5g, 11-5g1, 11-5g2, 11-5g3, or 11-5g4, wherein R₁ is hydrogen.

In still yet another aspect, the invention provides compounds of formula 11-5g6, i.e., compounds according to any one of formulas 11-4, 11-4a, 11-4b, 11-4c, 11-4c1, 11-4d, 11-4d1, 11-4d2, 11-5, 11-5a, 11-5b, 11-5c, 11-5d, 11-5d1, 11-5e, 11-5e1, 11-5f, 11-5f1, 11-5f2, 11-5f3, 11-5f4, 11-5f5, or 11-5f6, wherein R₁ and R₂ combined are oxo.

In still yet another aspect, the invention provides compounds of formula 11-5g7, i.e., compounds according to any one of formulas 11-4, 11-4a, 11-4b, 11-4c, 11-4c1, 11-4d, 11-4d1, 11-4d2, 11-5, 11-5a, 11-5b, 11-5c, 11-5d, 11-5d1, 11-5e, 11-5e1, 11-5f, 11-5f1, 11-5f2, 11-5f3, 11-5f4, 11-5f5, or 11-5f6, wherein R₁ and R₂ combined are =N-OR, where R is hydrogen, C₁-C₆ alkyl, aryl (such as phenyl) or arylalkyl (such as benzyl or phenethyl).

In yet another aspect, the invention provides compounds of formula 11-5g8, i.e., compounds according to any one of formulas 11-4, 11-4a, 11-4b, 11-4c, 11-4c1, 11-4d, 11-4d1, 11-4d2, 11-5, 11-5a, 11-5b, 11-5c, 11-5d, 11-5d1, 11-5e, 11-5e1, 11-5f, 11-5f1, 11-5f2, 11-5f3, 11-5f4, 11-5f5, or 11-5f6, wherein R₁ and R₂ together with the carbon to which they are attached form a C₃-C₆ cycloalkyl group.

In yet another aspect, the invention provides compounds of formula 11-5g9, i.e., compounds according to any one of formulas 11-4, 11-4a, 11-4b, 11-4c, 11-4c1, 11-4d, 11-4d1, 11-4d2, 11-5, 11-5a, 11-5b, 11-5c, 11-5d, 11-5d1, 11-5e, 11-5e1, 11-5f, 11-5f1, 11-5f2, 11-5f3, 11-5f4, 11-5f5, or 11-5f6, wherein R₁ and R₂ together with the carbon to which they are attached form a cyclopropyl group.

In yet another aspect, the invention provides compounds of formula 11-5g10, i.e., compounds according to any one of formulas 11-4, 11-4a, 11-4b, 11-4c, 11-4c1, 11-4d, 11-4d1, 11-4d2, 11-5, 11-5a, 11-5b, 11-5c, 11-5d, 11-5d1, 11-5e, 11-5e1, 11-5f, 11-5f1, 11-5f2, 11-5f3, 11-5f4, 11-5f5, or 11-5f6, wherein R₁ is hydrogen and R₂ is cyclopropyl. In one embodiment, the compound is racemic. In another embodiment, the compound is enantiomerically enriched.

In yet still another aspect, the invention provides compounds of formula 11-5h, i.e., compounds of formula 11-5g wherein,

X is O; and Y is -NR₆₀R₇₀ or -N(R₃₀)(CH₂)₂₋₆NR₆₀R₇₀; where R₆₀ and R₇₀ are independently selected from: hydrogen, methyl, ethyl, (C₃ -C₈)cycloalkyl, aryl-(C₁-C₆ alkyl) (such as benzyl or phenethyl), 4-pyridylmethyl,

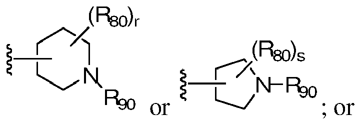

R₆₀ and R₇₀ taken together with the nitrogen atom to which they are bound form a heterocycloalkyl group selected from 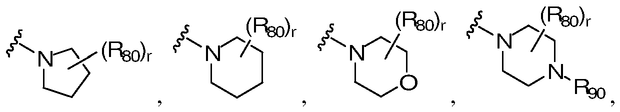 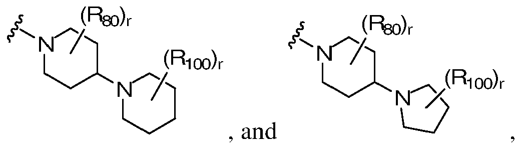 where
each R₈₀ is independently unsubstituted C₁-C₄ alkyl or C₁-C₄ alkyl substituted with hydroxyl or halogen;
each R₉₀ is independently hydrogen, unsubstituted C₁-C₄ alkyl, C₁-C₄ alkyl substituted with hydroxyl or halogen, unsubstituted C₃-C₈ cycloalkyl, C₃-C₈ cycloalkyl substituted with one or more (e.g., 1-4) R₅₀ groups, phenyl-(C₁-C₄ alkyl)-, pyridyl-(C₁-C₄ alkyl)-, thienyl-(C₁-C₄ alkyl)-, -C(O)O-(C₁-C₄ alkyl), -C(O)O-phenyl, -SO₂-(C₁-C₆ alkyl) -SO₂-phenyl unsubstituted phenyl, phenyl substituted with one or more (e.g., 1-4) R₅₀ groups, pyridyl, thienyl, pyridyl substituted with one or more (e.g., 1-4) R₅₀ groups, thienyl substituted with one or more (e.g., 1-4) R₅₀ groups, where
each R₅₀ is independently selected from: halogen, C₁-C₆ alkyl, C₁-C₆ haloalkyl, -OH, -O C₁-C₄ alkyl, OCF₃, -CN, - NR₆₀R₇₀, -C(O)O-(C₁-C₄ alkyl), -CONR₆₀R₇₀, -(C₁-C₆ alkyl)-NR₆₀R₇₀, -NR₆₀CO-(C₁-C₄ alkyl), -NR₆₀CO-phenyl, -NR₆₀CO-pyridyl, -NR₆₀CO-thienyl, and -NR₆₀CONR₆₀R₇₀,
each R₁₀₀ is independently hydrogen or C₁-C₄ alkyl;
each r is 0 to 4; and
each s is 0 to 3.

In yet still another aspect, the invention provides compounds of formula 11-5h1, i.e., compounds of formula 11-5g, wherein Y is -O-(C₁-C₆ alkyl). In one embodiment, Y is -O-(C₁-C₄ alkyl).

In yet still another aspect, the invention provides compounds of formula 11-5h2, i.e., compounds of formula 11-5g, wherein Y is -O-phenyl.

In yet still another aspect, the invention provides compounds of formula 11-5i, i.e., compounds of formula 11-5h, wherein said group 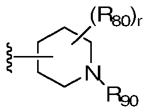 is a group of the formula: 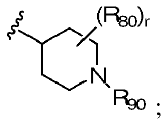 and said group 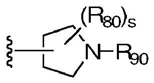 is a group of the formula:

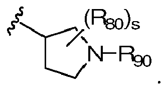

In a further aspect, the invention provides compounds of formula 11-5j, i.e., compounds according to any one of formulas, 11-5h, 11-5h1, 11-5h2, or 11-5i, wherein R₁ is H or C₁-C₄ alkyl. In one embodiment, R₁ is H or methyl. In another embodiment, R₁ is H.

In yet another aspect, the invention provides compounds of formula 11-6, i.e., compounds according to formula 9 or any one of formulas 10, 10-1, 10-2, 10-3, 10-4, 10-5, 10-6, 10-6a, 10-6b, 10-6c, 10-6d, 10-7, 10-8, 10-9, 10-10, 10-11, 10-11a, 10-11b, 10-11c, 10-12, 10-13, 10-14, or 10-15 11, 11-1, 11-1a, 11-2, 11-2a, 11-2b, 11-2c, 11-2d, 11-2e, 11-2f, 11-2g, 11-2h, 11-2i, 11-3, 11-3a, 11-3a1, 11-3a2, 11-3a3, 11-3a4, 11-3a5, 11-3a6, 11-3a7, 11-3a8, 11-3a9, 11-3a10, 11-3b, 11-3b1, 11-3b2, 11-3c, 11-3d, 11-4, 11-4a, 11-4b, 11-4c, 11-4c1, 11-4d, 11-4d1, 11-4d2, 11-5, 11-5a, 11-5b, 11-5c, 11-5d, 11-5d1, 11-5e, 11-5e1, 11-5f, 11-5f1, 11-5f2, 11-5f3, 11-5f4, 11-5f5, 11-5f6, 11-5g, 11-5g1, 11-5g2, 11-5g3, 11-5g4, 11-5g5, 11-5g6, 11-5g7, 11-5g8, 11-5g9, 11-5g10, 11-5h, 11-5h1, 11-5h2, 11-5i, or 11-5j, wherein the C₃-C₈ cycloalkyl (ring A of Formula I) group is cyclopropyl.

In yet another aspect, the invention provides compounds of formula 11-7, i.e., compounds according to formula 9 or any one of formulas 10, 10-1, 10-2, 10-3, 10-4, 10-5, 10-6, 10-6a, 10-6b, 10-6c, 10-6d, 10-7, 10-8, 10-9, 10-10, 10-11, 10-11a, 10-11b, 10-11c, 10-12, 10-13, 10-14, or 10-15 11, 11-1, 11-1a, 11-2, 11-2a, 11-2b, 11-2c, 11-2d, 11-2e, 11-2f, 11-2g, 11-2h, 11-2i, 11-3, 11-3a, 11-3a1, 11-3a2, 11-3a3, 11-3a4, 11-3a5, 11-3a6, 11-3a7, 11-3a8, 11-3a9, 11-3a10, 11-3b, 11-3b1, 11-3b2, 11-3c, 11-3d, 11-4, 11-4a, 11-4b, 11-4c, 11-4c1, 11-4d, 11-4d1, 11-4d2, 11-5, 11-5a, 11-5b, 11-5c, 11-5d, 11-5d1, 11-5e, 11-5e1, 11-5f, 11-5f1, 11-5f2, 11-5f3, 11-5f4, 11-5f5, 11-5f6, 11-5g, 11-5g1, 11-5g2, 11-5g3, 11-5g4, 11-5g5, 11-5g6, 11-5g7, 11-5g8, 11-5g9, 11-5g10, 11-5h, 11-5h1, 11-5h2, 11-5i, or 11-5j, wherein the C₃-C₈ cycloalkyl group (ring A of Formula I) is cyclobutyl.

In yet another aspect, the invention provides compounds of formula 11-8, i.e., compounds according to formula 9 or any one of formulas 10, 10-1, 10-2, 10-3, 10-4, 10-5, 10-6, 10-6a, 10-6b, 10-6c, 10-6d, 10-7, 10-8, 10-9, 10-10, 10-11, 10-11a, 10-11b, 10-11c, 10-12, 10-13, 10-14, or 10-15 11, 11-1, 11-1a, 11-2, 11-2a, 11-2b, 11-2c, 11-2d, 11-2e, 11-2f, 11-2g, 11-2h, 11-2i, 11-3, 11-3a, 11-3a1, 11-3a2, 11-3a3, 11-3a4, 11-3a5, 11-3a6, 11-3a7, 11-3a8, 11-3a9, 11-3a10, 11-3b, 11-3b1, 11-3b2, 11-3c, 11-3d, 11-4, 11-4a, 11-4b, 11-4c, 11-4c1, 11-4d, 11-4d1, 11-4d2, 11-5, 11-5a, 11-5b, 11-5c, 11-5d, 11-5d1, 11-5e, 11-5e1, 11-5f, 11-5f1, 11-5f2, 11-5f3, 11-5f4, 11-5f5, 11-5f6, 11-5g, 11-5g1, 11-5g2, 11-5g3, 11-5g4, 11-5g5, 11-5g6, 11-5g7, 11-5g8, 11-5g9, 11-5g10, 11-5h, 11-5h1, 11-5h2, 11-5i, or 11-5j, wherein the C₃-C₈ cycloalkyl group. (ring A of Formula I) is cyclopentyl.

In yet another aspect, the invention provides compounds of formula 11-9, i.e., compounds according to formula 9 or any one of formulas 10, 10-1, 10-2, 10-3, 10-4, 10-5, 10-6, 10-6a, 10-6b, 10-6c, 10-6d, 10-7, 10-8, 10-9, 10-10, 10-11, 10-11a, 10-11b, 10-11c, 10-12, 10-13, 10-14, or 10-15 11, 11-1, 11-1a, 11-2, 11-2a, 11-2b, 11-2c, 11-2d, 11-2e, 11-2f, 11-2g, 11-2h, 11-2i, 11-3, 11-3a, 11-3a1, 11-3a2, 11-3a3, 11-3a4, 11-3a5, 11-3a6, 11-3a7, 11-3a8, 11-3a9, 11-3a10, 11-3b, 11-3b1, 11-3b2, 11-3c, 11-3d, 11-4, 11-4a, 11-4b, 11-4c, 11-4c1, 11-4d, 11-4d1, 11-4d2, 11-5, 11-5a, 11-5b, 11-5c, 11-5d, 11-5d1, 11-5e, 11-5e1, 11-5f, 11-5f1, 11-5f2, 11-5f3, 11-5f4, 11-5f5, 11-5f6, 11-5g, 11-5g1, 11-5g2, 11-5g3, 11-5g4, 11-5g5, 11-5g6, 11-5g7, 11-5g8, 11-5g9, 11-5g10, 11-5h, 11-5h1, 11-5h2, 11-5i, or 11-5j, wherein the C₃-C₈ cycloalkyl group (ring A of Formula I) is cyclohexyl.

In yet another aspect, the invention provides compounds of formula 11-10, i.e., compounds according to any one of formulas formula 9 or 10, 10-1, 10-2, 10-3, 10-4, 10-5, 10-6, 10-6a, 10-6b, 10-6c, 10-6d, 10-7, 10-8, 10-9, 10-10, 10-11, 10-11a, 10-11b, 10-11c, 10-12, 10-13, 10-14, or 10-15 11, 11-1, 11-1a, 11-2, 11-2a, 11-2b, 11-2c, 11-2d, 11-2e, 11-2f, 11-2g, 11-2h, 11-2i, 11-3, 11-3a, 11-3a1, 11-3a2, 11-3a3, 11-3a4, 11-3a5, 11-3a6, 11-3a7, 11-3a8, 11-3a9, 11-3a10, 11-3b, 11-3b1, 11-3b2, 11-3c, 11-3d, 11-4, 11-4a, 11-4b, 11-4c, 11-4c1, 11-4d, 11-4d1, 11-4d2, 11-5, 11-5a, 11-5b, 11-5c, 11-5d, 11-5d1, 11-5e, 11-5e1, 11-5f, 11-5f1, 11-5f2, 11-5f3, 11-5f4, 11-5f5, 11-5f6, 11-5g, 11-5g1, 11-5g2, 11-5g3, 11-5g4, 11-5g5, 11-5g6, 11-5g7, 11-5g8, 11-5g9, 11-5g10, 11-5h, 11-5h1, 11-5h2, 11-5i, or 11-5j, wherein the C₃-C₈ cycloalkyl group (ring A of Formula I) is cycloheptyl.

In still yet another aspect, the invention provides compounds of formula 11-11, i.e., compounds according to formula 9 or any one of formulas 10, 10-1, 10-2, 10-3, 10-4, 10-5, 10-6, 10-6a, 10-6b, 10-6c, 10-6d, 10-7, 10-8, 10-9, 10-10, 10-11, 10-11a, 10-11b, 10-11c, 10-12, 10-13, 10-14, or 10-15 11, 11-1, 11-1a, 11-2, 11-2a, 11-2b, 11-2c, 11-2d, 11-2e, 11-2f, 11-2g, 11-2h, 11-2i, 11-3, 11-3a, 11-3a1, 11-3a2, 11-3a3, 11-3a4, 11-3a5, 11-3a6, 11-3a7, 11-3a8, 11-3a9, 11-3a10, 11-3b, 11-3b1, 11-3b2, 11-3c, 11-3d, 11-4, 11-4a, 11-4b, 11-4c, 11-4c1, 11-4d, 11-4d1, 11-4d2, 11-5, 11-5a, 11-5b, 11-5c, 11-5d, 11-5d1, 11-5e, 11-5e1, 11-Sf, 11-5f1, 11-5f2, 11-5f3, 11-5f4, 11-5f5, 11-5f6, 11-5g, 11-5g1, 11-5g2, 11-5g3, 11-5g4, 11-5g5, 11-5g6, 11-5g7, 11-5g8, 11-5g9, 11-5g10, 11-5h, 11-5h1, 11-5h2, 11-5i, or 11-5j, wherein the C₃-C₈ cycloalkyl group (ring A of Formula I) is cyclooctyl.

In another aspect, the invention provides compounds of formula 12, i.e., compounds of formula I, wherein
the A-ring is heteroaryl, which is pyridyl, pyrimidyl, quinolinyl, isoquinolinyl, thienyl, thiazolyl, imidazolyl, pyrazolyl, isoxazolyl, or oxazolyl, each of which is optionally substituted at a substitutable position with halogen, C₁-C₆ alkyl, C₂-C₆ alkenyl, C₂-C₆ alkynyl, C₁-C₆ alkoxy, C₁-C₆ haloalkyl, C₁-C₆ haloalkoxy, hydroxyl, hydroxy-(C₁-C₄ alkyl), CN, NO₂, aryloxy, aryl-(C₁-C₄ alkoxy) (such as benzyloxy),-SO₂-(C₁-C₆ alkyl), -NR'R", C₁-C₆ alkanoyl, -C(O)OR', -(C₁-C₄ alkyl)-C(O)OR', heteroaryl, aryl, aryl-(C₁-C₄ alkyl), -SO₂-NR'R", -C(O)NR'R", -OC(O)NR'R", -NR'C(O)R", -NR'C(O)NR'R", -NR'C(O)O-(C₁-C₆ alkyl) or -NR'C(O)O-phenyl, where each R' and R" is independently hydrogen or C₁-C₆ alkyl. In one embodiment, the B-ring is pyrazolyl, imidazolyl, pyrrolyl, triazolyl, pyrrolidinyl, pyrazolidinyl, imidazolidinyl, pyridyl, pyrimidyl, or isoxazolyl, each of which is optionally substituted at a substitutable position with a group that is independently C₁-C₆ alkyl, C₁-C₆ alkoxy, -NR'R", -SO₂-NR'R" -C(O)NR'R", -NR'C(O)R", -NR'C(O)NR'R", -NR'C(O)O-(C₁-C₆ alkyl), -NR'C(O)O-phenyl, NO₂, CN, -C(O)OR', hydroxyl, hydroxy-(C₁-C₆ alkyl), -S(O)_{z} (C₁-C₆ alkyl), -S(O)_{z} aryl, halogen, C₁-C₂ haloalkyl, C₁-C₂ haloalkoxy, benzyl or phenyl.

In still another aspect, the invention provides compounds of formula 13, i.e., compounds of formula I or 12 having the following formula: 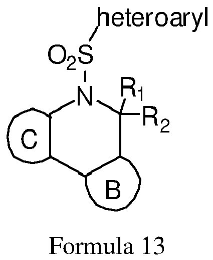 stereoisomers, tautomers, mixtures of stereoisomers and/or tautomers or pharmaceutically acceptable salts thereof. In one embodiment, the heteroaryl group is optionally substituted at a substitutable position with halogen, C₁-C₆ alkyl, C₂-C₆ alkenyl, C₂-C₆ alkynyl, C₁-C₆ alkoxy, C₁-C₆ haloalkyl, C₁-C₆ haloalkoxy, hydroxyl, hydroxy-(C₁-C₄ alkyl), CN, NO₂, aryloxy, aryl-(C₁-C₄alkoxy), -SO₂-(C₁-C₆ alkyl), -NR'R", C₁-C₆ alkanoyl, -C(O)OR', -(C₁-C₄ alkyl)-C(O)OR', pyridyl, phenyl, phenyl-(C₁-C₄ alkyl), -SO₂-NR'R", -C(O)NR'R", -OC(O)NR'R", -NR'C(O)R", -NR'C(O)NR'R", or -NR'C(O)OR', where each R' and R" is independently hydrogen or C₁-C₆ alkyl. In another embodiment the heteroaryl group is optionally substituted as described immediately above and the C-ring is cyclohexyl, phenyl, thienyl, imidazolinyl, thiazolyl, isothiazolyl, oxazolyl, isoxazolyl, pyrrolyl, pyrazolyl, pyrimidyl, pyrazinyl or pyridyl, each of which is optionally substituted at each substitutable position with groups that are independently halogen, C₁-C₆ alkyl, C₁-C₆ alkoxy, aryloxy, aryl-(C₁-C₄alkoxy), C₁-C₆ haloalkyl, C₁-C₆ haloalkoxy, hydroxyl, hydroxy-(C₁-C₆ alkyl), -NR'R", -(C₁-C₄ alkyl)-NR'R", -NR'C(O)O-(C₁-C₆ alkyl), -NR'C(O)O-phenyl, -COR', -C(O)OR', -C(O)NR'R", -OC(O)NR'R", -NR'C(O)NR'R"; -(C₁-C₆ alkyl)-C(O)OR', -(C₁-C₆ alkyl)-C(O)NR'R", -NR'C(O)R", NO₂, CN, oxo, benzyl, phenyl, oxazolyl, pyrazolyl, thiazolyl, pyridyl, furanyl, thienyl, C₂-C₆ alkenyl, C₂-C₆ alkynyl, -NR'SO₂R" -SO₂-NR'R", -S(O)_{z} aryl (where the aryl group is preferably naphthyl or phenyl, still more preferably, phenyl),or -S(O)_{z} (C₁-C₆ alkyl) where the alkyl, alkenyl and alkynyl portions of the above are unsubstituted or substituted with 1, 2 or 3 groups that are independently halogen, hydroxyl, -NR'R" or C₁-C₆ alkoxy, and where the aryl or heteroaryl portions of the above are optionally substituted with 1, or 2 groups that are independently C₁-C₄ alkyl, C₁-C₄ alkoxy, halogen, CF₃, OCF₃, hydroxyl, hydroxy-(C₁-C₄ alkyl), or -NR'R".

In still yet another aspect, the invention provides compounds of formula 13-1, i.e., compounds of formula 13, wherein the B-ring is pyrazolyl, imidazolyl, pyrrolyl, triazolyl, pyrrolidinyl, pyrazolidinyl, imidazolidinyl, pyridyl, pyrimidyl, or isoxazolyl, each of which is unsubstituted.

In still yet another aspect, the invention provides compounds of formula 13-2, i.e., compounds of formula 13, wherein the B-ring has the formula: 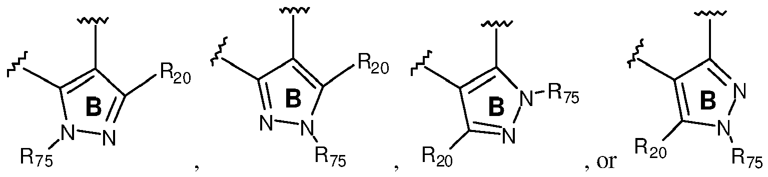 wherein
R₂₀ is hydrogen, C₁-C₆ alkyl, C₁-C₆ alkoxy, -NR'R", -(C₁-C₄ alkyl)-NR'R", -S(O)_{z} (C₁-C₆ alkyl), halogen, hydroxyl, CN, NO₂, CH₂F, CHF₂, CF₃, -C(O)OR', -C(O)NR'R", -(C₁-C₆ alkyl)-C(O)OR', -(C₁-C₆ alkyl)-C(O)NR'R", or phenyl, and
R₇₅ is hydrogen, C₁-C₆ alkyl, C₁-C₆ alkanoyl, phenyl-C₁-C₆ alkanoyl, -C(O)NR'R", -S(O)₂-(C₁-C₆ alkyl), -S(O)₂-aryl, -SO₂NR'R", phenyl, phenyl-(C₁-C₆ alkyl) (preferably phenethyl or benzyl, more preferably, benzyl), phenylcarbonyl, benzylcarbonyl, or heteroarylcarbonyl, where the heteroaryl group is pyridyl, pyrimidyl, thienyl or furanyl.

In still yet another aspect, the invention provides compounds of formula 13-3, i.e., compounds of formula 13-2, wherein the B-ring has the formula:

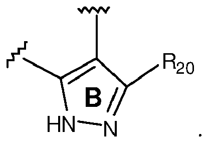

In still yet another aspect, the invention provides compounds of formula 13-4, i.e., compounds of formula 13-2, wherein the B-ring has the formula:

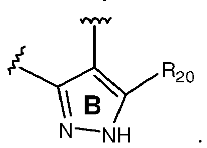

In still yet another aspect, the invention provides compounds of formula 13-5, i.e., compounds of formula 13-2, wherein the B-ring has the formula:

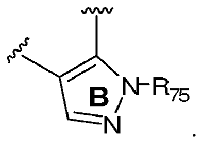

In still yet another aspect, the invention provides compounds of formula 13-6, i.e., compounds of formula 13-2, wherein the B-ring has the formula:

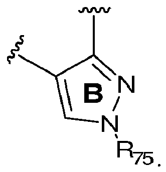

In a further aspect, the invention provides compounds of formula 13-6a, i.e., compounds according to any one of formulas 13-2, 13-3, or 13-4, wherein R₂₀ is hydrogen, C₁-C₄ alkyl, CH₂F, CHF₂, CF₃, -C(O)OR', -S(O)_{z} (C₁-C₄ alkyl), or hydroxyl.

In another aspect, the invention provides compounds of formula 13-6b, i.e., compounds of formula 13-6a, wherein R₂₀ is hydrogen or methyl.

In yet another aspect, the invention provides compounds of formula 13-6c, i.e., compounds according to any one of formulas 13-2, 13-5 or 13-6, wherein R₇₅ is hydrogen or methyl.

In still yet another aspect, the invention provides compounds of formula 13-6d, i.e., compounds according to any one of formulas 13-2, 13-5 or 13-6, wherein R₇₅ is hydrogen. In one embodiment, R₂₀ and R₇₅ are both hydrogen.

In still yet another aspect, the invention provides compounds of formula 13-7, i.e., compounds of formula 13-2, wherein the B-ring has the formula: 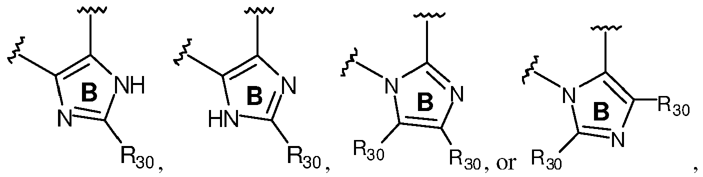 wherein R₃₀ is hydrogen, C₁-C₄ alkyl, C₁-C₄ alkoxy, -NR'R", C₁-C₄ alkythio, halogen, hydroxy, CH₂F, CHF₂, CF₃, or phenyl.

In still yet another aspect, the invention provides compounds of formula 13-8, i.e., compounds of formula 13-7, wherein the B-ring has the formula:

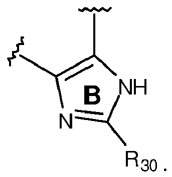

In still yet another aspect, the invention provides compounds of formula 13-9, i.e., compounds of formula 13-7, wherein the B-ring has the formula:

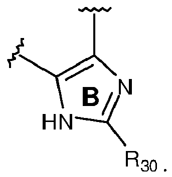

In still yet another aspect, the invention provides compounds of formula 13-10, i.e., compounds of formula 13-7, wherein the B-ring has the formula:

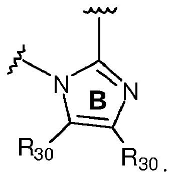

In still yet another aspect, the invention provides compounds of formula 13-11, i.e., compounds of formula 13-7, wherein the B-ring has the formula:

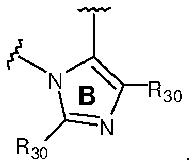

In a further aspect, the invention provides compounds of formula 13-11a, i.e., compounds according to any one of formulas 13-8, 13-9, 13-10, or 13-11, wherein each R₃₀ is independently hydrogen, C₁-C₄ alkyl, CH₂F, CHF₂, or CF₃.

In another aspect, the invention provides compounds of formula 13-11b, i.e., compounds of formula 13-11a, wherein each R₃₀ is independently hydrogen or methyl.

In still another aspect, the invention provides compounds of formula 13-11c, i.e., compounds of formula 13-11a, wherein each R₃₀ is hydrogen.

In still yet another aspect, the invention provides compounds of formula 13-12, i.e., compounds of formula 10, wherein the B-ring has the formula:

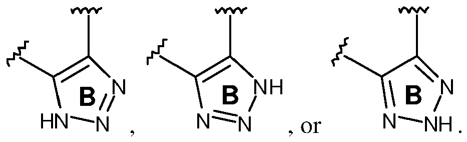

In still yet another aspect, the invention provides compounds of formula 13-13, i.e., compounds of formula 13-12, wherein the B-ring has the formula:

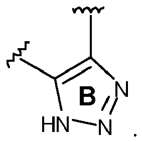

In still yet another aspect, the invention provides compounds of formula 13-14, i.e., compounds of formula 13-12, wherein the B-ring has the formula:

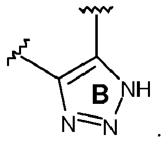

In still yet another aspect, the invention provides compounds of formula 13-15, i.e., compounds of formula 13-12, wherein the B-ring has the formula:

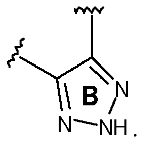

In yet still another aspect, the invention provides compounds of formula 14, i.e., compounds according to of formula 12 or any one of formulas 13, 13-1, 13-2, 13-3, 13-4, 13-5, 13-6, 13-6a, 13-6b, 13-6c, 13-6d, 13-7, 13-8, 13-9, 13-10, 13-11, 13-11a, 13-11b, 13-11c, 13-12, 13-13, 13-14, or 13-15, wherein the C-ring is phenyl or cyclohexyl, which is optionally substituted with 1, 2, 3, or 4 groups that are independently halogen, C₁-C₄ alkyl, C₁-C₄ alkoxy, aryloxy, aryl-(C₁-C₄ alkoxy), C₁-C₄ haloalkyl (in another aspect, CF₃), C₁-C₄ haloalkoxy (in another aspect, OCF₃), hydroxyl, hydroxy-(C₁-C₄ alkyl), -NR'R", -(C₁-C₄ alkyl)-NR'R", -NR'C(O)O-(C₁-C₆ alkyl), -NR'C(O)O-phenyl, -COR', -C(O)OR', -C(O)NR'R", -OC(O)NR'R", -NR'C(O)NR'R"; -(C₁-C₆ alkyl)-C(O)OR', -(C₁-C₆ alkyl)-C(O)NR'R", -NR'C(O)R", NO₂, CN, phenyl, oxazolyl, pyrazolyl, thiazolyl, pyridyl, furanyl, thienyl, C₂-C₆ alkenyl, C₂-C₆ alkynyl, -NR'SO₂R", -SO₂-NR'R", -S(O)_{z} aryl (such as phenyl), or -S(O)_{z} (C₁-C₆ alkyl), where the alkyl, alkenyl and alkynyl portions of the above are unsubstituted or substituted with 1 or 2 groups that are independently halogen, hydroxyl, -NR'R" or C₁-C₆ alkoxy, and where the aryl or heteroaryl portions of the above are optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R". In one embodiment, the C-ring is an optionally substituted phenyl.

In yet still another aspect, the invention provides compounds of formula 14-1, i.e., compounds of formula 14 wherein the C-ring is unsubstituted phenyl.

In yet still another aspect, the invention provides compounds of formula 14-1a, i.e., compounds of formula 14 wherein the C-ring is unsubstituted cyclohexyl.

In yet still another aspect, the invention provides compounds of formula 14-2, i.e., compounds of formula 14 wherein the C-ring is phenyl substituted with 1 or 2 groups that are independently Cl, F, methyl, ethyl, isopropyl, methoxy, ethoxy, CF₃, OCF₃, OH, CH₂OH, NH₂, NH(CH₃), N(CH₃)₂, or -OC(O)N(CH₃)₂. In one embodiment, the C-ring is bis-substituted with at least one halogen. In another embodiment, the halogen is F. In still another embodiment, the halogen is Cl. In another embodiment, the C-ring is bis-substituted with two halogens, which are the same or different. In still another embodiment, the C-ring is substituted at least at position 7. In another embodiment, the C-ring is substituted at positions 7 and 8. In another embodiment, the C-ring is monosubstituted with a halogen. In another embodiment, the halogen is F. In still another embodiment, the halogen is Cl. In still another embodiment, the C-ring is substituted at position 7. In yet another embodiment, the C-ring is substituted at position 8.

In still yet another aspect, the invention provides compounds of formula 14-2a, i.e., compounds of formula 14, wherein the C-ring is phenyl substituted with 1 or 2 groups that are independently -NR'C(O)O-(C₁-C₆ alkyl), -NR'C(O)O-phenyl, -C(O)OR', -C(O)NR'R", -OC(O)NR'R", -NR'C(O)NR'R"; -(C₁-C₆ alkyl)-C(O)OR', -(C₁-C₆ alkyl)-C(O)NR'R", -NR'C(O)R", NO₂, CN, phenyl, -S(O)_{z} phenyl, or -S(O)_{z} (C₁-C₆ alkyl), where the phenyl portions of the above are optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R". In one embodiment, the C-ring is substituted at least at the 7-position. In another embodiment, the C-ring is substituted at least at the 8-position.

In still another aspect, the invention provides compounds of formula 14-2b, i.e., compounds of formula 14, wherein the C-ring is phenyl substituted with 1 or 2 groups that are independently C₁-C₄ alkyl, C₂-C₆ alkenyl, C₂-C₆ alkynyl, where the alkyl, alkenyl and alkynyl portions of the above are unsubstituted or substituted with 1 or 2 groups that are independently halogen, hydroxyl, -NR'R" or C₁-C₄ alkoxy. In one embodiment, the C-ring is substituted at least at the 7-position. In another embodiment, the C-ring is substituted at least at the 8-position.

In still another aspect, the invention provides compounds of formula 14-2c, i.e., compounds of formula 14, wherein the C-ring is phenyl substituted with 1 or 2 groups that are independently -C(O)OH, -C(O)O-(C₁-C₄ alkyl), NO₂, CN, or phenyl, where the phenyl group is optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R". In one embodiment, the C-ring is substituted at least at the 7-position. In another embodiment, the C-ring is substituted at least at the 8-position.

In yet another aspect, the invention provides compounds of formula 14-2d, i.e., compounds of formula 14, wherein the C-ring is phenyl substituted with 1 or 2 groups that are independently C₁-C₄ alkyl, C₁-C₄ alkoxy, halogen, -C(O)NR'R", -NR'C(O)R", -OC(O)NR'R", -NR'C(O)NR'R"; or -NR'C(O)O-(C₁-C₆ alkyl), -NR'C(O)O-phenyl, where the phenyl group is optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R". In one embodiment, the C-ring is substituted at least at the 7-position. In another embodiment, the C-ring is substituted at least at the 8-position.

In yet still another aspect, the invention provides compounds of formula 14-2e, i.e., compounds of formula 14 wherein the C-ring is cyclohexyl substituted with 1 or 2 groups that are independently Cl, F, methyl, ethyl, isopropyl, methoxy, ethoxy, CF₃, OCF₃, OH, CH₂OH, NH₂, NH(CH₃), N(CH₃)₂, or -OC(O)N(CH₃)₂.

In still yet another aspect, the invention provides compounds of formula 14-2f, i.e., compounds of formula 14, wherein the C-ring is cyclohexyl substituted with 1 or 2 groups that are independently -NR'C(O)O-(C₁-C₆ alkyl), -NR'C(O)O-phenyl, -C(O)OR', -C(O)NR'R", -OC(O)NR'R", -NR'C(O)NR'R"; -(C₁-C₄ alkyl)-C(O)OR', -(C₁-C₄ alkyl)-C(O)NR'R", -NR'C(O)R", NO₂, CN, phenyl, -S(O)_{z} phenyl, or -S(O)_{z} (C₁-C₆ alkyl), where the phenyl portions of the above are optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R".

In still another aspect, the invention provides compounds of formula 14-2g, i.e., compounds of formula 14, wherein the C-ring is cyclohexyl substituted with 1 or 2 groups that are independently C₁-C₄ alkyl, C₂-C₆ alkenyl, C₂-C₆ alkynyl, where the alkyl, alkenyl and alkynyl portions of the above are unsubstituted or substituted with 1 or 2 groups that are independently halogen, hydroxyl, -NR'R" or C₁-C₄ alkoxy.

In still another aspect, the invention provides compounds of formula 14-2h, i.e., compounds of formula 14, wherein the C-ring is cyclohexyl substituted with 1 or 2 groups that are independently -C(O)OH, -C(O)O-(C₁-C₄ alkyl), NO₂, CN, or phenyl, where the phenyl group is optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R".

In yet another aspect, the invention provides compounds of formula 14-2i, i.e., compounds of formula 14, wherein the C-ring is cyclohexyl substituted with 1 or 2 groups that are independently C₁-C₄ alkyl, C₁-C₄ alkoxy, halogen, -C(O)NR'R", -NR'C(O)R", -OC(O)NR'R", -NR'C(O)NR'R"; -NR'C(O)O-(C₁-C₆ alkyl) or -NR'C(O)O-phenyl, where the phenyl group is optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R".

In yet still another aspect, the invention provides compounds of formula 14-3, i.e., compounds of formula 14 wherein the C-ring is phenyl or cyclohexyl substituted with 1 or 2 groups that are Cl, F, methyl, isopropyl, methoxy, CF₃, OCF₃, OH, or -OC(O)N(CH₃)₂.

In yet still another aspect, the invention provides compounds of formula 14-3a, i.e., compounds according to any one of formulas 14, 14-1, 14-1a, 14-2, 14-2a, 14-2b, 14-2d, 14-2c, 14-2d, 14-2e, 14-2f, 14-2g, 14-2h, 14-2i, or 14-3, wherein R₂ is -X(CO)Y or -(C(R₃)₂)₁-₄X(CO)Y.

In still another aspect, the invention provides compounds of formula 14-3a1, i.e., compounds according to any one of formulas 14, 14-1, 14-1a, 14-2, 14-2a, 14-2b, 14-2d, 14-2c, 14-2d, 14-2e, 14-2f, 14-2g, 14-2h, 14-2i, or 14-3, wherein R₂ is hydrogen, C₁-C₃ alkyl or C₃-C₆ cyclopropyl.

In still another aspect, the invention provides compounds of formula 14-3a2, i.e., compounds according to any one of formulas 14, 14-1, 14-1a, 14-2, 14-2a, 14-2b, 14-2d, 14-2c, 14-2d, 14-2e, 14-2f, 14-2g, 14-2h, 14-2i, or 14-3, wherein R₂ is NO₂ or CN.

In still another aspect, the invention provides compounds of formula 14-3a3, i.e., compounds according to any one of formulas 14, 14-1, 14-1a, 14-2, 14-2a, 14-2b, 14-2d, 14-2c, 14-2d, 14-2e, 14-2f, 14-2g, 14-2h, 14-2i, or 14-3, wherein R₂ is C₂-C₆ alkenyl or C₂-C₆ alkynyl.

In still another aspect, the invention provides compounds of formula 14-3a4, i.e., compounds according to any one of formulas 14, 14-1, 14-1a, 14-2, 14-2a, 14-2b, 14-2d, 14-2c, 14-2d, 14-2e, 14-2f, 14-2g, 14-2h, 14-2i, or 14-3, wherein R₂ is C₁-C₄ haloalkyl.

In still another aspect, the invention provides compounds of formula 14-3a5, i.e., compounds according to any one of formulas 14, 14-1, 14-1a, 14-2, 14-2a, 14-2b, 14-2d, 14-2c, 14-2d, 14-2e, 14-2f, 14-2g, 14-2h, 14-2i, 14-3, 14-3a, 14-3a1, 14-3a2, 14-3a3, or 14-3a4, wherein R₁ is hydrogen.

In still yet another aspect, the invention provides compounds of formula 14-3a6, i.e., compounds according to any one of formulas 14, 14-1, 14-1a, 14-2, 14-2a, 14-2b, 14-2d, 14-2c, 14-2d, 14-2e, 14-2f, 14-2g, 14-2h, 14-2i, or 14-3, wherein R₁ and R₂ combined are oxo.

In still yet another aspect, the invention provides compounds of formula 14-3a7, i.e., compounds according to any one of formulas 14, 14-1, 14-1a, 14-2, 14-2a, 14-2b, 14-2d, 14-2c, 14-2d, 14-2e, 14-2f, 14-2g, 14-2h, 14-2i, or 14-3, wherein R₁ and R₂ combined are =NOR, where R is hydrogen, C₁-C₆ alkyl, aryl (such as phenyl) or arylalkyl (such as benzyl or phenethyl).

In yet another aspect, the invention provides compounds of formula 14-3a8, i.e., compounds according to any one of formulas 14, 14-1, 14-1a, 14-2, 14-2a, 14-2b, 14-2d, 14-2c, 14-2d, 14-2e, 14-2f, 14-2g, 14-2h, 14-2i, or 14-3, wherein R₁ and R₂ together with the carbon to which they are attached form a C₃-C₆ cycloalkyl group.

In yet another aspect, the invention provides compounds of formula 14-3a9, i.e., compounds according to any one of formulas 14, 14-1, 14-1a, 14-2, 14-2a, 14-2b, 14-2d, 14-2c, 14-2d, 14-2e, 14-2f, 14-2g, 14-2h, 14-2i, or 14-3, wherein R₁ and R₂ together with the carbon to which they are attached form a cyclopropyl group.

In yet another aspect, the invention provides compounds of formula 14-3a10, i.e., compounds according to any one of formulas 14, 14-1, 14-1a, 14-2, 14-2a, 14-2b, 14-2d, 14-2c, 14-2d, 14-2e, 14-2f, 14-2g, 14-2h, 14-2i, or 14-3, wherein R₁ is hydrogen and R₂ is cyclopropyl. In one embodiment, the compound is racemic. In another embodiment, the compound is enantiomerically enriched.

In yet still another aspect, the invention provides compounds of formula 14-3b, i.e., compounds of formula 14-3a, wherein
X is O; and Y is -NR₆₀R₇₀ or -N(R₃₀)(CH₂)₂₋₆NR₆₀R₇₀; where R₆₀ and R₇₀ are independently selected from: hydrogen, methyl, ethyl, (C₃ -C₈)cycloalkyl, aryl-(C₁-C₆)alkyl (such as benzyl or phenethyl), 4-pyridylmethyl, 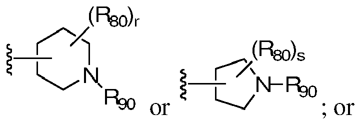
R₆₀ and R₇₀ taken together with the nitrogen atom to which they are bound form a heterocycloalkyl group selected from 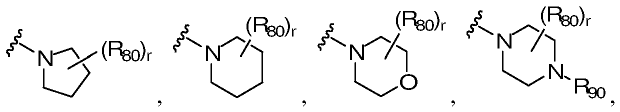 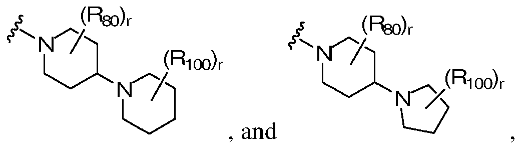 where,
   each R₈₀ is independently unsubstituted C₁-C₄ alkyl or C₁-C₄ alkyl substituted with hydroxyl or halogen;
   each R₉₀ is independently hydrogen, unsubstituted C₁-C₄ alkyl, C₁-C₄ alkyl substituted with hydroxyl or halogen, unsubstituted C₃-C₈ cycloalkyl, C₃-C₈ cycloalkyl substituted with one or more (e.g., 1-4) R₅₀, phenyl-(C₁-C₄ alkyl)-, pyridyl-(C₁-C₄ alkyl)-, thienyl-(C₁-C₄ alkyl), -C(O)O-(C₁-C₄ alkyl), -C(O)O-phenyl, -SO₂-(C₁-C₆ alkyl), -SO₂-phenyl, unsubstituted phenyl, phenyl substituted with one or more (e.g., 1-4) R₅₀ groups, pyridyl, thienyl, pyridyl substituted with one or more (e.g., 1-4) R₅₀ groups, thienyl substituted with one or more (e.g., 1-4) R₅₀ groups, where
   each R₅₀ is independently selected from: halogen, C₁-C₆ alkyl, C₁-C₆ haloalkyl, -OH, -O (C₁-C₄ alkyl), OCF₃, -CN, - NR₆₀R₇₀, -C(O)O-(C₁-C₄ alkyl), -CONR₆₀R₇₀, -(C₁-C₆ alkyl)-NR₆₀R₇₀, -NR₆₀CO-(C₁-C₄ alkyl), -NR₆₀CO-phenyl, -NR₆₀CO-pyridyl, -NR₆₀CO-thienyl, and -NR₆₀CONR₆₀R₇₀,
   each R₁₀₀ is independently hydrogen or C₁-C₄ alkyl;
   each r is 0 to 4; and
   each s is 0 to 3.

In yet still another aspect, the invention provides compounds of formula 14-3b1, i.e., compounds of formula 14-3a, wherein Y is -O-(C₁-C₆ alkyl). In one embodiment, Y is -O-(C₁-C₄ alkyl).

In yet still another aspect, the invention provides compounds of formula 14-3b2, i.e., compounds of formula 14-3a, wherein Y is -O-phenyl.

In yet still another aspect, the invention provides compounds of formula 14-3c, i.e., compounds of formula 14-3b, wherein said group 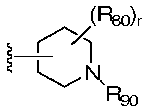 is a group of the formula: 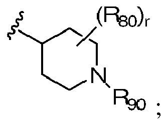 and, said group 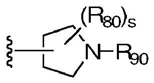 is a group of the formula:

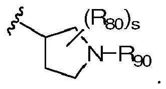

In a further aspect, the invention provides compounds of formula 14-3c1, i.e., compounds according to any one of formulas, 14-3b, 14-3b1, 14-3b2, or 14-3c, wherein R₁ is H or C₁-C₄ alkyl. In one embodiment, R₁ is H or methyl. In another embodiment, R₁ is H.

In yet still another aspect, the invention provides compounds of formula 14-4, i.e., compounds according to formula 12 or any one of formulas 13, 13-1, 13-2, 13-3, 13-4, 13-5, 13-6, 13-6a, 13-6b, 13-6c, 13-6d, 13-7, 13-8, 13-9, 13-10, 13-11, 13-11a, 13-11b, 13-11c, 13-12, 13-13, 13-14, or 13-15 the C-ring is 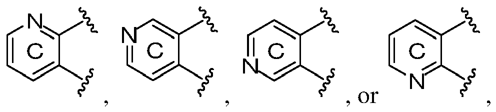 or N-oxide derivatives thereof,
each of which is optionally substituted with 1, 2, or 3 groups that are independently halogen, C₁-C₄ alkyl, C₁-C₄ alkoxy, aryloxy, aryl-(C₁-C₄ alkoxy), C₁-C₄ haloalkyl (in another aspect, CF₃), C₁-C₄ haloalkoxy (in another aspect, OCF₃), hydroxyl, hydroxy-(C₁-C₄ alkyl), -NR'R", -(C₁-C₆ alkyl)-NR'R", -NR'C(O)O-(C₁-C₆ alkyl), -NR'C(O)O-phenyl, -COR', -C(O)OR', -C(O)NR'R", OC(O)NR'R", -NR'C(O)NR'R"; -(C₁-C₆ alkyl)-C(O)OR', -(C₁-C₆ alkyl)-C(O)NR'R", -NR'C(O)R", NO₂, CN, benzyl, phenyl, oxazolyl, pyrazolyl, thiazolyl, pyridyl, furanyl, thienyl, C₂-C₆ alkenyl, C₂-C₆ alkynyl, -NR'SO₂R", -SO₂-NR'R", -S(O)_{z} aryl (where the aryl group is preferably naphthyl or phenyl, still more preferably, phenyl), or -S(O)_{z} (C₁-C₆ alky), where the alkyl, alkenyl and alkynyl portions of the above are unsubstituted or substituted with 1, 2 or 3 groups that are independently halogen, hydroxyl, - NR'R" or C₁-C₆ alkoxy, and where the aryl or heteroaryl portions of the above are optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R". In one embodiment, the invention provides compounds of formula 14-4 wherein the C-ring is pyridyl or pyridyl N-oxide substituted with 1 or 2 groups that are independently -NR'C(O)O-(C₁-C₆ alkyl), -NR'C(O)O-phenyl, -C(O)OR', -C(O)NR'R", -(C₁-C₆ alkyl)-C(O)OR', -(C₁-C₆ alkyl)-C(O)NR'R", -NR'C(O)R", -OC(O)NR'R", - NR'C(O)NR'R"; NO₂, CN, phenyl, -S(O)_{z} aryl (where the aryl group is preferably naphthyl or phenyl, still more preferably, phenyl) or -S(O)_{z} (C₁-C₆ alkyl) where the aryl portions of the above are optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R". In an alternative embodiment, the C-ring is pyridyl or pyridyl N-oxide substituted with 1 or 2 groups that are independently C₁-C₄ alkyl, C₂-C₆ alkenyl, C₂-C₆ alkynyl, where the alkyl, alkenyl and alkynyl portions of the above are unsubstituted or substituted with 1 or 2 groups that are independently halogen, hydroxyl, - NR'R" or C₁-C₄ alkoxy. In another alternative embodiment, the C-ring is pyridyl or pyridyl N-oxide substituted with 1 or 2 groups that are independently -C(O)OH, -C(O)O-(C₁-C₄ alkyl), NO₂, CN, or phenyl, where the phenyl group is optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R". In still another alternative embodiment, the C-ring is pyridyl or pyridyl N-oxide substituted with 1 or 2 groups that are independently C₁-C₄ alkyl, C₁-C₄ alkoxy, halogen, -C(O)NR'R", -NR'C(O)R", -OC(O)NR'R", -NR'C(O)NR'R"; or -NR'C(O)O-(C₁-C₆ alkyl), or -NR'C(O)O-phenyl, where the phenyl group is optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-C₁-C₆ alkyl, or -NR'R".

In still another aspect, the invention provides compounds of formula 14-4a, i.e., compounds of formula 12 or any one of formulas 13, 13-1, 13-2, 13-3, 13-4, 13-5, 13-6, 13-6a, 13-6b, 13-6c, 13-6d, 13-7, 13-8, 13-9, 13-10, 13-11, 13-11a, 13-11b, 13-11c, 13-12, 13-13, 13-14, or 13-15, wherein the C-ring is 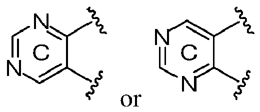 each of which is optionally substituted with 1 or 2 groups that are independently halogen, C₁-C₄ alkyl, C₁-C₄ alkoxy, C₁-C₆ alkanoyl, aryloxy, aryl-(C₁-C₄ alkoxy), C₁-C₄ haloalkyl (in another aspect, CF₃), C₁-C₄ haloalkoxy (in another aspect, OCF₃), hydroxyl, hydroxy-(C₁-C₄ alkyl), -NR'R", -NR'C(O)O-(C₁-C₆ alkyl), -NR'C(O)O-phenyl, -C(O)OR', -C(O)NR'R", -OC(O)NR'R", -NR'C(O)NR'R"; -(C₁-C₆ alkyl)-C(O)OR', -(C₁-C₆ alkyl)-C(O)NR'R", -NR'C(O)R", NO₂, CN, benzyl, phenyl, oxazolyl, pyrazolyl, thiazolyl, pyridyl, furanyl, thienyl, C₂-C₆ alkenyl, C₂-C₆ alkynyl, -NR'SO₂R" -SO₂-NR'R", -S(O)z aryl (where the aryl group is preferably naphthyl or phenyl, still more preferably, phenyl), or -S(O)_{z} (C₁-C₆ alkyl), where the alkyl, alkenyl and alkynyl portions of the above are unsubstituted or substituted with 1, 2 or 3 groups that are independently halogen, hydroxyl, -NR'R" or C₁-C₆ alkoxy, and where the aryl portions of the above are optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R". In one embodiment, the invention provides compounds of formula 14-4a, wherein the C-ring is pyrimidinyl substituted with 1 or 2 groups that are independently -NR'C(O)O-(C₁-C₆ alkyl), -NR'C(O)O-phenyl, -C(O)OR', -C(O)NR'R", -OC(O)NR'R", -NR'C(O)NR'R"; -(C₁-C₆ alkyl)-C(O)OR', -(C₁-C₆ alkyl)-C(O)NR'R", -NR'C(O)R", NO₂, CN, phenyl, -S(O)_{z} aryl (where the aryl group is preferably naphthyl or phenyl, still more preferably, phenyl) or -S(O)_{z} (C₁-C₆ alkyl) where the aryl portions of the above are optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R". In an alternative embodiment, the C-ring is pyrimidinyl substituted with 1 or 2 groups that are independently C₁-C₄ alkyl, C₂-C₆ alkenyl, C₂-C₆ alkynyl, where the alkyl, alkenyl and alkynyl portions of the above are unsubstituted or substituted with 1 or 2 groups that are independently halogen, hydroxyl, -NR'R" or C₁-C₄ alkoxy. In another alternative embodiment, the C-ring is pyrimidinyl substituted with 1 or 2 groups that are independently -C(O)OH, -C(O)O-(C₁-C₄ alkyl), NO₂, CN, or phenyl, where the phenyl group is optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R". In still another alternative embodiment, the C-ring is pyrimidinyl substituted with 1 or 2 groups that are independently C₁-C₄ alkyl, C₁-C₄ alkoxy, halogen, - C(O)NR'R", -NR'C(O)R", -OC(O)NR'R", -NR'C(O)NR'R"; -NR'C(O)O-(C₁-C₆ alkyl), or -NR'C(O)O-phenyl, where the phenyl group is optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R".

In still another aspect, the invention provides compounds of formula 14-4b, i.e., compounds of formula 12 or any one of formulas 13, 13-1, 13-2, 13-3, 13-4, 13-5, 13-6, 13-6a, 13-6b, 13-6c, 13-6d, 13-7, 13-8, 13-9, 13-10, 13-11, 13-11a, 13-11b, 13-11c, 13-12, 13-13, 13-14, or 13-15, wherein the C-ring is 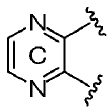 which is optionally substituted with 1 or 2 groups that are independently halogen, C₁-C₄ alkyl, C₁-C₄ alkoxy, aryloxy, aryl-(C₁-C₆ alkoxy), C₁-C₄ haloalkyl (in another aspect, CF₃), C₁-C₄ haloalkoxy (in another aspect, OCF₃), hydroxyl, hydroxy-(C₁-C₄ alkyl), -NR'R", -(C₁-C₄ alkyl)-NR'R", -NR'C(O)O-(C₁-C₆ alkyl), -NR'C(O)O-phenyl, -COR', -C(O)OR', -C(O)NR'R", -OC(O)NR'R", -NR'C(O)NR'R"; -(C₁-C₆ alkyl)-C(O)OR', -(C₁-C₆ alkyl)-C(O)NR'R", -NR'C(O)R", NO₂, CN, benzyl, phenyl, oxazolyl, pyrazolyl, thiazolyl, pyridyl, furanyl, thienyl, C₂-C₆ alkenyl, C₂-C₆ alkynyl, -NR'SO₂R", -SO₂-NR'R", -S(O)_{z} aryl (where the aryl group is preferably naphthyl or phenyl, still more preferably, phenyl), or -S(O)_{z} (C₁-C₆ alkyl), where the alkyl, alkenyl and alkynyl portions of the above are unsubstituted or substituted with 1, 2 or 3 groups that are independently halogen, hydroxyl, -NR'R" or C₁-C₆ alkoxy, and where the aryl or heteroaryl portions of the above are optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R". In one embodiment, the invention provides compounds of formula 14-4b wherein the C-ring is pyrazine substituted with 1 or 2 groups that are independently -NR'C(O)O-(C₁-C₆ alkyl), -NR'C(O)O-phenyl, -C(O)OR', -C(O)NR'R", -OC(O)NR'R", -NR'C(O)NR'R"; -(C₁-C₆ alkyl)-C(O)OR', -(C₁-C₆ alkyl)-C(O)NR'R", -NR'C(O)R", NO₂, CN, phenyl, S(O)_{Z} aryl (where the aryl group is preferably naphthyl or phenyl, still more preferably, phenyl) or -S(O)_{z} (C₁-C₆ alkyl) where the aryl portions of the above are optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R". In an alternative embodiment, the C-ring is pyrazine substituted with 1 or 2 groups that are independently C₁-C₄ alkyl, C₂-C₆ alkenyl, C₂-C₆ alkynyl, where the alkyl, alkenyl and alkynyl portions of the above are unsubstituted or substituted with 1 or 2 groups that are independently halogen, hydroxyl, -NR'R" or C₁-C₄ alkoxy. In another alternative embodiment, the C-ring is pyrazine substituted with 1 or 2 groups that are independently - C(O)OH, -C(O)O-(C₁-C₄ alkyl), NO₂, CN, or phenyl, where the phenyl group is optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R". In still another alternative embodiment, the C-ring is pyrazine substituted with 1 or 2 groups that are independently C₁-C₄ alkyl, C₁-C₄ alkoxy, halogen, - C(O)NR'R", -NR'C(O)R", -OC(O)NR'R", -NR'C(O)NR'R"; -NR'C(O)O-(C₁-C₆ alkyl) or -NR'C(O)O-phenyl, where the phenyl group is optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R".

In still another aspect, the invention provides compounds of formula 14-4c, i.e., compounds of formula 12 or any one of formulas 13, 13-1, 13-2, 13-3, 13-4, 13-5, 13-6, 13-6a, 13-6b, 13-6c, 13-6d, 13-7, 13-8, 13-9, 13-10, 13-11, 13-11a, 13-11b, 13-11c, 13-12, 13-13, 13-14, or 13-15, wherein the C-ring is: 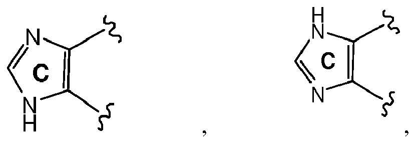 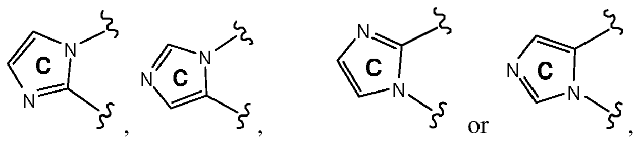 each of which is optionally substituted with 1 or 2 groups that are independently halogen, C₁-C₄ alkyl, C₁-C₄ alkoxy, aryloxy, aryl-(C₁-C₄ alkoxy), C₁-C₄ haloalkyl (in another aspect, CF₃), C₁-C₄ haloalkoxy (in another aspect, OCF₃), hydroxyl, hydroxy-(C₁-C₄ alkyl), - NR'R", -(C₁-C₆ alkyl)-NR'R", -NR'C(O)O-(C₁-C₆ alkyl), -NR'C(O)O-phenyl, -COR', -C(O)OR', -C(O)NR'R", -OC(O)NR'R", -NR'C(O)NR'R"; -(C₁-C₆ alkyl)-C(O)OR', -(C₁-C₆ alkyl)-C(O)NR'R", -NR'C(O)R", NO₂, CN, benzyl, phenyl, oxazolyl, pyrazolyl, thiazolyl, pyridyl, furanyl, thienyl, C₂-C₆ alkenyl, C₂-C₆ alkynyl, -NR'SO₂R" -SO₂-NR'R", -S(O)_{z} aryl (where the aryl group is preferably naphthyl or phenyl, still more preferably, phenyl), or -S(O)_{z} (C₁-C₆ alkyl), where the alkyl, alkenyl and alkynyl portions of the above are unsubstituted or substituted with 1, 2, or 3 groups that are independently halogen, hydroxyl, -NR'R" or C₁-C₆ alkoxy, and where the aryl or heteroaryl portions of the above are optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R".

In still another aspect, the invention provides compounds of formula 14-4c1, i.e., compounds of formula 14-4c wherein the C-ring is:

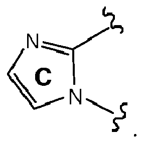

In still another aspect, the invention provides compounds of formula 14-4d, i.e., compounds of formula 12 or any one of formulas 13, 13-1, 13-2, 13-3, 13-4, 13-5, 13-6, 13-6a, 13-6b, 13-6c, 13-6d, 13-7, 13-8, 13-9, 13-10, 13-11, 13-11a, 13-11b, 13-11c, 13-12, 13-13, 13-14, or 13-15, wherein the C-ring is: 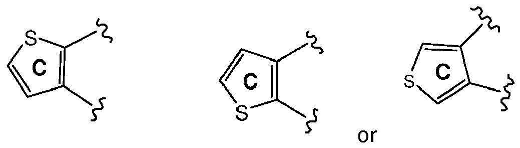 each of which is optionally substituted with 1 or 2 groups that are independently halogen, C₁-C₄ alkyl, C₁-C₄ alkoxy, aryloxy, aryl-(C₁-C₄ alkoxy), C₁-C₄ haloalkyl (in another aspect, CF₃), C₁-C₄ haloalkoxy (in another aspect, OCF₃), hydroxyl, hydroxy-(C₁-C₄ alkyl), -NR'R", -(C₁-C₆ alkyl)-NR'R", -NR'C(O)O-(C₁-C₆ alkyl), -NR'C(O)O-phenyl, -COR', -C(O)OR', -C(O)NR'R", -OC(O)NR'R", -NR'C(O)NR'R"; -(C₁-C₆ alkyl)-C(O)OR', -(C₁-C₆ alkyl)-C(O)NR'R", -NR'C(O)R", NO₂, CN, benzyl, phenyl, oxazolyl, pyrazolyl, thiazolyl, pyridyl, furanyl, thienyl, C₂-C₆ alkenyl, C₂-C₆ alkynyl, -NR'SO₂R", -SO₂-NR'R", -S(O)_{z} aryl (where the aryl group is preferably naphthyl or phenyl, still more preferably, phenyl), or -S(O)_{z} (C₁-C₆ alkyl), where the alkyl, alkenyl and alkynyl portions of the above are unsubstituted or substituted with 1, 2, or 3 groups that are independently halogen, hydroxyl, - NR'R" or C₁-C₆ alkoxy, and where the aryl or heteroaryl portions of the above are optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R".

In still another aspect, the invention provides compounds of formula 14-4d1, i.e., compounds of formula 14-4d wherein the C-ring is:

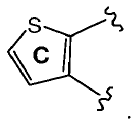

In still another aspect, the invention provides compounds of formula 14-4d2, i.e., compounds of formula 14-4d wherein the C-ring is:

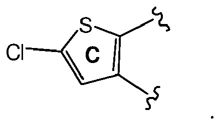

In yet still another aspect, the invention provides compounds of formula 14-5, i.e., compounds according to formula 14-4, 14-4a, 14-4b, 14-4c, or 14-4d, wherein the C-ring is unsubstituted.

In another aspect, the invention provides compounds of formula 14-5a, i.e., compounds of formula 14-4, 14-4a, 14-4b, 14-4c, or 14-4d, wherein the C-ring is substituted with 1 or 2 groups that are independently Cl, F, methyl, ethyl, isopropyl, methoxy, ethoxy, CF₃, OCF₃, OH, CH₂OH, or -C(O)CH₃.

In yet another aspect, the invention provides compounds of formula 14-5b, i.e., compounds of formula 14-4, 14-4a, 14-4b, 14-4c, or 14-4d, wherein the C-ring is substituted with 1 or 2 groups that are independently Cl, F, or methyl.

In still yet another aspect, the invention provides compounds of formula 14-5c, i.e., compounds according to any one of formulas 14-4, 14-5, 14-5a, or 14-4b, where the C-ring has the following structure:

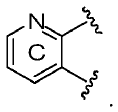

In still yet another aspect, the invention provides compounds of formula 14-5d, i.e., compounds according to any one of formulas 14-4, 14-5, 14-5a, or 14-5b, where the C-ring has the following structure:

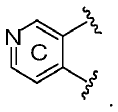

In still yet another aspect, the invention provides compounds of formula 14-5d1, i.e., compounds according to formula 14-4, where the C-ring has the following structure:

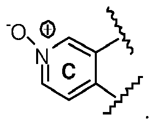

In still yet another aspect, the invention provides compounds of formula 14-5e, i.e., compounds according to any one of formulas 14-4, 14-5, 14-5a, or 14-5b, where the C-ring has the following structure:

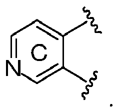

In still yet another aspect, the invention provides compounds of formula 14-5e1, i.e., compounds according to formula 14-4, 14-5, 14-5a, or 14-5b, where the C-ring has the following structure:

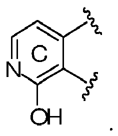

In still yet another aspect, the invention provides compounds of formula 14-5f, i.e., compounds according to formula 14-4, where the C-ring has the following structure:

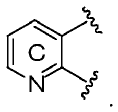

In a further aspect, the invention provides compounds of formula 14-5f1, i.e., compounds of formula 12 or any one of formulas 13, 13-1, 13-2, 13-3, 13-4, 13-5, 13-6, 13-6a, 13-6b, 13-6c, 13-6d, 13-7, 13-8, 13-9, 13-10, 13-11, 13-11a, 13-11b, 13-11c, 13-12, 13-13, 13-14, or 13-15, wherein the C-ring is thiazolyl, which is optionally substituted with 1 or 2 groups that are independently halogen, C₁-C₄ alkyl, C₁-C₄ alkoxy, aryloxy, aryl-(C₁-C₄ alkoxy), C₁-C₄ haloalkyl (in another aspect, CF₃), C₁-C₄ haloalkoxy (in another aspect, OCF₃), hydroxyl, hydroxy-(C₁-C₄ alkyl), -NR'R", -(C₁-C₆ alkyl)-NR'R", -NR'C(O)O-(C₁-C₆ alkyl), -NR'C(O)O-phenyl, -COR', -C(O)OR', -C(O)NR'R", -OC(O)NR'R", -NR'C(O)NR'R"; -(C₁-C₆ alkyl)-C(O)OR', -(C₁-C₆ alkyl)-C(O)NR'R", -NR'C(O)R", NO₂, CN, benzyl, phenyl, oxazolyl, pyrazolyl, thiazolyl, pyridyl, furanyl, thienyl, C₂-C₆ alkenyl, C₂-C₆ alkynyl, -NR'SO₂R" -SO₂-NR'R", -S(O)_{z} aryl (where the aryl group is preferably naphthyl or phenyl, still more preferably, phenyl), or -S(O)_{z} (C₁-C₆ alkyl), where the alkyl, alkenyl and alkynyl portions of the above are unsubstituted or substituted with 1, 2, or 3 groups that are independently halogen, hydroxyl, -NR'R" or C₁-C₆ alkoxy, and where the aryl or heteroaryl portions of the above are optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R".

In another aspect, the invention provides compounds of formula 14-5f2, i.e., compounds of formula 14-5f1, wherein the thiazolyl ring is substituted with one group that is halogen, C₁-C₄ alkyl, C₁-C₄ alkoxy, or benzyl.

In still another aspect, the invention provides compounds of formula 14-5f3, i.e., compounds of formula 14-5f1, wherein the thiazolyl ring is substituted with C₁-C₄ alkyl.

In yet another aspect, the invention provides compounds of formula 14-5f4, i.e., compounds of formula 14-5f1, wherein the thiazolyl ring is substituted with C₁-C₄ haloalkyl (in another aspect, CF₃), C₁-C₄ haloalkoxy (in another aspect, OCF₃), hydroxyl, hydroxy-(C₁-C₄ alkyl), -NR'R", or -(C₁-C₆ alkyl)-NR'R".

In yet still another aspect, the invention provides compounds of formula 14-5f5, i.e., compounds of formula 14-5f1, wherein the thiazolyl ring is substituted with C₂-C₄ alkyl, -(C₁-C₄ alkyl)-C(O)OR', or -(C₁-C₄ alkyl)-C(O)NR'R".

In another aspect, the invention provides compounds of formula 14-5f6, i.e., compounds of formula 14-5f1, wherein the thiazolyl ring is:

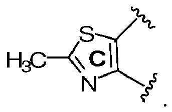

In yet still another aspect, the invention provides compounds of formula 14-5g, i.e., compounds according to any one of formulas 14-4, 14-4a, 14-4b, 14-4c, 14-4c1, 14-4d, 14-4d1, 14-4d2, 14-5, 14-5a, 14-5b, 14-5c, 14-5d, 14-5d1, 14-5e, 14-5e1, 14-5f, 14-5f1, 14-5f2, 14-5f3, 14-5f4, 14-5f5, or 14-5f6, wherein R₂ is -X(CO)Y or -(C(R₃)₂)₁₋₄X(CO)Y.

In still another aspect, the invention provides compounds of formula 14-5g1, i.e., compounds according to any one of formulas 14-4, 14-4a, 14-4b, 14-4c, 14-4c1, 14-4d, 14-4d1, 14-4d2, 14-5, 14-5a, 14-5b, 14-5c, 14-5d, 14-5d1, 14-5e, 14-5e1, 14-5f, 14-5f1, 14-5f2, 14-5f3, 14-5f4, 14-5f5, or 14-5f6, wherein R₂ is hydrogen, C₁-C₃ alkyl or C₃-C₆ cycloalkyl.

In still another aspect, the invention provides compounds of formula 14-5g2, i.e., compounds according to any one of formulas 14-4, 14-4a, 14-4b, 14-4c, 14-4c1, 14-4d, 14-4d1, 14-4d2, 14-5, 14-5a, 14-5b, 14-5c, 14-5d, 14-5d1, 14-5e, 14-5e1, 14-5f, 14-5f1, 14-5f2, 14-5f3, 14-5f4, 14-5f5, or 14-5f6, wherein R₂ is NO₂ or CN.

In still another aspect, the invention provides compounds of formula 14-5g3, i.e., compounds according to any one of formulas 14-4, 14-4a, 14-4b, 14-4c, 14-4c1, 14-4d, 14-4d1, 14-4d2, 14-5, 14-5a, 14-5b, 14-5c, 14-5d, 14-5d1, 14-5e, 14-5e1, 14-5f, 14-5f1, 14-5f2, 14-5f3, 14-5f4, 14-5f5, or 14-5f6, wherein R₂ is C₂-C₆ alkenyl or C₂-C₆ alkynyl.

In still another aspect, the invention provides compounds of formula 14-5g4, i.e., compounds according to any one of formulas 14-4, 14-4a, 14-4b, 14-4c, 14-4c1, 14-4d, 14-4d1, 14-4d2, 14-5, 14-5a, 14-5b, 14-5c, 14-5d, 14-5d1, 14-5e, 14-5e1, 14-5f, 14-5f1, 14-5f2, 14-5f3, 14-5f4, 14-5f5, or 14-5f6, wherein R₂ is C₁-C₄ haloalkyl.

In still another aspect, the invention provides compounds of formula 14-5g5, i.e., compounds according to any one of formulas 14-4, 14-4a, 14-4b, 14-4c, 14-4c1, 14-4d, 14-4d1, 14-4d2, 14-5, 14-5a, 14-5b, 14-5c, 14-5d, 14-5d1, 14-5e, 14-5e1, 14-5f, 14-5f1, 14-5f2, 14-5f3, 14-5f4, 14-5f5, 14-5f6, 14-5g, 14-5g1, 14-5g2, 14-5g3, or 14-5g4 wherein R₁ is hydrogen.

In still yet another aspect, the invention provides compounds of formula 14-5g6, i.e., compounds according to any one of formulas 14-4, 14-4a, 14-4b, 14-4c, 14-4c1, 14-4d, 14-4d1, 14-4d2, 14-5, 14-5a, 14-5b, 14-5c, 14-5d, 14-5d1, 14-5e, 14-5e1, 14-5f, 14-5f1, 14-5f2, 14-5f3, 14-5f4, 14-5f5, or 14-5f6, wherein R₁ and R₂ combined are oxo.

In still yet another aspect, the invention provides compounds of formula 14-5g7, i.e., compounds according to any one of formulas 14-4, 14-4a, 14-4b, 14-4c, 14-4c1, 14-4d, 14-4d1, 14-4d2, 14-5, 14-5a, 14-5b, 14-5c, 14-5d, 14-5d1, 14-5e, 14-5e1, 14-5f, 14-5f1, 14-5f2, 14-5f3, 14-5f4, 14-5f5, or 14-5f6, wherein R₁ and R₂ combined are =N-OR, where R is hydrogen, C₁-C₆ alkyl, aryl (such as phenyl) or arylalkyl (such as benzyl or phenethyl).

In yet another aspect, the invention provides compounds of formula 14-5g8, i.e., compounds according to any one of formulas 14-4, 14-4a, 14-4b, 14-4c, 14-4c1, 14-4d, 14-4d1, 14-4d2, 14-5, 14-5a, 14-5b, 14-5c, 14-5d, 14-5d1, 14-5e, 14-5e1, 14-5f, 14-5f1, 14-5f2, 14-5f3, 14-5f4, 14-5f5, or 14-5f6, wherein R₁ and R₂ together with the carbon to which they are attached form a C₃-C₆ cycloalkyl group.

In yet another aspect, the invention provides compounds of formula 14-5g9, i.e., compounds according to any one of formulas 14-4, 14-4a, 14-4b, 14-4c, 14-4c1, 14-4d, 14-4d1, 14-4d2, 14-5, 14-5a, 14-5b, 14-5c, 14-5d, 14-5d1, 14-5e, 14-5e1, 14-5f, 14-5f1, 14-5f2, 14-5f3, 14-5f4, 14-5f5, or 14-5f6, wherein R₁ and R₂ together with the carbon to which they are attached form a cyclopropyl group.

In yet another aspect, the invention provides compounds of formula 14-5g10, i.e., compounds according to any one of formulas 14-4, 14-4a, 14-4b, 14-4c, 14-4c1, 14-4d, 14-4d1, 14-4d2, 14-5, 14-5a, 14-5b, 14-5c, 14-5d, 14-5d1, 14-5e, 14-5e1, 14-5f, 14-5f1, 14-5f2, 14-5f3, 14-5f4, 14-5f5, or 14-5f6, wherein R₁ is hydrogen and R₂ is cyclopropyl. In one embodiment, the compound is racemic. In another embodiment, the compound is enantiomerically enriched.

In yet another aspect, the invention provides compounds of formula 14-5g11, i.e., compounds according to any one of formulas 14-4, 14-4a, 14-4b, 14-4c, 14-4c1, 14-4d, 14-4d1, 14-4d2, 14-5, 14-5a, 14-5b, 14-5c, 14-5d, 14-5d1, 14-5e, 14-5e1, 14-5f, 14-5f1, 14-5f2, 14-5f3, 14-5f4, 14-5f5, or 14-5f6, wherein R₂ is independently hydrogen, methyl, ethyl, propyl, isopropyl, or cyclopropyl; and R₁ is H or methyl.

In yet still another aspect, the invention provides compounds of formula 14-5h, i.e., compounds of formula 14-5g, wherein
X is O; and Y is -NR₆₀R₇₀ or -N(R₃₀)(CH₂)₂₋₆NR₆₀R₇₀; where R₆₀ and R₇₀ are independently selected from: hydrogen, methyl, ethyl, (C₃ -C₈)cycloalkyl, aryl(C₁-C₆ )alkyl (such as benzyl or phenethyl), 4-pyridylmethyl, 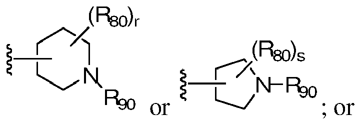
R₆₀ and R₇₀ taken together with the nitrogen atom to which they are bound form a heterocycloalkyl group selected from 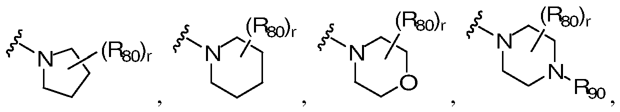 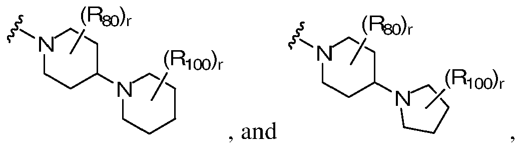 where
   each R₈₀ is independently unsubstituted C₁-C₄ alkyl or C₁-C₄ alkyl substituted with hydroxyl or halogen;
   each R₉₀ is independently hydrogen, unsubstituted C₁-C₄ alkyl, C₁-C₄ alkyl substituted with hydroxyl or halogen, unsubstituted C₃-C₈ cycloalkyl, C₃-C₈ cycloalkyl substituted with one or more (e.g., 1-4) R₅₀ groups, phenyl-(C₁-C₄ alkyl), pyridyl-(C₁-C₄ alkyl), thienyl-(C₁-C₄ alkyl), -C(O)O-(C₁-C₄ alkyl), -C(O)O-phenyl, -SO₂-(C₁-C₆ alkyl), -SO₂-phenyl, unsubstituted phenyl, phenyl substituted with one or more (e.g., 1-4) R₅₀ groups, pyridyl, thienyl, pyridyl substituted with one or more (e.g., 1-4) R₅₀ groups, thienyl substituted with one or more (e.g., 1-4) R₅₀ groups, where
   each R₅₀ is independently selected from: halogen, C₁-C₆ alkyl, - C₁-C₆ haloalkyl, -OH, -O C₁-C₄ alkyl, OCF₃, -CN, - NR₆₀R₇₀, -C(O)O-(C₁-C₄ alkyl), -CONR₆₀R₇₀, - (C₁-C₆ alkyl)-NR₆₀R₇₀, -NR₆₀CO-(C₁-C₄ alkyl), -NR₆₀CO-phenyl, -NR₆₀CO-pyridyl, -NR₆₀CO-thienyl, and -NR₆₀CONR₆₀R₇₀,
   each R₁₀₀ is independently hydrogen or C₁-C₄ alkyl;
   each r is 0 to 4; and
   each s is 0 to 3.

In yet still another aspect, the invention provides compounds of formula 14-5h1, i.e., compounds of formula 14-5g, wherein Y is -O-(C₁-C₆ alkyl). In one embodiment, Y is -O-(C₁-C₄ alkyl).

In yet still another aspect, the invention provides compounds of formula 14-5h2, i.e., compounds of formula 14-5g, wherein Y is -O-phenyl.

In yet still another aspect, the invention provides compounds of formula 14-5i, i.e., compounds of formula 14-5h, wherein said group 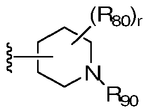 is a group of the formula: 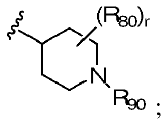 and said group 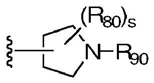 is a group of the formula:

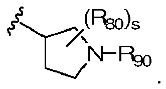

In a further aspect, the invention provides compounds of formula 14-5j, i.e., compounds according to any one of formulas, 14-5h, 14-5h1, 14-5h2, or 14-5i, wherein R₁ is H or C₁-C₄ alkyl. In one embodiment, R₁ is H or methyl. In another embodiment, R₁ is H.

In yet still another aspect, the invention provides compounds of formula 14-6, i.e., compounds according to any one of formulas 13, 13-1, 13-2, 13-3, 13-4, 13-5, 13-6, 13-6a, 13-6b, 13-6c, 13-6d, 13-7, 13-8, 13-9, 13-10, 13-11, 13-11a, 13-11b, 13-11c, 13-12, 13-13, 13-14, or 13-15, 14, 14-1, 14-1a, 14-2, 14-2a, 14-2b, 14-2d, 14-2c, 14-2d, 14-2e, 14-2f, 14-2g, 14-2h, 14-2i, 14-3, 14-3a, 14-3a1, 14-3a2, 14-3a3, 14-3a4, 14-3a5, 14-3a6, 14-3a7, 14-3a8, 14-3a9, 14-3a10, 14-3b, 14-3c, 14-3c1, 14-4, 14-4a, 14-4b, 14-4c, 14-4c1, 14-4d, 14-4d1, 14-4d2, 14-5, 14-5a, 14-5b, 14-5c, 14-5d, 14-5d1, 14-5e, 14-5e1, 14-5f, 14-5f1, 14-5f2, 14-5f3, 14-5f4, 14-5f5, 14-5f6, 14-5g, 14-5g1, 14-5g2, 14-5g3, 14-5g4, 14-5g5, 14-5g6, 14-5g7, 14-5g8, 14-5g9, 14-5g10, 14-5h, 14-5h1, 14-5h2, 14-5i, or 14-5j, wherein the heteroaryl group (ring A of Formula I) is pyridyl optionally substituted with 1 or 2 groups that are independently halogen, C₁-C₄ alkyl, C₁-C₄ alkoxy, C₁-C₄ haloalkyl, C₁-C₄ haloalkoxy, hydroxy, amino, or mono or di(C₁-C₄ alkyl)amino.

In yet still another aspect, the invention provides compounds of formula 14-7, i.e., compounds 14-6, wherein the pyridyl is substituted with one group that is halogen, C₁-C₄ alkyl, C₁-C₄ alkoxy, C₁-C₄ haloalkyl, C₁-C₄ haloalkoxy, hydroxy, amino, or mono or di(C₁-C₄ alkyl)amino.

In yet still another aspect, the invention provides compounds of formula 14-8, i.e., compounds of formula 14-7, wherein the pyridyl is substituted at the 4-position.

In yet still another aspect, the invention provides compounds of formula 14-8a, i.e., compounds of formula 14-7, wherein the pyridyl is substituted at the 2-position, i.e., immediately next to the pyridyl ring nitrogen. In one embodiment, the pyridyl ring is substituted at the 2-position, i.e., immediately next to the pyridyl ring nitrogen and the pyridyl ring is attached to the -SO₂- group at the 5-position.

In yet still another aspect, the invention provides compounds of formula 14-9, i.e., compounds of formula 14-8, wherein the pyridyl is substituted with one group that is halogen (preferably chloro) or C₁-C₄ haloalkyl (preferably CF₃).

In yet still another aspect, the invention provides compounds of formula 14-10, i.e., compounds of formula 14-9, wherein the heteroaryl group has the following structures:

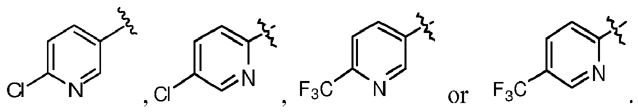

In yet still another aspect, the invention provides compounds of formula 14-10a, i.e., compounds of formula 14-9, wherein the heteroaryl group has the following structure:

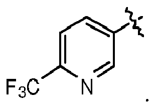

In yet still another aspect, the invention provides compounds of formula 14-lOb, i.e., compounds according to any one of formulas 13, 13-1, 13-2, 13-3, 13-4, 13-5, 13-6, 13-6a, 13-6b, 13-6c, 13-6d, 13-7, 13-8, 13-9, 13-10, 13-11, 13-11a, 13-11b, 13-llc, 13-12, 13-13, 13-14, or 13-15, 14, 14-1, 14-1a, 14-2, 14-2a, 14-2b, 14-2d, 14-2c, 14-2d, 14-2e, 14-2f, 14-2g, 14-2h, 14-2i, 14-3, 14-3a, 14-3a1, 14-3a2, 14-3a3, 14-3a4, 14-3a5, 14-3a6, 14-3a7, 14-3a8, 14-3a9, 14-3a10, 14-3b, 14-3c, 14-3c1, 14-4, 14-4a, 14-4b, 14-4c, 14-4c1, 14-4d, 14-4d1, 14-4d2, 14-5, 14-5a, 14-5b, 14-5c, 14-5d, 14-5d1, 14-5e, 14-5e1, 14-5f, 14-5f1, 14-5f2, 14-5f3, 14-5f4, 14-5f5, 14-5f6, 14-5g, 14-5g1, 14-5g2, 14-5g3, 14-5g4, 14-5g5, 14-5g6, 14-5g7, 14-5g8, 14-5g9, 14-5g10, 14-5h, 14-5h1, 14-5h2, 14-5i, or 14-5j, wherein the heteroaryl group is pyridyl, or thienyl, each of which is optionally substituted with 1 or 2 groups that are independently halo, methyl, methoxy, CF₃, or OCF₃.

In yet still another aspect, the invention provides compounds of formula 14-11, i.e., compounds according to any one of formulas 13, 13-1, 13-2, 13-3, 13-4, 13-5, 13-6, 13-6a, 13-6b, 13-6c, 13-6d, 13-7, 13-8, 13-9, 13-10, 13-11, 13-11a, 13-11b, 13-11c, 13-12, 13-13, 13-14, or 13-15, 14, 14-1, 14-1a, 14-2, 14-2a, 14-2b, 14-2d, 14-2c, 14-2d, 14-2e, 14-2f, 14-2g, 14-2h, 14-2i, 14-3, 14-3a, 14-3a1, 14-3a2, 14-3a3, 14-3a4, 14-3a5, 14-3a6, 14-3a7, 14-3a8, 14-3a9, 14-3a10, 14-3b, 14-3c, 14-3c1, 14-4, 14-4a, 14-4b, 14-4c, 14-4c1, 14-4d, 14-4d1, 14-4d2, 14-5, 14-5a, 14-5b, 14-5c, 14-5d, 14-5d1, 14-5e, 14-5e1, 14-5f, 14-5f1, 14-5f2, 14-5f3, 14-5f4, 14-5f5, 14-5f6, 14-5g, 14-5g1, 14-5g2, 14-5g3, 14-5g4, 14-5g5, 14-5g6, 14-5g7, 14-5g8, 14-5g9, 14-5g10, 14-5h, 14-5h1, 14-5h2, 14-5i, or 14-5j, wherein the heteroaryl group is an unsubstituted pyridyl.

In yet still another aspect, the invention provides compounds of formula 14-12, i.e., compounds according to any one of formulas 13, 13-1, 13-2, 13-3, 13-4, 13-5, 13-6, 13-6a, 13-6b, 13-6c, 13-6d, 13-7, 13-8, 13-9, 13-10, 13-11, 13-11a, 13-11b, 13-11c, 13-12, 13-13, 13-14, or 13-15, 14, 14-1, 14-1a, 14-2, 14-2a, 14-2b, 14-2d, 14-2c, 14-2d, 14-2e, 14-2f, 14-2g, 14-2h, 14-2i, 14-3, 14-3a, 14-3a1, 14-3a2, 14-3a3, 14-3a4, 14-3a5, 14-3a6, 14-3a7, 14-3a8, 14-3a9, 14-3a10, 14-3b, 14-3c, 14-3c1, 14-4, 14-4a, 14-4b, 14-4c, 14-4c1, 14-4d, 14-4d1, 14-4d2, 14-5, 14-5a, 14-5b, 14-5c, 14-5d, 14-5d1, 14-5e, 14-5e1, 14-5f, 14-5f1, 14-5f2, 14-5f3, 14-5f4, 14-5f5, 14-5f6, 14-5g, 14-5g1, 14-5g2, 14-5g3, 14-5g4, 14-5g5, 14-5g6, 14-5g7, 14-5g8, 14-5g9, 14-5g10, 14-5h, 14-5h1, 14-5h2, 14-5i, or 14-5j, wherein the heteroaryl group (ring A of Formula I) is thienyl optionally substituted with 1 or 2 groups that are independently halogen, C₁-C₄ alkyl, C₁-C₄ alkoxy, C₁-C₄ haloalkyl, C₁-C₄ haloalkoxy, hydroxy, amino, or mono or di(C₁-C₄ alkyl)amino.

In yet still another aspect, the invention provides compounds of formula 14-13, i.e., compounds of formula 14-12 where the thienyl group is substituted with one group that is halogen, C₁-C₄ alkyl, C₁-C₄ alkoxy, C₁-C₄ haloalkyl, C₁-C₄ haloalkoxy, hydroxy, amino, or mono or di(C₁-C₄ alkyl)amino.

In yet still another aspect, the invention provides compounds of formula 14-13a, i.e., compounds of formula 14-12 where the thienyl group is substituted with one group that is optionally substituted heteroaryl (for example pyridyl).

In yet still another aspect, the invention provides compounds of formula 14-14, i.e., compounds of formula 14-13, wherein the thienyl group is substituted with one halogen (preferably Cl).

In yet still another aspect, the invention provides compounds of formula 14-15, i.e., compounds of formula 14-14, wherein the thienyl group has the formula:

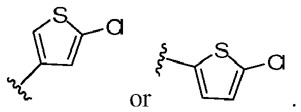

In yet still another aspect, the invention provides compounds of formula 14-16, i.e., compounds of formula 14-12, wherein the thienyl group is unsubstituted.

In yet still another aspect, the invention provides compounds of formula 14-17, i.e., compounds of formula 14-16, wherein the thienyl group has the formula:

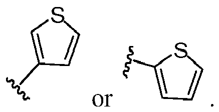

In yet still another aspect, the invention provides compounds of formula 14-18, i.e., compounds according to any one of formulas 13, 13-1, 13-2, 13-3, 13-4, 13-5, 13-6, 13-6a, 13-6b, 13-6c, 13-6d, 13-7, 13-8, 13-9, 13-10, 13-11, 13-11a, 13-11b, 13-11c, 13-12, 13-13, 13-14, or 13-15, 14, 14-1, 14-1a, 14-2, 14-2a, 14-2b, 14-2d, 14-2c, 14-2d, 14-2e, 14-2f, 14-2g, 14-2h, 14-2i, 14-3, 14-3a, 14-3a1, 14-3a2, 14-3a3, 14-3a4, 14-3a5, 14-3a6, 14-3a7, 14-3a8, 14-3a9, 14-3a10, 14-3b, 14-3c, 14-3c1, 14-4, 14-4a, 14-4b, 14-4c, 14-4c1, 14-4d, 14-4d1, 14-4d2, 14-5, 14-5a, 14-5b, 14-5c, 14-5d, 14-5d1, 14-5e, 14-5e1, 14-5f, 14-5f1, 14-5f2, 14-5f3, 14-5f4, 14-5f5, 14-5f6, 14-5g, 14-5g1, 14-5g2, 14-5g3, 14-5g4, 14-5g5, 14-5g6, 14-5g7, 14-5g8, 14-5g9, 14-5g10, 14-5h, 14-5h1, 14-5h2, 14-5i, or 14-5j, wherein the heteroaryl group (ring A of Formula I) comprises thiazolyl, imidazolyl, pyrazolyl, isoxazolyl, or oxazolyl optionally substituted with 1 or 2 groups that are independently halogen, C₁-C₄ alkyl, C₁-C₄ alkoxy, C₁-C₄ haloalkyl, C₁-C₄ haloalkoxy, hydroxy, amino, or mono or di(C₁-C₄ alkyl)amino.

In yet still another aspect, the invention provides compounds of formula 14-19, i.e., compounds of formula 14-18, wherein the thiazolyl group is unsubstituted and has the formula:

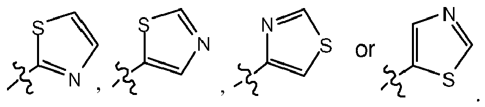

In yet still another aspect, the invention provides compounds of formula 14-19a, i.e., compounds of formula 14-18 where the heteroaryl group (ring A of Formula I) is a thiazolyl group that is substituted with one or two groups that are halogen, C₁-C₄ alkyl, C₁-C₄ alkoxy, C₁-C₄ haloalkyl, C₁-C₄ haloalkoxy, hydroxy, amino, or mono or di(C₁-C₄ alkyl)amino.

In yet still another aspect, the invention provides compounds of formula 14-19b, i.e., compounds of formula 14-18, wherein the heteroaryl group (ring A of Formula I) is a thiazolyl group that has the formula:

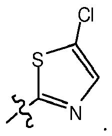

In yet still another aspect, the invention provides compounds of formula 14-20, i.e., compounds of formula 14-18, wherein the heteroaryl group (ring A of Formula I) is a pyrazolyl group that is unsubstituted and has the formula:

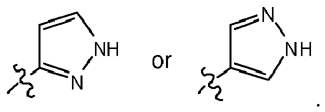

In yet still another aspect, the invention provides compounds of formula 14-20a, i.e., compounds of formula 14-18 where the heteroaryl group (ring A of Formula I) is a pyrazolyl group that is substituted with one or two groups that arehalogen, C₁-C₄ alkyl, C₁-C₄ alkoxy, C₁-C₄ haloalkyl, C₁-C₄ haloalkyoxy, hydroxy, amino, or mono or di(C₁-C₄ alkyl)amino.

In yet still another aspect, the invention provides compounds of formula 14-20b, i.e., compounds of formula 14-18, wherein the heteroaryl group (ring A of Formula I) is a pyrazolyl group that has the formula:

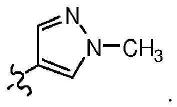

In yet still another aspect, the invention provides compounds of formula 14-21, i.e., compounds of formula 14-18, wherein the heteroaryl group (ring A of Formula I) is an isoxazolyl group that is unsubstituted and has the formula:

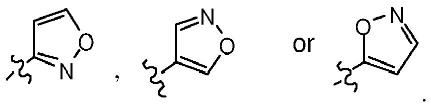

In yet still another aspect, the invention provides compounds of formula 14-21a, i.e., compounds of formula 14-18 where the heteroaryl group (ring A of Formula I) is an isoxazolyl group that is substituted with one or two groups that are halogen, C₁-C₄ alkyl, C₁-C₄ alkoxy, C₁-C₄ haloalkyl, C₁-C₄ haloalkoxy, hydroxy, amino, or mono or di(C₁-C₄ alkyl)amino.

In yet still another aspect, the invention provides compounds of formula 14-21b, i.e., compounds of formula 14-18, wherein the heteroaryl group (ring A of Formula I) is an isoxazolyl group that has the formula:

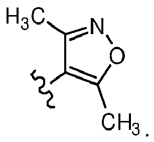

In yet still another aspect, the invention provides compounds of formula 14-22, i.e., compounds of formula 14-18, wherein the heteroaryl group (ring A of Formula I) is an imidazolyl group that is unsubstituted and has the formula:

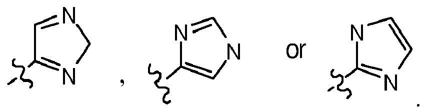

In yet still another aspect, the invention provides compounds of formula 14-22a, i.e., compounds of formula 14-18 where the heteroaryl group (ring A of Formula I) is an imidazolyl group that is substituted with one or two groups that are halogen, C₁-C₄ alkyl, C₁-C₄ alkoxy, C₁-C₄ haloalkyl, C₁-C₄ haloalkoxy, hydroxy, amino, or mono or di(C₁-C₄ alkyl)amino.

In yet still another aspect, the invention provides compounds of formula 14-22b, i.e., compounds of formula 14-18, wherein the heteroaryl group (ring A of Formula I) is an imidazolyl group that has the formula:

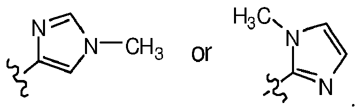

In yet still another aspect, the invention provides compounds of formula 14-23, i.e., compounds according to any one of formulas 12, 13, 13-1, 13-2, 13-3, 13-4, 13-5, 13-6, 13-6a, 13-6b, 13-6c, 13-6d, 13-7, 13-8, 13-9, 13-10, 13-11, 13-11a, 13-11b, 13-11c, 13-12, 13-13, 13-14, or 13-15, 14, 14-1, 14-2, 14-2a, 14-2b, 14-2c, 14-2d, 14-3, 14-4, 14-4a, 14-4b, 14-4c, 14-4c1, 14-4d, 14-4d1, 14-4d2, 14-5, 14-5a, 14-5b, 14-5c, 14-5d, 14-5d1, 14-5e, 14-5e1, 14-5f, 14-5f1, 14-5f2, 14-5f3, 14-5f4, 14-5f5, or 14-5f6, wherein R₁ is H; R₂ is H, or C₃-C₆ cycloalkyl; R₂₀ is H or methyl; R₇₅ is H or methyl; and the C-ring is phenyl substituted with two halogens. In one embodiment, R₂₀ and R₇₅ are both hydrogen.

In another aspect, the invention provides compounds of formula 14-24, i.e., compounds according to formula 13, wherein the A-ring is a heteroaryl group, which has the following structures:

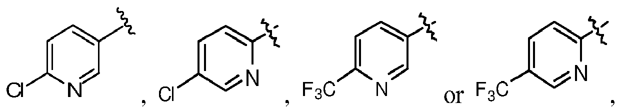

R₁ is hydrogen or methyl; R₂ is H, methyl, or cyclopropyl; the C-ring is phenyl substituted with 1 or 2 groups that are independently Cl, F, methyl, ethyl, isopropyl, methoxy, ethoxy, CF₃, OCF₃, OH, CH₂OH, NH₂, NH(CH₃), or N(CH₃)₂; and the B-ring has the formula:

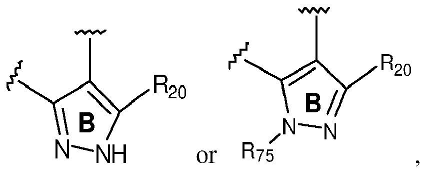

where R₂₀ and R₇₅ are independently H, methyl or ethyl. In one embodiment, the C-ring is bis-substituted with at least one halogen. In another embodiment, the halogen is F. In still another embodiment, the halogen is Cl. In another embodiment, the C-ring is bis-substituted with two halogens, which are the same or different. In still another embodiment, the C-ring is substituted at least at position 7. In another embodiment, the C-ring is substituted at positions 7 and 8. In yet another embodiment, the C-ring is substituted with two halogens, which are the same, and are attached to positions 7 and 8. In another embodiment, the C-ring is monosubstituted with a halogen. In another embodiment, the halogen is F. In still another embodiment, the halogen is Cl. In still another embodiment, the C-ring is substituted at position 7. In yet another embodiment, the C-ring is substituted at position 8.

In another aspect, the invention provides compounds of formula 15, i.e., compounds of formula I, wherein
the A-ring is heterocycloalkyl, which is optionally substituted at a substitutable position with halogen, C₁-C₆ alkyl, C₂-C₆ alkenyl, C₂-C₆ alkynyl, C₁-C₆ alkoxy, C₁-C₆ haloalkyl, C₁-C₆ haloalkoxy, hydroxyl, hydroxy-(C₁-C₄ alkyl), CN, aryloxy, aryl-(C₁-C₄alkoxy) such as benzyloxy, -SO₂-(C₁-C₆ alkyl), -NR'R", C₁-C₆ alkanoyl, -C(O)OR', -(C₁-C₄ alkyl)-C(O)OR', heteroaryl, aryl, aryl C₁-C₄ alkyl, -SO₂-NR'R", -C(O)NR'R", -OC(O)NR'R", -NR'C(O)R", -NR'C(O)NR'R", -NR'C(O)O-(C₁-C₆ alkyl), or -NR'C(O)O-phenyl, where each R' and R" is independently hydrogen or C₁-C₆ alkyl; and
the B-ring is pyrazolyl, imidazolyl, pyrrolyl, triazolyl, pyrrolidinyl, pyrazolidinyl, imidazolidinyl, pyridyl, pyrimidyl, or isoxazolyl, each of which is optionally substituted at a substitutable position with a group that is independently C₁-C₆ alkyl, C₁-C₆ alkoxy, -NR'R", -SO₂-NR'R", -C(O)NR'R", -NR'C(O)R", -NR'C(O)NR'R", - NR'C(O)O-(C₁-C₆ alkyl), -NR'C(O)O-phenyl, NO₂, CN, -C(O)OR', hydroxyl, hydroxy-(C₁-C₆ alkyl), -S(O)_{z} (C₁-C₆ alkyl), -S(O)_{z} aryl, halogen, C₁-C₂ haloalkyl, C₁-C₂ haloalkoxy, benzyl, or phenyl.

In another aspect, the invention provides compounds of formula 16, i.e., compounds of either formula I or formula 15, having the structure 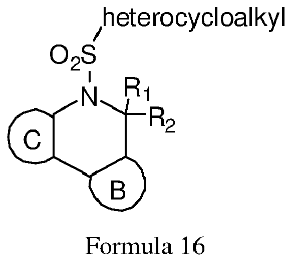 stereoisomers, tautomers, mixtures of stereoisomers and/or tautomers or pharmaceutically acceptable salts thereof, wherein
the heterocycloalkyl group is optionally substituted at each substitutable position with halogen, C₁-C₆ alkyl, C₂-C₆ alkenyl, C₂-C₆ alkynyl, C₁-C₆ alkoxy, C₁-C₆ haloalkyl, C₁-C₆ haloalkoxy, hydroxyl, hydroxy-(C₁-C₄ alkyl), CN, NO₂, aryloxy, aryl-(C₁-C₄alkoxy),-SO₂-(C₁-C₆ alkyl), -NR'R", C₁-C₆ alkanoyl, -C(O)OR', -(C₁-C₄alkyl)-C(O)OR', pyridyl, phenyl, phenyl-(C₁-C₄ alkyl), -SO₂-NR'R", -C(O)NR'R", -OC(O)NR'R", -NR'C(O)R", -NR'C(O)NR'R", or -NR'CO(O)R', where each R' and R" is independently hydrogen or C₁-C₆ alkyl; and
wherein the C-ring is cyclohexyl, phenyl, thienyl, imidazolinyl, thiazolyl, isothiazolyl, oxazolyl, isoxazolyl, pyrrolyl, pyrazolyl, pyrimidyl, pyrazinyl or pyridyl, each of which is optionally substituted at each substitutable position with groups that are independently halogen, C₁-C₆ alkyl, C₁-C₆ alkoxy, aryloxy, aryl-(C₁-C₄ alkoxy), C₁-C₆ haloalkyl, C₁-C₆ haloalkoxy, hydroxyl, hydroxy-(C₁-C₆ alkyl)-, -NR'R", -(C₁-C₆ alkyl)-NR'R", -NR'C(O)O-(C₁-C₆ alkyl), -NR'C(O)O-phenyl, -COR', -C(O)OR', -C(O)NR'R", -OC(O)NR'R", -NR'C(O)NR'R"; -(C₁-C₆ alkyl)-C(O)OR', -(C₁-C₆ alkyl)-C(O)NR'R", -NR'C(O)R", NO₂, CN, oxo, benzyl, phenyl, oxazolyl, pyrazolyl, thiazolyl, pyridyl, furanyl, thienyl, C₂-C₆ alkenyl, C₂-C₆ alkynyl, -NR'SO₂R", -SO₂-NR'R", -S(O)_{z} aryl (where the aryl group is preferably naphthyl or phenyl, still more preferably, phenyl),or -S(O)_{z} (C₁-C₆ alkyl), where the alkyl, alkenyl and alkynyl portions of the above are unsubstituted or substituted with 1, 2 or 3 groups that are independently halogen, hydroxyl, -NR'R" or C₁-C₆ alkoxy, and where the aryl or heteroaryl portions of the above are optionally substituted with 1, or 2 groups that are independently C₁-C₄ alkyl, C₁-C₄ alkoxy, halogen, CF₃, OCF₃, hydroxyl, hydroxy-(C₁-C₄ alkyl), or -NR'R".

In another aspect, the invention provides compounds of formula 16-1, i.e., compounds of formula 16, wherein the B-ring is pyrazolyl, imidazolyl, pyrrolyl, triazolyl, pyrrolidinyl, pyrazolidinyl, imidazolidinyl, pyridyl, pyrimidyl, or isoxazolyl, each of which is unsubstituted.

In another aspect, the invention provides compounds of formula 16-2, i.e., compounds of formula 16, wherein the B-ring has the formula: 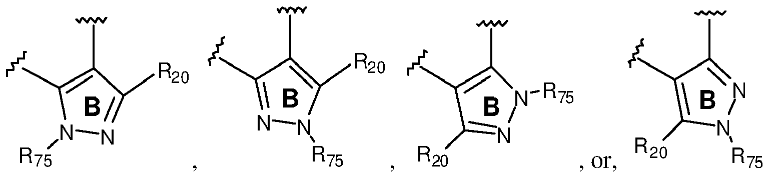 wherein
R₂₀ is hydrogen, C₁-C₆ alkyl, C₁-C₆ alkoxy, -NR'R", -(C₁-C₄ alkyl)-NR'R", -S(O)_{z} (C₁-C₆ alkyl), hydroxyl, halogen, CN, NO₂, CH₂F, CHF₂, CF₃, -C(O)OR', -C(O)NR'R", -(C₁-C₆ alkyl)-C(O)OR', -(C₁-C₆ alkyl)-C(O)NR'R", or phenyl, and
R₇₅ is hydrogen, C₁-C₆ alkyl, C₁-C₆ alkanoyl, phenyl-(C₁-C₆ alkanoyl), -C(O)NR'R", -S(O)₂-(C₁-C₆) alkyl, -S(O)₂-aryl, -SO₂NR'R, phenyl, phenyl-(C₁-C₆ alkyl) (preferably phenethyl or benzyl, more preferably, benzyl), phenylcarbonyl, benzylcarbonyl, or heteroarylcarbonyl" where the heteroaryl group is pyridyl, pyrimidyl, thienyl or furanyl.

In another aspect, the invention provides compounds of formula 16-3, i.e., compounds of formula 16-2, wherein the B-ring has the formula:

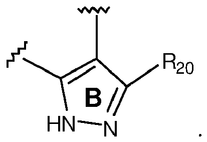

In another aspect, the invention provides compounds of formula 16-4, i.e., compounds of formula 16-2, wherein the B-ring has the formula:

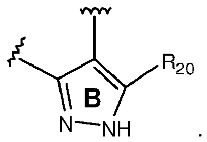

In another aspect, the invention provides compounds of formula 16-5, i.e., compounds of formula 16-2, wherein the B-ring has the formula:

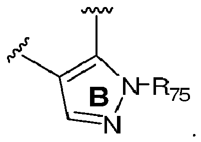

In another aspect, the invention provides compounds of formula 16-6, i.e., compounds of formula 16-2, wherein the B-ring has the formula:

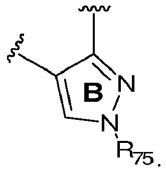

In a further aspect, the invention provides compounds of formula 16-6a, i.e., compounds according to any one of formulas 16-2, 16-3, or 16-4, wherein R₂₀ is hydrogen, C₁-C₄ alkyl, CH₂F, CHF₂, CF₃, -C(O)OR', -S(O)_{z} (C₁-C₄ alkyl), or hydroxyl.

In another aspect, the invention provides compounds of formula 16-6b, i.e., compounds of formula 16-6a, wherein R₂₀ is hydrogen or methyl.

In yet another aspect, the invention provides compounds of formula 16-6c, i.e., compounds according to any one of formulas 16-2, 16-5 or 16-6, wherein R₇₅ is hydrogen or methyl.

In still yet another aspect, the invention provides compounds of formula 16-6d, i.e., compounds according to any one of formulas 16-2, 16-5 or 16-6, wherein R₇₅ is hydrogen.

In another aspect, the invention provides compounds of formula 16-7, i.e., compounds of formula 16, wherein the B-ring has the formula: 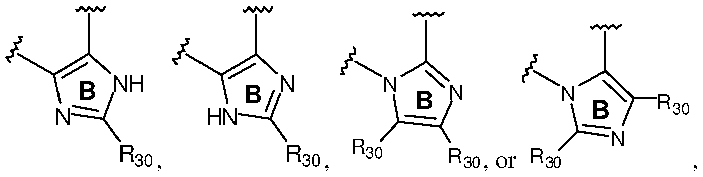 wherein R₃₀ is hydrogen, C₁-C₄ alkyl, C₁-C₄ alkoxy, -NR'R", C₁-C₄ alkythio, hydroxyl, halogen, CH₂F, CHF₂, CF₃, or phenyl.

In another aspect, the invention provides compounds of formula 16-8, i.e., compounds of formula 16-7, wherein the B-ring has the formula:

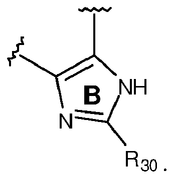

In another aspect, the invention provides compounds of formula 16-9, i.e., compounds of formula 16-7, wherein the B-ring has the formula:

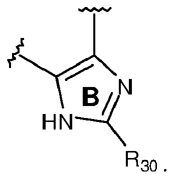

In another aspect, the invention provides compounds of formula 16-10, i.e., compounds of formula 16-7, wherein the B-ring has the formula:

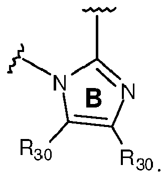

In another aspect, the invention provides compounds of formula 16-11, i.e., compounds of formula 16-7, wherein the B-ring has the formula:

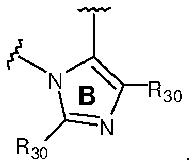

In a further aspect, the invention provides compounds of formula 16-11a, i.e., compounds according to any one of formulas 16-8, 16-9, 16-10, or 16-11, wherein each R₃₀ is independently hydrogen, C₁-C₄ alkyl, CH₂F, CHF₂, or CF₃.

In another aspect, the invention provides compounds of formula 16-11b, i.e., compounds of formula 16-11a, wherein each R₃₀ is independently hydrogen or methyl.

In still another aspect, the invention provides compounds of formula 16-11c, i.e., compounds of formula 16-11a, wherein each R₃₀ is hydrogen.

In another aspect, the invention provides compounds of formula 16-12, i.e., compounds of formula 16, wherein the B-ring has the formula:

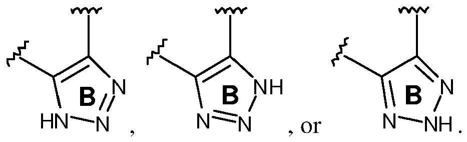

In another aspect, the invention provides compounds of formula 16-13, i.e., compounds of formula 16-12, wherein the B-ring has the formula:

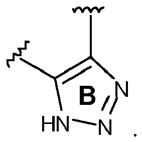

In another aspect, the invention provides compounds of formula 16-14, i.e., compounds of formula 16-12, wherein the B-ring has the formula:

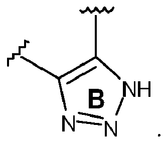

In another aspect, the invention provides compounds of formula 16-15, i.e., compounds of formula 16-12, wherein the B-ring has the formula:

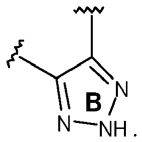

In another aspect, the invention provides compounds of formula 17, i.e., compounds of formulas 15, 16, 16-1, 16-2, 16-3, 16-4, 16-5, 16-6, 16-6a, 16-6b, 16-6c, 16-6d, 16-7, 16-8, 16-9, 16-10, 16-11, 16-11a, 16-11b, 16-11c, 16-12, 16-13, 16-14, or 16-15 wherein the C-ring is phenyl or cyclohexyl, which is optionally substituted with 1, 2, 3, or 4 groups that are independently halogen, C₁-C₄ alkyl, C₁-C₄ alkoxy, aryloxy, aryl-(C₁-C₆ alkoxy), C₁-C₄ haloalkyl (in another aspect, CF₃), C₁-C₄ haloalkoxy (in another aspect, OCF₃), hydroxyl, hydroxy-(C₁-C₄ alkyl), -NR'R", -(C₁-C₄ alkyl)-NR'R", -NR'C(O)O-(C₁-C₆ alkyl), -NR'C(O)O-phenyl, -COR', -C(O)OR', -C(O)NR'R", -OC(O)NR'R", -NR'C(O)NR'R"; -(C₁-C₆ alkyl)-C(O)OR', -(C₁-C₆ alkyl)-C(O)NR'R", -NR'C(O)R", NO₂, CN, oxo, benzyl, phenyl, oxazolyl, pyrazolyl, thiazolyl, pyridyl, furanyl, thienyl, C₂-C₆ alkenyl, C₂-C₆ alkynyl, -S(O)_{z} aryl, or -S(O)_{z} (C₁-C₆ alkyl), where the alkyl, alkenyl and alkynyl portions of the above are unsubstituted or substituted with 1 or 2 groups that are independently halogen, hydroxyl, -NR'R" or C₁-C₆ alkoxy, and where the aryl or heteroaryl portions of the above are optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R". In one embodiment, the C-ring is an optionally substituted phenyl.

In another aspect, the invention provides compounds of formula 17-1, i.e., compounds of formula 17 wherein the C-ring is unsubstituted phenyl.

In another aspect, the invention provides compounds of formula 17-1a, i.e., compounds of formula 17 wherein the C-ring is unsubstituted cyclohexyl.

In yet another aspect, the invention provides compounds of formula 17-2, i.e., compounds of formula 17 wherein the C-ring is phenyl substituted with 1 or 2 groups that are independently Cl, F, methyl, ethyl, isopropyl, methoxy, ethoxy, CF₃, OCF₃, OH, CH₂OH, NH₂, NH(CH₃), or N(CH₃)₂, or -OC(O)N(CH₃)₂. In one embodiment, the C-ring is bis-substituted with at least one halogen. In another embodiment, the halogen is F. In still another embodiment, the halogen is Cl. In another embodiment, the C-ring is bis-substituted with two halogens, which are the same or different. In still another embodiment, the C-ring is substituted at least at position 7. In another embodiment, the C-ring is substituted at positions 7 and 8. In another embodiment, the C-ring is monosubstituted with a halogen. In another embodiment, the halogen is F. In still another embodiment, the halogen is Cl. In still another embodiment, the C-ring is substituted at position 7. In yet another embodiment, the C-ring is substituted at position 8.

In still yet another aspect, the invention provides compounds of formula 17-2a, i.e., compounds of formula 17, wherein the C-ring is phenyl substituted with 1 or 2 groups that are independently -NR'C(O)O-(C₁-C₆ alkyl), -NR'C(O)O-phenyl, -C(O)OR', -C(O)NR'R", -OC(O)NR'R", -NR'C(O)NR'R", -(C₁-C₆ alkyl)-C(O)OR', -(C₁-C₆ alkyl)-C(O)NR'R", -NR'C(O)R", NO₂, CN, phenyl, -S(O)_{z} phenyl, or -S(O)_{z} (C₁-C₆ alkyl), where the phenyl portions of the above are optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-C₁-C₆ alkyl, or -NR'R". In one embodiment, the C-ring is substituted at least at the 7-position. In another embodiment, the C-ring is substituted at least at the 8-position.

In still another aspect, the invention provides compounds of formula 17-2b, i.e., compounds of formula 17, wherein the C-ring is phenyl substituted with 1 or 2 groups that are independently C₁-C₄ alkyl, C₂-C₆ alkenyl, C₂-C₆ alkynyl, where the alkyl, alkenyl and alkynyl portions of the above are unsubstituted or substituted with 1 or 2 groups that are independently halogen, hydroxyl, -NR'R" or C₁-C₄ alkoxy. In one embodiment, the C-ring is substituted at least at the 7-position. In another embodiment, the C-ring is substituted at least at the 8-position.

In still another aspect, the invention provides compounds of formula 17-2c, i.e., compounds of formula 17, wherein the C-ring is phenyl substituted with 1 or 2 groups that are independently -C(O)OH, -C(O)O-(C₁-C₄ alkyl), NO₂, CN, or phenyl, where the phenyl group is optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R". In one embodiment, the C-ring is substituted at least at the 7-position. In another embodiment, the C-ring is substituted at least at the 8-position.

In yet another aspect, the invention provides compounds of formula 17-2d, i.e., compounds of formula 17, wherein the C-ring is phenyl substituted with 1 or 2 groups that are independently C₁-C₄ alkyl, C₁-C₄ alkoxy, halogen, -C(O)NR'R", -NR'C(O)R", -OC(O)NR'R", -NR'C(O)NR'R", -NR'C(O)O-(C₁-C₆ alkyl) or -NR'C(O)O-phenyl, where the phenyl group is optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R". In one embodiment, the C-ring is substituted at least at the 7-position. In another embodiment, the C-ring is substituted at least at the 8-position.

In yet another aspect, the invention provides compounds of formula 17-2e, i.e., compounds of formula 17 wherein the C-ring is cyclohexyl substituted with 1 or 2 groups that are independently Cl, F, methyl, ethyl, isopropyl, methoxy, ethoxy, CF₃, OCF₃, OH, CH₂OH, NH₂, NH(CH₃), N(CH₃)₂, or -OC(O)N(CH₃)₂.

In still yet another aspect, the invention provides compounds of formula 17-2f, i.e., compounds of formula 17, wherein the C-ring is cyclohexyl substituted with 1 or 2 groups that are independently -NR'C(O)O-(C₁-C₆ alkyl), -NR'C(O)O-phenyl, -C(O)OR', -C(O)NR'R", -OC(O)NR'R", -NR'C(O)NR'R", -(C₁-C₆ alkyl)-C(O)OR', -(C₁-C₆ alkyl)-C(O)NR'R", -NR'C(O)R", NO₂, CN, phenyl, -S(O)_{z} aryl, or -S(O)_{z} (C₁-C₆ alkyl), where the phenyl portions of the above are optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R".

In still another aspect, the invention provides compounds of formula 17-2g, i.e., compounds of formula 17, wherein the C-ring is cyclohexyl substituted with 1 or 2 groups that are independently C₁-C₄ alkyl, C₂-C₆ alkenyl, C₂-C₆ alkynyl, where the alkyl, alkenyl and alkynyl portions of the above are unsubstituted or substituted with 1 or 2 groups that are independently halogen, hydroxyl, -NR'R" or C₁-C₄ alkoxy.

In still another aspect, the invention provides compounds of formula 17-2h, i.e., compounds of formula 17, wherein the C-ring is cyclohexyl substituted with 1 or 2 groups that are independently -C(O)OH, -C(O)O-(C₁-C₄ alkyl), NO₂, CN, or phenyl, where the phenyl group is optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R"..

In yet another aspect, the invention provides compounds of formula 17-2i, i.e., compounds of formula 17, wherein the C-ring is cyclohexyl substituted with 1 or 2 groups that are independently C₁-C₄ alkyl, C₁-C₄ alkoxy, halogen, -C(O)NR'R", -NR'C(O)R", -OC(O)NR'R", -NR'C(O)NR'R", -NR'C(O)O-(C₁-C₆ alkyl) or -NR'C(O)O-phenyl, where the phenyl group is optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R".

In yet another aspect, the invention provides compounds of formula 17-3, i.e., compounds of formula 17 wherein the C-ring is phenyl or cyclohexyl substituted with one or two groups that are Cl, F, methyl, ethyl, isopropyl, methoxy, CF₃, OCF₃, OH, or -OC(O)N(CH₃)₂.

In yet still another aspect, the invention provides compounds of formula 17-3a, i.e., compounds according to any one of formulas 17, 17-1, 17-1a, 17-2, 17-2a, 17-2b, 17-2c, 17-2d, 17-2e, 17-2f, 17-2g, 17-2h, 17-2i, or 17-3, wherein R₂ is -X(CO)Y or -(C(R₃)₂)₁₋₄X(CO)Y.

In still another aspect, the invention provides compounds of formula 17-3a1, i.e., compounds according to any one of formulas 17, 17-1, 17-1a, 17-2, 17-2a, 17-2b, 17-2c, 17-2d, 17-2e, 17-2f, 17-2g, 17-2h, 17-2i, or 17-3, wherein R₂ is hydrogen, C₁-C₃ alkyl or C₃-C₆ cycloalkyl.

In still another aspect, the invention provides compounds of formula 17-3a2, i.e., compounds according to any one of formulas 17, 17-1, 17-1a, 17-2, 17-2a, 17-2b, 17-2c, 17-2d, 17-2e, 17-2f, 17-2g, 17-2h, 17-2i, or 17-3, wherein R₂ is NO₂ or CN.

In still another aspect, the invention provides compounds of formula 17-3a3, i.e., compounds according to any one of formulas 17, 17-1, 17-1a, 17-2, 17-2a, 17-2b, 17-2c, 17-2d, 17-2e, 17-2f, 17-2g, 17-2h, 17-2i, or 17-3, wherein R₂ is C₂-C₆ alkenyl or C₂-C₆ alkynyl.

In still another aspect, the invention provides compounds of formula 17-3a4, i.e., compounds according to any one of formulas 17, 17-1, 17-1a, 17-2, 17-2a, 17-2b, 17-2c, 17-2d, 17-2e, 17-2f, 17-2g, 17-2h, 17-2i, or 17-3, wherein R₂ is C₁-C₄ haloalkyl.

In still another aspect, the invention provides compounds of formula 17-3a5, i.e., compounds according to any one of formulas 17, 17-1, 17-1a, 17-2, 17-2a, 17-2b, 17-2c, 17-2d, 17-2e, 17-2f, 17-2g, 17-2h, 17-2i, 17-3, 17-3a, 17-3a1, 17-3a2, 17-3a3, or 17-3a4, wherein R₁ is hydrogen.

In still yet another aspect, the invention provides compounds of formula 17-3a6, i.e., compounds according to any one of formulas 17, 17-1, 17-1a, 17-2, 17-2a, 17-2b, 17-2c, 17-2d, 17-2e, 17-2f, 17-2g, 17-2h, 17-2i, or 17-3, wherein R₁ and R₂ combined are oxo.

In still yet another aspect, the invention provides compounds of formula 17-3a7, i.e., compounds according to any one of formulas 17, 17-1, 17-1a, 17-2, 17-2a, 17-2b, 17-2c, 17-2d, 17-2e, 17-2f, 17-2g, 17-2h, 17-2i, or 17-3, wherein R₁ and R₂ combined are =N-OR, where R is hydrogen, C₁-C₆ alkyl, aryl (such as phenyl) or arylalkyl (such as benzyl or phenethyl).

In yet another aspect, the invention provides compounds of formula 17-3a8, i.e., compounds according to any one of formulas 17, 17-1, 17-1a, 17-2, 17-2a, 17-2b, 17-2c, 17-2d, 17-2e, 17-2f, 17-2g, 17-2h, 17-2i, or 17-3, wherein R₁ and R₂ together with the carbon to which they are attached form a C₃-C₆ cycloalkyl group.

In yet another aspect, the invention provides compounds of formula 17-3a9, i.e., compounds according to any one of formulas 17, 17-1, 17-1a, 17-2, 17-2a, 17-2b, 17-2c, 17-2d, 17-2e, 17-2f, 17-2g, 17-2h, 17-2i, or 17-3, wherein R₁ and R₂ together with the carbon to which they are attached form a cyclopropyl group.

In yet another aspect, the invention provides compounds of formula 17-3a10, i.e., compounds according to any one of formulas 17, 17-1, 17-1a, 17-2, 17-2a, 17-2b, 17-2c, 17-2d, 17-2e, 17-2f, 17-2g, 17-2h, 17-2i, or 17-3, wherein R₁ is hydrogen and R₂ is cyclopropyl. In one embodiment, the compound is racemic. In another embodiment, the compound is enantiomerically enriched.

In yet still another aspect, the invention provides compounds of formula 17-3b, i.e., compounds of formula 17-3a, wherein
X is O; and Y is -NR₆₀R₇₀ or -N(R₃₀)(CH₂)₂₋₆NR₆₀R₇₀; where R₆₀ and R₇₀ are independently selected from: hydrogen, methyl, ethyl, (C₃ -C₈)cycloalkyl, aryl-(C₁-C₆ alkyl) (such as benzyl or phenethyl), 4-pyridylmethyl, 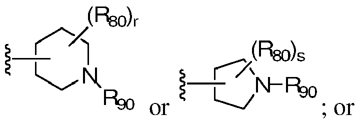
R₆₀ and R₇₀ taken together with the nitrogen atom to which they are bound form a heterocycloalkyl group selected from 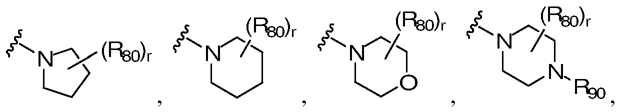 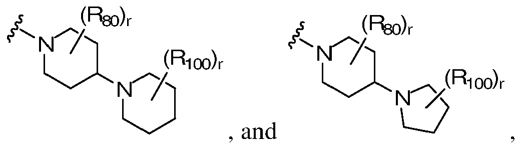 where
   each R₈₀ is independently unsubstituted C₁-C₄ alkyl or C₁-C₄ alkyl substituted with hydroxyl or halogen groups;
   each R₉₀ is independently hydrogen, unsubstituted C₁-C₄ alkyl, C₁-C₄ alkyl substituted with hydroxyl or halogen, unsubstituted C₃-C₈ cycloalkyl, C₃-C₈ cycloalkyl substituted with one or more (e.g., 1-4) R₅₀, phenyl-(C₁-C₄ alkyl), pyridyl-(C₁-C₄ alkyl), thienyl-(C₁-C₄ alkyl), -C(O)O-(C₁-C₄ alkyl), -C(O)O-phenyl, -SO₂-(C₁-C₆alkyl), -SO₂-phenyl, unsubstituted phenyl, phenyl substituted with one or more (e.g., 1-4) R₅₀ groups, pyridyl, thienyl, pyridyl substituted with one or more (e.g., 1-4) R₅₀ groups, thienyl substituted with one or more (e.g., 1-4) R₅₀ groups, where
   each R₅₀ is independently selected from: halogen, C₁-C₆ alkyl, - C₁-C₆ haloalkyl, -OH, -O (C₁-C₄ alkyl), OCF₃, -CN, - NR₆₀R₇₀, -C(O)O-(C₁-C₄ alkyl), -CONR₆₀R₇₀, - (C₁-C₆ alkyl)-NR₆₀R₇₀, -NR₆₀CO-(C₁-C₄ alkyl), -NR₆₀CO-phenyl, -NR₆₀CO-pyridyl, -NR₆₀CO-thienyl, and -NR₆₀CONR₆₀R₇₀,
   each R₁₀₀ is independently hydrogen or C₁-C₄ alkyl;
   each r is 0 to 4; and
   each s is 0 to 3.

In yet still another aspect, the invention provides compounds of formula 17-3b1, i.e., compounds of formula 17-3a, wherein Y is -O-(C₁-C₆ alkyl). In one embodiment, Y is -O-(C₁-C₄ alkyl).

In yet still another aspect, the invention provides compounds of formula 17-3b2, i.e., compounds of formula 17-3a, wherein Y is -O-phenyl.

In yet still another aspect, the invention provides compounds of formula 17-3c, i.e., compounds of formula 17-3b, wherein said group 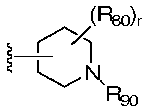 is a group of the formula: 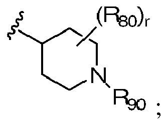 and, said group 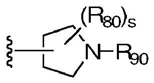 is a group of the formula:

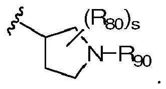

In a further aspect, the invention provides compounds of formula 17-3d, i.e., compounds according to any one of formulas, 17-3b, 17-3b1, 17-3b2, or 17-3c, wherein R₁ is H or C₁-C₄ alkyl. In one embodiment, R₁ is H or methyl. In another embodiment, R₁ is H.

In yet another aspect, the invention provides compounds of formula 17-4, i.e., compounds according to any one of formulas 15, or any of the formulas 16, 16-1, 16-2, 16-3, 16-4, 16-5, 16-6, 16-6a, 16-6b, 16-6c, 16-6d, 16-7, 16-8, 16-9, 16-10, 16-11, 16-11a, 16-11b, 16-11c, 16-12, 16-13, 16-14, or 16-15, wherein the C-ring is 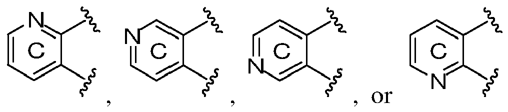 or N-oxide derivatives therof,
each of which is optionally substituted with 1, 2, or 3 groups that are independently halogen, C₁-C₄ alkyl, C₁-C₄ alkoxy, aryloxy, aryl-(C₁-C₄ alkoxy), C₁-C₄ haloalkyl (in another aspect, CF₃), C₁-C₄ haloalkoxy (in another aspect, OCF₃), hydroxyl, hydroxy-(C₁-C₄ alkyl), -NR'R", -(C₁-C₄ alkyl)-NR'R", -NR'C(O)O-(C₁-C₆ alkyl), -NR'C(O)O-phenyl, -COR', -C(O)OR', -C(O)NR'R", -(C₁-C₆ alkyl)-C(O)OR', -(C₁-C₆ alkyl)-C(O)NR'R", -NR'C(O)R", -OC(O)NR'R", -NR'C(O)NR'R", NO₂, CN, benzyl, phenyl, oxazolyl, pyrazolyl, thiazolyl, pyridyl, furanyl, thienyl, C₂-C₆ alkenyl, C₂-C₆ alkynyl, -NR'SO₂R", -SO₂-NR'R", -S(O)_{z} aryl (where the aryl group is preferably naphthyl or phenyl, still more preferably, phenyl), or -S(O)_{z} (C₁-C₆ alkyl), where the alkyl, alkenyl and alkynyl portions of the above are unsubstituted or substituted with 1, 2 or 3 groups that are independently halogen, hydroxyl, - NR'R" or C₁-C₆ alkoxy, and where the aryl or heteroaryl portions of the above are optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R". In one embodiment, the invention provides compounds wherein the C-ring is pyridyl or pyridyl N-oxide substituted with 1 or 2 groups that are independently -NR'C(O)O-(C₁-C₆ alkyl), -NR'C(O)O-phenyl, -C(O)OR', -C(O)NR'R", -(C₁-C₆ alkyl)-C(O)OR', -(C₁-C₆ alkyl)-C(O)NR'R", -NR'C(O)R", -OC(O)NR'R", -NR'C(O)NR'R", NO₂, CN, phenyl, -S(O)_{z} aryl (where the aryl group is preferably naphthyl or phenyl, still more preferably, phenyl) or -S(O)_{z} (C₁-C₆ alkyl) where the aryl portions of the above are optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R". In an alternative embodiment, the C-ring is pyridyl or pyridyl N-oxide substituted with 1 or 2 groups that are independently C₁-C₄ alkyl, C₂-C₆ alkenyl, C₂-C₆ alkynyl, where the alkyl, alkenyl and alkynyl portions of the above are unsubstituted or substituted with 1 or 2 groups that are independently halogen, hydroxy, -NR'R" or C₁-C₄ alkoxy. In another alternative embodiment, the C-ring is pyridyl or pyridyl N-oxide substituted with 1 or 2 groups that are independently -C(O)O-(C₁-C₄ alkyl), NO₂, CN, or phenyl, where the phenyl group is optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R". In still another alternative embodiment, the C-ring is pyridyl or pyridyl N-oxide substituted with 1 or 2 groups that are independently C₁-C₄ alkyl, C₁-C₄ alkoxy, halogen, -C(O)NR'R", NR'C(O)R", -OC(O)NR'R", -NR'C(O)NR'R", -NR'C(O)O-(C₁-C₆ alkyl) or -NR'C(O)O-phenyl, where the phenyl group is optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R".

In still another aspect, the invention provides compounds of formula 17-4a, i.e., compounds of formula 15 or any one of formulas 15, 16, 16-1, 16-2, 16-3, 16-4, 16-5, 16-6, 16-6a, 16-6b, 16-6c, 16-6d, 16-7, 16-8, 16-9, 16-10, 16-11, 16-11a, 16-11b, 16-11c, 16-12, 16-13, 16-14, or 16-15, wherein the C-ring is 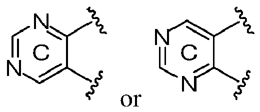 each of which is optionally substituted with 1, 2, or 3 groups that are independently halogen, C₁-C₄ alkyl, C₁-C₄ alkoxy, aryloxy, aryl-(C₁-C₄ alkoxy), C₁-C₄ haloalkyl (in another aspect, CF₃), C₁-C₄ haloalkoxy (in another aspect, OCF₃), hydroxyl, hydroxy-(C₁-C₄ alkyl), -NR'R", -(C₁-C₄ alkyl)-NR'R", -NR'C(O)O-(C₁-C₆ alkyl), -NR'C(O)O-phenyl, -COR', -C(O)OR', -C(O)NR'R", -OC(O)NR'R", -NR'C(O)NR'R"; -(C₁-C₆ alkyl)-C(O)OR', -(C₁-C₆ alkyl)-C(O)NR'R", -NR'C(O)R", NO₂, CN, benzyl, phenyl, oxazolyl, pyrazolyl, thiazolyl, pyridyl, furanyl, thienyl, C₂-C₆ alkenyl, C₂-C₆ alkynyl, -NR'SO₂R" -SO₂-NR'R", -S(O)_{z} aryl (where the aryl group is preferably naphthyl or phenyl, still more preferably, phenyl), or -S(O)_{z} (C₁-C₆ alkyl), where the alkyl, alkenyl and alkynyl portions of the above are unsubstituted or substituted with 1, 2 or 3 groups that are independently halogen, hydroxyl, -NR'R" or C₁-C₆ alkoxy, and where the aryl or heteroaryl portions of the above are optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R". In one embodiment, the invention provides compounds wherein the C-ring is pyrimidinyl substituted with 1 or 2 groups that are independently -NR'C(O)O-(C₁-C₆ alkyl), -NR'C(O)O-phenyl, -C(O)OR', -C(O)NR'R", -OC(O)NR'R", -NR'C(O)NR'R", -(C₁-C₆ alkyl)-C(O)OR', -(C₁-C₆ alkyl)-C(O)NR'R", -NR'C(O)R", NO₂, CN, phenyl, -S(O)_{z} aryl (where the aryl group is preferably naphthyl or phenyl, still more preferably, phenyl) or -S(O)_{z} (C₁-C₆ alkyl) where the aryl portions of the above are optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R". In an alternative embodiment, the C-ring is pyrimidinyl substituted with 1 or 2 groups that are independently C₁-C₄ alkyl, C₂-C₆ alkenyl, C₂-C₆ alkynyl, where the alkyl, alkenyl and alkynyl portions of the above are unsubstituted or substituted with 1 or 2 groups that are independently halogen, hydroxyl, -NR'R" or C₁-C₄ alkoxy. In another alternative embodiment, the C-ring is pyrimidinyl substituted with 1 or 2 groups that are independently -C(O)OH, -C(O)O-(C₁-C₄ alkyl), NO₂, CN, or phenyl, where the phenyl group is optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R". In still another alternative embodiment, the C-ring is pyrimidinyl substituted with 1 or 2 groups that are independently C₁-C₄ alkyl, C₁-C₄ alkoxy, halogen, -C(O)NR'R", -NR'C(O)R", -OC(O)NR'R", -NR'C(O)NR'R", -NR'C(O)O-(C₁-C₆ alkyl) or - NR'C(O)O-phenyl, where the phenyl group is optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R".

In still another aspect, the invention provides compounds of formula 17-4b, i.e., compounds of formula 15 or any one of formulas 15, 16, 16-1, 16-2, 16-3, 16-4, 16-5, 16-6, 16-6a, 16-6b, 16-6c, 16-6d, 16-7, 16-8, 16-9, 16-10, 16-11, 16-11a, 16-11b, 16-11c, 16-12, 16-13, 16-14, or 16-15, wherein the C-ring is 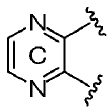 which is optionally substituted with 1, 2, or 3 groups that are independently halogen, C₁-C₄ alkyl, C₁-C₄ alkoxy, aryloxy, aryl-(C₁-C₄ alkoxy), C₁-C₄ haloalkyl (in another aspect, CF₃), C₁-C₄ haloalkoxy (in another aspect, OCF₃), hydroxyl, hydroxy-(C₁-C₄ alkyl), -NR'R", -(C₁-C₄ alkyl)-NR'R", -NR'C(O)O-(C₁-C₆ alkyl), -NR'C(O)O-phenyl, -COR', -C(O)OR', -C(O)NR'R", -OC(O)NR'R", -NR'C(O)NR'R"; -(C₁-C₄ alkyl)-C(O)OR', -(C₁-C₄ alkyl)-C(O)NR'R", -NR'C(O)R", NO₂, CN, benzyl, phenyl, pyridyl, thienyl, C₂-C₆ alkenyl, C₂-C₆ alkynyl, -NR'SO₂R" -SO₂-NR'R", -S(O)_{z} aryl (where the aryl group is preferably naphthyl or phenyl, still more preferably, phenyl), or -S(O)_{z} (C₁-C₆ alkyl), where the alkyl, alkenyl and alkynyl portions of the above are unsubstituted or substituted with 1, 2 or 3 groups that are independently halogen, hydroxyl, -NR'R" or C₁-C₆ alkoxy, and where the aryl or heteroaryl portions of the above are optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R". In one embodiment, the invention provides compounds wherein the C-ring is pyrazine substituted with 1 or 2 groups that are independently -NR'C(O)O-(C₁-C₆ alkyl), -NR'C(O)O-phenyl, -C(O)OR', -C(O)NR'R", - OC(O)NR'R", -NR'C(O)NR'R", -(C₁-C₆ alkyl)-C(O)OR', -(C₁-C₆ alkyl)-C(O)NR'R", - NR'C(O)R", NO₂, CN, phenyl, -S(O)_{z} aryl (where the aryl group is preferably naphthyl or phenyl, still more preferably, phenyl) or -S(O)_{z} (C₁-C₆ alkyl) where the aryl portions of the above are optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R". In an alternative embodiment, the C-ring is pyrazine substituted with 1 or 2 groups that are independently C₁-C₄ alkyl, C₂-C₆ alkenyl, C₂-C₆ alkynyl, where the alkyl, alkenyl and alkynyl portions of the above are unsubstituted or substituted with 1 or 2 groups that are independently halogen, hydroxyl, -NR'R" or C₁-C₄ alkoxy. In another alternative embodiment, the C-ring is pyrazine substituted with 1 or 2 groups that are independently -C(O)OH, -C(O)O-(C₁-C₄ alkyl), NO₂, CN, or phenyl, where the phenyl group is optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R". In still another alternative aspect, the C-ring is pyrazine substituted with 1 or 2 groups that are independently C₁-C₄ alkyl, C₁-C₄ alkoxy, halogen, -C(O)NR'R", -NR'C(O)R", -OC(O)NR'R", -NR'C(O)NR'R", or -NR'C(O)O-(C₁-C₆ alkyl), -NR'C(O)O-phenyl, where the phenyl group is optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R".

In still another aspect, the invention provides compounds of formula 17-4c, i.e., compounds of formula 15 or any of the formulas 15, 16, 16-1, 16-2, 16-3, 16-4, 16-5, 16-6, 16-6a, 16-6b, 16-6c, 16-6d, 16-7, 16-8, 16-9, 16-10, 16-11, 16-11a, 16-11b, 16-11c, 16-12, 16-13, 16-14, or 16-15, wherein the C-ring is: 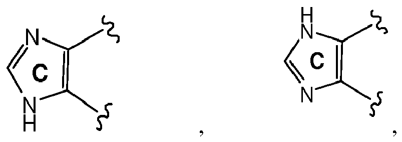 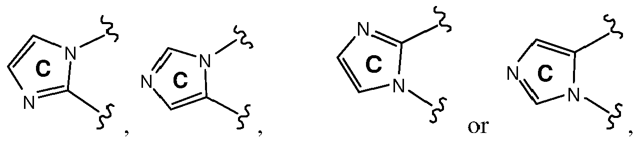 each of which is optionally substituted with 1 or 2 groups that are independently halogen, C₁-C₄ alkyl, C₁-C₄ alkoxy, aryloxy, aryl-(C₁-C₄ alkoxy), C₁-C₄ haloalkyl (in another aspect, CF₃), C₁-C₄ haloalkoxy (in another aspect, OCF₃), hydroxyl, hydroxy-(C₁-C₄ alkyl), -NR'R", -(C₁-C₆ alkyl)-NR'R", -NR'C(O)O-(C₁-C₆ alkyl), -NR'C(O)O-phenyl, -COR', -C(O)OR', -C(O)NR'R", -OC(O)NR'R", -NR'C(O)NR'R"; -(C₁-C₆ alkyl)-C(O)OR', -(C₁-C₆ alkyl)-C(O)NR'R", -NR'C(O)R", NO₂, CN, benzyl, phenyl, oxazolyl, pyrazolyl, thiazolyl, pyridyl, furanyl, thienyl, C₂-C₆ alkenyl, C₂-C₆ alkynyl, -NR'SO₂R", -SO₂-NR'R", -S(O)_{z} aryl (where the aryl group is preferably naphthyl or phenyl, still more preferably, phenyl), or -S(O)_{z} (C₁-C₆ alkyl), where the alkyl, alkenyl and alkynyl portions of the above are unsubstituted or substituted with 1, 2, or 3 groups that are independently halogen, hydroxyl, -NR'R" or C₁-C₆ alkoxy, and where the aryl or heteroaryl portions of the above are optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R".

In still another aspect, the invention provides compounds of formula 17-4c1, i.e., compounds of formula 17-4c wherein the C-ring is:

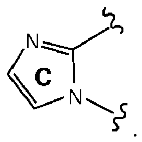

In still another aspect, the invention provides compounds of formula 17-4d, i.e., compounds of formula 15 or any of the formulas 15, 16, 16-1, 16-2, 16-3, 16-4, 16-5, 16-6, 16-6a, 16-6b, 16-6c, 16-6d, 16-7, 16-8, 16-9, 16-10, 16-11, 16-11a, 16-11b, 16-11c, 16-12, 16-13, 16-14, or 16-15, wherein the C-ring is 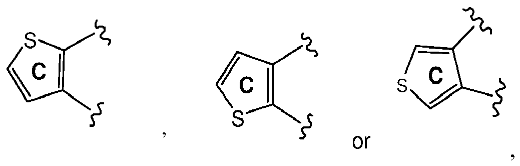 each of which is optionally substituted with 1 or 2 groups that are independently halogen, C₁-C₄ alkyl, C₁-C₄ alkoxy, aryloxy, aryl-(C₁-C₄ alkoxy), C₁-C₄ haloalkyl (in another aspect, CF₃), C₁-C₄ haloalkoxy (in another aspect, OCF₃), hydroxyl, hydroxy-(C₁-C₄ alkyl), -NR'R", -(C₁-C₆ alkyl)-NR'R", -NR'C(O)O-(C₁-C₆ alkyl), -NR'C(O)O-phenyl, -COR', -C(O)OR', -C(O)NR'R", -OC(O)NR'R", -NR'C(O)NR'R"; -(C₁-C₆ alkyl)-C(O)OR', -(C₁-C₆ alkyl)-C(O)NR'R", -NR'C(O)R", NO₂, CN, benzyl, phenyl, oxazolyl, pyrazolyl, thiazolyl, pyridyl, furanyl, thienyl, C₂-C₆ alkenyl, C₂-C₆ alkynyl, -NR'SO₂R", -SO₂-NR'R", -S(O)_{z} aryl (where the aryl group is preferably naphthyl or phenyl, still more preferably, phenyl), or -S(O)_{z} (C₁-C₆ alkyl), where the alkyl, alkenyl and alkynyl portions of the above are unsubstituted or substituted with 1, 2, or 3 groups that are independently halogen, hydroxyl, -NR'R" or C₁-C₆ alkoxy, and where the aryl or heteroaryl portions of the above are optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R".

In still another aspect, the invention provides compounds of formula 17-4d1, i.e., compounds of formula 17-4d wherein the C-ring is:

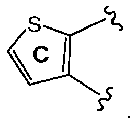

In still another aspect, the invention provides compounds of formula 17-4d2, i.e., compounds of formula 17-4d wherein the C-ring is:

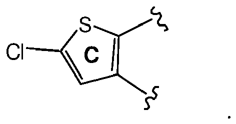

In another aspect, the invention provides compounds of formula 17-5, i.e., compounds according to formula 17-4, 17-4a, 17-4b, 17-4c, or 17-4d wherein the C-ring is unsubstituted.

In another aspect, the invention provides compounds of formula 17-5a, i.e., compounds of formula 17-4, 17-4a, 17-4b, 17-4c, or 17-4d wherein the C-ring is substituted with 1 or 2 groups that are independently Cl, F, methyl, ethyl, isopropyl, methoxy, ethoxy, CF₃, OCF₃, OH, or -C(O)CH₃.

In yet another aspect, the invention provides compounds of formula 17-5b, i.e., compounds of formula 17-4, 17-4a, 17-4b, 17-4c, or 17-4d wherein the C-ring is substituted with 1 or 2 groups that are Cl, F, or methyl.

In still yet another aspect, the invention provides compounds of formula 17-5c, i.e., compounds according to any one of formulas 17-4, 17-5, 17-5a, or 17-5b, where the C-ring has the following structure:

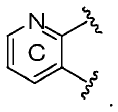

In still yet another aspect, the invention provides compounds of formula 17-5d, i.e., compounds according to any one of formulas 17-4, 17-5, 17-5a, or 17-5b, where the C-ring has the following structure:

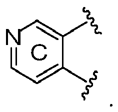

In still yet another aspect, the invention provides compounds of formula 17-5d1, i.e., compounds according to any one of formulas 17-4, 17-5, 17-5a, or 17-5b, where the C-ring has the following structure:

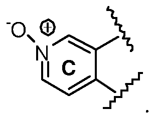

In still yet another aspect, the invention provides compounds of formula 17-5e, i.e., compounds according to any one of formulas 17-4, 17-5, 17-5a, or 17-5b, where the C-ring has the following structure:

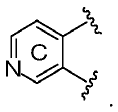

In still yet another aspect, the invention provides compounds of formula 17-5e1, i.e., compounds according to formula 17-4, where the C-ring has the following structure:

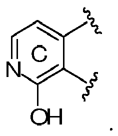

In still yet another aspect, the invention provides compounds of formula 17-5f, i.e., compounds according to any one of formulas 17-4, 17-5, 17-5a, or 17-5b, where the C-ring has the following structure:

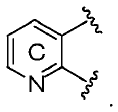

In a further aspect, the invention provides compounds of formula 17-5f1, i.e., compounds of formula 15 or any of the formulas 15, 16, 16-1, 16-2, 16-3, 16-4, 16-5, 16-6, 16-6a, 16-6b, 16-6c, 16-6d, 16-7, 16-8, 16-9, 16-10, 16-11, 16-11a, 16-11b, 16-11c, 16-12, 16-13, 16-14, or 16-15, wherein the C-ring is thiazolyl, which is optionally substituted with 1 or 2 groups that are independently halogen, C₁-C₄ alkyl, C₁-C₄ alkoxy, aryloxy, aryl-(C₁-C₄ alkoxy), C₁-C₄ haloalkyl (in another aspect, CF₃), C₁-C₄ haloalkoxy (in another aspect, OCF₃), hydroxyl, hydroxy-(C₁-C₄ alkyl), -NR'R", -(C₁-C₆ alkyl)-NR'R", -NR'C(O)O-(C₁-C₆ alkyl), -NR'C(O)O-phenyl, -COR', -C(O)OR', -C(O)NR'R", -OC(O)NR'R", -NR'C(O)NR'R"; -(C₁-C₆ alkyl)-C(O)OR', -(C₁-C₆ alkyl)-C(O)NR'R", -NR'C(O)R", NO₂, CN, benzyl, phenyl, oxazolyl, pyrazolyl, thiazolyl, pyridyl, furanyl, thienyl, C₂-C₆ alkenyl, C₂-C₆ alkynyl, -NR'SO₂R", -SO₂-NR'R", -S(O)_{z} aryl (where the aryl group is preferably naphthyl or phenyl, still more preferably, phenyl), or -S(O)_{z} (C₁-C₆ alkyl), where the alkyl, alkenyl and alkynyl portions of the above are unsubstituted or substituted with 1, 2, or 3 groups that are independently halogen, hydroxyl, -NR'R" or C₁-C₆ alkoxy, and where the aryl or heteroaryl portions of the above are optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R".

In another aspect, the invention provides compounds of formula 17-5f2, i.e., compounds of formula 17-5f1, wherein the thiazolyl ring is substituted with one group that is halogen, C₁-C₄ alkyl, C₁-C₄ alkoxy, or benzyl.

In still another aspect, the invention provides compounds of formula 17-5f3, i.e., compounds of formula 17-5f1, wherein the thiazolyl ring is substituted with C₁-C₄ alkyl.

In yet another aspect, the invention provides compounds of formula 17-5f4, i.e., compounds of formula 17-5f1, wherein the thiazolyl ring is substituted with C₁-C₄ haloalkyl (in another aspect, CF₃), C₁-C₄ haloalkoxy (in another aspect, OCF₃), hydroxyl, hydroxy-(C₁-C₄ alkyl), -NR'R", or -(C₁-C₆ alkyl)-NR'R".

In yet still another aspect, the invention provides compounds of formula 17-5f5, i.e., compounds of formula 17-5f1, wherein the thiazolyl ring is substituted with C₂-C₄ alkyl, -(C₁-C₄ alkyl)-C(O)OR', or -(C₁-C₄ alkyl)-C(O)NR'R".

In another aspect, the invention provides compounds of formula 17-5f6, i.e., compounds of formula 17-5f1, wherein the thiazolyl ring is:

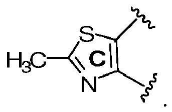

In yet still another aspect, the invention provides compounds of formula 17-5g, i.e., compounds according to any one of formulas 17-4, 17-4a, 17-4b, 17-4c, 17-4c1, 17-4d, 17-4d1, 17-4d2, 17-5, 17-5a, 17-5b, 17-5c, 17-5d, 17-5d1, 17-5e, 17-5e1, 17-5f, 17-5f1, 17-5f2, 17-5f3, 17-5f4, 17-5f5, or 17-5f6, wherein R₂ is -X(CO)Y or -(C(R₃)₂)₁₋₄X(CO)Y.

In still another aspect, the invention provides compounds of formula 17-5g1, i.e., compounds according to any one of formulas 17-4, 17-4a, 17-4b, 17-4c, 17-4c1, 17-4d, 17-4d1, 17-4d2, 17-5, 17-5a, 17-5b, 17-5c, 17-5d, 17-5d1, 17-5e, 17-5e1, 17-5f, 17-5f1, 17-5f2, 17-5f3, 17-5f4, 17-5f5, or 17-5f6, wherein R₂ is hydrogen, C₁-C₃ alkyl or C₃-C₆ cycloalkyl.

In still another aspect, the invention provides compounds of formula 17-5g2, i.e., compounds according to any one of formulas 17-4, 17-4a, 17-4b, 17-4c, 17-4c1, 17-4d, 17-4d1, 17-4d2, 17-5, 17-5a, 17-5b, 17-5c, 17-5d, 17-5d1, 17-5e, 17-5e1, 17-5f, 17-5f1, 17-5f2, 17-5f3, 17-5f4, 17-5f5, or 17-5f6, wherein R₂ is NO₂ or CN.

In still another aspect, the invention provides compounds of formula 17-5g3, i.e., compounds according to any one of formulas 17-4, 17-4a, 17-4b, 17-4c, 17-4c1, 17-4d, 17-4d1, 17-4d2, 17-5, 17-5a, 17-5b, 17-5c, 17-5d, 17-5d1, 17-5e, 17-5e1, 17-5f, 17-5f1, 17-5f2, 17-5f3, 17-5f4, 17-5f5, or 17-5f6, wherein R₂ is C₂-C₆ alkenyl or C₂-C₆ alkynyl.

In still another aspect, the invention provides compounds of formula 17-5g4, i.e., compounds according to any one of formulas 17-4, 17-4a, 17-4b, 17-4c, 17-4c1, 17-4d, 17-4d1, 17-4d2, 17-5, 17-5a, 17-5b, 17-5c, 17-5d, 17-5d1, 17-5e, 17-5e1, 17-5f, 17-5f1, 17-5f2, 17-5f3, 17-5f4, 17-5f5, or 17-5f6, wherein R₂ is C₁-C₄ haloalkyl.

In still another aspect, the invention provides compounds of formula 17-5g5, i.e., compounds according to any one of formulas 17-4, 17-4a, 17-4b, 17-4c, 17-4c1, 17-4d, 17-4d1, 17-4d2, 17-5, 17-5a, 17-5b, 17-5c, 17-5d, 17-5d1, 17-5e, 17-5e1, 17-5f, 17-5f1, 17-5f2, 17-5f3, 17-5f4, 17-5f5, 17-5f6, 17-5g, 17-5g1, 17-5g2, 17-5g3 or 17-5g4 wherein R₁ is hydrogen.

In still yet another aspect, the invention provides compounds of formula 17-5g6, i.e., compounds according to any one of formulas 17-4, 17-4a, 17-4b, 17-4c, 17-4c1, 17-4d, 17-4d1, 17-4d2, 17-5, 17-5a, 17-5b, 17-5c, 17-5d, 17-5d1, 17-5e, 17-5e1, 17-5f, 17-5f1, 17-5f2, 17-5f3, 17-5f4, 17-5f5, or 17-5f6, wherein R₁ and R₂ combined are oxo.

In still yet another aspect, the invention provides compounds of formula 17-5g7, i.e., compounds according to any one of formulas 17-4, 17-4a, 17-4b, 17-4c, 17-4c1, 17-4d, 17-4d1, 17-4d2, 17-5, 17-5a, 17-5b, 17-5c, 17-5d, 17-5d1, 17-5e, 17-5e1, 17-5f, 17-5f1, 17-5f2, 17-5f3, 17-5f4, 17-5f5, or 17-5f6, wherein R₁ and R₂ combined are =N-OR, where R is hydrogen, C₁-C₆ alkyl, aryl (such as phenyl) or arylalkyl (such as benzyl or phenethyl).

In yet another aspect, the invention provides compounds of formula 17-5g8, i.e., compounds according to any one of formulas 17-4, 17-4a, 17-4b, 17-4c, 17-4c1, 17-4d, 17-4d1, 17-4d2, 17-5, 17-5a, 17-5b, 17-5c, 17-5d, 17-5d1, 17-5e, 17-5e1, 17-5f, 17-5f1, 17-5f2, 17-5f3, 17-5f4, 17-5f5, or 17-5f6, wherein R₁ and R₂ together with the carbon to which they are attached form a C₃-C₆ cycloalkyl group.

In yet another aspect, the invention provides compounds of formula 17-5g9, i.e., compounds according to any one of formulas 17-4, 17-4a, 17-4b, 17-4c, 17-4c1, 17-4d, 17-4d1, 17-4d2, 17-5, 17-5a, 17-5b, 17-5c, 17-5d, 17-5d1, 17-5e, 17-5e1, 17-5f, 17-5f1, 17-5f2, 17-5f3, 17-5f4, 17-5f5, or 17-5f6, wherein R₁ and R₂ together with the carbon to which they are attached form a cyclopropyl group.

In yet another aspect, the invention provides compounds of formula 17-5g10, i.e., compounds according to any one of formulas 17-4, 17-4a, 17-4b, 17-4c, 17-4c1, 17-4d, 17-4d1, 17-4d2, 17-5, 17-5a, 17-5b, 17-5c, 17-5d, 17-5d1, 17-5e, 17-5e1, 17-5f, 17-5f1, 17-5f2, 17-5f3, 17-5f4, 17-5f5, or 17-5f6, wherein R₁ is hydrogen and R₂ is cyclopropyl. In one embodiment, the compound is racemic. In another embodiment, the compound is enantiomerically enriched.

In yet still another aspect, the invention provides compounds of formula 17-5h, i.e., compounds of formula 17-5g, wherein
X is O; and Y is -NR₆₀R₇₀ or -N(R₃₀)(CH₂)₂₋₆NR₆₀R₇₀; where R₆₀ and R₇₀ are independently selected from: hydrogen, methyl, ethyl, (C₃-C₈)cycloalkyl, aryl-(C₁-C₆)alkyl (such as benzyl or phenethyl), 4-pyridylmethyl, 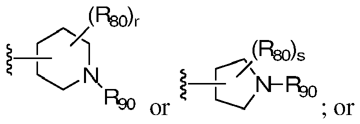
R₆₀ and R₇₀ taken together with the nitrogen atom to which they are bound form a heterocycloalkyl group selected from 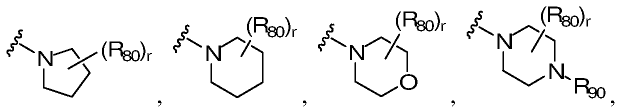 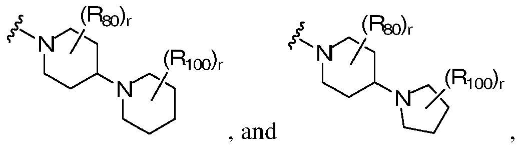 where
   each R₈₀ is independently unsubstituted C₁-C₄ alkyl or C₁-C₄ alkyl substituted with hydroxyl or halgoen;
   each R₉₀ is independently hydrogen, unsubstituted C₁-C₄ alkyl, C₁-C₄ alkyl substituted with hydroxyl or halogen, unsubstituted C₃-C₈ cycloalkyl, C₃-C₈ cycloalkyl substituted with one or more (e.g., 1-4) R₅groups, phenyl-(C₁-C₄ alkyl), pyridyl-(C₁-C₄ alkyl), thienyl-(C₁-C₄ alkyl), -C(O)O-(C₁-C₄ alkyl), -C(O)O-phenyl, -SO₂-(C₁-C₆ alkyl), -SO₂-phenyl, unsubstituted phenyl, phenyl substituted with one or more (e.g., 1-4) R₅₀ groups, pyridyl, thienyl, pyridyl substituted with one or more (e.g., 1-4) R₅₀ groups, thienyl substituted with one or more (e.g., 1-4) R₅₀ groups, where
   each R₅₀ is independently selected from: halogen, C₁-C₆ alkyl, - C₁-C₆ haloalkyl, -OH, -O- (C₁-C₄ alkyl), OCF₃, -CN, - NR₆₀R₇₀, -C(O)O-(C₁-C₄ alkyl), -CONR₆₀R₇₀, -(C₁-C₆ alkyl)-NR₆₀R₇₀, -NR₆₀CO-(C₁-C₄ alkyl), -NR₆₀CO-phenyl, -NR₆₀CO-pyridyl, -NR₆₀CO-thienyl, and -NR₆₀CONR₆₀R₇₀,
   each R₁₀₀ is independently hydrogen or C₁-C₄ alkyl;
   each r is 0 to 4; and
   each s is 0 to 3.

In yet still another aspect, the invention provides compounds of formula 17-5h1, i.e., compounds of formula 17-5g, wherein Y is -O-(C₁-C₆ alkyl). In one embodiment, Y is -O-(C₁-C₄ alkyl).

In yet still another aspect, the invention provides compounds of formula 17-5h2, i.e., compounds of formula 17-5g, wherein Y is -O-phenyl.

In yet still another aspect, the invention provides compounds of formula 17-5i, i.e., compounds of formula 17-5h, wherein said group 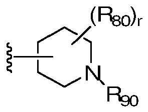 is a group of the formula: 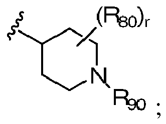 and, said group 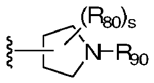 is a group of the formula:

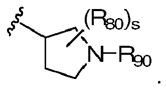

In a further aspect, the invention provides compounds of formula 17-5j, i.e., compounds according to any one of formulas, 17-5h, 17-5h1, 17-5h2, or 17-5i, wherein R₁ is H or C₁-C₄ alkyl. In one embodiment, R₁ is H or methyl. In another embodiment, R₁ is H.

In yet another aspect, the invention provides compounds of formula 17-6, i.e., compounds of formulas 15, 16, 16-1, 16-2, 16-3, 16-4, 16-5, 16-6, 16-6a, 16-6b, 16-6c, 16-6d, 16-7, 16-8, 16-9, 16-10, 16-11, 16-11a, 16-11b, 16-11c, 16-12, 16-13, 16-14, or 16-15, 17, 17-1, 17-1a, 17-2, 17-2a, 17-2b, 17-2c, 17-2d, 17-2e, 17-2f, 17-2g, 17-2h, 17-2i, 17-3, 17-3a, 17-3a1, 17-3a2, 17-3a3, 17-3a4, 17-3a5, 17-3a6, 17-3a7, 17-3a8, 17-3a9, 17-3a10, 17-3b, 17-3b1, 17-3b2, 17-3c, 17-3d, 17-4, 17-4a, 17-4b, 17-4c, 17-4c1, 17-4d, 17-4d1, 17-4d2, 17-5, 17-5a, 17-5b, 17-5c, 17-5d, 17-5d1, 17-5e, 17-5e1, 17-5f, 17-5f1, 17-5f2, 17-5f3, 17-5f4, 17-5f5, 17-5f6, 17-5g, 17-5g1, 17-5g2, 17-5g3, 17-5g4, 17-5g5, 17-5g6, 17-5g7, 17-5g8, 17-5g9, 17-5g10, 17-5h, 17-5h1, 17-5h2, or 17-5i, or 17-5j wherein the heterocycloalkyl group (ring A of Formula I) is morpholinyl optionally substituted with one or more groups that are independently halogen, C₁-C₆ alkyl, C₁-C₆ alkoxy, haloalkyl, haloalkoxy, hydroxyl, CN, aryloxy, -SO₂-(C₁-C₆ alkyl), -NR'R", C₁-C₆ alkanoyl, pyridyl, phenyl, or -SO₂-NR'R", where each R' and R" is independently hydrogen or C₁-C₆ alkyl.

In still another aspect, the invention provides compounds of formula 17-6a, i.e., compounds of formula 17-6 where the morpholinyl group is not attached to the sulfur of the SO₂ group via the ring nitrogen.

In still another aspect, the invention provides compounds of formula 17-6b, i.e., compounds of formula 17-6 where the morpholinyl group is attached to the sulfur of the SO₂ group via the ring nitrogen.

In yet another aspect, the invention provides compounds of formula 17-7, i.e., compounds of formulas 15, 16, 16-1, 16-2, 16-3, 16-4, 16-5, 16-6, 16-6a, 16-6b, 16-6c, 16-6d, 16-7, 16-8, 16-9, 16-10, 16-11, 16-11a, 16-11b, 16-11c, 16-12, 16-13, 16-14, or 16-15, 17, 17-1, 17-1a, 17-2, 17-2a, 17-2b, 17-2c, 17-2d, 17-2e, 17-2f, 17-2g, 17-2h, 17-2i, 17-3, 17-3a, 17-3a1, 17-3a2, 17-3a3, 17-3a4, 17-3a5, 17-3a6, 17-3a7, 17-3a8, 17-3a9, 17-3a10, 17-3b, 17-3b1, 17-3b2, 17-3c, 17-3d, 17-4, 17-4a, 17-4b, 17-4c, 17-4c1, 17-4d, 17-4d1, 17-4d2, 17-5, 17-5a, 17-5b, 17-5c, 17-5d, 17-5d1, 17-5e, 17-5e1, 17-5f, 17-5f1, 17-5f2, 17-5f3, 17-5f4, 17-5f5, 17-5f6, 17-5g, 17-5g1, 17-5g2, 17-5g3, 17-5g4, 17-5g5, 17-5g6, 17-5g7, 17-5g8, 17-5g9, 17-5g10, 17-5h, 17-5h1, 17-5h2, or 17-5i, or 17-5j wherein the heterocycloalkyl group (ring A of Formula I) is thiomorpholinyl optionally substituted with one or more groups that are independently halogen, C₁-C₆ alkyl, C₁-C₆ alkoxy, haloalkyl, haloalkoxy, hydroxyl, CN, aryloxy, -SO₂-(C₁-C₆ alkyl), -NR'R", C₁-C₆ alkanoyl, pyridyl, phenyl, or -SO₂-NR'R", where each R' and R" is independently hydrogen or C₁-C₆ alkyl.

In still another aspect, the invention provides compounds of formula 17-7a, i.e., compounds of formula 17-7 where the thiomorpholinyl group is not attached to the sulfur of the SO₂ group via the ring nitrogen.

In still another aspect, the invention provides compounds of formula 17-7b, i.e., compounds of formula 17-7 where the thiomorpholinyl group is attached to the sulfur of the SO₂ group via the ring nitrogen.

In yet another aspect, the invention provides compounds of formula 17-8, i.e., compounds of formulas 15, 16, 16-1, 16-2, 16-3, 16-4, 16-5, 16-6, 16-6a, 16-6b, 16-6c, 16-6d, 16-7, 16-8, 16-9, 16-10, 16-11, 16-11a, 16-11b, 16-11c, 16-12, 16-13, 16-14, or 16-15, 17, 17-1, 17-1a, 17-2, 17-2a, 17-2b, 17-2c, 17-2d, 17-2e, 17-2f, 17-2g, 17-2h, 17-2i, 17-3, 17-3a, 17-3a1, 17-3a2, 17-3a3, 17-3a4, 17-3a5, 17-3a6, 17-3a7, 17-3a8, 17-3a9, 17-3a10, 17-3b, 17-3b1, 17-3b2, 17-3c, 17-3d, 17-4, 17-4a, 17-4b, 17-4c, 17-4c1, 17-4d, 17-4d1, 17-4d2, 17-5, 17-5a, 17-5b, 17-5c, 17-5d, 17-5d1, 17-5e, 17-5e1, 17-5f, 17-5f1, 17-5f2, 17-5f3, 17-5f4, 17-5f5, 17-5f6, 17-5g, 17-5g1, 17-5g2, 17-5g3, 17-5g4, 17-5g5, 17-5g6, 17-5g7, 17-5g8, 17-5g9, 17-5g10, 17-5h, 17-5h1, 17-5h2, or 17-5i, or 17-5j , wherein the heterocycloalkyl group (ring A of Formula I) is thiomorpholinyl S,S-dioxide optionally substituted with one or more groups that are independently halogen, C₁-C₆ alkyl, C₁-C₆ alkoxy, haloalkyl, haloalkoxy, hydroxyl, CN, aryloxy, -SO₂-(C₁-C₆ alkyl), -NR'R", C₁-C₆ alkanoyl, pyridyl, phenyl, or -SO₂-NR'R", where each R' and R" is independently hydrogen or C₁-C₆ alkyl.

In still another aspect, the invention provides compounds of formula 17-8a, i.e., compounds of formula 17-8 where the thiomorpholinyl S,S-dioxide group is not attached to the sulfur of the SO₂ group via the ring nitrogen.

In still another aspect, the invention provides compounds of formula 17-8b, i.e., compounds of formula 17-8 where the thiomorpholinyl S,S-dioxide group is attached to the sulfur of the SO₂ group via the ring nitrogen.

In yet another aspect, the invention provides compounds of formula 17-9, i.e., compounds of formulas 15, 16, 16-1, 16-2, 16-3, 16-4, 16-5, 16-6, 16-6a, 16-6b, 16-6c, 16-6d, 16-7, 16-8, 16-9, 16-10, 16-11, 16-11a, 16-11b, 16-11c, 16-12, 16-13, 16-14, or 16-15, 17, 17-1, 17-1a, 17-2, 17-2a, 17-2b, 17-2c, 17-2d, 17-2e, 17-2f, 17-2g, 17-2h, 17-2i, 17-3, 17-3a, 17-3a1, 17-3a2, 17-3a3, 17-3a4, 17-3a5, 17-3a6, 17-3a7, 17-3a8, 17-3a9, 17-3a10, 17-3b, 17-3b1, 17-3b2, 17-3c, 17-3d, 17-4, 17-4a, 17-4b, 17-4c, 17-4c1, 17-4d, 17-4d1, 17-4d2, 17-5, 17-5a, 17-5b, 17-5c, 17-5d, 17-5d1, 17-5e, 17-5e1, 17-5f, 17-5f1, 17-5f2, 17-5f3, 17-5f4, 17-5f5, 17-5f6, 17-5g, 17-5g1, 17-5g2, 17-5g3, 17-5g4, 17-5g5, 17-5g6, 17-5g7, 17-5g8, 17-5g9, 17-5g10, 17-5h, 17-5h1, 17-5h2, or 17-5i, or 17-5j, wherein the heterocycloalkyl group (ring A of Formula I) is piperidinyl optionally substituted with one or more groups that are independently halogen, C₁-C₆ alkyl, C₁-C₆ alkoxy, haloalkyl, haloalkoxy, hydroxyl, CN, aryloxy, -SO₂-(C₁-C₆ alkyl), -NR'R", C₁-C₆ alkanoyl, pyridyl, phenyl, or -SO₂-NR'R", where each R' and R" is independently hydrogen or C₁-C₆ alkyl.

In still another aspect, the invention provides compounds of formula 17-9a, i.e., compounds of formula 17-9 where the piperidinyl group is not attached to the sulfur of the SO₂ group via the ring nitrogen.

In still another aspect, the invention provides compounds of formula 17-9b, i.e., compounds of formula 17-9 where the piperidinyl group is attached to the sulfur of the SO₂ group via the ring nitrogen.

In yet another aspect, the invention provides compounds of formula 17-10, i.e., compounds of formulas 15, 16, 16-1, 16-2, 16-3, 16-4, 16-5, 16-6, 16-6a, 16-6b, 16-6c, 16-6d, 16-7, 16-8, 16-9, 16-10, 16-11, 16-11a, 16-11b, 16-11c, 16-12, 16-13, 16-14, or 16-15, 17, 17-1, 17-1a, 17-2, 17-2a, 17-2b, 17-2c, 17-2d, 17-2e, 17-2f, 17-2g, 17-2h, 17-2i, 17-3, 17-3a, 17-3a1, 17-3a2, 17-3a3, 17-3a4, 17-3a5, 17-3a6, 17-3a7, 17-3a8, 17-3a9, 17-3a10, 17-3b, 17-3b1, 17-3b2, 17-3c, 17-3d, 17-4, 17-4a, 17-4b, 17-4c, 17-4c1, 17-4d, 17-4d1, 17-4d2, 17-5, 17-5a, 17-5b, 17-5c, 17-5d, 17-5d1, 17-5e, 17-5e1, 17-5f, 17-5f1, 17-5f2, 17-5f3, 17-5f4, 17-5f5, 17-5f6, 17-5g, 17-5g1, 17-5g2, 17-5g3, 17-5g4, 17-5g5, 17-5g6, 17-5g7, 17-5g8, 17-5g9, 17-5g10, 17-5h, 17-5h1, 17-5h2, or 17-5i, or 17-5j wherein the heterocycloalkyl group (ring A of Formula I) is piperazinyl optionally substituted with one or more groups that are independently halogen, C₁-C₆ alkyl, C₁-C₆ alkoxy, haloalkyl, haloalkoxy, hydroxyl, CN, aryloxy, -SO₂-(C₁-C₆ alkyl), -NR'R", C₁-C₆ alkanoyl, pyridyl, phenyl, or -SO₂-NR'R", where each R' and R" is independently hydrogen or C₁-C₆ alkyl.

In still another aspect, the invention provides compounds of formula 17-10a, i.e., compounds of formula 17-10 where the piperazinyl group is not attached to the sulfur of the SO₂ group via the ring nitrogen.

In still another aspect, the invention provides compounds of formula 17-10b, i.e., compounds of formula 17-10 where the piperazinyl group is attached to the sulfur of the S0₂ group via the ring nitrogen.

In yet still another aspect, the invention provides compounds of formula 18, i.e., compounds according to any of the preceding formulas wherein, when R₂ is a cycloalkyl group, R₂ is cyclopropyl group.

In yet still another aspect, the invention provides compounds of formula 19, i.e., compounds according to any of the preceding formulas wherein, when R₂ is a cycloalkyl group, R₂ is cyclobutyl group.

In yet still another aspect, the invention provides compounds of formula 20, i.e., compounds according to any of the preceding formulas wherein, when R₂ is a cycloalkyl group, R₂ is cyclopentyl group.

In yet still another aspect, the invention provides compounds of formula 21 i.e., compounds according to any of the preceding formulas wherein, when R₂ is a cycloalkyl group, unless specifically identified as being other than cycloalkyl. In one embodiment, R₂ is cyclohexyl group. In a further embodiment, R₂ is cyclopentyl. In a still further embodiment, R₂ is cyclopropyl.

In another aspect, the invention provides compounds of formula 22, i.e., compounds according to any of the formulas 18, 19, 20, or 21 wherein R₁ is hydrogen, unless specifically identified as being other than hydrogen.

In another aspect, the invention provides compounds that are:
5-[(4-chlorophenyl)sulfonyl]-4,5-dihydro-1*H*-pyrazolo[4,3-*c*]quinoline;
5-[(4-chlorophenyl)sulfonyl]-8-fluoro-4,5-dihydro-2*H*-pyrazolo[4,3-*c*]quinoline;
5-[(4-chlorophenyl)sulfonyl]-7-fluoro-4,5-dihydro-2*H*-pyrazolo[4,3-*c*]quinoline;
5-[(4-chlorophenyl)sulfonyl]-9-fluoro-4,5-dihydro-2*H*-pyrazolo[4,3-*c*]quinoline;
5-[(4-chlorophenyl)sulfonyl]-4-methyl-4,5-dihydro-2*H*-pyrazolo[4,3-*c*]quinoline;
5-[(4-chlorophenyl)sulfonyl]-7-methoxy-4-methyl-4,5-dihydro-1*H*-pyrazolo[4,3-*c*]quinoline;
5-[(4-chlorophenyl)sulfonyl]-9-methoxy-4-methyl-4,5-dihydro-1*H*-pyrazolo[4,3-*c*]quinoline;
5-[(4-chlorophenyl)sulfonyl]-3-methyl-4,5-dihydro-1*H*-pyrazolo[4,3-*c*]quinoline;
6-(4-chlorophenylsulfonyl)-4-methoxy-5,6-dihydropyrimido[5,4-*c*]quinolin-2-amine;
5-[(4-chlorophenyl)sulfonyl]-3-(difluoromethyl)-4,5-dihydro-1*H*-pyrazolo[4,3-*c*]quinoline;
5-(4-Chloro-benzenesulfonyl)-8-fluoro-4,5-dihydro-isoxazolo[4,5-*c*]quinoline;
6-(4-chlorophenylsulfonyl)-5,6-dihydrobenzo[*f*][1,7]naphthyridine;
ethyl 5-[(4-chlorophenyl)-sulfonyl]-4,5-dihydro-1*H*-pyrazolo[4,3-*c*]quinoline-3-carboxylate;
ethyl 5-[(4-chlorophenyl)-sulfonyl]-8-fluoro-4,5-dihydro-1*H*-pyrazolo[4,3-*c*]quinoline-3-carboxylate;
5-[(4-chlorophenyl)sulfonyl]-3-(methylthio)-4,5-dihydro-1*H*-pyrazolo[4,3-*c*] quinoline;
5-[(4-chlorophenyl)sulfonyl]-3-(methylsulfonyl)-4,5-dihydro-1*H*-pyrazolo[4,3-*c*] quinoline;
8-chloro-5-[(4-chlorophenyl)sulfonyl]-4,5-dihydro-2*H*-pyrazolo[4,3-*c*]quinoline;
4-methyl-5-(pyridin-2-ylsulfonyl)-4,5-dihydro-2*H*-pyrazolo[4,3-*c*]quinoline;
5-[(4-chlorophenyl)sulfonyl]-4,5-dihydro-1*H*-pyrazolo[4,3-*c*]quinolin-3-ol;
{5-[(4-chlorophenyl)sulfonyl]-8-fluoro-4,5-dihydro-1*H*-pyrazolo[4,3-*c*]quinolin-3-yl}methanol;
5-[(4-chlorophenyl)sulfonyl]-7,9-dimethoxy-4-methyl-4,5-dihydro-1*H*-pyrazolo[4,3-*c*] quinoline;
5-[(4-chlorophenyl)sulfonyl]-8-fluoro-4,5-dihydro-1*H*-pyrazolo[4,3-*c*]quinoline-3-carboxamide;
5-[(4-chlorophenyl)sulfonyl]-8-fluoro-4,5-dihydro-1*H*-pyrazolo[4,3-*c*]quinoline-3-carboxylic acid;
6-(4-chlorophenylsulfonyl)-5-ethyl-5,6-dihydropyrazolo[1,5-*c*]quinazoline;
5-[(4-chlorophenyl)sulfonyl]-8-fluoro-4,5-dihydro-1*H*-pyrazolo[4,3-*c*]quinoline-3-carbonitrile;
5-[(4-chlorophenyl)sulfonyl]-4-methyl-4,5-dihydro-2*H*-pyrazolo[4,3-*c*]quinoline; (resolved enantiomer, with a retention time of about 9.3 min* ;)
5-[(4-chlorophenyl)sulfonyl]-4-methyl-4,5-dihydro-2*H*-pyrazolo[4,3-*c*]quinoline (resolved enantiomer, with a retention time of about 20.5 min* ;)
5-[(4-chlorophenyl)sulfonyl]-8-fluoro-4-methyl-4,5-dihydro-2*H*-pyrazolo[4,3-*c*]quinoline (racemate);
5-[(4-chlorophenyl)sulfonyl]-8-fluoro-4-methyl-4,5-dihydro-2*H*-pyrazolo[4,3-*c*]quinoline (resolved enantiomer, with a retention time of about 9.8 min*;)
5-[(4-chlorophenyl)sulfonyl]-8-fluoro-4-methyl-4,5-dihydro-2*H*-pyrazolo[4,3-*c*]quinoline (resolved enantiomer, with a retention time of about 27.1 min*;)
5-[(4-chlorophenyl)sulfonyl]-8-fluoro-4,4-dimethyl-4,5-dihydro-2*H*-pyrazolo[4,3-*c*]quinoline;
8-fluoro-4-methyl-5-(pyridin-3-ylsulfonyl)-4,5-dihydro-1*H*-pyrazolo[4,3-*c*]quinoline (racemate);
8-fluoro-4-methyl-5-(pyridin-2-ylsulfonyl)-4,5-dihydro-1*H*-pyrazolo[4,3-*c*]quinoline;
4-methyl-5-(pyridin-3-ylsulfonyl)-4,5-dihydro-1*H*-pyrazolo[4,3-c]quinoline;
8-fluoro-4-methyl-5-(pyridin-3-ylsulfonyl)-4,5-dihydro-1*H*-pyrazolo[4,3-*c*]quinoline (resolved enantiomer, with a retention time of about 15.2 min* ;)
8-fluoro-4-methyl-5-(pyridin-3-ylsulfonyl)-4,5-dihydro-1*H*-pyrazolo[4,3-*c*]quinoline (resolved enantiomer, with a retention time of about 17.2 min* ;)
5-[(4-chlorophenyl)sulfonyl]-1,5-dihydro-4*H*-pyrazolo[4,3-*c*]quinolin-4-one;
5-[(4-chlorophenyl)sulfonyl]-4-methyl-4,5-dihydro-1*H*-pyrazolo[4,3-*c*]-1,5-naphthyridine;
8-fluoro-4-methyl-5-(pyridin-2-ylsulfonyl)-4,5-dihydro-1*H*-pyrazolo[4,3-c]quinoline; (resolved enantiomer, with a retention time of about 16.3 min*;)
5-(4-chlorophenylsulfonyl)-4-ethyl-4,5-dihydro-1*H*-pyrazolo[4,3-c]quinoline; (resolved enantiomer, with a retention time of about 21.4 min* ;)
5-(4-chlorophenylsulfonyl)-4-methyl-4,5-dihydro-1*H*-pyrazolo[4,3-c]quinolin-7-ol;
5-(4-chlorophenylsulfonyl)-8-fluoro-1*H*-pyrazolo[4,3-c]quinolin-4(5*H*)-one;
5-(4-chlorophenylsulfonyl)-4-methyl-4,5,5a,6,7,8,9,9a-octahydro-1*H*-pyrazolo[4,3-c]quinoline;
5-(4-chlorophenylsulfonyl)-9-fluoro-4-methyl-4,5-dihydro-2*H*-pyrazolo[4,3-c]quinoline;
5-(4-chlorophenylsulfonyl)-7-fluoro-4-methyl-4,5-dihydro-2*H*-pyrazolo[4,3-c]quinoline;
8-fluoro-5-(4-fluorophenylsulfonyl)-4-methyl-4,5-dihydro-1*H*-pyrazolo[4,3-c]quinoline;
4-methyl-5-(pyridin-2-ylsulfonyl)-4,5-dihydro-1*H*-pyrazolo[4,3-c]quinoline; (racemate and resolved enantiomer, with a retention time of about 7.9 min* .)5-(4-chlorophenylsulfonyl)-8-fluoro-4-deutero-4-methyl-4,5-dihydro-1*H*-pyrazolo[4,3-c]quinoline;
9-fluoro-6-(4-fluorophenylsulfonyl)-5-methyl-5,6-dihydropyrimido[5,4-c]quinolin-2-amine;
5-(4-chlorophenylsulfonyl)-4-cyclopropyl-4,5-dihydro-2*H*-pyrazolo[4,3-c]quinoline;
5-(4-chlorophenylsulfonyl)-4-(trifluoromethyl)-4,5-dihydro-2*H*-pyrazolo[4,3-c]quinoline;
5-(4-chlorophenylsulfonyl)-4-methyl-4,5-dihydro-1*H*-pyrazolo[4,3-c]quinolin-7-ol; (resolved enantiomer, with a retention time of about 13.4 min* ;)5-(4-chlorophenylsulfonyl)-4-methyl-4,5-dihydro-1*H*-pyrazolo[4,3-c]quinolin-7-ol; (resolved enantiomer, with a retention time of about 17.0 min*;)5-(4-chlorophenylsulfonyl)-4-isopropyl-4,5-dihydro-2*H-*pyrazolo[4,3-c]quinoline;
1,5-bis(4-chlorophenylsulfonyl)-1*H*-pyrazolo[4,3-c]quinolin-4(5*H*)-one;
5-(4-chlorophenylsulfonyl)-4,5-dihydro-1*H*-imidazo[1,2-a]pyrazolo[4,3-e]pyrimidine;
8-fluoro-5-(4-fluorophenylsulfonyl)-4-methyl-4,5-dihydro-1*H*-pyrazolo[4,3-c]quinoline; (resolved enantiomer, with a retention time of about 7.6 min* ;)
8-fluoro-5-(4-fluorophenylsulfonyl)-4-methyl-4,5-dihydro-1*H*-pyrazolo[4,3-c]quinoline; (resolved enantiomer, with a retention time of about 10.1 min*;)
5'-(4-chlorophenylsulfonyl)-2',5'-dihydrospiro[cyclopropane-1,4'-pyrazolo[4,3-c] quinoline] ;
5-(4-chlorophenylsulfonyl)-7,8-difluoro-4-methyl-4,5-dihydro-2*H*-pyrazolo[4,3-c]quinoline;
7,8-difluoro-4-methyl-5-(thiophen-2-ylsulfonyl)-4,5-dihydro-2*H*-pyrazolo[4,3-c]quinoline;
5-(4-chlorophenylsulfonyl)-4-methyl-4,5-dihydro-2*H*-pyrazolo[4,3-c][1,8]naphthyridine;
5-(4-chlorophenylsulfonyl)-4-ethyl-4,5-dihydro-1*H*-pyrazolo[4,3-c]quinoline; (resolved enantiomer, with a retention time of about 6.0 min* ;)
5-(4-chlorophenylsulfonyl)-4-ethyl-4,5-dihydro-1*H*-pyrazolo[4,3-c]quinoline; (resolved enantiomer, with a retention time of about 9.3 min* ;)
5-(5-chlorothiophen-2-ylsulfonyl)-7,8-difluoro-4-methyl-4,5-dihydro-1*H-*pyrazolo[4,3-c]quinoline;
5-(4-chlorophenylsulfonyl)-4-methyl-4,5-dihydro-1*H*-pyrazolo[4,3-c]quinolin-7-yl dimethylcarbamate;
4-cyclopropyl-7,8-difluoro-5-(4-(trifluoromethyl)phenylsulfonyl)-4,5-dihydro-1*H-*pyrazolo[4,3-c]quinoline; (resolved enantiomer, with a retention time of about 6.0 min* ;)
4-cyclopropyl-7,8-difluoro-5-(4-(trifluoromethyl)phenylsulfonyl)-4,5-dihydro-1*H-*pyrazolo[4,3-c]quinoline; (resolved enantiomer, with a retention time of about 9.3 min*;)
4-cyclopropyl-7,8-difluoro-5-(4-(trifluoromethyl)phenylsulfonyl)-4,5-dihydro-1*H-*pyrazolo[4,3-c]quinoline; (racemate;)
7-chloro-4-methyl-5-(4-(trifluoromethyl)phenylsulfonyl)-4,5-dihydro-1*H-*pyrazolo[3,4-d]thieno[2,3-b]pyridine;
5-(4-chlorophenylsulfonyl)-4-cyclopropyl-4,5-dihydro-1*H*-imidazo[1,2-a]pyrazolo[4,3-e]pyrimidine;
4-ethyl-7,8-difluoro-5-(5-(trifluoromethyl)pyridin-2-ylsulfonyl)-4,5-dihydro-1*H-*pyrazolo[4,3-c]quinoline;
7,8-difluoro-4-methyl-5-(4-(trifluoromethyl)phenylsulfonyl)-4,5-dihydro-1*H-*pyrazolo[4,3-c]quinoline; (resolved enantiomer, with a retention time of about 7.0 min* ;)
7,8-difluoro-4-methyl-5-(4-(trifluoromethyl)phenylsulfonyl)-4,5-dihydro-1*H-*pyrazolo[4,3-c]quinoline; (resolved enantiomer, with a retention time of about 9.5 min*;)
4-(4-cyclopropyl-1*H*-pyrazolo[4,3-c]quinolin-5(4*H*)-ylsulfonyl)benzonitrile;
5-(4-chlorophenylsulfonyl)-4-methyl-4,5-dihydro-2*H*-pyrazolo[4,3-c] [1,7]naphthyridine;
5'-(4-(trifluoromethyl)phenylsulfonyl)-1',5'-dihydrospiro[cyclopropane-1,4'-pyrazolo[4,3-c]quinoline];
5-(4-chlorophenylsulfonyl)-4-methyl-4,5-dihydro-1*H*-imidazo[1,2-a]pyrazolo[4,3-e]pyrimidine;
4-cyclopropyl-7,8-difluoro-5-(5-(trifluoromethyl)pyridin-2-ylsulfonyl)-4,5-dihydro-1*H*-pyrazolo[4,3-c]quinoline;
5-(5-chlorothiophen-2-ylsulfonyl)-4-cyclopropyl-7,8-difluoro-4,5-dihydro-2*H-*pyrazolo[4,3-c]quinoline;
4-cyclopropyl-7,8-difluoro-5-(4-fluorophenylsulfonyl)-4,5-dihydro-2*H*-pyrazolo[4,3-c]quinoline;
4-cyclopropyl-5-(4-(trifluoromethyl)phenylsulfonyl)-4,5-dihydro-2*H*-pyrazolo[4,3-c][1,8]naphthyridine;
4-cyclopropyl-5-(4-fluorophenylsulfonyl)-4,5-dihydro-2*H*-pyrazolo[4,3-c][1,8]naphthyridine;
4-cyclopropyl-5-(4-fluorophenylsulfonyl)-4,5-dihydro-2*H*-pyrazolo[4,3-c]quinoline;
4-cyclopropyl-7,8-difluoro-1-(methoxymethyl)-5-(6-(trifluoromethyl)pyridin-3-ylsulfonyl)-4,5-dihydro-1*H*-pyrazolo[4,3-c]quinoline;
4-cyclopropyl-7,8-difluoro-5-(thiophen-2-ylsulfonyl)-4,5-dihydro-2*H*-pyrazolo[4,3-c]quinoline;
4-cyclopropyl-5-(4-(trifluoromethyl)phenylsulfonyl)-4,5-dihydro-1*H*-imidazo[1,2-a]pyrazolo[4,3-e]pyrimidine;
4-cyclopropyl-7,8-difluoro-5-(6-(trifluoromethyl)pyridin-3-ylsulfonyl)-4,5-dihydro-1*H*-pyrazolo[4,3-c]quinoline;
5-(4-fluorophenylsulfonyl)-4-(trifluoromethyl)-4,5-dihydro-2*H*-pyrazolo[4,3-c][1,8]naphthyridine;
7,8-difluoro-4-(pyridin-3-yl)-5-(4-(trifluoromethyl)phenylsulfonyl)-4,5-dihydro-1*H-*pyrazolo[4,3-c]quinoline;
4-cyclopropyl-8-fluoro-5-(4-(trifluoromethyl)phenylsulfonyl)-4,5-dihydro-1*H-*pyrazolo[4,3-c]quinoline; (racemate and resolved enantiomers, with a retention time of about 8.0 min* and 12.6;)
5-(4-chlorophenylsulfonyl)-4-methyl-4,5-dihydro-2*H*-[1,3]dioxolo[4,5-g]pyrazolo[4,3-c]quinoline;
5-(5-chloropyridin-2-ylsulfonyl)-4-cyclopropyl-7,8-difluoro-4,5-dihydro-1*H-*pyrazolo[4,3-c]quinoline;
5-(5-chlorothiophen-2-ylsulfonyl)-4-cyclopropyl-7,8-difluoro-4,5-dihydro-2*H-*pyrazolo[4,3-c]quinoline; (resolved enantiomer, with a retention time of about 10.3 min* ;)
5-(5-chlorothiophen-2-ylsulfonyl)-4-cyclopropyl-7,8-difluoro-4,5-dihydro-2*H-*pyrazolo[4,3-c]quinoline; (resolved enantiomer, with a retention time of about 16.1 min*;)
4-cyclopropyl-7,8-difluoro-5-(4-fluorophenylsulfonyl)-4,5-dihydro-2*H*-pyrazolo[4,3-c]quinoline; (resolved enantiomer, with a retention time of about 16.9 min* ;)
4-cyclopropyl-7,8-difluoro-5-(4-fluorophenylsulfonyl)-4,5-dihydro-2*H*-pyrazolo[4,3-c]quinoline; (resolved enantiomer, with a retention time of about 19.7 min* ;)
4-cyclopropyl-8-fluoro-5-(6-(trifluoromethyl)pyridin-3-ylsulfonyl)-4,5-dihydro-2*H-*pyrazolo[4,3-c]quinoline; (Enantiomer A)
4-cyclopropyl-8-fluoro-5-(6-(trifluoromethyl)pyridin-3-ylsulfonyl)-4,5-dihydro-2*H-*pyrazolo[4,3-c]quinoline; (Enantiomer B)
4-cyclopropyl-7,8-difluoro-5-(6-(trifluoromethyl)pyridin-3-ylsulfonyl)-4,5-dihydro-1*H*-pyrazolo[4,3-c]quinoline; (resolved enantiomer, with a retention time of about 17.3 min* ;)
4-cyclopropyl-7,8-difluoro-5-(6-(trifluoromethyl)pyridin-3-ylsulfonyl)-4,5-dihydro-1*H*-pyrazolo[4,3-c]quinoline;(resolved enantiomer, with a retention time of about 13.0 min* ;)
7,8-difluoro-4-isopropyl-5-(4-(trifluoromethyl)phenylsulfonyl)-4,5-dihydro-2*H-*pyrazolo[4,3-c]quinoline;
4-cyclopropyl-7,8-difluoro-5-(4-(trifluoromethoxy)phenylsulfonyl)-4,5-dihydro-1*H-*pyrazolo[4,3-c]quinoline;
7,8-difluoro-5-(4-(trifluoromethyl)phenylsulfonyl)-4,5-dihydro-2*H*-pyrazolo[4,3-c]quinoline;
5-(4-chlorophenylsulfonyl)-4-methyl-4,5-dihydro-1*H*-pyrazolo[4,3-c]quinoline-7,8-diol;
5-(5-chloropyridin-2-ylsulfonyl)-4-cyclopropyl-8-fluoro-4,5-dihydro-1*H-*pyrazolo[4,3-c]quinoline;
4-cyclopropyl-5-(6-(trifluoromethyl)pyridin-3-ylsulfonyl)-4,5-dihydro-1*H-*pyrazolo[4,3-c]quinoline;
(R)-4-cyclopropyl-7-(trifluoromethoxy)-5-(6-(trifluoromethyl)pyridin-3-ylsulfonyl)-4,5-dihydro-2*H*-pyrazolo[4,3-c]quinoline;
4-cyclopropyl-5-(4-(trifluoromethyl)phenylsulfonyl)-4,5-dihydro-2*H*-pyrazolo[4,3-c][1,7]naphthyridine 7-oxide;
5-(4-chlorophenylsulfonyl)-4-methyl-4,5-dihydro-2*H*-pyrazolo[4,3-c]quinolin-8-ol;
4-(8-fluoro-4-methyl-1H-pyrazolo[4,3-c]quinolin-5(4*H*)-ylsulfonyl)-3,5-dimethylisoxazole;
8-fluoro-4-methyl-5-(1-methyl-1*H*-pyrazol-4-ylsulfonyl)-4,5-dihydro-1*H-*pyrazolo[4,3-c]quinoline;
8-fluoro-4-methyl-5-(5-(pyridin-2-yl)thiophen-2-ylsulfonyl)-4,5-dihydro-1*H-*pyrazolo[4,3-c]quinoline;
(*R*)-2-tert-butyl-4-cyclopropyl-7,8-difluoro-5-(4-(trifluoromethyl)phenylsulfonyl)-4,5-dihydro-2*H*-pyrazolo[4,3-c]quinoline;
(*R*)-1-tert-butyl-4-cyclopropyl-7,8-difluoro-5-(4-(trifluoromethyl)phenylsulfonyl)-4,5-dihydro-1*H*-pyrazolo [4,3-c] quinoline;
(*R*)-4-cyclopropyl-7-(trifluoromethyl)-5-(6-(trifluoromethyl)pyridin-3-ylsulfonyl)-4,5-dihydro-1*H*-pyrazolo[4,3-c]quinoline;
(*R*)-4-cyclopropyl-7-(trifluoromethoxy)-5-(4-(trifluoromethoxy)phenylsulfonyl)-4,5-dihydro-2*H*-pyrazolo[4,3-c]quinoline;
(*R*)-4-(4-cyclopropyl-7,8-difluoro-1*H*-pyrazolo[4,3-c]quinolin-5(4*H*)-ylsulfonyl)aniline;
(*R*)-4-cyclopropyl-7,8-difluoro-5-(4-nitrophenylsulfonyl)-4,5-dihydro-1*H-*pyrazolo[4,3-c]quinoline; (*R*)-4-cyclopropyl-8-(trifluoromethyl)-5-(6-(trifluoromethyl)pyridin-3-ylsulfonyl)-4,5-dihydro-1*H*-pyrazolo[4,3-c]quinoline;
5-chloro-2-(4-cyclopropyl-7,8-difluoro-1*H*-pyrazolo[4,3-c]quinolin-5(4*H*)-ylsulfonyl)thiazole;
(*R*)-4-cyclopropyl-5-(4-(difluoromethoxy)phenylsulfonyl)-7,8-difluoro-4,5-dihydro-1*H*-pyrazolo[4,3-c]quinoline;
4-cyclopropyl-5-(4-(trifluoromethyl)phenylsulfonyl)-4,5-dihydro-2*H*-pyrazolo[4,3-c][1,5]naphthyridine;
4-cyclopropyl-8-fluoro-5-(5-(trifluoromethyl)pyridin-2-ylsulfonyl)-4,5-dihydro-1*H-*pyrazolo[4,3-c]quinoline;
4-cyclopropyl-8-fluoro-5-(5-(trifluoromethyl)pyridin-2-ylsulfonyl)-4,5-dihydro-1*H-*pyrazolo[4,3-c]quinoline;
4-cyclopropyl-5-(4-(difluoromethoxy)phenylsulfonyl)-8-fluoro-4,5-dihydro-1*H-*pyrazolo[4,3-c]quinoline;
4-cyclopropyl-7,8-difluoro-5-(5-(trifluoromethyl)pyridin-2-ylsulfonyl)-4,5-dihydro-1*H*-pyrazolo[4,3-c]quinoline;
5-(4-chlorophenylsulfonyl)-4-methyl-4,5-dihydro-1*H*-pyrazolo[4,3-c]quinoline-7,8-diol;
8-fluoro-4-methyl-5-(1-methyl-1H-imidazol-4-ylsulfonyl)-4,5-dihydro-1*H-*pyrazolo[4,3-c]quinoline;
(*R*)-1-tert-butyl-4-ethyl-7,8-difluoro-5-(4-(trifluoromethyl)phenylsulfonyl)-4,5-dihydro-1*H*-pyrazolo[4,3-c]quinoline;
(*R*)-4-ethyl-7,8-difluoro-5-(4-(trifluoromethyl)phenylsulfonyl)-4,5-dihydro-1*H-*pyrazolo[4,3-c]quinoline;
4-(4-(trifluoromethyl)phenylsulfonyl)-4,5,8,8b-tetrahydro-3a*H*-isothiazolo[4,5-b]pyrazolo[3,4-d]pyridine; and
7-methyl-5-(4-(trifluoromethyl)phenylsulfonyl)-4,5,5a,7-tetrahydro-1*H*-oxazolo[5,4-b]pyrazolo[3,4-d]pyridine;
(R)-4-cyclopropyl-8-fluoro-5-(4-(trifluoromethoxy)phenylsulfonyl)-4,5-dihydro-1*H*-pyrazolo[4,3-c]quinoline
4-cyclopropyl-7-methyl-5-(4-(trifluoromethyl)phenylsulfonyl)-4,5-dihydro-1*H-*pyrazolo[3,4-d]thiazolo[5,4-b]pyridine
(R)-4-cyclopropyl-8-fluoro-5-(4-methoxyphenylsulfonyl)-4,5-dihydro-1*H-*pyrazolo[4,3-c]quinoline;
and stereoisomers, tautomers, mixtures of stereoisomers and/or tautomers, or pharmaceutically acceptable salts thereof.

In another aspect, the invention provides a method of treating Alzheimer's disease comprising administering a therapeutically effective amount of a compound or salt of formula I, where formula I also encompasses:
5-tosyl-4,5-dihydro-2*H*-pyrazolo[4,3-c]quinoline,
2-phenyl-5-tosyl-4,5-dihydro-2*H*-pyrazolo[4,3-c]quinoline,
7-methoxy-2-phenyl-5-tosyl-4,5-dihydro-2*H*-pyrazolo[4,3-c]quinoline,
8-methoxy-5-tosyl-4,5-dihydro-2*H*-pyrazolo[4,3-c]quinoline,
8-methoxy-2-phenyl-5-tosyl-4,5-dihydro-2*H*-pyrazolo[4,3-c]quinoline,
3-(methylthio)-5-tosyl-4,5-dihydro-2*H*-pyrazolo[4,3-c]quinoline,
7-methoxy-5-tosyl-4,5-dihydro-2*H*-pyrazolo[4,3-c]quinoline,
5-tosyl-4,5-dihydro-2*H*-pyrazolo[4,3-c]quinoline,
ethyl 1-(4-sulfamoylphenyl)-5-tosyl-4,5-dihydro-1*H*-pyrazolo[4,3-c]quinoline-3-carboxylate, 1-(4-sulfamoylphenyl)-5-tosyl-4,5-dihydro-1*H*-pyrazolo[4,3-c]quinoline-3-carboxamide, and ethyl 1-methyl-5-tosyl-4,5-dihydro-1*H*-pyrazolo[4,3-c]quinoline-3-carboxylate;
to a patient in need of such treatment.

In still another aspect, the invention provides a composition comprising a compound or salt of formula I, where formula I further comprises 3-(methylthio)-5-tosyl-4,5-dihydro-2*H*-pyrazolo[4,3-c]quinoline, 7-methoxy-5-tosyl-4,5-dihydro-2*H*-pyrazolo[4,3-c]quinofine, and 5-tosyl-4,5-dihydro-2*H*-pyrazolo[4,3-c]quinoline, and at least one pharmaceutically acceptable solvent, adjuvant, excipient, carrier, binder or disintegrant.

In still another aspect, the invention provides a method of treating Alzheimer's disease comprising administering a therapeutically effective amount of a compound or salt of formula I, where formula I also encompasses
5-tosyl-4,5-dihydro-2*H*-pyrazolo[4,3-c]quinoline,
2-phenyl-5-tosyl-4,5-dihydro-2*H*-pyrazolo[4,3-c]quinoline,
7-methoxy-2-phenyl-5-tosyl-4,5-dihydro-2*H*-pyrazolo[4,3-c]quinoline,
8-methoxy-5-tosyl-4,5-dihydro-2*H*-pyrazolo[4,3-c]quinoline,
8-methoxy-2-phenyl-5-tosyl-4,5-dihydro-2*H*-pyrazolo[4,3-c]quinoline,
3-(methylthio)-5-tosyl-4,5-dihydro-2*H*-pyrazolo[4,3-c]quinoline,
7-methoxy-5-tosyl-4,5-dihydro-2*H*-pyrazolo[4,3-c]quinoline,
5-tosyl-4,5-dihydro-2*H*-pyrazolo[4,3-c]quinoline,
ethyl 1-(4-sulfamoylphenyl)-5-tosyl-4,5-dihydro-1*H*-pyrazolo[4,3-c]quinoline-3-carboxylate, 1-(4-sulfamoylphenyl)-5-tosyl-4,5-dihydro-1*H*-pyrazolo[4,3-c]quinoline-3-carboxamide; and ethyl 1-methyl-5-tosyl-4,5-dihydro-1*H*-pyrazolo[4,3-c]quinoline-3-carboxylate
to a patient in need of such treatment.

In another aspect, the compounds of the invention have minimal interaction or preferably, no interaction with Notch.

### Definitions

The definitions and explanations below are for the terms as used throughout this entire document including both the specification and the claims.

It should be noted that, as used in this specification and the appended claims, the singular forms "a," "an," and "the" include plural referents unless the content clearly dictates otherwise. Thus, for example, reference to a composition containing "a compound" includes a mixture of two or more compounds. It should also be noted that the term "or" is generally employed in its sense including "and/or" unless the content clearly dictates otherwise.

Where multiple substituents are indicated as being attached to a structure, it is understood that the substituents can be the same or different. Thus for example "Rₘ optionally substituted with 1, 2 or 3 Rq groups" indicates that Rₘ is substituted with 1, 2, or 3 Rq groups where the Rq groups can be the same or different. It will be understood by those skilled in the art with respect to any group containing one or more substituents that such groups are not intended to introduce any substitution or substitution patterns that are sterically impractical and /or synthetically non-feasable.

APP, amyloid precursor protein, is defined as any APP polypeptide, including APP variants, mutations, and isoforms, for example, as disclosed in U.S. Patent No. 5,766,846. A beta, amyloid beta peptide, is defined as any peptide resulting from beta-secretase mediated cleavage of APP, including peptides of 39, 40, 41, 42, and 43 amino acids, and extending from the beta-secretase cleavage site to amino acids 39, 40, 41, 42, or 43.

Pharmaceutically acceptable refers to those properties and/or substances that are acceptable to the patient from a toxicological and/or safety point of view.

A therapeutically effective amount is defined as an amount effective to reduce or lessen at least one symptom of the disease being treated or to reduce or delay onset of one or more clinical markers or symptoms of the disease.

By "A beta-related disease" is meant diseases or disorders which are associated with extracellular accumulation of A beta (amyloid) in various organs and tissues of the body and includes diseases, disorders, conditions, pathologies, and other abnormalities of the structure or function of the brain, including components thereof, such as the stroma, including the vasculature or the parenchyma including functional or anatomical regions, or neurons themselves, in which the causative agent is A beta (amyloid). The 4 kDa beta-amyloid (A beta) protein, a 39-43 amino-acid protein, is a major component of cerebral and cerebrovascular deposits, such as plaques, found in, among others, Alzheimer's disease, cerebral amyloid angiopathy, mild cognitive impairment (MCI), and Down's syndrome and is derived from the proteolytic cleavage of a larger, membrane-bound precursor, the A beta precursor protein (APP) by several aspartyl proteases, one of which is gamma secretase.

By "alkanoyl" is meant an acyl radical Alk-C(O)-, wherein Alk is an alkyl radical as defined herein. Examples of alkanoyl include acetyl, propionyl, butyryl, isobutyryl, valeryl, isovaleryl, 2-methyl-butyryl, 2,2-dimethylpropionyl, valeryl, hexanoyl, heptanoyl, octanoyl and the like. By "C₁-C₆ alkanoyl" in the present invention is meant an acyl radical Alk-C(O)-, wherein Alk is a straight or branched chain alkyl group having 1-6 carbon atoms.

By "alkyl" and "C₁-C₆ alkyl" in the present invention is meant straight or branched chain alkyl groups having 1-6 carbon atoms, such as, methyl, ethyl, propyl, isopropyl, n-butyl, sec-butyl, tert-butyl, pentyl, 2-pentyl, isopentyl, neopentyl, hexyl, 2-hexyl, 3-hexyl, and 3-methylpentyl. It is understood that in cases where an alkyl chain of a substituent (e.g. of an alkyl, alkoxy or alkenyl group) is shorter or longer than 6 carbons, it will be so indicated in the second "C" as, for example, "C₁-C₁₀" indicates a maximum of 10 carbons. Alkyl groups may be mono or di-radicals. The meaning will be clear to one of skill in the art. The term also includes substituted alkyl groups, and refers to an alkyl group in which 1 or more hydrogen atoms is replaced by a substituent independently selected from the group: acyl, acyloxy, alkoxy, amino (wherein the amino group may be a cyclic amine), aryl, heteroaryl, heterocycoalkyl, carboxyl, oxo, amido, cyano, cycloalkyl, cycloalkenyl, halogen, hydroxyl, nitro, sulfamoyl, sulfanyl, sulfinyl, sulfonyl, and sulfonic acid.

By "alkylene" is meant a diradical alkyl group, whereby alkyl is as defined above.

By "alkoxy" and "C₁-C₆ alkoxy" in the present invention is meant straight or branched chain alkyl groups having 1-6 carbon atoms, attached through at least one divalent oxygen atom, such as, for example, methoxy, ethoxy, propoxy, isopropoxy, n-butoxy, sec-butoxy, tert-butoxy, pentoxy, isopentoxy, neopentoxy, hexoxy, and 3-methylpentoxy.

By the term "halogen" in the present invention is meant fluorine, bromine, chlorine, and/or iodine.

"Alkenyl" and "C₂-C₆ alkenyl" means straight and branched hydrocarbon radicals having from 2 to 6 carbon atoms and from one to three double bonds and includes, for example, ethenyl, propenyl, 1-but-3-enyl, 1-pent-3-enyl, 1-hex-5-enyl and the like. The term also includes substituted alkenyl groups, and refers to an alkenyl group in which 1 or more hydrogen atoms is replaced by a substituent independently selected from the group: acyl, acyloxy, alkoxy, amino (wherein the amino group may be a cyclic amine), aryl, heteroaryl, heterocycloalkyl, carboxyl, oxo, amido, cyano, cycloalkyl, cycloalkenyl, halogen, hydroxyl, nitro, sulfamoyl, sulfanyl, sulfinyl, sulfonyl, and sulfonic acid, for example 1*H*-pyrrol-2-ylmethylene.

"Alkynyl" and "C₂-C₆ alkynyl" means straight and branched hydrocarbon radicals having from 2 to 6 carbon atoms and one or two triple bonds and includes ethynyl, propynyl, butynyl, pentyn-2-yl and the like. The term also includes substituted alkynyl groups, and refers to an alkynyl group in which 1 or more hydrogen atoms is replaced by a substituent independently selected from the group: acyl, acyloxy, alkoxy, amino (wherein the amino group may be a cyclic amine), aryl, heteroaryl, heterocyclyl, carboxyl, oxo, amido, cyano, cycloalkyl, cycloalkenyl, halogen, hydroxyl, nitro, sulfamoyl, sulfanyl, sulfinyl, sulfonyl, and sulfonic acid.

As used herein, the term "cycloalkyl" refers to saturated carbocyclic radicals having three to twelve carbon atoms. The cycloalkyl can be monocyclic, a polycyclic fused system, or a bi or polycyclic bridged system, such as adamantyl or bicyclo[2.2.1] heptyl. Examples of such radicals include cyclopropyl, cyclobutyl, cyclopentyl and cyclohexyl. Preferred cycloalkyl groups are cyclopentyl, cyclohexyl, and cycloheptyl. The cycloalkyl groups herein are unsubstituted or, substituted in one or more substitutable positions with various groups. For example, such cycloalkyl groups may be optionally substituted with, for example, C₁-C₆ alkyl, C₁-C₆ alkoxy, halogen, hydroxyl, oxo, cyano, nitro, amino, mono(C₁-C₆)alkylamino, di(C₁-C₆)alkylamino, C₂-C₆alkenyl, C₂-C₆alkynyl, C₁-C₆ haloalkyl, C₁-C₆ haloalkoxy, amino(C₁-C₆)alkyl, mono(C₁-C₆)alkylamino(C₁-C₆)alkyl or di(C₁-C₆)alkylamino(C₁-C₆)alkyl.

By "aryl" is meant an aromatic carbocyclic group having a single ring (e.g., phenyl) or multiple condensed rings in which at least one is aromatic, (e.g., 1,2,3,4-tetrahydronaphthyl, naphthyl), which is optionally substituted at one or more substitutable positions. Preferred aryl groups of the present invention are phenyl, 1-naphthyl, 2-naphthyl, indanyl, indenyl, dihydronaphthyl, fluorenyl, tetralinyl or 6,7,8,9-tetrahydro-5H-benzo[a]cycloheptenyl. More preferred aryl groups include phenyl and naphthyl. The most preferred aryl group is phenyl. The aryl groups herein are unsubstituted or, substituted in one or more substitutable positions with various groups. For example, such aryl groups may be optionally substituted with, for example, C₁-C₆ alkyl, C₁-C₆ alkoxy, halogen, hydroxyl, cyano, nitro, amino, mono(C₁-C₆)alkylamino, di(C₁-C₆)alkylamino, C₂-C₆alkenyl, C₂-C₆alkynyl, C₁-C₆ haloalkyl, C₁-C₆ haloalkoxy, amino(C₁-C₆)alkyl, mono(C₁-C₆)alkylamino(C₁-C₆)alkyl or di(C₁-C₆)alkylamino(C₁-C₆)alkyl.

By "arylalkyl" or "aralkyl" is meant the group -alkylene-aryl, or -alkyl-aryl, wherein alkylene, alkyl, and aryl are defined herein.

By "aryloxy" is meant the group -O-aryl wherein the term aryl is as defined herein.

By "arylalkyloxy" "arylalkyloxy" or "aralkyloxy" is meant the group -O-C₁₋₄-alkylene-aryl, or -O-C₁₋₄-alkyl-aryl, wherein the terms aryl, alkyl, and alkylene are as defined herein. An example of arylalkyloxy is benzyloxy (or -O-CH₂-phenyl).

By the term "halogen" or "halo" in the present invention is meant fluorine, bromine, chlorine, and/or iodine.

By "haloalkyl" is meant an alkyl radical having the meaning as defined above wherein one or more hydrogens are replaced by a halogen. Examples of such haloalkyls include chloromethyl, 1-bromoethyl, fluoromethyl, difluoromethyl, trifluoromethyl, 1,1,1-trifluoroethyl and the like.

By "heteroaryl" is mean at least one or more aromatic ring systems of 5-, 6-, or 7-membered rings which includes fused ring systems of 9-11 atoms containing at least one and up to four heteroatoms selected from nitrogen, oxygen, or sulfur. A heteroaryl ring may be a mono-radical, or di-radical. For example, when the B-ring is pyrazolyl, the pyrazolyl ring is fused to the piperidine ring in the core, and the pyrazolyl ring is a diradical. The meaning will be apparent to one of ordinary skill in the art. Preferred heteroaryl groups of the present invention include pyridyl, pyrimidinyl, quinolinyl, benzothienyl, indolyl, indolinyl, pryidazinyl, pyrazinyl, isoindolyl, isoquinolyl, quinazolinyl, quinoxalinyl, phthalazinyl, imidazolyl, isoxazolyl, pyrazolyl, oxazolyl, thiazolyl, indolizinyl, indazolyl, benzothiazolyl, benzimidazolyl, benzofuranyl, furanyl, thienyl, pyrrolyl, oxadiazolyl, thiadiazolyl, triazolyl, tetrazolyl, isothiazolyl, naphthyridinyl, isochromanyl, chromanyl, tetrahydroisoquinolinyl, isoindolinyl, isobenzotetrahydrofuranyl, isobenzotetrahydrothienyl, isobenzothienyl, benzoxazolyl, pyridopyridyl, benzotetrahydrofuranyl, benzotetrahydrothienyl, purinyl, benzodioxolyl, triazinyl, pteridinyl, benzothiazolyl, imidazopyridyl, imidazothiazolyl, dihydrobenzisoxazinyl, benzisoxazinyl, benzoxazinyl, dihydrobenzisothiazinyl, benzopyranyl, benzothiopyranyl, chromonyl, chromanonyl, pyridyl-N-oxide, tetrahydroquinolinyl, dihydroquinolinyl, dihydroquinolinonyl, dihydroisoquinolinonyl, dihydrocoumarinyl, dihydroisocoumarinyl, isoindolinonyl, benzodioxanyl, benzoxazolinonyl, pyrrolyl N-oxide" pyrimidinyl N-oxide, pyridazinyl N-oxide, pyrazinyl N-oxide, quinolinyl N-oxide, indolyl N-oxide, indolinyl N-oxide, isoquinolyl N-oxide, quinazolinyl N-oxide, quinoxalinyl N-oxide, phthalazinyl N-oxide, imidazolyl N-oxide, isoxazolyl N-oxide, oxazolyl N-oxide, thiazolyl N-oxide, indolizinyl N-oxide, indazolyl N-oxide, benzothiazolyl N-oxide, benzimidazolyl N-oxide, pyrrolyl N-oxide, oxadiazolyl N-oxide, thiadiazolyl N-oxide, triazolyl N-oxide, tetrazolyl N-oxide, benzothiopyranyl S-oxide, benzothiopyranyl S,S-dioxide. Preferred heteroaryl groups include pyridyl, pyridazinyl, pyrimidyl, pyrazinyl, pyrrolyl, thiazolyl, imidazolyl, pyrazolyl, triazolyl, oxazolyl, isoxazolyl, thienyl, furanyl, quinolinyl, and isoquinolinyl. The heteroaryl groups herein are unsubstituted or substituted in one or more substitutable positions with various groups. For example, such heteroaryl groups may be optionally substituted with, for example, C₁-C₆ alkyl, C₁-C₆ alkoxy, halogen, hydroxyl, cyano, nitro, amino, mono(C₁-C₆)alkylamino, di(C₁-C₆)alkylamino, C₂-C₆alkenyl, C₂-C₆alkynyl, C₁-C₆ haloalkyl, C₁-C₆ haloalkoxy, amino(C₁-C₆)alkyl, mono(C₁-C₆)alkylamino(C₁-C₆)alkyl or di(C₁-C₆)alkylamino(C₁-C₆)alkyl.

By "heterocycle", "heterocycloalkyl" or "heterocyclyl" is meant one or more carbocyclic ring systems of 4-, 5-, 6-, or 7-membered rings which includes fused ring systems of 9-11 atoms containing at least one and up to four heteroatoms selected from nitrogen, oxygen, or sulfur. Preferred heterocycles of the present invention include morpholinyl, thiomorpholinyl, thiomorpholinyl S-oxide, thiomorpholinyl S,S-dioxide, piperazinyl, homopiperazinyl, pyrrolidinyl, pyrrolinyl, tetrahydropyranyl, piperidinyl, tetrahydrofuranyl, tetrahydrothienyl, homopiperidinyl, homomorpholinyl, homothiomorpholinyl, homothiomorpholinyl S,S-dioxide, oxazolidinonyl, dihydropyrazolyl, dihydropyrrolyl, dihydropyrazolyl, dihydropyridyl, dihydropyrimidinyl, dihydrofuryl, dihydropyranyl, tetrahydrothienyl, tetrahydrothienyl S-oxide, tetrahydrothienyl S,S-dioxide and homothiomorpholinyl S-oxide. The heterocycle groups herein are unsubstituted or substituted in one or more substitutable positions with various groups. For example, such heterocycle groups may be optionally substituted with, for example, C₁-C₆ alkyl, C₁-C₆ alkoxy, halogen, hydroxyl, oxo, cyano, nitro, amino, mono(C₁-C₆)alkylamino, di(C₁-C₆)alkylamino, C₂-C₆ alkenyl, C₂-C₆ alkynyl, C₁-C₆ haloalkyl, C₁-C₆ haloalkoxy, amino(C₁-C₆)alkyl, mono(C₁-C₆)alkylamino(C₁-C₆)alkyl, di(C₁-C₆)alkylamino(C₁-C₆)alkyl or oxo.

By "hydroxyalkyl" is meant an alkyl substituted with a hydroxyl, such as hydroxymethyl, 1-hydroxypropyl, 2-hydroxyethyl, 3-hydroxyethyl, or 3-hydroxybutyl.

Numbering on the tricyclic core when the C-ring is a phenyl and the B-ring is pyrazolyl:

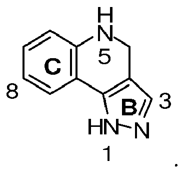

Most compounds were named using Autonom 2000 4.01.305, which is available from Beilstein Information Systems, Inc, Englewood, Colorado, or ChemDraw v.10.0, (available from Cambridgesoft at 100 Cambridge Park Drive, Cambridge, MA 02140). Alternatively, the names were generated based on the IUPAC rules.

The compounds of this invention may contain one or more asymmetric carbon atoms, so that the compounds can exist in different stereoisomeric forms. These compounds can be, for example, racemates, chiral non-racemic or diastereomers. In these situations, the single enantiomers, i.e., optically active forms, can be obtained by asymmetric synthesis or by resolution of the racemates. Resolution of the racemates can be accomplished, for example, by conventional methods such as crystallization in the presence of a resolving agent; chromatography, using, for example a chiral HPLC column; or derivatizing the racemic mixture with a resolving reagent to generate diastereomers, separating the diastereomers via chromatography, and removing the resolving agent to generate the original compound in enantiomerically enriched form. Any of the above procedures can be repeated to increase the enantiomeric purity of a compound.

Non-toxic pharmaceutically acceptable salts include, but are not limited to salts of inorganic acids such as hydrochloric, sulfuric, phosphoric, diphosphoric, hydrobromic, and nitric or salts of organic acids such as formic, citric, malic, maleic, fumaric, tartaric, succinic, acetic, lactic, methanesulfonic, p-toluenesulfonic, 2-hydroxyethylsulfonic, salicylic, besylate and stearic. Similarly, pharmaceutically acceptable cations include, but are not limited to sodium, potassium, calcium, aluminum, lithium and ammonium. Those skilled in the art will recognize a wide variety of non-toxic pharmaceutically acceptable addition salts. The invention also encompasses prodrugs of the compounds of Formula I.

The invention also encompasses the prodrugs of the compounds of Formula I. Those skilled in the art will recognize various synthetic methodologies that may be employed to prepare non-toxic pharmaceutically acceptable prodrugs of the compounds encompassed by Formula I. Those skilled in the art will recognize a wide variety of non-toxic pharmaceutically acceptable solvates, such as water, ethanol, mineral oil, vegetable oil, and dimethylsulfoxide... The invention also encompasses the acylated prodrugs of the compounds of Formula I. Those skilled in the art will recognize various synthetic methodologies, which may be employed to prepare non-toxic pharmaceutically acceptable addition salts and acylated prodrugs of the compounds encompassed by Formula I.

The term "acid prodrug group" denotes a moiety that is converted in vivo into an active carboxylic acid compound of formula I. Such prodrug groups are generally known in the art and include ester forming groups, to form an ester prodrug, such as benzyloxy, di(C₁-C₆)alkylaminoethyloxy, acetoxymethyl, pivaloyloxymethyl, phthalidoyl, ethoxycarbonyloxyethyl, 5-methyl-2-oxo-1,3-dioxol-4-yl methyl, and (C₁-C₆)alkoxy optionally substituted by N-morpholino and amide-forming groups such as di(C₁-C₆)alkylamino. Preferred prodrug groups include C₁-C₆ alkoxy forming an ester, and O⁻M⁺ where M⁺ represents a cation to form a salt of the acid. Preferred cations include sodium, potassium, and ammonium. Other cations include magnesium and calcium. Further preferred prodrug groups include O⁼M⁺⁺ where M⁺⁺ is a divalent cation such as magnesium or calcium.

It is understood by those practicing the art that the definition of compounds of Formula I contain asymmetric carbons, encompass all possible stereoisomers, mixtures and regioisomers thereof which possess the activity discussed below. Such regioisomers may be obtained pure by standard separation methods known to those skilled in the art. The definition encompasses any optical isomers and diastereomers as well as the racemic and resolved enantiomercially pure R and S stereoisomers as well as other mixtures of the R and S stereoisomers and pharmaceutically acceptable salts thereof, which possess the activity discussed below. Optical isomers may be obtained in pure form by standard separation techniques or enantiomer specific synthesis. It is understood that this invention encompasses all crystalline forms of compounds of Formula I.

When the compounds described herein contain olefinic double bonds or other centers of geometric asymmetry, and unless otherwise specified, it is intended that the compounds include the cis, trans, Z- and E- configurations. Likewise, all tautomeric forms are also intended to be included.

The compounds of general Formula I may be administered orally, topically, parenterally, by inhalation or spray or rectally in dosage unit formulations containing conventional non-toxic pharmaceutically acceptable carriers, adjuvants and vehicles. The term parenteral as used herein includes percutaneous, subcutaneous, intravascular (e.g., intravenous), intramuscular, or intrathecal injection or infusion techniques and the like. In addition, there is provided a pharmaceutical formulation comprising a compound of general Formula I and a pharmaceutically acceptable carrier. One or more compounds of general Formula I may be present in association with one or more non-toxic pharmaceutically acceptable carriers and/or diluents and/or adjuvants, and if desired other active ingredients. The pharmaceutical compositions containing compounds of general Formula I may be in a form suitable for oral use, for example, as tablets, troches, lozenges, aqueous or oily suspensions, dispersible powders or granules, emulsion, hard or soft capsules, or syrups or elixirs.

Compositions intended for oral use may be prepared according to any method known to the art for the manufacture of pharmaceutical compositions and such compositions may contain one or more agents selected from the group consisting of sweetening agents, flavoring agents, coloring agents and preservative agents in order to provide pharmaceutically elegant and palatable preparations. Tablets contain the active ingredient in admixture with non-toxic pharmaceutically acceptable excipients that are suitable for the manufacture of tablets. These excipients may be for example, inert diluents, such as calcium carbonate, sodium carbonate, lactose, calcium phosphate or sodium phosphate; granulating and disintegrating agents, for example, corn starch, or alginic acid; binding agents, for example starch, gelatin or acacia, and lubricating agents, for example magnesium stearate, stearic acid or talc. The tablets may be uncoated or they may be coated by known techniques. In some cases such coatings may be prepared by known techniques to delay disintegration and absorption in the gastrointestinal tract and thereby provide a sustained action over a longer period. For example, a time delay material such as glyceryl monosterate or glyceryl distearate may be employed.

Formulations for oral use may also be presented as hard gelatin capsules, wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin or olive oil.

Formulations for oral use may also be presented as lozenges.

Aqueous suspensions contain the active materials in admixture with excipients suitable for the manufacture of aqueous suspensions. Such excipients are suspending agents, for example sodium carboxymethylcellulose, methylcellulose, hydropropyl-methylcellulose, sodium alginate, polyvinylpyrrolidone, gum tragacanth and gum acacia; dispersing or wetting agents may be a naturally-occurring phosphatide, for example, lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl p-hydroxybenzoate, one or more coloring agents, one or more flavoring agents, and one or more sweetening agents, such as sucrose or saccharin.

Oily suspensions may be formulated by suspending the active ingredients in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol. Sweetening agents and flavoring agents may be added to provide palatable oral preparations. These compositions may be preserved by the addition of an anti-oxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents or suspending agents are exemplified by those already mentioned above. Additional excipients, for example sweetening, flavoring and coloring agents, may also be present.

Pharmaceutical compositions of the invention may also be in the form of oil-in-water emulsions. The oily phase may be a vegetable oil or a mineral oil or mixtures of these. Suitable emulsifying agents may be naturally-occurring gums, for example gum acacia or gum tragacanth, naturally-occurring phosphatides, for example soy bean, lecithin, and esters or partial esters derived from fatty acids and hexitol, anhydrides, for example sorbitan monooleate, and condensation products of the said partial esters with ethylene oxide, for example polyoxyethylene sorbitan monooleate. The emulsions may also contain sweetening and flavoring agents.

Syrups and elixirs may be formulated with sweetening agents, for example glycerol, propylene glycol, sorbitol, glucose or sucrose. Such formulations may also contain a demulcent, a preservative and flavoring and coloring agents. The pharmaceutical compositions may be in the form of a sterile injectable aqueous or oleaginous suspension. This suspension may be formulated according to the known art using those suitable dispersing or wetting agents and suspending agents that have been mentioned above. The sterile injectable preparation may also be a sterile injectable solution or suspension in a non-toxic parentally acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono-or diglycerides. In addition, fatty acids such as oleic acid find use in the preparation of injectables.

The compounds of general Formula I may also be administered in the form of suppositories, e.g., for rectal administration of the drug. These compositions can be prepared by mixing the drug with a suitable non-irritating excipient that is solid at ordinary temperatures but liquid at the rectal temperature and will therefore melt in the rectum to release the drug. Such materials include cocoa butter and polyethylene glycols.

Compounds of general Formula I may be administered parenterally in a sterile medium. The drug, depending on the vehicle and concentration used, can either be suspended or dissolved in the vehicle. Advantageously, adjuvants such as local anesthetics, preservatives and buffering agents can be dissolved in the vehicle.

For disorders of the eye or other external tissues, e.g., mouth and skin, the formulations are preferably applied as a topical gel, spray, ointment or cream, or as a suppository, containing the active ingredients in a total amount of, for example, 0.075 to 30% w/w, preferably 0.2 to 20% w/w and most preferably 0.4 to 15% w/w. When formulated in an ointment, the active ingredients may be employed with either paraffinic or a water-miscible ointment base.

Alternatively, the active ingredients may be formulated in a cream with an oil-in-water cream base. If desired, the aqueous phase of the cream base may include, for example at least 30% w/w of a polyhydric alcohol such as propylene glycol, butane-1,3-diol, mannitol, sorbitol, glycerol, polyethylene glycol and mixtures thereof. The topical formulation may desirably include a compound which enhances absorption or penetration of the active ingredient through the skin or other affected areas. Examples of such dermal penetration enhancers include dimethylsulfoxide and related analogs. The compounds of this invention can also be administered by a transdermal device. Preferably topical administration will be accomplished using a patch either of the reservoir and porous membrane type or of a solid matrix variety. In either case, the active agent is delivered continuously from the reservoir or microcapsules through a membrane into the active agent permeable adhesive, which is in contact with the skin or mucosa of the recipient. If the active agent is absorbed through the skin, a controlled and predetermined flow of the active agent is administered to the recipient. In the case of microcapsules, the encapsulating agent may also function as the membrane. The transdermal patch may include the compound in a suitable solvent system with an adhesive system, such as an acrylic emulsion, and a polyester patch. The oily phase of the emulsions of this invention may be constituted from known ingredients in a known manner. While the phase may comprise merely an emulsifier, it may comprise a mixture of at least one emulsifier with a fat or oil or with both a fat and an oil. Preferably, a hydrophilic emulsifier is included together with a lipophilic emulsifier, which acts as a stabilizer. It is also preferred to include both an oil and a fat. Together, the emulsifier(s) with or without stabilizer(s) make-up the so-called emulsifying wax, and the wax together with the oil and fat make up the so-called emulsifying ointment base, which forms the oily, dispersed phase of the cream formulations. Emulsifiers and emulsion stabilizers suitable for use in the formulation of the invention include Tween 60, Span 80, cetostearyl alcohol, myristyl alcohol, glyceryl monostearate, and sodium lauryl sulfate, among others. The choice of suitable oils or fats for the formulation is based on achieving the desired cosmetic properties, since the solubility of the active compound in most oils likely to be used in pharmaceutical emulsion formulations is very low. Thus, the cream should preferably be a non-greasy, non-staining and washable product with suitable consistency to avoid leakage from tubes or other containers. Straight or branched chain, mono- or dibasic alkyl esters such as di-isoadipate, isocetyl stearate, propylene glycol diester of coconut fatty acids, isopropyl myristate, decyl oleate, isopropyl palmitate, butyl stearate, 2-ethylhexyl palmitate or a blend of branched chain esters may be used. These may be used alone or in combination depending on the properties required. Alternatively, high melting point lipids such as white soft paraffin and/or liquid paraffin or other mineral oils can be used.

Formulations suitable for topical administration to the eye also include eye drops wherein the active ingredients are dissolved or suspended in suitable carrier, especially an aqueous solvent for the active ingredients. The anti-inflammatory active ingredients are preferably present in such formulations in a concentration of 0.5 to 20%, advantageously 0.5 to 10% and particularly about 1.5% w/w. For therapeutic purposes, the active compounds of this combination invention are ordinarily combined with one or more adjuvants appropriate to the indicated route of administration. The compounds may be admixed with lactose, sucrose, starch powder, cellulose esters of alkanoic acids, cellulose alkyl esters, talc, stearic acid, magnesium stearate, magnesium oxide, sodium and calcium salts of phosphoric and sulfuric acids, gelatin, acacia gum, sodium alginate, polyvinylpyrrolidone, and/or polyvinyl alcohol, and then tableted or encapsulated for convenient administration. Such capsules or tablets may contain a controlled-release formulation as may be provided in a dispersion of active compound in hydroxypropylmethyl cellulose. Formulations for parenteral administration may be in the form of aqueous or non-aqueous isotonic sterile injection solutions or suspensions. These solutions and suspensions may be prepared from sterile powders or granules having one or more of the carriers or diluents mentioned for use in the formulations for oral administration. The compounds may be dissolved in water, polyethylene glycol, propylene glycol, ethanol, corn oil, cottonseed oil, peanut oil, sesame oil, benzyl alcohol, sodium chloride, and/or various buffers. Other adjuvants and modes of administration are well and widely known in the pharmaceutical art.

Dosage levels of the order of from about 0.1 mg to about 140 mg per kilogram of body weight per day are useful in the treatment of the above-indicated conditions (about 0.5 mg to about 7 g per patient per day). The amount of active ingredient that may be combined with the carrier materials to produce a single dosage form will vary depending upon the host treated and the particular mode of administration. Dosage unit forms will generally contain between from about 1 mg to about 500 mg of an active ingredient. The daily dose can be administered in one to four doses per day. In the case of skin conditions, it may be preferable to apply a topical preparation of compounds of this invention to the affected area two to four times a day.

It will be understood, however, that the specific dose level for any particular patient will depend upon a variety of factors including the activity of the specific compound employed, the age, body weight, general health, sex, diet, time of administration, route of administration, and rate of excretion, drug combination and the severity of the particular disease undergoing therapy.

For administration to non-human animals, the composition may also be added to the animal feed or drinking water. It may be convenient to formulate the animal feed and drinking water compositions so that the animal takes in a therapeutically appropriate quantity of the composition along with its diet. It may also be convenient to present the composition as a premix for addition to the feed or drinking water.

The disclosures in this document of all articles and references, including patents, are incorporated herein by reference in their entirety.

The invention is illustrated further by the following examples, which are not to be construed as limiting the invention in scope or spirit to the specific procedures described in them.

The starting materials and various intermediates may be obtained from commercial sources, prepared from commercially available compounds, and/or prepared using known synthetic methods.

### General Synthetic Procedures

The compounds of the invention can be prepared using methods known in the art of organic synthesis. For example, the compounds of the invention, as well as all intermediates, can be synthesized by known processes using either solution or solid phase techniques as shown below. Representative procedures for preparing compounds of the invention are outlined in the following schemes.

Additionally, as will be apparent to those skilled in the art, conventional protecting groups may be necessary to prevent certain functional groups from undergoing undesired reactions. Suitable protecting groups for various functional groups as well as suitable conditions for protecting and deprotecting particular functional groups are well known in the art. For example, numerous protecting groups are described in T. W. Greene and G. M. Wuts, Protecting Groups in Organic Synthesis, Second Edition, Wiley, New York, 1991, and references cited therein.

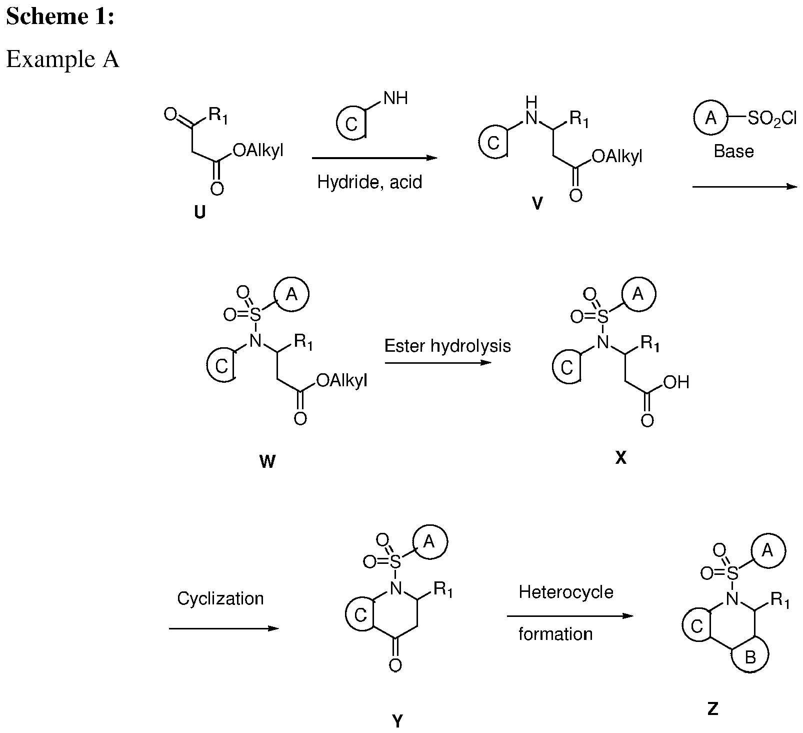

Compounds **V** can be prepared by reductive amination of an appropriate aniline and a β-keto ester **U** in an appropriate solvent such as dichloromethane, 1,2-dichloroethane, benzene, toluene, tetrahydrofuran, or chloroform, an acid source such as acetic acid, propionic acid, formic acid, hydrochloric acid, or trifluoroacetic acid, and a hydride source such as sodium borohydride, sodium cyanoborohydride, sodium triacetoxyborohydride, decaborane, or dibutyl chloride hydride complex. After reaction completion, the excess hydride can be quenched with a base such as saturated aqueous NaHCO₃ or saturated aqueous Na₂CO₃, or saturated aqueous K₂CO₃, and the resulting solution or biphasic mixture is then treated by standard extraction procedures to isolate the products **V.**

Compounds **W** can be prepared by reacting compounds **V** with a sulfonyl chloride in a suitable solvent such as dichloromethane, 1,2-dichloroethane, benzene, toluene, tetrahydrofuran, chloroform, or pyridine and a suitable base such as triethylamine, diisopropylethylamine, pyridine, or dimethylamino pyridine. The reaction mixture can then be concentrated and the residue taken up in a suitable solvent for aqueous extraction such as diethyl ether, ethyl acetate, chloroform, or dichloromethane and then treated by standard extraction procedures to isolate the products **W.**

Compounds **X** can be prepared by standard acid or base catalyzed ester hydrolysis methods or by other methods as outlined in 'Protective Groups in Organic Synthesis' third edition by Greene and Wuts, Wiley interscience, 1999.

Compounds **Y** can be prepared by cyclization of compounds **X** by heating in trifluoroacetic anhydride and trifluoroacetic acid (3:1 mixture) followed by addition of water and standard aqueous workup, or by the Inverse Friedel-Crafts Method described in W.S. Johnson and H.J. Glenn, JACS, 71, 1092 (1949).

Compounds **Z** can be prepared by a variety of methods for construction of the desired ring B-ring. Compounds **Z** were the B-ring is pyrazolyl can be made by treatment of compounds **Y** with acylating agents such as dimethylformamide dimethyl acetal, ethyl formate, or dimethylacetamide dimethyl acetal followed by cyclization with hydrazines in a suitable solvent such as acetic acid to obtain compounds **Z.**

In the above scheme, each of the variables independently contains the definitions as described above, while P is a protecting group. One of ordinary skill in the art will appreciate that the above scheme can be used to selectively produce a single diastereomer and/or a single enantiomer.

Compounds may be deprotected by standard means appropriate to the type of protection utilized as described in Greene, Theodora W.; Wuts, Peter G. M. Protective Groups in Organic Synthesis. 2nd Ed. (1991), 473 pp. (treatment with trimethylsilyl iodide, catalytic hydrogenation, sodium alkoxide, etc.)

### Experimental Procedures

Certain compounds of this invention are prepared from other compounds of this invention via-known reactions and functional group transformations. Examples of such transformations include ester hydrolysis, amide formation, and reductive alkylation; examples of these are described in the preparations below. Starting materials are obtained from commercial sources or prepared by known methods as described in the examples below.

Compounds included in this invention are exemplified by the following examples, which should not be construed as limiting the scope of this disclosure. Analogous structures and alternative synthetic routes within the scope of the invention will be apparent to those skilled in the art.

### Examples

The following synthetic and biological examples are offered to illustrate this invention and are not to be construed in any way as limiting the scope of this invention. Unless otherwise stated, all temperatures are in degrees Celsius

Reagents and solvents obtained from commercial suppliers were used without further purification unless otherwise stated. Thin layer chromatography was performed on precoated 0.25 mm silica gel plates (E. Merck, silica gel 60, F₂₅₄). Visualization was achieved using UV illumination or staining with phosphomolybdic acid, ninhydrin or other common staining reagents. Flash chromatography was performed using either a Biotage Flash 40 system and prepacked silica gel columns or hand packed columns (E. Merck silica gel 60, 230-400 mesh). Preparatory HPLC was performed on a Varian Prepstar high performance liquid chromatograph. ¹H NMR spectra were recorded on either a Varian Gemini 300 MHz spectrometer or a Bruker Avance 300 MHz spectrometer. Chemical shifts are reported in ppm (δ) and were calibrated using the undeuterated solvent resonance as internal standard. Mass spectra were recorded on an Agilent series 1100 mass spectrometer connected to an Agilent series 1100 HPLC.

In the examples below, the following abbreviations have the following meanings. If an abbreviation is not defined, it has its generally accepted meaning.

| | | |
|---|---|---|
| BSA | = | bovine serum albumin |
| DBU | = | 1,8-diazabicyclo[5.4.0]undec-7-ene |
| DCE | = | 1,2-dichloroethane |
| DEAD | = | diethyl azodicarboxylate |
| DMAP | = | 4-dimethylaminopyridine |
| DME | = | 1,2-dimethoxyethane |
| DMF | = | dimethylformamide |
| DMF-DMA | = | N,N-dimethylformamide dimethyl acetal |
| DMSO | = | dimethylsulfoxide |
| DPPA | = | diphenylphosphoryl azide |
| EDTA | = | ethylenediamine tetraacetic acid |
| EtOAc | = | ethyl acetate |
| EtOH | = | ethanol |
| Et₃N | = | triethylamine |
| HBSS | = | Hank's balanced salt solution |
| HEPES | = | 4-(2-hydroxyethyl)-1-piperazineethanesulfonic acid |
| HOAc | = | Acetic acid |
| HPLC | = | high performance liquid chromatography |
| i-PrOH | = | *iso*-propanol |
| LC/MS | = | liquid chromatography/mass spectroscopy |
| MTBE | = | methyl tert-butylether |
| PBS | = | phosphate buffered saline |
| PBS++ | = | PBS with calcium and magnesium |
| R_{f} | = | retention factor (ratio of distance traveled by substance/distance traveled by solvent front) |
| Rₜ | = | retention time |
| SEM | = | 2-(trimethylsilyl)ethoxymethyl |
| TFA | = | trifluoroacetic acid |
| THF | = | tetrahydrofuran |
| TLC | = | thin layer chromatography |

### Exemplary HPLC Procedures

Purity of compounds were determined by HPLC/MS analysis employing a variety of analytical methods:
Method 1 = 20% [B]: 80% [A] to 70% [B]: 30% [A] gradient in 1.75 min, then hold, at 2 mL/min, where [A]=0.1 % trifluoroacetic acid in water; [B]=0.1 % trifluoroacetic acid in acetonitrile on a Phenomenex Luna C18 (2) 4.6-mm X 30-cm column, 3 micron packing, 210 nm detection, at 35 °C.
Method 2 = 50% [B]: 50% [A] to 95% [B] : 5% [A] gradient in 2.5 min, then hold, at 2 mL/min, where [A]=0.1% trifluoroacetic acid in water; [B]=0.1% trifluoroacetic acid in acetonitrile on a Phenomenex Luna C18 (2) 4.6-mm X 30-cm column, 3 micron packing, 210 nm detection, at 35 °C.
Method 3 = 5% [B]: 95% [A] to 20% [B]: 80% [A] gradient in 2.5 min, then hold, at 2 mL/min, where [A]=0.1% trifluoroacetic acid in water; [B]=0.1% trifluoroacetic acid in acetonitrile on a Phenomenex Luna C18 (2) 4.6-mm X 30-cm column, 3 micron packing, 210 nm detection, at 35 °C.
Method 4 = 20% [B]: 80% [A] to 70% [B]: 30% [A] gradient in 2.33 min, then hold, at 1.5 mL/min, where [A]=0.1% trifluoroacetic acid in water; [B]=0.1% trifluoroacetic acid in acetonitrile on a Phenomenex Luna C18 (2) 4.6-mm X 30-cm column, 3 micron packing, 210 nm detection, at 35 °C.
Method 5 = 50% [B]: 50% [A] to 95% [B] : 5% [A] gradient in 3.33 min, then hold, at 1.5 mL/min, where [A]=0.1% trifluoroacetic acid in water; [B]=0.1% trifluoroacetic acid in acetonitrile on a Phenomenex Luna C18 (2) 4.6-mm X 30-cm column, 3 micron packing, 210 nm detection, at 35 °C.
Method 6 = 5% [B]: 95% [A] to 20% [B] : 80% [A] gradient in 3.33 min, then hold, at 1.5 mL/min, where [A]=0.1% trifluoroacetic acid in water; [B]=0.1% trifluoroacetic acid in acetonitrile on a Phenomenex Luna C18 (2) 4.6-mm X 30-cm column, 3 micron packing, 210 nm detection, at 35 °C.
Method 7= 20% [B]: 80% [A] to 70% [B]: 30% [A] gradient in 10.0 min, then hold, at 1.5 mL/min, where [A]=0.1% trifluoroacetic acid in water; [B]=0.1% trifluoroacetic acid in acetonitrile on a Phenomenex Luna C18 (2) 4.6-mm X 3-cm column, 3 micron packing, 210 nm detection, at 35 °C.
Method 8= 10% [B]: 90% [A] to 40% [B]: 60% [A] gradient in 10.0 min, then hold, at 1.5 mL/min, where [A]=0.1% trifluoroacetic acid in water; [B]=0.1% trifluoroacetic acid in acetonitrile on a Phenomenex Luna C18 (2) 4.6-mm X 3-cm column, 3 micron packing, 210 nm detection, at 35 °C.
Method 9 = 23% [B]: 77% [A] to 30% [B]: 70% [A] gradient in 15.0 min, then hold, at 1.0 mL/min, where [A]=0.1% trifluoroacetic acid in water; [B]=0.1% trifluoroacetic acid in acetonitrile on a Zorbex SB-phenyl C18 2.1-mm X 5-cm column, 5 micron packing, 210 nm detection, at 30 °C.
Method 10 = 5%[A]: 95% [B] isocrat, 1.0 mL/min, where [A]=isopropanol; [B]=hexanes; on a Chiral Technologies AD column (4.6 x 250 mm), 10 micron packing, 220 nm detection at ambient temperature.
Method 11 = 10%[A]: 90% [B] isocrat, 1.0 mL/min, where [A]=ethanol; [B]=hexanes; on a Chiral Technologies AD column (4.6 x 250 mm), 10 micron packing, 220 nm detection at ambient temperature.
Method 12 = 10%[A]: 90% [B] isocrat, 0.8 mL/min, where [A]=ethanol; [B]=hexanes; on a Chiral Technologies IA column (4.6 x 250 mm), 5 micron packing, 220 nm detection at ambient temperature.
Method 13 = 10%[A]: 90% [B] isocrat, 1.0 mL/min, where [A]=isopropanol; [B]=hexanes; on a Chiral Technologies IA column (4.6 x 250 mm), 5 micron packing, 220 nm detection at ambient temperature.
Method 14 = 10%[A]: 90% [B] isocrat, 1.0 mL/min, where [A]=isopropanol; [B]=hexanes; on a Chiral Technologies IB column (4.6 x 250 mm), 5 micron packing, 220 nm detection at ambient temperature.
Method 15 = 10%[A]: 90% [B] isocrat, 1.0 mL/min, where [A]=ethanol; [B]=hexanes; on a Chiral Technologies OD column (4.6 x 250 mm), 10 micron packing, 220 nm detection at ambient temperature.
Method 16 = 10%[A]: 90% [B] isocrat, 1.0 mL/min, where [A]=isopropanol; [B]=hexanes; on a Chiral Technologies OD column (4.6 x 250 mm), 10 micron packing, 220 nm detection at ambient temperature.
Method 17 = 15%[A]: 85% [B] isocrat, 1.0 mL/min, where [A]=ethanol; [B]=hexanes; on a Chiral Technologies OD column (4.6 x 250 mm), 10 micron packing, 220 nm detection at ambient temperature.
Method 18 = 5%[A]: 95%[B] isocrat, 55 mL/min, where [A]=isopropanol; [B]=hexanes on a Chiral Technologies AD column (5 x 50 cm), 20 micron packing, 220 nM detection at ambient temperature
Method 19 = 10%[A]: 90%[B] isocrat, 0.8 mL/min, where [A]=ethanol; [B]=hexanes; on a Chiral Technologies IB column (4.6 x 250 mm), 10 micron packing, 220 nm detection at ambient temperature.
Method 20 = 10%[A]: 90% [B] isocrat, 1.0 mL/min, where [A]=ethanol; [B]=hexanes; on a Chiral Technologies OD-H column (4.6 x 250 mm), 5 micron packing, 220 nm detection at ambient temperature.
Method 21 = 20%[A]: 80% [B] isocrat, 1.0 mL/min, where [A]=ethanol; [B]=hexanes; on a Chiral Technologies OD column (4.6 x 250 mm), 10 micron packing, 220 nm detection at ambient temperature.
Method 22 = 20%[A]: 80% [B] isocrat, 1.0 mL/min, where [A]=isopropanol; [B]=hexanes; on a Chiral Technologies OD column (4.6 x 250 mm), 10 micron packing, 220 nm detection at ambient temperature.

### Example 1

### 5-[(4-Chlorophenyl)sulfonyl]-4,5-dihydro-1H-pyrazolo[4,3-c]quinoline (7).

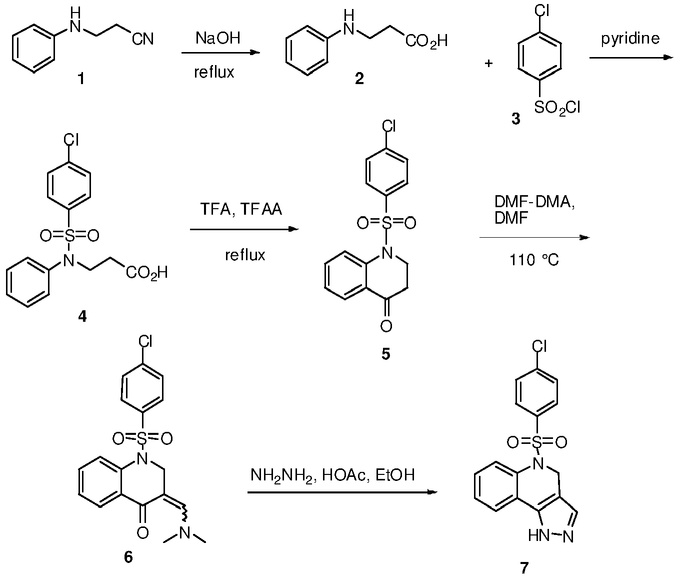

### 3-(Phenylamino)propanoic acid (2).

To a 10% aqueous solution of NaOH (120 mL) was added 3-anilinopropionitrile (1) (10.3 g, 10.7 mmol). The suspension was heated at reflux for 6 h and the resulting homogenous solution cooled to room temperature and stirred overnight. The solution was washed with ether (3 x 50 mL) and the pH of the aqueous phase was then adjusted to approximately 3 with conc. HCl. The aqueous phase was extracted with EtOAc (4 x 100 mL) and the combined organic phase was washed with water (1 x 50 mL), brine (1 x 50 mL), dried (MgSO₄), filtered and concentrated to yield (2) (11.6 g, 100%) as a brown solid. ¹H NMR (CDCl₃) 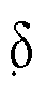 7.20 (2 H, t, J = 8.2 Hz), 6.77 (1H, t, J = 8.8 Hz), 6.69 (2H, d, J = 7.7 Hz), 3.46, (2H, t, J = 6.6 Hz), 2.67 (2H, t, J = 6.6 Hz); ¹³C NMR (CDCl₃) 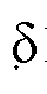 178.5, 147.1, 129.4, 118.5, 113.6, 39.4, 33.5; MS *m*/*z:* 166 (M+H)⁺.

### 3-(4-Chloro-N-phenylphenylsulfonamido)propanoic acid (4).

To a solution of (2) (2.0 g, 12.3 mmol) in pyridine (70 mL) at 0 °C was added 4-chlorobenzenesulfonyl chloride (3) (2.6 g, 12.3 mmol) portionwise. The reaction was warmed to room temperature and stirred for 3 hours. The reaction mixture was then concentrated and the residue taken up in EtOAc (100 mL). The organic phase was washed with 1N HCl (5 x 100 mL), water (1 x 100 mL), brine (1 x 50 mL), dried (MgSO₄), filtered, and concentrated to yield (4) (3.2g, 77%) as a tan solid. ¹H NMR (CDCl₃) 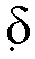 7.58-7.54 (2H, m), 7.48-7.45 (2H, m), 7.38-7.36 (3H, m), 7.08-7.06 (2H, m), 3.39 (2H, t, J = 7.5 Hz), 2.64 (2H, t, J = 7.2 Hz); MS *m*/*z* 340 (M+H)⁺.

### 1-(4-Chlorophenylsulfonyl)-2,3-dihydroquinolin-4(1H)-one (5).

Trifluoroacetic acid (5 mL) and trifluoroacetic anhydride (2 mL) were added to (4) (1.64 g, 4.83 mmol). The homogenous solution was heated at reflux for 3.5 hours, cooled and poured into ice water and the product extracted with EtOAc (1 x 50 mL, 2 x 10 mL). The combined organic phase was washed with water (1 x 20 mL), saturated aqueous NaHCO₃ (5 x 50 mL), brine (1 x 10 mL), dried (MgSO₄), filtered, and concentrated to yield (5) (1.48 g, 95%) as a tan solid. TLC: Rf = 0.30 (3:1 hexanes/EtOAc, silica gel). ¹H NMR (CDCl₃) 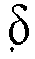 7.98 (1H, dd, J = 7.7, 1.6 Hz), 7.86 (1H, d, J = 8.2 Hz), 7.65-7.61 (3H, m), 7.45-7.42 (2 H, m), 7.31 (1H, d, J = 8.2 Hz), 4.27 (2H, t, J = 6.6 Hz), 2.48 (2H, t, J = 6.6 Hz); MS *m*/*z* 322 (M+H)⁺.

### 5-(4-Chlorophenylsulfonyl)-4,5-dihydro-1H-pyrazolo[4,3-c]quinoline (7).

To a DMF (800 µL) solution of (5) (151 mg, 0.470 mmol) was added dimethylformamide dimethyl acetal (62 µL, 0.470 mmol). The reaction was heated at 110 °C for 4 hours and then cooled to room temperature and set aside overnight. HPLC/MS analysis of the reaction mixture determined compound (6) to be present, (M+H)+ = 377, with no remaining (5). Volatiles were removed under a stream of nitrogen and the crude product was used directly in the next reaction. To an EtOH/glacial HOAc solution (25:1, 2 mL) of crude (6) (177 mg, 0.470 mmol) was added hydrazine hydrate (73 µL, 2.34 mmol). The reaction mixture was stirred overnight at room temperature, concentrated and the residue taken up in EtOAc (10 mL). The organic phase was washed with sat. aq. NaHCO₃ ( 3 x 20 mL), brine (1 x 10 mL), dried over MgSO₄, filtered and concentrated. The crude product was purified by reverse phase HPLC to give (7) (26 mg, 12% from (5)) as a yellow TFA salt. TLC: Rf = 0.35 (1:1 hexanes/EtOAc, silica gel). ¹H NMR (DMSO-d₆) δ 7.68-7.62 (2H, m), 7.46-7.41 (3H, m), 7.32 (2H, d, J = 8.2 Hz), 7.16 (2H, d, J = 8.8 Hz), 4.93 (2H, s); ¹³C NMR (DMSO-d₆) δ 138.9, 137.1, 135.0, 129.6, 129.5, 129.3, 128.9, 128.6, 123.3, 112.3, 44.2; MS *m*/*z* 346 (M+H)⁺.

### Example 2

### 5-[(4-Chlorophenyl)sulfonyl]-8-fluoro-4,5-dihydro-2H-pyrazolo[4,3-c]quinoline (14).

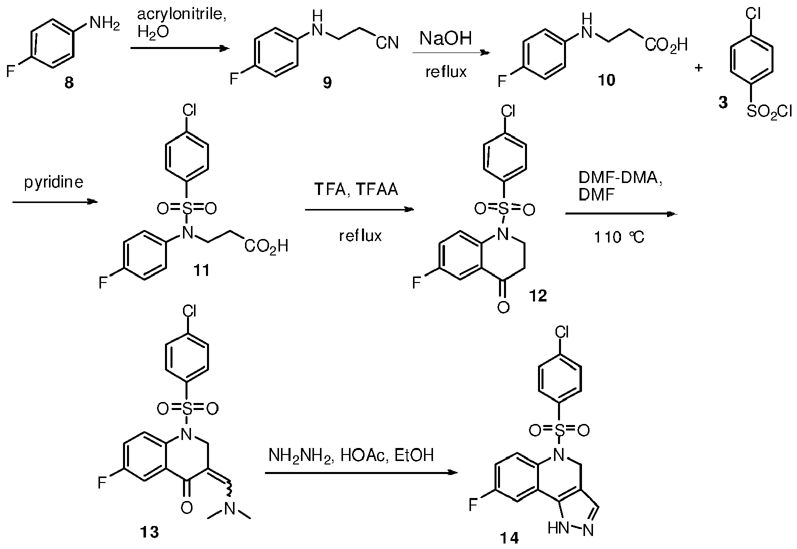

### 3-(4-Fluorophenylamino)propanoic acid (10).

Acrylonitrile (7.89 mL, 120 mmol) was added to 4-fluoroaniline (8), (11.1 g, 100 mmol), and H₂O (80 mL). The biphasic mixture was heated to reflux and stirred overnight. The reaction mixture was cooled to room temp. HPLC/MS analysis of the reaction mixture confirmed compound (9) to be present, (M+H)⁺ = 165.1. NaOH (12 g, 300 mmol) was added to the reaction mixture, and the reaction was heated to reflux for 1.5 hours. HPLC/MS analysis of the reaction mixture confirmed compound (10), to be present with no remaining (9). The reaction was cooled to room temperature and extracted with diethyl ether (3 x 50 mL). The pH was adjusted to approximately 3 with 3N aqueous HCl and then the aqueous layer was extracted with EtOAc (2 x 50 mL). The aqueous layer was then adjusted to pH 5 with 1 N NaOH and extracted with EtOAc (1 x 100 mL). The combined organic extracts were dried over MgSO₄, filtered, and concentrated to yield (10) as a brown solid, (16 g, 87.6 mmol, 87%) MS *m*/*z* 184.1 (M+H)⁺.

### 3-(4-Chloro-N-(4-fluorophenyl)phenylsulfonamido)propanoic acid (11).

To a rapidly stirring room temperature pyridine (0.5 M, 166 mL) solution of (10) (15.19 g, 83 mmol) was added 4-chlorobenzenesulfonyl chloride (3), (17.5 g, 83 mmol). After 30 minutes, pyridine was removed and the residue was taken into EtOAc, and washed with water (5 x 50 mL), brine (2 x 50 mL), and 1M HCl (3 x 50 mL). The organic phase was dried (MgSO₄), filtered, and concentrated to yield (11) as a tan solid (22.2 g, 62.3 mmol, 75 %). MS *m*/*z* 357.9 (M+H)⁺, 379.9 (M+Na)⁺.

### 1-(4-Chlorophenylsulfonyl)-6-fluoro-2,3-dihydroquinolin-4(1H)-one (12).

Trifluoroacetic acid (10 mL) and trifluoroacetic anhydride (3.2 mL) were added to (11) (3.3 g, 9.2 mmol). The homogenous solution was heated to 80 °C for 6 hours, cooled and poured into ice water. The product was extracted with EtOAc. The organic phase was washed with sat. aq. NaHCO₃ (3 x 25 mL), and concentrated. The residue was triturated with diethyl ether, and then chromatographed with EtOAc to afford (12) (0.8 g, 26%). MS *m*/*z* 339.9 (M+H)⁺.

### 5-(4-Chlorophenylsulfonyl)-8-fluoro-4,5-dihydro-1H-pyrazolo[4,3-c]quinoline (14).

Dimethylformamide dimethyl acetal (2 mL) was added to (12) (454 mg, 1.34 mmol). The reaction was heated at 103 °C for 20 minutes and then cooled to room temperature. HPLC/MS analysis of the reaction mixture determined compound (13) was present, (M+H)⁺ = 394.9, with no remaining (12). Volatiles were removed and the crude product was used directly in the next reaction. To an HOAc solution (4 mL) of crude (13) (1.34 mmol) was added hydrazine hydrate (78 µL, 1.61 mmol). The reaction mixture was heated to 95 °C for 1 hour. Solvent was removed and the residue was chromatographed with 1:1 hexane/EtOAc to afford (14) (432 mg, 89%). MS *m*/*z* 364 (M+H)⁺. ¹H NMR (CD₃OD) δ 7.77-7.74 (dd, J = 8.9, 5.0 Hz, 1H), 7.40(s, 1H), 7.36-7.33 (dd, J = 8.8, 3.0 Hz, 1H), 7.20-7.14 (m, 5H), 4.95 (s, 2H); ¹³C NMR (CD₃OD) δ 163.6, 160.3, 138.8, 136.0, 130.8, 130.7, 128.6, 128.1, 114.6, 114.3, 112.2, 108.7, 108.4, 43.0.

### Example 3

### 5-[(4-Chlorophenyl)sulfonyl]-7-fluoro-4,5-dihydro-2H-pyrazolo[4,3-c]quinoline (23) and 5-[(4-Chlorophenyl)sulfonyl]-9-fluoro-4,5-dihydro-2H-pyrazolo[4,3-c]quinoline (24).

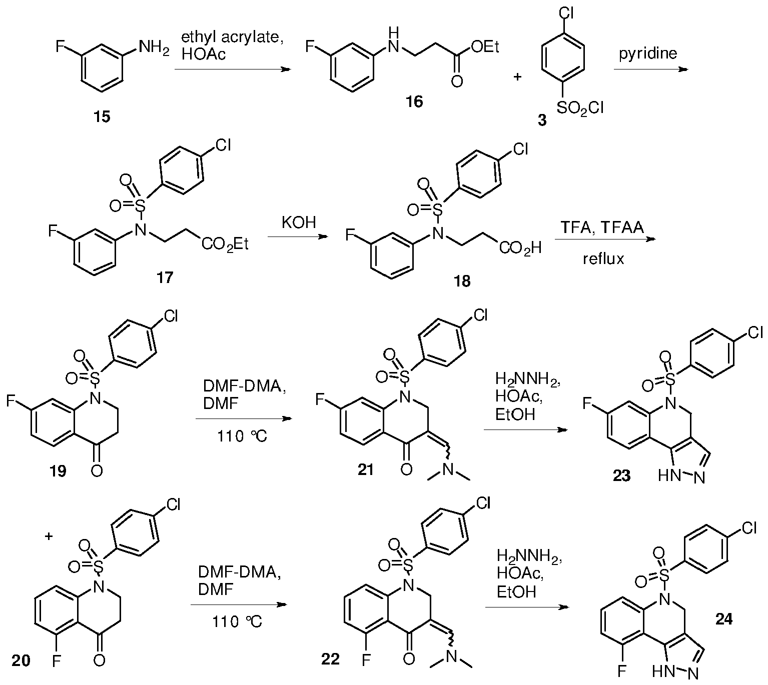

### Ethyl 3-(3-fluorophenylamino)propanoate (16).

A mixture of 3-fluoroaniline (15), 10 g, 90 mmol), ethyl acrylate (9.9 g, 10.8 mL, 95 mmmol) and HOAc (400 µL) was heated at reflux for 17 h. The mixture was cooled to ambient temperature then distilled (b.p 120-130 °C at 0.25 Torr) to give (16), 12.35 g (65%).

### Ethyl 3-(4-chloro-N-(3-fluorophenyl)phenylsulfonamido)propanoate (17).

Ethyl 3-(3'-fluoroanilinyl)propionate (16), 12.3 g, 58.2 mmol) was dissolved in pyridine (125 mL) and cooled in an ice bath. 4-Chlorobenzenesulfonyl chloride (3), 13.5 g, 64 mmol) was added in portions over 30 min. The mixture was stirred for 1 h in an ice bath and then allowed to warm to ambient temperature for 1h. The volatiles were removed *in vacuo* and the residue was diluted with water (200 mL) and then extracted with EtOAc (2 x 200 mL). The combined organic extracts were washed with 1N aq HCl (2 x 100) then dried (Na₂SO₄), filtered and evaporated *in vacuo* to give 23.2 g of (17) (quantitative).

### 3-(4-Chloro-N-(3-fluorophenyl)phenylsulfonamido)propanoic acid (18).

The ethyl ester (17) (23.2 g, 58 mmol) was taken up into MeOH (200 mL) and treated dropwise with a solution of potassium hydroxide (4.10 g, 73 mmol) in water (75 mL). The mixture was then stirred for 1 h until TLC analysis indicated the consumption of starting material. The mixture was concentrated *in vacuo,* diluted with water (200 mL), then made acidic with 6 N aq. HCl. The mixture was extracted with EtOAc (2 x 200 mL) and the combined organic extracts were washed with water (2 x 100 mL), then dried (Na₂SO₄), filtered and concentrated *in vacuo* to give a residue of (18). The residue was recrystallized from EtOAc/hexanes to give two small crops of crystals (2.35g, 1.21 g), but the remaining mother liquors were concentrated *in vacuo* to give a residue (16 g), which was taken on to the next step.

### 1-(4-Chlorophenylsulfonyl)-7-fluoro-2,3-dihydroquinolin-4(1H)-one (19) and 1-(4-Chlorophenylsulfonyl)-5-fluoro-2,3-dihydroquinolin-4(1H)-one (20).

The carboxylic acid (18) (16 g) was taken up into trifluoroacetic acid (75 mL) and treated with trifluoroacetic anhydride (30 mL). The mixture was heated to reflux for 3 hours before cooling to ambient temperature. The volatiles were removed *in vacuo* to give a residue which was combined with two earlier reactions and purified by silica gel chromatography (twice, eluted 4:1 hexanes/EtOAc) to give (19) (5.5 g) and (20) (1.05 g).

### 5-(4-Chlorophenylsulfonyl)-7-fluoro-4,5-dihydro-1H-pyrazolo[4,3-c]quinoline (23).

Dimethylformamide dimethyl acetal (2 mL) was added to (19) (518 mg, 1.53 mmol). The reaction was heated at 103 °C for 20 minutes and then cooled to room temperature. HPLC/MS analysis of the reaction mixture determined compound (21) present, (M+H)⁺ - 394.9, with no remaining (19). Volatiles were removed and the crude product was used directly in the next reaction. To an acetic acid solution (4 mL) of crude (21) (1.53 mmol) was added hydrazine hydrate (74 µL, 1.53 mmol). The reaction mixture was heated to 95 °C for 1 hour. Solvent was removed and the residue was chromatographed with HPLC to afford (23) (272 mg, 0.75 mmol). MS *m*/*z* 363.9 (M+H)⁺. ¹H NMR (CD₃OD) δ 7.68-7.63 (dd, J = 8.6, 6.2 Hz, 1H), 7.53-7.49(dd, J = 10.1, 2.6 Hz, 1H), 7.38 (s, 1H), 7.22-7.14 (m, 5H), 4.96 (s, 2H); ¹³C NMR (CD₃OD) δ 163.4, 160.2, 139.0, 136.6, 136.4.8, 136.2, 128.5, 128.2, 123.7, 123.6, 115.7, 115.4, 114.8, 114.5,43.1.

### 5-(4-Chlorophenylsulfonyl)-9-fluoro-4,5-dihydro-1H-pyrazolo[4,3-c]quinoline (24).

Dimethylformamide dimethyl acetal (2 mL) was added to (20) (530 mg, 1.56 mmol). The reaction was heated at 103 °C for 20 minutes and then cooled to room temperature. HPLC/MS analysis of the reaction mixture determined compound (22) present, (M+H)⁺ = 394.9, with no remaining (20). Volatiles were removed and the crude product was used directly in the next reaction. To an acetic acid solution (4 mL) of crude (22) (1.56 mmol) was added hydrazine hydrate (76 µL, 1.56 mmol). The reaction mixture was heated to 95 °C for 1 hour. Solvent was removed and the residue was chromatographed with HPLC to afford (24) (253 mg, 45 %). MS *m*/*z* 364.0 (M+H)⁺. ¹H NMR (CD₃OD) δ 7.64-7.61 (d, J = 7.7 Hz, 1H), 7.47-7.39 (m, 2H), 7.24-7.19 (m, 5H), 4.96 (s, 2H); ¹³C NMR (CD₃OD) δ 159.2, 155.9, 139.0, 136.2, 128.5, 128.3, 128.2, 127.7, 124.5, 124.4, 114.8, 114.5, 112.7, 43.0.

### Example 4

### 5-(4-Chlorophenylsulfonyl)-4-methyl-4,5-dihydro-2H-pyrazolo[4,3-c]quinoline (31).

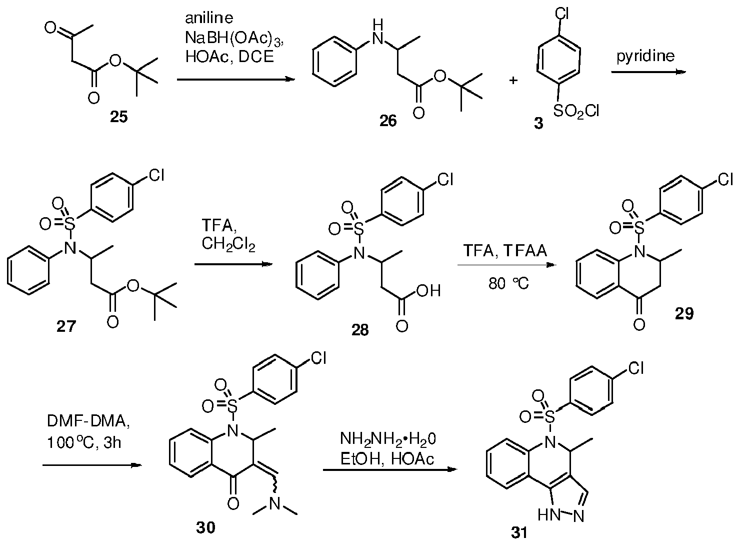

### tert-Butyl 3-(phenylamino)butanoate (26).

To a 1,2-dichloroethane solution (100 mL) of aniline (2.52 g, 27.1 mmol) was added t-butyl acetoacetate (25), 4.49 mL, 27.1 mmol) and glacial acetic acid (2.0 mL, 35.2 mmol). Sodium triacetoxyborohydride (8.60 g, 40.6 mmol) was added in portions and the reaction was stirred overnight at room temperature. Saturated aqueous NaHCO₃ (200 mL) was slowly added and the biphasic solution was well stirred for 45 min. The aqueous solution was extracted with dichloroethane (1 x 30 mL) and the combined organic portions were washed with brine (1 x 20 mL), dried (MgSO₄), filtered and concentrated to yield (26) (5.2, 81%) as an oil which solidified upon standing. MS *m*/*z* 236.2 (M+H)⁺.

### tert-Butyl 3-(4-chloro-N-phenylphenylsulfonamido)butanoate (27).

To a solution of (26) (2.34 g, 9.94 mmol) in pyridine (25 mL) at 0 °C was added 4-chlorobenzenesulfonyl chloride (3), (6.29 g, 29.8 mmol) portionwise. The reaction was warmed to room temperature and then heated at 70 °C for 5 hours, cooled, and stirred overnight at room temperature. The reaction mixture was then concentrated and the residue taken up in EtOAc (150 mL). The organic phase was washed with cold 1N HCl (5 x 100 mL), water (1 x 50 mL), sat. aq. NaHCO₃ (3 x 50 mL), brine (1 x 20 mL), dried (MgSO₄), filtered, and concentrated to an oil. ¹H NMR revealed the presence of unreacted 4-chlorobenzenesulfonyl chloride, so the product was dissolved in pyridine (25 mL) and dimethylaminopropylamine (35 mL) was added and the resulting solution was stirred overnight. The reaction mixture was concentrated and the residue taken up in EtOAc (150 mL) and washed with cold 1N HCl (6 x 60 mL), water (1 x 50 mL), sat. aq. NaHCO₃ (3 x 60 mL), brine (1 x 20 mL), dried (MgSO₄), filtered, and concentrated to yield (27) (3.7 g, 91%) as a light yellow oil. MS *m*/*z*: 432.1 (M+Na)⁺.

### 3-(4-Chloro-N-phenylphenylsulfonamido)butanoic acid (28).

To a solution of (27) (3.35 g, 8.17 mmol) in CH₂Cl₂ (15 mL) at 0 °C was added TFA (45 mL). The reaction mixture was allowed to warm to room temperature. After stirring for 72 hours the reaction mixture was concentrated to give (28) (2.63 g, 91%) as a light yellow oil which solidified upon standing. MS *m*/*z* 354.1(M+H)⁺.

### 5-(4-Chlorophenylsulfonyl)-4-methyl-4,5-dihydro-1H-pyrazolo[4,3-c]quinoline (31).

To a solution of (28) (1.02 g, 2.89 mmol) in TFA (3 mL) was added trifluoroacetic anhydride (1.2 mL, 8.8 mmol). The reaction mixture was heated at 80 °C for one hour, cooled and pour onto cracked ice. The product was extracted with EtOAc (3 x 15 mL) and the combined organic portion was washed with water (1 x 20 mL), sat. aq. NaHCO₃ (3 x 40 mL), brine (1 x 20 mL), dried (MgSO₄), filtered, and concentrated to a tacky yellow solid. The crude product was purified by flash chromatography eluting with 6:1 hexanes/EtOAc to give impure (29) (351 mg) as a light yellow foam. MS *m*/*z*: 336.1 (M+H)⁺.

A dimethylformamide dimethyl acetal (1.5 mL) solution of (29) (263 mg, 0.782 mmol) was heated at 100 °C for 3 hours. HPLC/MS analysis of the reaction mixture determined no starting material remained so the reaction was cooled to room temperature. Following concentration, the crude product (30) was used directly in the next reaction. To an EtOH/glacial acetic acid solution (25:1 v/v, 2 mL) of (30) (306 mg, 0.782 mmol) was added hydrazine hydrate (189 µL, 3.91 mmol). The reaction mixture was stirred overnight at room temperature, concentrated, and the residue taken up in EtOAc (10 mL). The organic phase was washed with sat. aq. NaHCO₃ ( 3 x 20 mL), brine (1 x 10 mL), dried over MgSO₄, filtered and concentrated to a semi-solid. The crude product was purified by flash chromatography eluting with 10:1 hexanes/EtOAc and then 2:1 hexanes/EtOAc to give (31) as a light golden solid. MS *m*/*z* 360.0 (M+H)⁺. ¹H NMR (CDCl₃) δ 7.82 (1H, dd), 7.66 (1H, dd), 7.44-7.33 (2H, m), 7.21 (1H, s), 7.12-7.02 (4H, m), 5.62 (1H, q), 1.30 (3H, d) ¹³C NMR (CDCl₃) 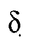 138.9, 136.0, 132.4, 129.8, 128.6, 128.4, 128.3, 127.8, 124.8, 122.4, 118.0, 49.8, 23.1.

### Example 5

### 1-(4-Chlorophenylsulfonyl)-7-methoxy-2-methyl-2,3-dihydroquinolin-4(1H)-one (35) and 1-(4-Chlorophenylsulfonyl)-5-methoxy-2-methyl-2,3-dihydroquinolin-4(1H)-one (36).

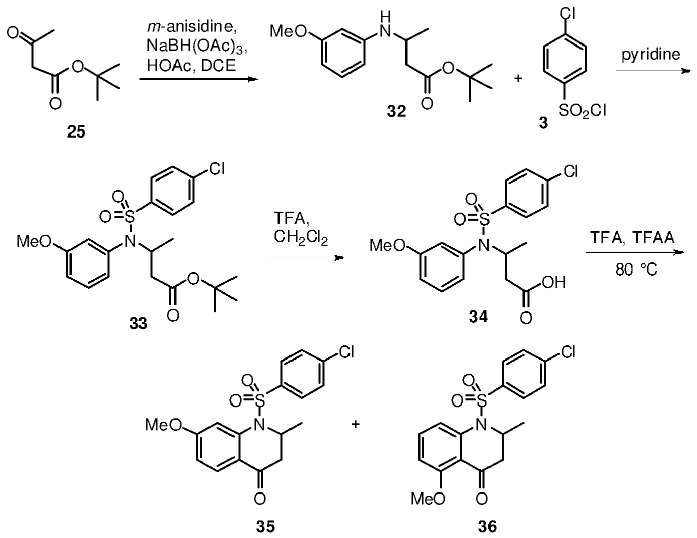

### tert-Butyl 3-(3-methoxyphenylamino)butanoate (32).

To a solution of m-anisidine (1.00 g, 8.12 mmol) in CH₂Cl₂ (10 mL) was added *tert-*butyl acetoacetate (25), (1.28 g, 8.12 mmol), followed by HOAc (0.47 mL, 8.12 mmol). The reaction mixture was stirred for 45 minutes. Sodium triacetoxyborohydride (2.41 g, 11.3 mmol) was added and the reaction mixture was stirred overnight at room temperature. The reaction mixture was quenched by the slow addition of saturated aqueous NaHCO₃. The two layers were separated. The aqueous solution was extracted with CH₂Cl₂ (10 mL). The combined organic layers were washed with saturated aqueous NaCl, dried over MgSO₄, filtered and concentrated. The resulting residue was purified by flash chromatography (20% EtOAc in hexanes) to afford (32) (1.09 g, 51%) MS *m*/*z* 266.1 (M+H)⁺.

### tert-Butyl 3-(4-chloro-N-(3-methoxyphenyl)phenylsulfonamido) butanoate (33).

To a solution of (32) (1.00 g, 3.76 mmol) in pyridine (10 mL) was added 4-chlorobenzenesulfonyl chloride (3), (0.79 g, 3.76 mmol). The reaction mixture was stirred overnight at room temperature. The reaction mixture was concentrated, and the resulting residue was dissolved in CH₂Cl₂ (15 mL). The organic phase was washed with water (10 mL), and saturated aqueous NaCl (10 mL), then dried over MgSO₄, filtered and concentrated. The resulting residue was purified by flash chromatography (20% EtOAc in hexanes) to afford (33) (1.32 g, 80%) MS *m*/*z* 462.1 (M+Na)⁺.

### 3-(4-Chloro-N-(3-methoxyphenyl)phenylsulfonamido)butanoic acid (34).

To a solution of (33) (1.25 g, 2.86 mmol) in CH₂Cl₂ (10 mL) was added TFA (10 mL). The reaction mixture was stirred at room temperature overnight, and then concentrated. The resulting residue was dissolved in CH₂Cl₂ (10 mL) and washed with water (5 mL). The organic layer was dried with MgSO₄, filtered and concentrated to afford (34) (0.93g, 85%), which solidified upon standing. MS *m*/*z* 384.1 (M+H)⁺.

### 1-(4-Chlorophenylsulfonyl)-7-methoxy-2-methyl-2,3-dihydroquinolin-4(1H)-one (35) and 1-(4-Chlorophenylsulfonyl)-5-methoxy-2-methyl-2,3-dihydroquinolin-4(1H)-one (36).

To a solution of (34) (0.93 g, 2.43 mmol) in trifluoroacetic acid (5 mL) was added trifluoroacetic anhydride (2 mL). The reaction mixture was heated to 80 °C for 3h, and then cooled to room temperature. The reaction mixture was poured into water and crushed ice. The product was extracted with EtOAc (3 x 15 mL). The combined organic layers were washed with saturated aqueous NaHCO₃ (3 x 15mL), saturated aqueous NaCl (15 mL), dried over MgSO₄, filtered and concentrated. The resulting residue was purified by flash chromatography (20% EtOAc in hexanes) to yield the two regioisomers (35) (0.66 g, 69%) MS m/z 366.0 (M+H)⁺, and (36) (0.06 g, 6%) MS *m*/*z* 366.0 (M+H)⁺.

### Example 6

### 5-(4-Chlorophenylsulfonyl)-7-methoxy-4-methyl-4,5-dihydro-2H-pyrazolo[4,3-c]quinoline (38).

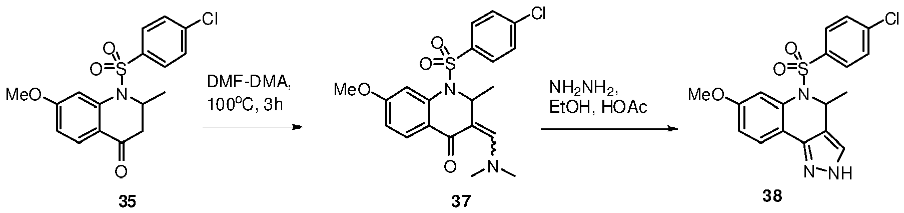

### 5-(4-Chlorophenylsulfonyl)-7-methoxy-4-methyl-4,5-dihydro-2H-pyrazolo[4,3-c]quinoline (38).

To (35) (0.25 g, 0.68 mmol) was added dimethylformamide dimethylacetal (5 mL). The reaction mixture was heated to 100 °C for 3h, then cooled to room temperature and concentrated. The resulting residue of crude (37) was used without purification in the next reaction. To a solution of (37) in MeOH (5 mL) was added HOAc (0.12 mL, 2.05 mmol), followed by anhydrous hydrazine (0.06 g, 2.05 mmol). The reaction mixture was stirred at room temperature overnight, then concentrated. The resulting residue was dissolved in EtOAc (15 mL), washed with saturated aqueous NaHCO₃ (10 mL) and saturated aqueous NaCl (10 mL). The organic layer was dried with MgSO₄, filtered and concentrated. The resulting residue was purified by flash chromatography (30% EtOAc in hexanes) to yield (38) (0.16 g, 61%); ¹H NMR (CDCl₃) δ 7.55 (d, J = 8.3 Hz, 1H), 7.39 (d, J = 2.5 Hz, 1H), 7.16 (m, 3H), 7.03 (d, J = 8.7 Hz, 2H), 6.91 (dd, J = 8.5, 2.5 Hz, 1H), 5.60 (q, J = 6.9 Hz, 1H), 3.91 (s, 3H), 1.32 (d, J = 6.9 Hz, 3H); ¹³C NMR (CDCl₃) δ 159.7, 139.0, 136.1, 133.9, 128.5, 128.3, 123.3, 117.7, 116.9, 114.6, 114.4, 60.5, 55.7, 50.1, 23.2, 14.2; MS *m*/*z* 389.0 (M+H)⁺.

### Example 7

### 5-(4-Chlorophenylsulfonyl)-9-methoxy-4-methyl-4,5-dihydro-2H-pyrazolo[4,3-c]quinoline (40).

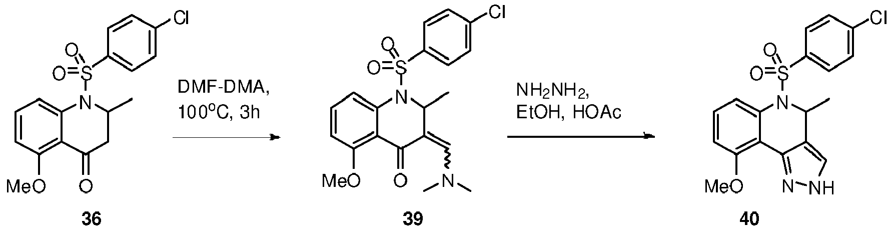

### 5-(4-Chlorophenylsulfonyl)-9-methoxy-4-methyl-4,5-dihydro-2H-pyrazolo[4,3-c]quinoline (40).

To (36) (0.06 g, 0.15 mmol) was added dimethylformamide dimethylacetal (2 mL). The reaction mixture was heated to 100 °C for 3h, then cooled to room temperature and concentrated. The resulting residue of crude (39) was used without purification in the next reaction. To a solution of (39) in MeOH (2 mL) was added HOAc (0.02 mL, 0.45 mmol), followed by anhydrous hydrazine (0.02 g, 045 mmol). The reaction mixture was stirred at room temperature overnight, then concentrated. The resulting residue was dissolved in EtOAc (15 mL), washed with saturated aqueous NaHCO₃ (10 mL) and saturated aqueous NaCl (10 mL). The organic layer was dried with MgSO₄, filtered and concentrated. The resulting residue was purified by flash chromatography (30% EtOAc in hexanes) to yield (40) (0.03 g, 59%); ¹H NMR (CDCl₃) δ 7.52 (dd, J, 8.1, 0.8 Hz, 1H), 7.36 (d, J = 8.3 Hz, 1H), 7.28 (s, 1H), 7.17 (m, 4H), 6.92 (dd, J = 8.3, 0.5 Hz, 1H), 5.68 (q, J = 6.9 Hz, 1H), 3.96 (s, 3H), 1.28 (d, J = 6.9 Hz, 3H); ¹³C NMR (75 MHz, CDCl₃) δ 154.4, 139.0, 136.1, 134.0, 132.7, 129.5, 128.7, 122.3, 117.5, 111.3, 109.5, 60.5, 56.1, 50.5, 23.0, 14.3; MS *m*/*z* 390.0 (M+H)⁺.

### Example 8

### 5-[(4-Chlorophenyl)sulfonyl]-3-methyl-4,5-dihydro-1H-pyrazolo[4,3-c]quinoline (42).

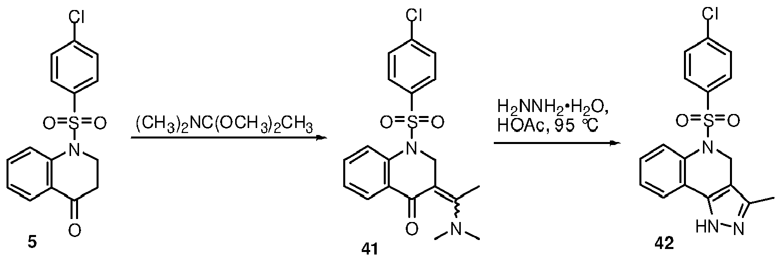

### 5-(4-Chlorophenylsulfonyl)-3-methyl-4,5-dihydro-1H-pyrazolo[4,3-c]quinoline (42).

Ketone (5) (357 mgs, 1.11 mmol) was warmed to dissolution in neat *N,N-*dimethylacetamide dimethyl acetal (1.63 mL, 11.11 mmol) and stirred under nitrogen at 103 °C for 30 min. The amber solution was cooled to ambient temperature and subsequently dried via rotary evaporation to afford compound (41) MS (ES) *m*/*z* 391.0 (M+H)⁺.

To a suspension of intermediate (41) (156 mgs, 0.4 mmol) and glacial HOAc (8 mL) was added hydrazine hydrate (82.4 µL, 1.0 mmol). The solution was stirred under nitrogen and heated at 95 °C for 18h. Additional hydrazine hydrate was then added (82.4 µL, 1.0 mmol) and the reaction stirred for an additional 4h at 95 °C. Upon complete consumption of (41) (0.3h-22h LC-MS), the reaction mixture was cooled to ambient temperature and the solvent was removed by rotary evaporation. The resulting residue was dissolved in CH₂Cl₂ (20 mL), washed with water (2 x 20 mL) and dried over MgSO₄. After filtration and concentration by rotary evaporation, the residue was dissolved in MeOH (6 mL) and purified by reverse-phase preparative HPLC, resulting in 33 mgs of (42). MS (ES) *m*/*z* 359.9 (M+H)⁺. ¹H NMR (CDCl₃) δ 7.81-7.78 (dd, J = 6.2, 1.5 Hz, 1H), 7.66-7.63 (dd, J = 5.4, 1.6 Hz, 1H), 7.43-7.32 (m, 2H), 7.16-7.07 (m, 4H), 4.81 (s, 2H), 2.21 (s, 3H); ¹³C NMR (CDCl₃) δ 158.3, 156.2, 143.7, 138.9, 136.5, 135.2, 134.8, 128.7, 128.3, 128.2, 128.1, 127.8, 125.7, 122.4, 109.9, 43.0, 9.6.

### Example 9

### 5-(4-Chlorophenylsulfonyl)-9-methoxy-4-methyl-4,5-dihydro-2H-pyrazolo[4,3-c]quinoline (49).

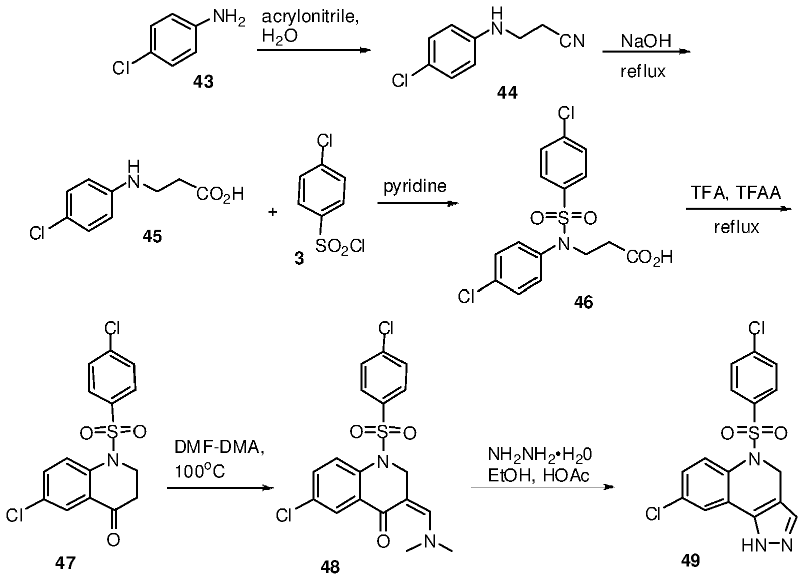

### 3-(4-Chlorophenylamino)propanenitrile (44).

Acrylonitrile (10.3 mL, 157 mmol) was added to 4-fluoroaniline (43), (10 g, 78 mmol), and H₂O (62 mL). The biphasic mixture was heated to reflux and stirred for 4 days. An additional 10 mL of acrylonitrile was added to the reaction mixture and reflux continued overnight. Cooled reaction was extracted with CH₂Cl₂ (100 mL) and organic phase was washed with 0.1 M aqueous citric acid (25 mL) and dried over MgSO₄ to obtain compound (44) (12.7 g, 90%) MS *m*/*z* 181(M+H)⁺.

### 3-(4-Chlorophenylamino)propanoic acid (45).

To (44) (12.7 g, 70.8 mmol) was added 1M aqueous NaOH (71 mL), and the reaction was heated to reflux for 4 hours. HPLC/MS analysis of reaction mixture confirmed compound (45), to be present with no remaining (44). The reaction was cooled to room temperature and the precipitate was filtered. The pH was adjusted to approximately 5 with 37% HCl and the product was filtered yielding (45) as a white solid, (10.0 g, 72%) MS *m*/*z* 200(M+H)+.

### 3-(4-Chloro-N-(4-chlorophenyl)phenylsulfonamido)propanoic acid (46).

To a rapidly stirring 0 °C pyridine (0.5 M, 166 mL) solution of (45) (15.19 g, 83 mmol) was added 4-chlorobenzenesulfonyl chloride (3), 17.5 g, 83 mmol). After 1 h pyridine was removed and the residue was taken into EtOAc, washed with water (2 x 50 mL) and dried (MgSO₄), filtered, and concentrated. The sample was crystallized with 1:1 hexane:EtOAc and (46) (1.6 g, 46 %) was obtained MS *m*/*z* 395.9 (M+Na)⁺.

### 6-Chloro-1-(4-chlorophenylsulfonyl)-2,3-dihydroquinolin-4(1H)-one (47).

Trifluoroacetic acid (10 mL) and trifluoroacetic anhydride (3.2 mL) were added to (46) (0.8 g, 2.14 mmol). The homogenous solution was heated to reflux for 7 hours, cooled and poured into ice water. The product was extracted with EtOAc. The organic phase was washed with saturated aqueous NaHCO₃ (3 x 25 mL), dried over MgSO₄, and concentrated. The residue was chromatographed with HPLC to afford (47) (0.192 g, 25%). MS *m*/*z* 355.9 (M+H)⁺.

### 8-Chloro-5-(4-chlorophenylsulfonyl)-4,5-dihydro-1H-pyrazolo[4,3-c]quinoline (49).

Dimethylformamide dimethyl acetal (1 mL) was added to (47) (192 mg, 0.54 mmol). The reaction was heated at 103 °C for 20 minutes and then cooled to room temperature. HPLC/MS analysis of the reaction mixture determined compound (48) present, (M+H)⁺ = 394.9, with no remaining (47). Volatiles were removed and the crude product was used directly in the next reaction. To an acetic acid solution (1.35 mL) of crude (48) was added hydrazine hydrate (26 µL, 0.54 mmol). The reaction mixture was heated at 95 °C for 1 hour. Solvent was removed and the residue was chromatographed on HPLC to afford (49) (153 mg, 75 %). MS m/z 379.9 (M+H)⁺. ¹H NMR (CD₃OD) δ 7.75-7.72 (d, J = 8.6 Hz, 1H), 7.64-7.63(d, J = 2.4 Hz, 1H) 7.44-7.40(m 2H), 7.19-7.18 (m, 4H), 4.95 (s, 2H); ¹³C NMR (CD₃OD) δ 138.9, 136.1, 133.5, 133.4, 130.1, 128.5, 128.2, 127.6, 121.9, 112.3, 43.0.

### Example 10

### 4-Methyl-5-(pyridin-3-ylsulfonyl)-4,5-dihydro-2H-pyrazolo[4,3-c]quinoline (55).

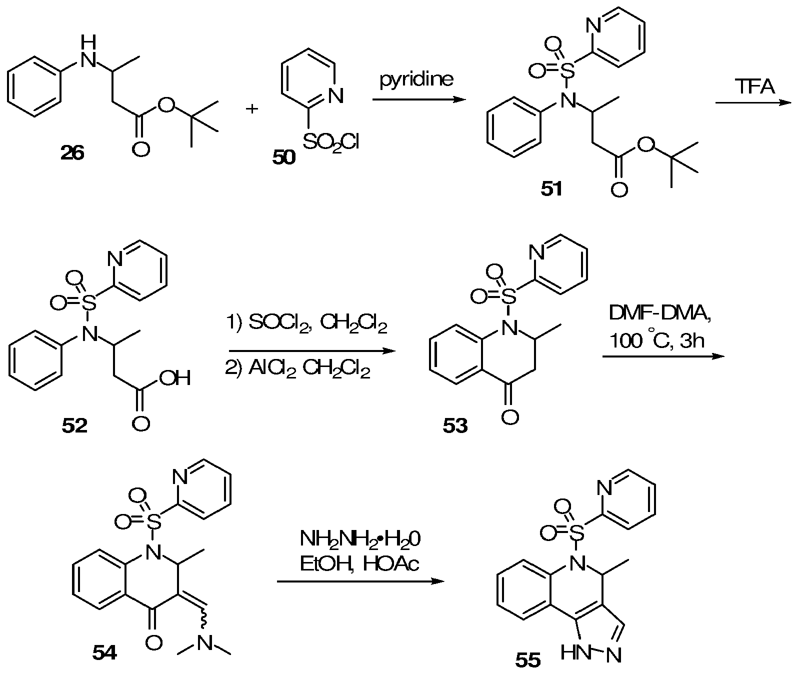

### tert-Butyl 3-(N-phenylpyridine-2-sulfonamido)butanoate (51).

To a solution of (26) (2.74 g, 11.6 mmol) in pyridine (46 mL) at 0 °C was added 2-pyridylsulfonyl chloride (50), (5 g, 23.4 mmol). The reaction was warmed to room temperature and stirred overnight. The reaction mixture was then concentrated and the residue taken up in diethyl ether (150 mL). The organic phase was washed with water (1 x 200 mL), 3N HCl (1 x 50 mL), sat. aq. NaHCO₃ (1 x 50 mL), water (1 x 50mL), brine (1 x 20 mL), dried (MgSO₄), filtered, and concentrated to yield (51) (4.192 g, 96%) as a light yellow oil. MS *m*/*z* 377.1 (M+H)⁺.

### 3-(N-Phenylpyridine-2-sulfonamido)butanoic acid (52).

To (51) (4.19 g, 11.1 mmol) was added TFA (8.5 mL) at room temperature and the mixture was sonicated to form a homogeneous solution. After stirring for 1.5 hours the reaction mixture was concentrated, then dissolved in CH₂Cl₂ (200 mL) and extracted with sat. aq. NaHCO₃ (4 x 100 mL). Collected aqueous extracts were extracted with CH₂Cl₂ and then the pH was adjusted to 1. The aqueous layer was then extracted with EtOAc (4 x 100 mL). Collected organic extracts were dried over MgSO₄, filtered, and concentrated to afford (52) (3.26 g, 92%). MS *m*/*z* 321.0 (M+H)⁺.

### 2-Methyl-1-(pyridin-2-ylsulfonyl)-2,3-dihydroquinolin-4(1H)-one (53).

To a solution of (52) (1.32 g, 4.11 mmol) in CH₂Cl₂ (30 mL) was added thionylchloride (0.45 mL, 6.16 mmol). After 20 min at room temperature, the reaction mixture was transferred over 15 min to a 0 °C CH₂Cl₂ (40 mL) solution of AlCl₃ (1.36 g, 10.27 mmol). The reaction mixture was then allowed to warm to room temperature and stirred overnight. In the morning, 6N HCl (30 mL) was slowly added to the reaction mixture at 0°C. The resulting biphasic mixture was rapidly stirred for 1.5 hours, and then the layers were separated. The organic phase was washed with water (1 x 50 mL), sat. aq. NaHCO₃ (1 x 50 mL), water (1 x 50 mL), brine (1 x 50 mL), dried (MgSO₄), filtered, and concentrated to obtain (53) (1.22 g, 98%) MS *m*/*z* 303.0 (M+H)⁺.

### 4-Methyl-5-(pyridin-2-ylsulfonyl)-4,5-dihydro-1H-pyrazolo[4,3-c]quinoline (55).

A dimethylformamide dimethyl acetal (6.5 mL) solution of (53) (1.46 g, 4.83 mmol) was heated at 100 °C for 2.5 hours. HPLC/MS analysis of the reaction mixture determined no starting material remained so the reaction was cooled to room temperature and concentrated to obtain the crude product (54), which was used directly in the next reaction. To a glacial acetic acid solution (12 mL) of (54) was added hydrazine hydrate (257 µL, 5.31 mmol). The reaction mixture was heated to 80 °C for 45 min, concentrated, and the residue taken up in diethyl ether (100 mL). The organic phase was washed with water (1 x 25 mL), sat. aq. NaHCO₃ (1 x 20 mL), brine (1 x 25 mL), dried over MgSO₄, filtered and concentrated. The crude product was purified by preparative HPLC chromatography to obtain (55) as a light yellow solid. MS m/z 327.0 : (M+H)⁺. ¹H NMR (CDCl₃) δ 8.45-8.43 (m, 2H), 7.86-7.83 (dd, J = 7.8, 1.3 Hz, 1H), 7.66-7.63 (dd, J = 7.4, 1.6 Hz, 1H), 7.47-7.28 (m, 4H), 7.03-6.99 (m, 1H), 5.75-5.68 (q, J = 6.9 Hz, 1H), 1.35-1.33 (d, J = 6.9 Hz, 3H); ¹³C NMR (CDCl₃) δ 155.7, 149.3, 137.0, 132.5, 129.3, 128.4, 127.3, 126.4, 124.6, 122.6, 122.1, 118.7, 50.2, 23.1.

### Example 11

### 5-(4-Chlorophenylsulfonyl)-4-methyl-4,5-dihydro-1H-pyrazolo[4,3-c] [1,5]naphthyridine (62).

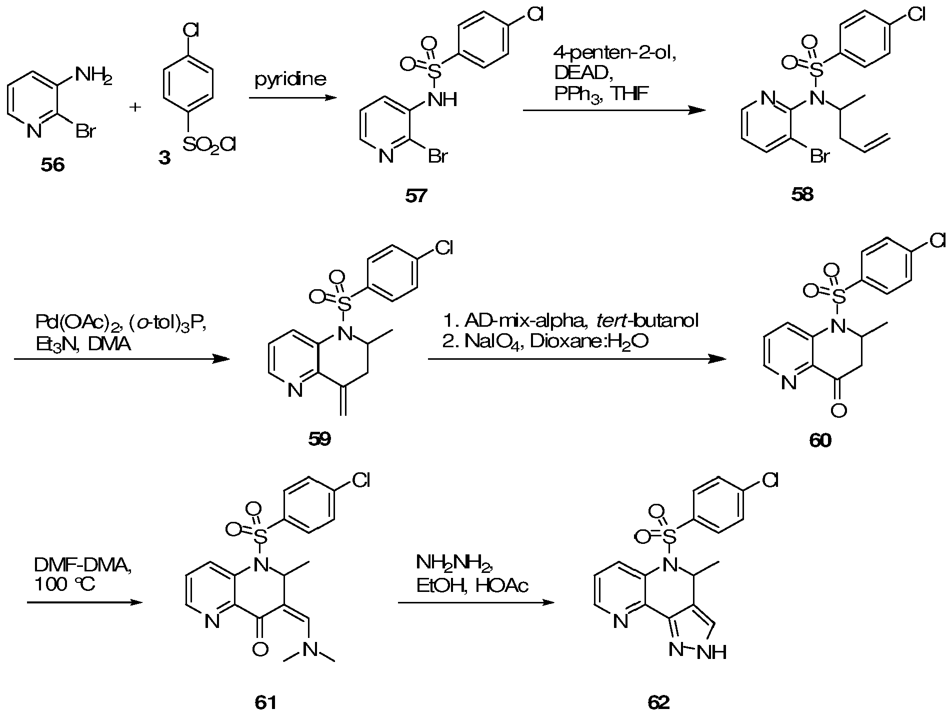

### N-(2-Bromopyridin-3-yl)-4-chlorobenzenesulfonamide (57).

To a solution of 3-amino-2-bromopyridine (56), (2.00 g, 11.6 mmol) in pyridine (10 mL) was added *p*-chlorobenzenesulfonyl chloride (3), 2.44 g, 11.6 mmol). The reaction mixture was stirred at room temperature for 24h, and then filtered and concentrated. The resulting residue was dissolved in CH₃CN (10 mL) and purified by reverse-phase HPLC. The resulting residue was dissolved in CH₂Cl₂ (20 mL) and washed with saturated aqueous NaHCO₃ (2 x 30 mL). The organic layer was dried with MgSO₄, filtered and concentrated to afford (57) (0.59 g, 25%). MS (ES) *m*/*z* 348.8 (M+H)⁺.

### N-(3-Bromopyridin-2-yl)-4-chloro-N-(pent-4-en-2-yl)benzenesulfonamide (58).

To a solution of (57) (1.71 g, 4.93 mmol) in THF (10 mL) was added 4-penten-2-ol (0.51 mL, 4.93 mmol), followed by triphenylphosphine (1.55 g, 5.92 mmol). Diethyl azodicarboxylate (1.08 mL, 5.92 mmol) was added drop-wise to the reaction mixture. The reaction mixture was stirred at room temperature for 24h, and then concentrated. The resulting residue was dissolved in CH₃CN (10 mL) and purified by reverse-phase HPLC. The resulting residue was dissolved in CH₂Cl₂ (20 mL) and washed with saturated aqueous NaHCO₃ (2 x 30 mL). The organic layer was dried over MgSO₄, filtered and concentrated to afford (58) (0.68 g, 33%). MS (ES) *m*/*z* 416.9 (M+H)⁺.

### 1-(4-Chlorophenylsulfonyl)-2-methyl-4-methylene-1,2,3,4-tetrahydro-1,5-naphthyridine (59).

To a solution of (58) (0.68 g, 1.64 mmol) in DMA (5 mL) was added palladium acetate (0.03 g, 0.16 mmol), tri(*o*-tolyl)phosphine (0.16 g, 0.54 mmol), and triethylamine (0.41 mL, 2.95 mmol). The reaction mixture was heated to 150 °C for 8h. The reaction mixture was then cooled to room temperature, diluted with CH₂Cl₂ (10 mL), and washed with H₂O (15 mL) and brine (15 mL). The organic layer was dried over MgSO₄, filtered and concentrated. The resulting residue was purified by flash chromatography (20% EtOAc in hexanes) to afford (59) (0.31 g, 57%). MS (ES) *m*/*z* 335.0 (M+H)⁺.

### 1-(4-Chlorophenylsulfonyl)-2-methyl-2,3-dihydro-1,5-naphthyridin-4(1H)-one (60).

To a solution of (59) (0.21 g, 0.63 mmol) in a 1:1 mix of *tert*-butanol (3 mL) and H₂O (3 mL) at 0 °C was added AD-mix-α (0.88 g, 1.4 g/mmol). The reaction mixture was stirred for 24h while slowly warming to room temperature. The temperature was then decreased to 0 °C and sodium sulfite (1.51 g, 1.5 g/mmol) was added. After 10 minutes at 0 °C, the ice bath was removed and the reaction mixture was stirred at room temperature for 45 minutes. The reaction mixture was diluted with CH₂Cl₂ (10 mL) and H₂O (10 mL) and the two layers were separated. The organic layer was washed with brine (15 mL), dried over MgSO₄, filtered and concentrated. The resulting residue was dissolved in a 1:1 mixture of dioxane (2 mL) and H₂O (2 mL), and sodium periodate (0.13 g, 0.63 mmol) was added. The reaction mixture was stirred at room temperature for 30 minutes. The reaction mixture was diluted with CH₂Cl₂ (10 mL) and H₂O (10 mL) and the two layers were separated. The organic layer was dried over MgSO₄, filtered and concentrated. The resulting residue was purified by flash chromatography (70% EtOAc in hexanes) to afford (60) (0.13 g, 61%). MS (ES) *m*/*z* 337.0 (M+H)⁺.

### 5-(4-Chlorophenylsulfonyl)-4-methyl-4,5-dihydro-2H-pyrazolo[4,3-c][1,5]naphthyridine (62).

Dimethylformamide dimethylacetal (1 mL) and (60) (0.09 g, 0.28 mmol) were combined and heated to 100 °C for 2h. The reaction mixture was then cooled to room temperature and concentrated. The resulting residue was dissolved in CH₂Cl₂ (15 mL) and washed with H₂O (10 mL) and brine (15 mL). The organic layer was dried with MgSO₄, filtered and concentrated. The resulting residue (61) was dissolved in MeOH (1 mL) and the temperature was decreased to 0 °C. Anhydrous hydrazine (0.02 mL, 0.55 mmol) was added to the reaction mixture, followed by HOAc (0.03 mL, 0.55 mmol). The reaction mixture was stirred for 18h while warming to room temperature. The reaction mixture was concentrated. The resulting residue was dissolved in CH₂Cl₂ (15 mL) and washed with H₂O (15 mL), saturated aqueous NaHCO₃ (15 mL), and brine (15 mL). The organic layer was dried over MgSO₄, filtered and concentrated. The resulting residue was purified by flash chromatography on alumina (5% MeOH in CH₂Cl₂) to afford (62) (0.03 g, 32%); ¹H NMR (CDCl₃) δ 8.64 (dd, J = 4.8, 1.5 Hz, 1H), 8.21 (dd, J = 8.2, 1.5 Hz, 1H), 7.38 (dd, J = 8.2, 4.8 Hz, 1H), 7.36 (s, 1H), 7.17 (m, 2H), 7.09 (m, 2H), 5.67 (q, J = 7.0 Hz, 1H), 1.37 (d, J = 6.8 Hz, 3H); ¹³C NMR (CDCl₃) δ 147.7, 141.6, 139.6, 137.0, 136.0, 135.3, 129.2, 128.8, 128.3, 123.2, 121.0, 50.7, 23.8. MS (ES) *m*/*z* 361.0 (M+H)⁺.

### Example 12

### 4-Cyclopropyl-7,8-difluoro-5-(4-(trifluoromethyl)phenylsulfonyl)-4,5-dihydro-2H-pyrazolo[4,3-c]quinoline (76).

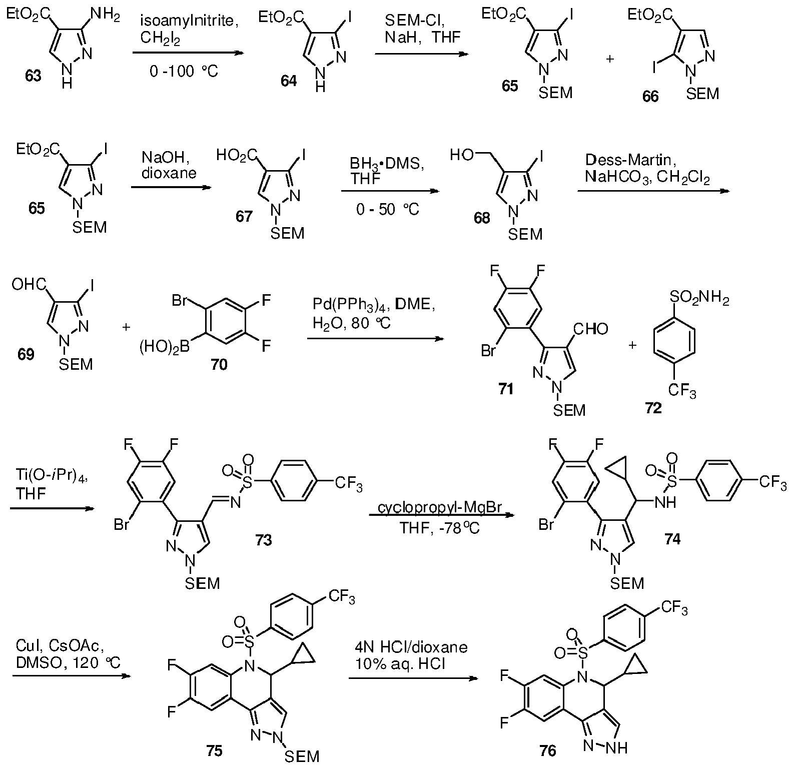

### Ethyl 3-iodo-1H-pyrazole-4-carboxylate (64).

Isoamylnitrite (400 mL, 2.98 mol) was added to a heterogenous mixture of ethyl 3-amino-1*H*-pyrazole-4-carboxylate (63), 47.69 g, 307 mmol) in diiodomethane (800 mL) at - 10 °C over a period of 30 minutes. The heterogeneous mixture was placed into a preheated oil bath at 100 °C for 2 hours. The solution was cooled to ambient temperature, diluted with saturated aqueous sodium sulfite, and extracted with EtOAc. The combined organic extracts were dried over MgSO₄, filtered, and concentrated under reduced pressure. The residue was flash chromatographed on silica using 9:1, 4:1, 7:3, 3:2, and 1:1 hexanes:EtOAc as the eluant to yield 53.85 g (66%) of (64) as a light yellow solid.
Method 1: Retention time 1.26 min by HPLC. MS *m*/*z* 267 (M+H)⁺.

### Ethyl 3-iodo-1-((2-(trimethylsilyl)ethoxy)methyl)-1H-pyrazole-4-carboxylate (65) and Ethyl 5-iodo-1-((2-(trimethylsilyl)ethoxy)methyl)-1H-pyrazole-4-carboxylate (66).

Sodium hydride (6.00 g, 150 mmol) as a 60% dispersion in mineral oil was added to a solution of ethyl 3-iodo-1*H*-pyrazole-4-carboxylate (64), (28.21 g, 106 mmol) in tetrahydrofuran (400 mL). After stirring for 1 hour, (2-(chloromethoxy)ethyl)trimethylsilane (25.0 mL, 142 mmol) was added. After stirring for 18 hours, the heterogeneous mixture was diluted with saturated aqueous NaHCO₃ and extracted with diethyl ether. The combined organic extracts were dried over MgSO₄, filtered, and concentrated under reduced pressure. The residue was flash chromatographed with 49:1, 24:1, 23:2, 22:3, 21:4 and 4:1 hexanes:EtOAc as the eluant to yield 22.50 g (54%) of (65) as a light yellow liquid and 18.00 g (43%) of (66) as a light yellow liquid.
¹H NMR (CDCl₃) δ 8.02 (s, 1H), 5.58 (s, 2H), 4.33 (q, J=7.2 Hz, 2H), 3.60 (t, J=8.1 Hz, 2H), 1.37 (t, J=7.2 Hz, 3H), 0.91 (t, J=8.1 Hz, 2H), -0.03 (s, 9H).
¹H NMR (CDCl₃) δ 7.97 (s, 1H), 5.40 (s, 2H), 4.31 (q, J=7.2 Hz, 2H), 3.58 (t, J=8.4 Hz, 2H), 1.35 (t, J=7.2 Hz, 3H), 0.90 (t, J=8.4 Hz, 2H), -0.03 (s, 9H).

### 3-Iodo-1-((2-(trimethylsilyl)ethoxy)methyl)-1H-pyrazole-4-carboxylic acid (67).

Ethyl 3-iodo-1-((2-(trimethylsilyl)ethoxy)methyl)-1*H*-pyrazole-4-carboxylate (65), (22.50 g, 56.8 mmol) and 3N aqueous sodium hydroxide (50.0 mL, 150 mmol) in dioxane (200 mL) was stirred for four days. The solution was brought to pH=3 and extracted with CH₂Cl₂. The combined organic extracts were dried over MgSO₄, filtered, and concentrated under reduced pressure to yield 20.91 g (100%) of (67) as a light yellow solid.
Method 7: Retention time 6.22 min by HPLC. MS *m*/*z* 369 (M+H)⁺, 391 (M+Na)⁺.

### 3-Iodo-1-((2-(trimethylsilyl)ethoxy)methyl)-1H-pyrazol-4-yl)methanol (68).

Neat borane dimethylsulfide *ca*. 10M (25 mL, 250 mmol) was slowly added to 3-iodo-1-((2-(trimethylsilyl)ethoxy)methyl)-1*H*-pyrazole-4-carboxylic acid (67), (20.91 g, 56.8 mmol) in THF (250 mL) at 0 °C. After stirring for 30 minutes, the solution was placed into a preheated oil bath at 50 °C. After stirring for 18 hours, the solution was cooled to 0°C and ice was added. After stirring for 30 minutes, saturated aqueous NH₄Cl was added and stirred for an additional 30 minutes. The solution was extracted with CH₂Cl₂ and the combined organic extracts were dried over MgSO₄, filtered, and concentrated under reduced pressure to yield 19.50 g (97%) of (68) as a light yellow liquid.
Method 7: Retention time 5.72 min by HPLC MS *m*/*z* 355 (M+H)⁺.

### 3-Iodo-1-((2-(trimethylsilyl)ethoxy)methyl)-1H-pyrazole-4-carbaldehyde (69).

Dess-Martin periodinane (13.80 g, 32.5 mmol) was added to a heterogeneous mixture of (3-iodo-1-((2-(trimethylsilyl)ethoxy)methyl)-1*H*-pyrazol-4-yl)methanol (68), 7.19 g, 20.3 mmol) and NaHCO₃ (17.44 g, 208 mmol) in CH₂Cl₂ (200 mL). After stirring for 24 hours, the heterogeneous mixture was diluted with saturated aqueous sodium sulfite and water, and then extracted with diethyl ether. The combined organic extracts were dried over MgSO₄, filtered, and concentrated. The residue was flash chromatographed on silica using 99:1 49:1, 24:1, 23:2, 22:3, 21:4, and 4:1 hexanes:EtOAc as the eluant to yield 7.15 g (100%) of (69) as a white solid.
Method 1: Retention time 2.43 min by HPLC. MS *m*/*z* 353, 375(M+Na)⁺.
¹H NMR (CDCl₃) δ 9.71 (s, 1H), 8.00 (s, 1H), 5.44 (s, 2H), 3.58 (m, 2H), 1.35 0.90 (m, 2H), - 0.03 (s, 9H).

### 3-(2-Bromo-4,5-difluorophenyl)-1-((2-(trimethylsilyl)ethoxy)methyl)-1H-pyrazole-4-carbaldehyde (71).

Tetrakis(triphenylphosphine)palladium(0) (1.29 g, 1.12 mmol), 3-iodo-1-((2-(trimethylsilyl)ethoxy)methyl)-1*H*-pyrazole-4-carbaldehyde (69), 3.53 g, 10.0 mmol), 2-bromo-4,5-difluorophenylboronic acid (70), 10.17 g, 42.9 mmol), and K₂CO₃ (15.91 g, 115 mmol) was placed into a flask that was then evacuated and refilled with nitrogen three times. Water (40 mL) and 1,2-dimethoxyethane (40 mL) were added and the solution placed into a preheated oil bath at 80 °C. After stirring for 18 hours, the solution was extracted with diethyl ether. The combined organic extracts were dried over MgSO₄, filtered, and concentrated. The residue was flash chromatographed on silica using 49:1, 24:1, 23:2, 22:3, 21:4, and 4:1 hexanes:EtOAc as the eluant to yield 2.10 g pure (50%) and 1.00 g slightly impure (<24%) of (71) as an orange oil.
Method 2: Retention time 2.04 min by HPLC MS *m*/*z* 359 and 361(M+H)⁺.

### N-((3-(2-Bromo-4,5-difluorophenyl)-1-((2-(trimethylsilyl)ethoxy)methyl)-1H-pyrazol-4-yl)(cyclopropyl)methyl)-4-(trifluoromethyl)benzenesulfonamide (74).

Titanium(IV) isopropoxide (3.0 mL, 10.2 mmol) was added to a solution of 3-(2-bromo-4,5-difluorophenyl)-1-((2-(trimethylsilyl)ethoxy)methyl)-1*H*-pyrazole-4-carbaldehyde (71), (1.00 g, 2.40 mmol) and 4-(trifluoromethyl)benzenesulfonamide (72), (970 mg, 4.31 mmol) in THF (10 mL). After stirring for 18 hours, the solution was diluted with CH₂Cl₂, then water, and the heterogeneous solution was filtered through Celite. The filtrate was extracted with CH₂Cl₂, the combined organic extracts were dried over MgSO₄, filtered, and concentrated. The residue was flash chromatographed on silica using 9:1, 4:1, 7:3, and 3:2 hexanes:EtOAc as the eluant to afford a mixture of products containing (73), which was used without further purification.
Method 2: Retention time 2.58 min by HPLC (M+=624 and 626).

A 1.0M solution of cyclopropyl magnesium bromide in THF (20.0 mL, 20.0 mmol) was slowly added along the walls of the flask to a solution of (73) in THF (20 mL) at -78 °C. After stirring for 30 minutes, the dry ice/acetone bath was removed and saturated aqueous NH₄Cl was added followed by water until the heterogeneous mixture became homogeneous. The biphasic solution was extracted with CH₂Cl₂. The combined organic extracts were dried over MgSO₄, filtered, and concentrated. The residue was flash chromatographed on silica using 9:1, 4:1, 7:3, and 3:2 hexanes:EtOAc as the eluant to yield 1.13 g (>60%, two steps) of (74) as a yellow oil.
Method 2: Retention time 2.55 min by HPLC. MS *m*/*z* 666 and 668(M+H)⁺

### 4-Cyclopropyl-7,8-difluoro-5-(4-(trifluoromethyl)phenylsulfonyl)-2-((2-(trimethylsilyl)ethoxy)-methyl)-4,5-dihydro-2H-pyrazolo[4,3-c]quinoline (75).

Cesium acetate (2.71g, 14.1 mmol), cuprous iodide (797 mg, 4.19 mmol), and N-((3-(2-bromo-4,5-difluorophenyl)-1-((2-(trimethylsilyl)ethoxy)methyl)-1*H*-pyrazol-4-yl)(cyclopropyl)methyl)-4-(trifluoromethyl)benzenesulfonamide (74), 1.13 g, 1.69 mmol) were placed into a flask that was then evacuated and refilled with nitrogen. Degassed DMSO (1.7 mL) was added and the heterogeneous mixture was placed into a preheated oil bath at 120 °C. After stirring for 2 hours, the solution was cooled to ambient temperature. The solid that formed was washed with CH₂Cl₂, and filtered. The filtrate was concentrated and the residue was flash chromatographed on silica using 19:1, 9:1, 17:3, and 4:1 hexanes:EtOAc as the eluant to yield 645 mg (65%) of (75) as a yellow oil.
Method 2: Retention time 2.82 min by HPLC MS *m*/*z* 586 (M+H)⁺ 608, (M+Na)⁺.

### 4-Cyclopropyl-7,8-difluoro-5-(4-(trifluoromethyl)phenylsulfonyl)-4,5-dihydro-2H-pyrazolo[4,3-c]quinoline (76).

4-Cyclopropyl-7,8-difluoro-5-(4-(trifluoromethyl)phenylsulfonyl)-2-((2-(trimethylsilyl)ethoxy)methyl)-4,5-dihydro-2*H*-pyrazolo[4,3-c]quinoline (75), 645 mg, 1.10 mmol) was stirred in 4N HCl in dioxane (10 mL) and 10% aqueous HCl (2 mL) for 18 hours. The solution was concentrated to yield (76) as a light yellow solid. Method 7: Retention time 8.34 min by HPLC. MS m/z 456.0(M+H)⁺, 478(M+Na)⁺.
¹H NMR (CDCl₃) δ 7.76 (dd, J = 11.1, 7.2 Hz, 1H), 7.47 (m, J = 10.2, 8.7 Hz, 1H), 7.39 (d, J = 8.7 Hz, 2H), 7.32 (d, J = 8.7 Hz, 2H), 7.20 (s, 1H), 4.95f (d, J = 7.8 Hz, 1H), 1.03 (m, 1H), 0.54 (m, 1H), 0.41 (m, 2H), 0.08 (m, 1H).

### Example 13

### (R)-4-Cyclopropyl-8-fluoro-5-(6-(trifluoromethyl)pyridin-3-ylsulfonyl)-4,5-dihydro-2H-pyrazolo[4,3-c]quinoline (86).

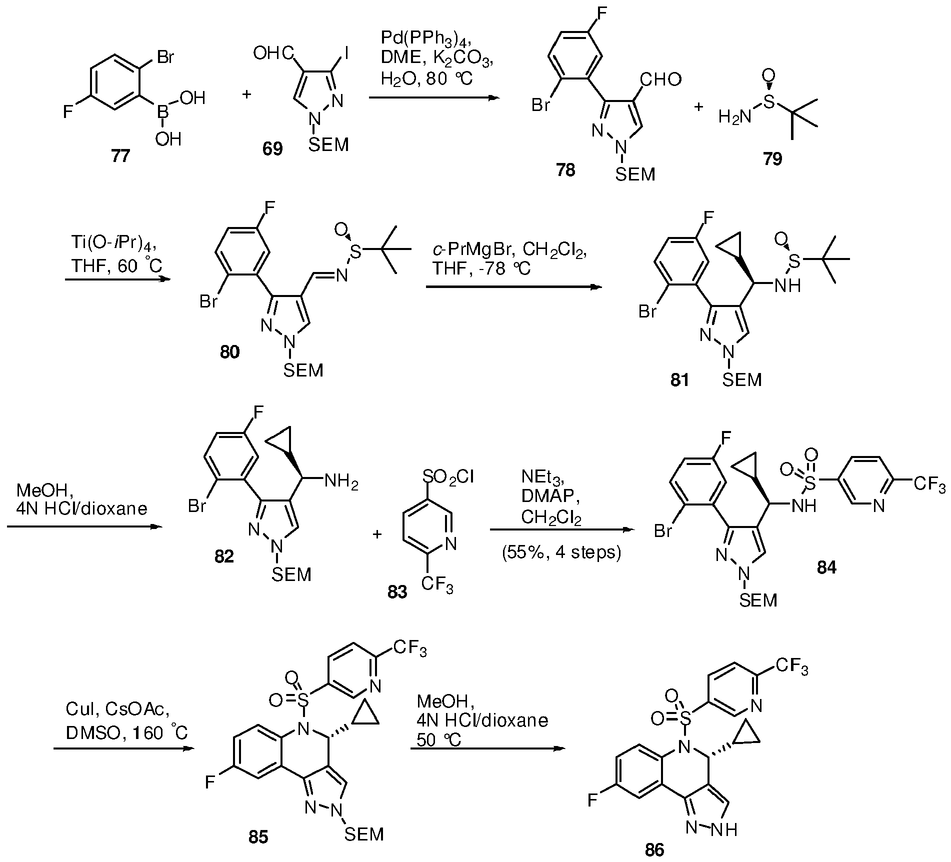

### 3-(2-Bromo-5-fluorophenyl)-1-((2-(trimethylsilyl)ethoxy)methyl)-1H-pyrazole-4-carbaldehyde (78).

Tetrakis(triphenylphosphine)palladium(0) (224 mg, 194 µmol), 3-iodo-1-((2-(trimethylsilyl)ethoxy)methyl)-1*H*-pyrazole-4-carbaldehyde (69), (1.71 g, 10.0 mmol), 2-bromo-5-fluorophenylboronic acid (77), 1.36 g, 6.23 mmol), and K₂CO₃ (2.86 g, 20.7 mmol) were placed into a flask that was evacuated and refilled with nitrogen three times. Water (5 mL) and 1,2-dimethoxyethane (5 mL) were added and the solution placed into a preheated oil bath at 80 °C. After stirring for 18 hours, the solution was extracted with diethyl ether. The combined organic extracts were dried over MgSO₄, filtered, and concentrated. The residue was flash chromatographed on silica using 49:1, 24:1, 23:2, 22:3, 21:4, and 4:1 hexanes:EtOAc as eluant to yield 590 mg (73%) of (78) as a brown oil.
Method 2: Retention time 1.92 min by HPLC MS *m*/*z* 399 and 401 (M+H)⁺.

### (S)-(E)-N-((3-(2-Bromo-5-fluorophenyl)-1-((2-(trimethylsilyl)ethoxy)methyl)-1H-pyrazol-4-yl)methylene)-2-methylpropane-2-sulfinamide (80).

Titanium(IV) isopropoxide (1.1 mL, 3.75 mmol) was added to a solution of 3-(2-bromo-5-fluorophenyl)-1-((2-(trimethylsilyl)ethoxy)methyl)-1*H*-pyrazole-4-carbaldehyde (78), 590 mg, 1.48 mmol) and (S)-(-)-*tert*-butanesulfinamide (79), 254 mg, 2.10 mmol) in THF (10 mL). After stirring for 18 hours, the solution was placed into a preheated oil bath at 60 °C. After stirring for 2 hours, the solution was cooled to ambient temperature, diluted with CH₂Cl₂, then water, and the heterogeneous solution was filtered through Celite. The filtrate was extracted with CH₂Cl₂, the combined organic extracts were dried over MgSO₄, filtered, and concentrated to yield (80) as an oil which was used without further purification.
Method 2: Retention time 2.28 min by HPLC. MS *m*/*z* 502 and 504(M+H)⁺.

### (S)-N-((R)-(3-(2-Bromo-5-fluorophenyl)-1-((2-(trimethylsilyl)ethoxy)methyl)-1H-pyrazol-4-yl)(cyclopropyl)methyl)-2-methylpropane-2-sulfinamide (81).

A 1.0M solution of cyclopropyl magnesium bromide in THF (15.0 mL, 15.0 mmol) was slowly added dropwise to a solution of (*S*)-(*E*)-*N*-((3-(2-bromo-5-fluorophenyl)-1-((2-(trimethylsilyl)ethoxy)methyl)-1*H*-pyrazol-4-yl)methylene)-2-methylpropane-2-sulfinamide (80) in CH₂Cl₂ (15 mL) at -78°C. After stirring for 30 minutes, the dry ice/acetone bath was removed and saturated aqueous NaHCO₃ was added followed by water until the heterogeneous mixture became homogeneous. The biphasic solution was extracted with CH₂Cl₂, the combined organic extracts were dried over MgSO₄, filtered, and concentrated to yield (81) as a brown oil which was used without further purification.
Method 7: Retention time 9.63 min by HPLC, MS *m*/*z* 544 and 546 (M+H)⁺. for the major diastereomer and 10.03 min by HPLC, MS *m*/*z* 544 and 546(M+H)⁺. for the minor diastereomer.

### (R)-(3-(2-Bromo-5-fluorophenyl)-1-((2-(trimethylsilyl)ethoxy)methyl)-1H-pyrazol-4-yl)(cyclopropyl)methanamine (82).

4 N HCl in dioxane (1 mL) was added to a solution of (*S*)-*N*-((*R*)-(3-(2-bromo-5-fluorophenyl)-1-((2-(trimethylsilyl)ethoxy)methyl)-1*H*-pyrazol-4-yl)(cyclopropyl)methyl)-2-methylpropane-2-sulfinamide (81) in MeOH (20 mL). After stirring for 1 hour, the solution was concentrated. The residue was diluted with 1 N aqueous NaOH, extracted with CH₂Cl₂, the combined organic extracts were dried over MgSO₄, filtered, and concentrated to yield (82) as a brown oil which was used without further purification.
Method 7: Retention time 5.57 min by HPLC MS *m*/*z* 423 and 425 (M-NH₂)⁺ and MS *m*/*z* 440 and 442(M+H)⁺.

### (R)-N-((3-(2-Bromo-5-fluorophenyl)-1-((2-(trimethylsilyl)ethoxy)methyl)-1H-pyrazol-4-yl)(cyclopropyl)methyl)-6-(trifluoromethyl)pyridine-3-sulfonamide (84).

4-(Trifluoromethyl)benzene-1-sulfonyl chloride (83), (835 mg, 3.40 mmol) was added to a solution of (*R*)-(3-(2-bromo-5-fluorophenyl)-1-((2-(trimethylsilyl)ethoxy)methyl)-1*H-*pyrazol-4-yl)(cyclopropyl)methanamine (82) and DMAP (120 mg, 982 µmol) in CH₂Cl₂ (5 mL) and triethylamine (1 mL). After stirring for 18 hours, the solution was concentrated, and the residue was flash chromatographed on silica using 9:1, 4:1, 7:3, and 3:2 hexanes:EtOAc as eluant to yield 525 mg (55%, 4 steps) of (84) as an oil.
Method 7: Retention time 11.00 min by HPLC MS *m*/*z* 649 and 651(M+H)⁺.

### (R)-4-Cyclopropyl-8-fluoro-5-(6-(trifluoromethyl)pyridin-3-ylsulfonyl)-2-((2-(trimethylsilyl)ethoxy)methyl)-4,5-dihydro-2H-pyrazolo[4,3-c]quinoline (85).

Cesium acetate (1.46 g, 7.61 mmol), cuprous iodide (446 mg, 2.34 mmol), and (*R*)-*N-*((3-(2-bromo-5-fluorophenyl)-1-((2-(trimethylsilyl)ethoxy)methyl)-1*H*-pyrazol-4-yl)(cyclopropyl)methyl)-6-(trifluoromethyl)pyridine-3-sulfonamide (84), 613 mg, 944 µmol) were placed into a flask that was evacuated and refilled with nitrogen. Degassed DMSO (1.0 mL) was added and the heterogeneous mixture was placed into a preheated oil bath at 160 °C. After stirring for 30 minutes, the solution was cooled to ambient temperature; the solid that formed was washed with CH₂Cl₂, and filtered through Celite. The filtrate was concentrated and the residue was flash chromatographed on silica using 19:1, 9:1, 17:3, 4:1, and 3:1 hexanes: EtOAc as eluant to yield 405 mg (75%) of (85) as a yellow oil.
Method 2: Retention time 2.66 min by HPLC MS *m*/*z* 569 (M+H)⁺, 591(M+Na)⁺.

### (R)-4-Cyclopropyl-8-fluoro-5-(6-(trifluoromethyl)pyridin-3-ylsulfonyl)-4,5-dihydro-2H-pyrazolo[4,3-c]quinoline (86).

4 N HCl in dioxane (10 mL) was added to a solution of (*R*)-4-cyclopropyl-8-fluoro-5-(6-(trifluoromethyl)pyridin-3-ylsulfonyl)-2-((2-(trimethylsilyl)ethoxy)methyl)-4,5-dihydro-2*H*-pyrazolo[4,3-c]quinoline (85), (1.30 g, 2.29 mmol) in MeOH (20 mL) was placed into a preheated oil bath at 50 0°C. After stirring for 1.5 hours, the solution was concentrated. The residue was diluted with saturated aqueous NaHCO₃, extracted with CH₂Cl₂, the combined organic extracts were dried over MgSO₄, filtered, and concentrated. The residue was flash chromatographed on silica using 9:1, 4:1, 7:3, and 3:2 hexanes:EtOAc as eluant to yield 1.00 g of (86) as a light yellow oil.
Method 7: Retention time 7.04 min by HPLC MS *m*/*z* 439 (M+H)⁺; 461(M+Na)⁺.
¹H NMR (CDCl₃) δ 10.13 (broad s, 1H), 8.51 (s, 1H), 7.85 (dd, J=12.0 and 6.4 Hz, 1H), 7.40 (m, 2H), 7.36 (d, J=3.0 Hz, 1H), 7.28 (s, 1H), 7.14 (dt, J=3.0 and 8.7 Hz, 1H), 4.99 (d, J=7.8 Hz, 1H), 1.01 (m, 1H), 0.54 (m, 1H), 0.39 (m, 2H), 0.09 (m, 1H).
Separation of the major and minor enantiomers was achieved using HPLC Method 18.

### Example 14

### 2-Bromo-5-fluorophenylboronic acid (88).

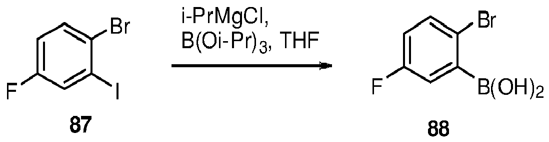

### 2-Bromo-5-fluorophenylboronic acid (88).

Isopropylmagnesium chloride (36.5 mmol, 2.0 M in THF) was slowly added to a -42 °C THF (2.0 M) solution of 1-bromo-4-fluoro-2-iodobenzene (87), (10 g, 33.2 mmol) under N₂, and stirred at -42 °C for 2 h. Triisopropylborate (11.4 mL, 49.8 mmol) was added and the reaction was warmed to room temperature and stirred overnight. 1N NaOH (20 mL) was added and the mixture stirred for 1h. The pH was adjusted to 3 with 3N HCl, and the sample was extracted (2 x 20 mL) with EtOAc. The combined organics were dried over MgSO₄, filtered through Celite, and the solvent was removed to obtain (5.75 g, 26.39 mmol) of 2-bromo-5-fluorophenylboronic acid (88) as a white solid. ¹H NMR (CDCl₃) δ 7.68-7.64 (1H, dd, J = 9.1, 3.2 Hz), 7.54-7.49 (1H, dd, J = 8.8, 4.8 Hz), 7.08-7.01 (1H, m), 5.76 (2H, s).

### Example 15

### 5-(4-chlorophenylsulfonyl)-4-(trifluoromethyl)-4,5-dihydro-2H-pyrazolo[4,3-c]quinoline (94).

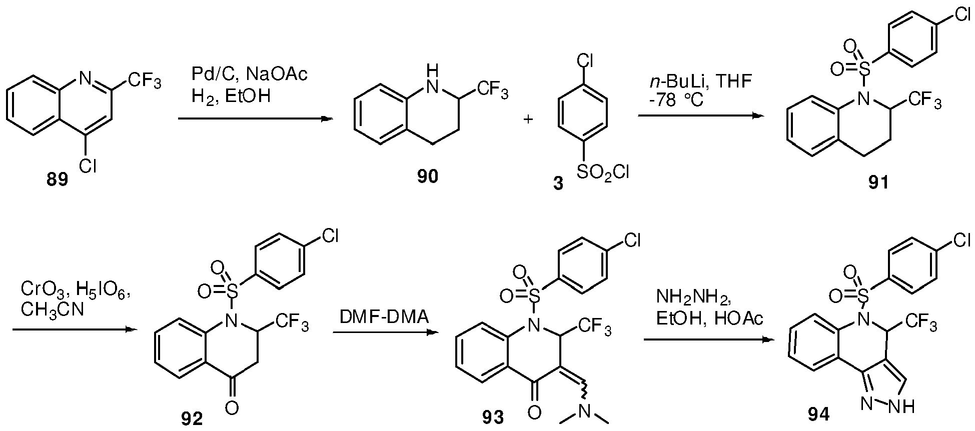

### 2-(Trifluoromethyl)-1,2,3,4-tetrahydroquinoline (90).

An ethanol solution (70 mL) of 4-chloro-2-(trifluoromethyl)quinoline (89) (5.48 g, 23.66 mmol), NaOAc (3.88 g, 47.32 mmol) and 10% Pd/C (500 mg) was placed under 45 psi of H₂ for one day. The reaction mixture was then filtered through a pad of Celite and the filtrate was concentrated. The residue was taken-up in EtOAc and washed with water (3 x 50 ml), brine (1 x 10 ml), dried over MgSO₄, filtered and concentrated to yield (90) (4.43 g, 93%) as a clear oil that solidified upon standing and was used without further purification. ¹H NMR (CDCl₃) δ 7.07-7.02 (m, 2H), 6.75-6.70 (m, 1H), 6.60 (d, J = 8.0Hz, 1H), 4.09 (br s, 1H), 3.92-3.84 (m, 1H), 2.85-2.81 (m, 2H), 2.15-2.08 (m, 2H); MS *m*/*z* 202.1 (M+H)⁺.

### 1-(4-Chlorophenylsulfonyl)-2-(trifluoromethyl)-1,2,3,4-tetrahydroquinoline (91).

To a THF solution (5.5 mL) of (90) (271 mg, 1.34 mmol) at -78 °C was added n-BuLi (2.5M in hexanes, 566 µL, 1.41 mmol). After 10 minutes a THF solution (2mL) of 4-chlorobenzenesulfonyl chloride (341 mg, 1.62 mmol) was added over a 5 minute period to give a black solution. The reaction mixture was held at -78 °C one hour and then warmed to - 30 °C. After 30 minutes the mixture was cooled to -78 °C, quenched with the addition of saturated NH₄Cl, warmed to room temperature and diluted with EtOAc and water. The organic portion was washed with 0.2 N citric acid (3 x 15 mL), water (1 x 15 mL), sat. NaHCO₃ (3 x 15 mL), brine (1 x 10 mL), dried over MgSO₄, filtered and concentrated to yield the crude product which was purified by flash chromatography eluting with 25:1 hexanes/EtOAc to give (91) (178mg, 35%).
¹H NMR (CDCl₃) δ 7.59 (d, J = 7.7 Hz, 1H), 7.42-7.27 (m, 5H), 7.22-7.18 (m, 1H), 7.00, (d, J = 7.1 Hz, 1H), 4.91-4.84 (m, 1H), 2.40-2.27 (m, 2H), 1.81-1.67 (m, 1H), 1.51-1.42 (m, 1H); MS *m*/*z* 376.0 (M+H)⁺.

### 1-(4-Chlorophenylsulfonyl)-2-(trifluoromethyl)-2,3-dihydroquinolin-4(1H)-one (92).

To a solution of H₅IO₆ (594 mg, 2.61mmol) in CH₃CN (3 mL) was added CrO₃ (7 mg, 70.1 µmol). After stirring for a few minutes (91) (178 mg, 0.47 mmol) was added to the reddish solution to give a heterogeneous reaction mixture solution. TLC analysis (10:1 hexane/EtOAc) after 6 hours determined no starting material remained so the reaction mixture was diluted with EtOAc (10 mL) and washed with water (2 x 10 mL), 5% NaHSO₃ (2 x 10 mL), brine (1 x 20 mL), dried over MgSO₄, filtered and concentrated to give the crude product. Purification by preparative TLC (2:1 hexanes/EtOAc) gave 92 (37.6 mg, 20%). ¹H NMR (CDCl₃) δ 7.95-7.92 (m, 1H), 7.86-7.83 (m, 1H), 7.66-7.56 (m, 3H), 7.45-7.32 (m, 3H), 5.28-5.23 (m, 1H), 2.84 (dd, J = 19.9, 1.6 Hz, 1H), 2.64 (dd, J = 18.7, 7.70 Hz, 1H); MS *m*/*z* 390.0 (M+H)⁺.

### 5-(4-Chlorophenylsulfonyl)-4-(trifluoromethyl)-4,5-dihydro-2H-pyrazolo[4,3-c]quinoline (94).

A dimethylformamide dimethyl acetal (173 µL) solution of (92) (34 mg, 87 µmol) was heated at 100 °C for 1 hour. HPLC/MS analysis of the reaction mixture determined no starting material remained so the reaction was cooled to room temperature and diluted with EtOAc (5 mL). The organic phase was washed with water (3 x 10 mL), brine (1 x 20 mL), dried over MgSO₄, filtered and concentrated to give (93) which was used directly in the next reaction. To an EtOH/glacial HOAc solution (25:1 v/v, 400 µL) of (93) (39 mg, 90 µmol) was added hydrazine hydrate (21 µL, 0.45 mmol). The reaction mixture was stirred 5 days at room temperature, transferred to a sealed tube and heated at 75 °C for 5 days. The reaction mixture was then cooled to room temperature, concentrated, and the residue taken up in EtOAc (10 mL). The organic phase was washed with sat. NaHCO₃ (3 x 20 mL), brine (1 x 10 mL), dried over MgSO₄, filtered and concentrated. The crude product was purified by preparative HPLC to give (94) (6.7 mg, 19% over two steps) as a white powder. ¹H NMR (CDCl₃) δ 7.88 (dd, J = 7.70, 1.1 Hz, 1H), 7.67 (dd, J = 8.5, 1.6 Hz, 1H), 7.53 (s, 1H), 7.43-7.36 (m, 2H), 7.25-7.17 (m, 4H), 6.03 (q, J = 7.1 Hz, 1H); MS *m*/*z* 414.0 (M+H)⁺.

### Example 16

### 6-(Trifluoromethyl)pyridine-3-sulfonyl chloride (83).

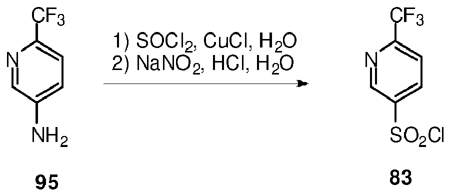

### 6-(Trifluoromethyl)pyridine-3-sulfonyl chloride (83).

Thionyl chloride (31 mL, 0.43 mol) was slowly added to 175 mL of water at 0 °C. During the addition the temperature was maintained between 0-5 °C. After addition the solution was warmed to 15 °C and 0.47 g (4.8 mmol) of CuCl was added. The solution was diluted with 100 mL of water and cooled back to 0 °C.

A solution of 7.21 g (0.10 mol) of NaNO₂ in 100 mL of water was slowly added to a solution of 15.39 g (0.10 mol) of 5-amino-2-(trifluoromethyl)pyridine (95) in 125 mL of conc. HCl at 0 °C. During addition the temperature was maintained between 0-5 °C. This mixture was then slowly added to the above prepared solution so as to maintain a temperature between 0-5 °C. A voluminous precipitate formed. The mixture was stirred for an additional 30 min after addition and the solid was then collected by filtration. The solid was washed with water and dissolved in CHCl₃. The solution was dried over MgSO₄, filtered and the solvent was removed to afford 18.03 g (77%) of sulfonyl chloride (83) as a tan solid. ¹H NMR (CDCl₃) δ 9.34 (d, J = 2.2 Hz, 1H), 8.53 (dd, J = 8.4, 2.2 Hz, 1H), 7.98 (d, J = 8.4 Hz, 1H).

### Example 17

### 5-(4-Chlorophenylsulfonyl)-4-methyl-4,5-dihydro-1H-imidazo[1,2-a]pyrazolo[4,3-e]pyrimidine (104).

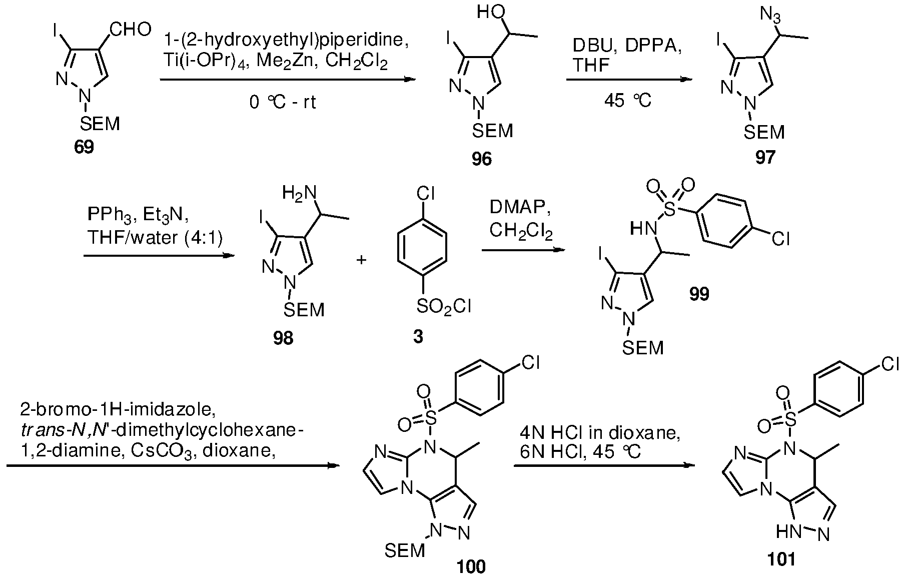

### 1-(3-Iodo-1-((2-(trimethylsilyl)ethoxy)methyl)-1H-pyrazol-4-yl)ethanol (96).

To a stirring mixture of 1-(2-hydroxyethyl)piperidine (149 mg, 1.09 mmol) in dry CH₂Cl₂ (25 mL) under N₂ at room temperature was added Ti(iOPr)₄. After 1 h of stirring, the reaction mixture was cooled to 0 °C and a 2.0 M solution of dimethyl zinc in toluene (17 mL, 33 mmol) was added. After 30 min, a solution of aldehyde (69) (1.45 g, 5.45 mmol) in CH₂Cl₂ (2 mL) was added and the reaction was stirred overnight. The reaction mixture was quenched with 1N HCl and the layers were separated. The aqueous layer was extracted with CH₂Cl₂ (3 x 10 mL). The combined organic layers were dried over MgSO₄, filtered, and concentrated under reduced pressure. The residue was flash chromatographed with 9:1, 4:1, and 3:1 hexanes:EtOAc as the eluant to yield 1.3 g (85%) of (96) as a colorless oil. The product was analyzed by HPLC/MS using Method 1. Retention time = 2.199 min. MS *m*/*z* 369.0 (M+H)⁺. ¹H NMR (300 MHz, CDCl₃) δ 7.45 (s, 1H), 5.38 (s, 2H), 4.79 (q, *J* = 6.1 Hz, 1H), 3.58 (t, *J* = 7.7 Hz, 2H), 1.53 (d, *J* = 6.1 Hz, 3H), 0.91 (t, *J* = 8.2 Hz, 2H), -0.03 (s, 9H).

### 4-(1-Azidoethyl)-3-iodo-1-((2-(trimethylsilyl)ethoxy)methyl)-1H-pyrazole (97).

To a stirring mixture of the alcohol (96) (1.2 g, 3.26 mmol) in THF (0.5 M, 6.5 mL) at room temperature was added DBU (843 mg, 5.54 mmol), followed by DPPA (1.5 g, 5.54 mmol). The resulting mixture slowly turned dark brown, and it was slowly warmed up to 45 °C. The reaction mixture was cooled to room temperature and then diluted with CH₂Cl₂ and quenched with brine. The layers were separated. The aqueous layer was extracted with CH₂Cl₂ (3 x 25 mL). The combined organic extracts were dried over MgSO₄, filtered, and concentrated under reduced pressure. The residue was flash chromatographed with 10:1 and 9:1 hexanes:EtOAc as eluant to yield 1.1 g of (97) as a colorless oil. The product was analyzed by HPLC/MS using Method 1. Retention time = 2.076 min. MS *m*/*z* 394.0 (M+H)⁺; ¹H NMR (300 MHz, CDCl₃) δ 7.47 (s, 1H), 5.41 (s, 2H), 4.47 (q, *J* = 7.0 Hz, 1H), 3.58 (t, *J* = 8.1 Hz, 2H), 1.53 (d, *J* = 6.6 Hz, 3H), 0.91 (t, *J* = 8.2 Hz, 2H), -2.0 (s, 9H).

### 1-(3-Iodo-1-((2-(trimethylsilyl)ethoxy)methyl)-1H-pyrazol-4-yl)ethanamine (98).

To a stirring mixture of the azide (97) (270 mg, 0.7 mmol) in THF/water was added PPh₃ (545 mg, 2.08 mmol) and Et₃N (0.58 mL, 4.15 mmol). The reaction mixture was warmed to 50 °C and stirred overnight. The reaction mixture was quenched with water and extracted with EtOAc. The combined organic layers were dried over MgSO₄, filtered, and concentrated under reduced pressure. The residue was flash chromatographed with 2:1, 1:1 hexanes/EtOAc and 10:1:0.1 EtOAc/MeOH/NH₄OH to give 240 mg of compound (98) as a colorless oil. The product was analyzed by HPLC/MS using Method 1. Retention time = 1.576 min. MS *m*/*z* 368.1 (M+H)⁺; ¹H NMR (300 MHz, CDCl₃) δ 7.66 (s, 1H), 5.37 (s, 2H), 4.17 (q, *J* = 6.9 Hz, 1H), 3.59 (t, *J* = 7.8 Hz, 2H), 1.56 (d, *J* = 6.5 Hz, 3H), 0.91 (d, *J* = 8.2 Hz, 2H), -1.3 (s, 9H).

### 4-Chloro-N-(1-(3-iodo-1-((2-(trimethylsilyl)ethoxy)methyl)-1H-pyrazol-4-yl)ethyl)benzenesulfonamide (99).

To a stirring mixture of amine (98) (100 mg, 0.27 mmol) in CH₂Cl₂ (1.0 mL) at room temperature was added 4-chlorobenzenesulfonyl chloride (3), (111 mg, 0.55 mmol), Et₃N, and DMAP (4 mg). The reaction mixture was reacted for 4 h or until all the starting material was consumed. The resulting mixture was diluted with NaHCO₃ and extracted with EtOAc. The residue was flash chromatographed with 2:1, 1:1 and 1:2 hexanes/EtOAc to give 70 mg of (99) as a colorless oil. The product was analyzed by HPLC/MS using Method 1. Retention time = 2.906 min. MS *m*/*z* 564.0 (M+Na)⁺.

### 5-(4-Chlorophenylsulfonyl)-4-methyl-1-((2-(trimethylsilyl)ethoxy)methyl)-4,5-dihydro-1H-imidazo[1,2-a]pyrazolo[4,3-e]pyrimidine (100).

To a stirring mixture of sulfonamide (99) (110 mg, 0.2 mmol) in deoxygenated dioxane (0.5 M) was added *trans-N,N*'-dimethylcyclohexane-1,2-diamine (114 mg, 0.812 mmol), CuI (38 mg, 0.2 mmol), CsCO₃ (263 mg, 0.81 mmol), and 2-bromo-1*H*-imidazole (38 mg, 0.24 mmol). The reaction mixture was quickly warmed to 110 °C and stirred for 1 h. The crude mixture was directly loaded onto a silica gel column and chromatographed to give 78 mg (80%) of (100) as a colorless oil. The product was analyzed by HPLC/MS using Method 2. Retention time = 1.429 min. MS *m*/*z* 480.1 (M+H)⁺.

### 5-(4-Chlorophenylsulfonyl)-4-methyl-4,5-dihydro-1H-imidazo[1,2-a]pyrazolo[4,3-e]pyrimidine (101).

Pyrimidine (100) was stirred in 4N HCl in dioxane (10 mL) and 6 N aqueous HCl (2 mL) for 18 hours. The solution was concentrated to yield compound (101) as a yellow solid. The desired product was further purified by preparative HPLC using the specified column type and analyzed using Method 1 to give a white solid. MS(ESI) *m*/*z* 350.0 (M+H)⁺. Retention time = 1.139 min. ¹H NMR (300 MHz, CDCl₃) δ 7.74-7.72 (m, 2H), 7.37-7.35 (m, 2H), 7.32 (s, 2H), 7.08-7.07 (m, 1H), 5.78 (q, *J* = 5.7 Hz, 1H), 1.36 (d, *J* = 6.6 Hz, 3H).

### Example 18

### 4-Cyclopropyl-5-(4-(trifluoromethyl)phenylsulfonyl)-4,5-dihydro-2H-pyrazolo[4,3-c][1,8]naphthyridine (108).

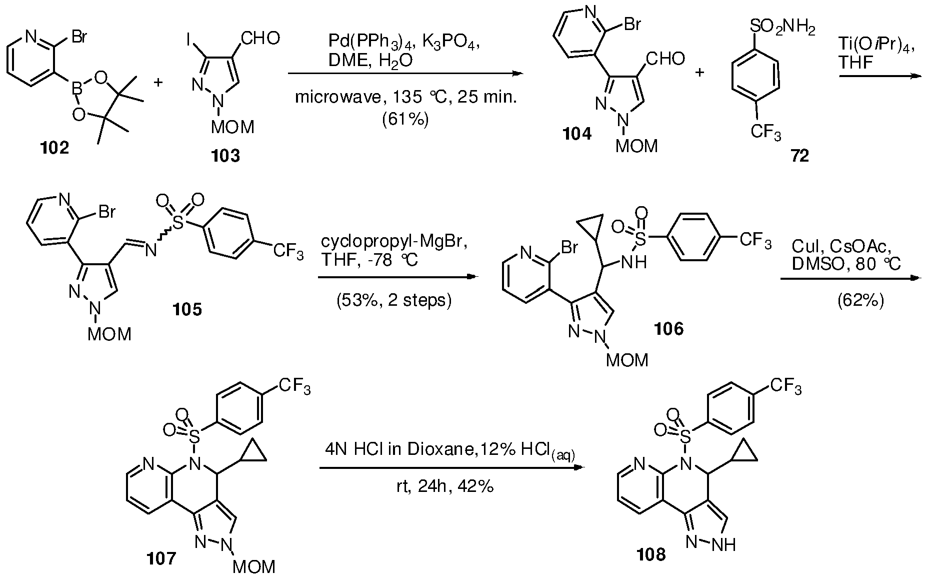

### 3-(2-Bromopyridin-3-yl)-1-(methoxymethyl)-1H-pyrazole-4-carbaldehyde (104).

To a solution of potassium phosphate (4.78 g, 22.5 mmol) in a 1:1 ethylene glycol dimethylether (10 mL) and H₂O (10 mL) mixture was added 2-bromopyridine-3-boronic acid pinacol ester (102) (2.13 g, 7.52 mmol). The reaction mixture was stirred at room temperature for 10 minutes before aldehyde (103) (1.00 g, 3.75 mmol) and palladium tetrakistriphenylphosphine (0.43 g, 0.37 mmol) were added. The reaction mixture was microwaved at 125 °C for 25 minutes. The reaction mixture was then diluted with EtOAc (25 mL) and the two layers were separated. The organic layer was washed with saturated aqueous NaCl (15 mL), dried over MgSO₄, filtered and concentrated. The resulting residue was purified by flash chromatography (50% EtOAc in hexanes) to afford (104) (0.68 g, 61%). MS (ES) *m*/*z* 295.9, 297.9 (M+H)⁺.

### N-((3-(2-Bromopyridin-3-yl)-1-(methoxymethyl)-1H-pyrazol-4-yl)( cyclopropyl)methyl)-4-(trifluoromethyl)benzenesulfonamide (106).

To a solution of (104) (0.68 g, 2.30 mmol) in THF (10 mL) was added sulfonamide (72) (0.77 g, 3.45 mmol), followed by titanium (IV) isopropoxide (1.30 g, 4.6 mmol). The reaction mixture was stirred at room temperature for 18 h. The reaction mixture was then diluted with EtOAc (15 mL), and then quenched with saturated aqueous NaCl (3 mL). The mixture was stirred for 10 minutes, and then was filtered through a pad of Celite. The two layers were separated. The organic layer was dried over MgSO₄, filtered and concentrated. The resulting imine, (105), was used in the next reaction without purification. To a solution of (105) in THF at -78 °C was added slowly cyclopropylmagnesium bromide (0.5M in THF, 9.20 mL, 4.60 mmol). The reaction mix was stirred for 2 h at -78 °C, then diluted with EtOAc (20 mL) and quenched with saturated aqueous NH₄Cl (10 mL). The mixture was warmed to room temperature and the two layers were separated. The organic layer was washed with saturated aqueous NaCl (15mL), dried over MgSO₄, filtered and concentrated. The resulting residue was purified by flash chromatography (50% EtOAc in hexanes) to afford (106) (0.66 g, 53% over two steps). MS (ES) *m*/*z* 544.8 and 546.8 (M+H)⁺.

### 4-Cyclopropyl-2-(methoxymethyl)-5-(4-(trifluoromethyl)phenylsulfonyl)-4,5-dihydro-2H-pyrazolo[4,3-c][1,8]naphthyridine (107).

Sulfonamide (106) (0.30 g, 0.55 mmol) was dissolved in DMSO (1 mL) and nitrogen was bubbled through the solution for 1 h. In a separate flame-dried flask, copper iodide (0.42 g, 2.20 mmol) and cesium acetate (1.05 g, 5.50 mmol) were combined. The reaction flask was evacuated, then refilled with nitrogen. This pump/purge process was repeated three times. The DMSO solution of sulfonamide (106) was added to the reaction flask and the flask was plunged into a preheated, 80 °C oil bath. The reaction mixture was stirred for 2 h at 80 °C, and then cooled to room temperature. The mixture was diluted with Et₂O (15 mL) and washed with H₂O (10 mL). The organic layer was dried over MgSO₄, filtered and concentrated. The resulting residue was purified by flash chromatography (50% EtOAc in hexanes) to afford (107) (0.16 g, 62%). MS (ES) *m*/*z* 464.9 (M+H)⁺.

### 4-Cyclopropyl-5-(4-(trifluoromethyl)phenylsulfonyl)-4,5-dihydro-2H-pyrazolo[4,3-c][1,8]naphthyridine (108).

To a solution of pyrazolonaphthyridine (107) (0.21 g, 0.45 mmol) in 4N HCl in dioxane (5 mL) was added 12% HCl (0.15 mL). The reaction mixture was stirred for 18 h at room temperature. The reaction mixture was diluted with EtOAc (15 mL), and the temperature was decreased to 0 °C. The reaction mixture was quenched with the addition of 1M NaOH until pH 7. The two layers were separated. The aqueous layer was washed with EtOAc (20 mL, 3x). The combined organic layers were dried over MgSO₄, filtered and concentrated. The resulting residue was purified by flash chromatography (50% EtOAc in hexanes) to afford (108) (0.14 g, 42%). ¹H NMR (CDCl₃) δ 8.32 (dd, *J* = 4.8, 1.6 Hz, 1H), 8.19 (d, *J* = 8.3 Hz, 2H), 8.07 (dd, *J* = 7.6, 1.5 Hz, 1H), 7.72 (d, *J* = 8.3 Hz, 2H), 7.52 (s, 1H), 7.16 (dd, *J* = 7.6, 4.9 Hz, 1H), 5.45 (d, *J* = 8.1 Hz, 1H), 1.15 (m, 1H), 0.51 (m, 2H), 0.38 (m, 1H), 0.21 (m, 1H); ¹³C NMR (75 MHz, CDCl₃) δ 159.9, 148.5, 148.1, 144.7, 141.2, 134.6, 134.2, 131.4, 128.9, 126.3, 125.8, 125.7, 121.5, 120.9, 116.0, 57.7, 18.0, 5.07, 2.67. MS (ES) *m*/*z* 420.9 (M+H)⁺.

### Example 19

### 4-Cyclopropyl-5-(4-(trifluoromethyl)phenylsulfonyl)-4,5-dihydro-2H-pyrazolo[4,3-c][1,5]naphthyridine (115).

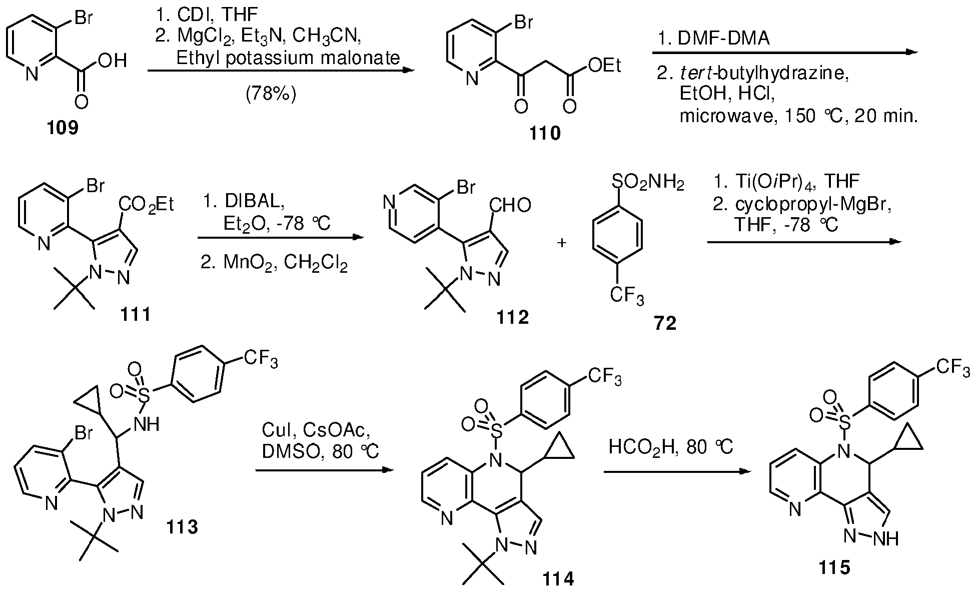

### Ethyl 3-(3-bromopyridin-2-yl)-3-oxopropanoate (110).

To a suspension of 3-bromo-2-pyridine-carboxylic acid (109) (2.00 g, 9.90 mmol) in THF (10 mL) was added carbonyl diimidazole (1.61 g, 9.90 mmol). The reaction mix was stirred for 18 h at room temperature. In a separate, flame-dried flask, potassium ethylmalonate (3.37 g, 19.8 mmol) was suspended in CH₃CN (40 mL) and Et₃N (3.00 g, 29.7 mmol) was added. The temperature was decreased to 0 °C. Magnesium chloride (2.35 g, 24.7 mmol) was added in two portions and the reaction mix was stirred at room temperature for 5 h. The temperature was then decreased to 0 °C and the THF solution of (109) was added slowly. The reaction mix was stirred at 0 °C for 10 minutes, then was warmed to room temperature and stirred for 18 h. The reaction mix was concentrated, and the resulting residue was suspended in toluene (70 mL). The temperature was decreased to 0 °C and the reaction mix was quenched by the slow addition of 12% HCl (50 mL). The reaction mix was warmed to room temperature and stirred for 30 minutes. The two layers were separated, and the aqueous layer was washed with EtOAc (50 mL, 3x). The combined organic layers were washed with saturated aqueous NaHCO₃ (50 mL), dried over MgSO₄, filtered and concentrated. The resulting residue was purified by flash chromatography (30% EtOAc in hexanes) to afford (110) (2.09 g, 78%). MS (ES) *m*/*z* 271.9, 273.9 (M+H)⁺.

### Ethyl 5-(3-bromopyridin-2-yl)-1-tert-butyl-1H-pyrazole-4-carboxylate (111).

The β-ketoester (110) (0.90 g, 3.31 mmol) was dissolved in dimethylformamide dimethylacetal (3 mL), and was heated to 50 °C for 15 min. The reaction mix was cooled to room temperature and concentrated. The resulting residue was dissolved in EtOH (15 mL) and *tert*-butylhydrazine hydrochloride (0.49 g, 4.97 mmol) was added followed by three drops of concentrated HCl. The reaction mix was stirred at room temperature for 10 minutes, and then was microwaved at 150 °C for 25 min. The reaction mixture was concentrated. The resulting residue was dissolved in EtOAc (25 mL) and was washed with H₂O (10 mL) and saturated aqueous NaHCO₃ (10 mL). The organic layer was dried with MgSO₄, filtered and concentrated. The resulting residue was purified by flash chromatography (20% EtOAc in hexanes) to afford (111) (1.47 g, 60%). MS (ES) *m*/*z* 351.9, 353.9 (M+H)⁺.

### 5-(3-Bromopyridin-4-yl)-1-tert-butyl-1H-pyrazole-4-carbaldehyde (112).

To a solution of (111) (1.00 g, 2.83 mmol) in Et₂O (10 mL) at -78 °C was added diisobutylaluminum hydride (1M in toluene, 7.09 mmol). The reaction mix was stirred at -78 °C for 1.5 h, and then was quenched with a 1:1 mixture of Celite and Na₂S₂O₃•5H₂O (2.5 g). The reaction mix was stirred while warming to room temperature and then was filtered and concentrated. The resulting residue was dissolved in CH₂Cl₂ (20 mL) and MnO₂ (2.46 g, 28.3 mmol) was added. The reaction mix was stirred at room temperature for 48 h. The reaction mixture was filtered through a pad of Celite and was concentrated. The resulting residue was purified by flash chromatography (50% EtOAc in hexanes) to afford (112) (0.27 g, 32%). MS (ES) *m*/*z* 307.9, 309.9 (M+H)⁺.

### N-((5-(3-Bromopyridin-2-yl)-1-tert-butyl-1H-pyrazol-4-yl)(cyclopropyl)methyl)-4-(trifluoromethyl)benzenesulfonamide (113).

To a solution of (112) (0.25 g, 0.81 mmol) in THF (5 mL) was added sulfonamide (72) (0.27 g, 1.21 mmol), followed by titanium (IV) isopropoxide (0.46 g, 1.62 mmol). The reaction mix was stirred at room temperature for 18 h. The reaction mix was diluted with EtOAc (10 mL), and then quenched with saturated aqueous NaCl (3 mL). The reaction mix was stirred for 10 minutes, and then was filtered through a pad of Celite. The two layers were separated. The organic layer was dried with MgSO₄, filtered and concentrated. To a solution of the resulting residue THF (5 mL) at -78 °C was added slowly cyclopropylmagnesium bromide (0.5M in THF, 16.2 mL, 8.11 mmol). The reaction mixture was stirred for 2 h at -78 °C, then diluted with EtOAc (20 mL) and was quenched with saturated NH₄Cl_{(aq)} (10 mL). The reaction mix was warmed to room temperature and the two layers were separated. The organic layer was washed with saturated aqueous NaCl (15 mL), dried over MgSO₄, filtered and concentrated. The resulting residue was purified by flash chromatography (30% EtOAc in hexanes) to afford (113) (0.17 g, 45% over two steps). MS (ES) m/z 556.9 and 558.9 (M+H)⁺.

### 1-tert-Butyl-4-cyclopropyl-5-(4-(trifluoromethyl)phenylsulfonyl)-4,5-dihydro-1H-pyrazolo[4,3-c][1,5]naphthyridine (114).

Sulfonamide (113) (0.82 g, 0.15 mmol) was dissolved in DMSO (1 mL) and nitrogen was bubbled through for 1 h. In a separate flame-dried flask, copper iodide (0.11 g, 0.59 mmol) and cesium acetate (0.28 g, 1.48 mmol) were combined. The reaction flask was evacuated, and then refilled with nitrogen. The pump/purge process was repeated three times. The DMSO solution of sulfonamide (113) was added to the reaction flask and the flask was plunged into a preheated, 80 °C oil bath. The reaction mixture was stirred for 1 h at 80 °C, and then cooled to room temperature. The reaction mixture was diluted with Et₂O (15 mL) and washed with water (10 mL). The organic layer was dried over MgSO₄, filtered and concentrated. The resulting residue was purified by flash chromatography (30% EtOAc in hexanes) to afford (114) (0.05 g, 66%). MS (ES) *m*/*z* 477.0 (M+H)⁺.

### 4-Cyclopropyl-5-(4-(trifluoromethyl)phenylsulfonyl)-4,5-dihydro-2H-pyrazolo[4,3-c][1,5]naphthyridine (115).

A solution of (114) (0.05 g, 0.09 mmol) in formic acid (1 mL) was plunged into a preheated 80 °C oil bath. The reaction mixture stirred for 2 h at 80 °C and then cooled to room temperature. The mixture was concentrated and the resulting residue was dissolved in CH₂Cl₂ (10 mL) and washed with water (5 mL). The organic layer was dried over MgSO₄, filtered and concentrated. The resulting residue was purified by flash chromatography (50% EtOAc in hexanes) to afford (115) (0.03 g, 63%). ¹H NMR (300 MHz, CDCl₃) δ 8.62 (dd, *J* = 4.8, 1.2 Hz, 1H), 8.27 (dd, *J* = 8.2, 1.2 Hz, 1H), 7.36 (m, 6H), 5.12 (d, *J* = 7.5 Hz, 1H), 1.05 (m, 1H), 0.45 (m, 2H), 0.19 (m, 1H); ¹³C NMR (75 MHz, CDCl₃) δ 147.7, 141.6, 141.0, 136.7, 135.6, 133.5, 129.9, 127.3, 125.5, 125.4, 123.2, 117.7, 58.7, 30.3, 17.0, 3.32, 2.82; MS (ES) *m*/*z* 420.9 (M+H)⁺.

### Example 20

### 5-(Trifluoromethyl)pyridine-2-sulfonamide (117).

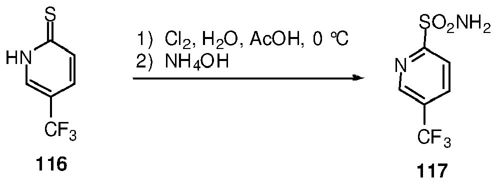

5-(Trifluoromethyl)pyridine-2(1H)-thione (116), 10.0g, 55.8 mmol) was placed in a flask containing 100 mL of water and 50 mL of acetic acid. The mixture was cooled to 0 °C and chlorine gas was bubbled into the mixture for 45-60 minutes. Water (100 mL) was added and a precipitate formed which was collected by filtration. This solid was cooled in an ice bath and in a separate flask 100 mL of conc. NH₄OH was also cooled in an ice bath. After 10 minutes, the cooled NH₄OH solution was added to the solid and the mixture was stirred overnight at room temperature. The mixture was then extracted with Et₂O (100 mL). The aqueous layer was collected and concentrated to give crude product that was purified by column chromatography on silica gel using EtOAc/hexanes gradients to afford 1.30 g of (117) (10%) as an off-white solid.

### Example 21

### 5-(4-Chlorophenylsulfonyl)-4-methyl-4,5-dihydro-1H-pyrazolo[4,3-c]quinolin-7-ol (119).

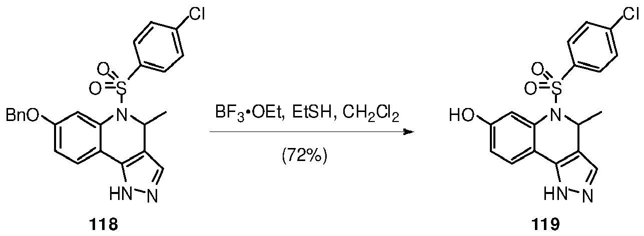

To a solution of (118) (0.37 g, 0.78 mmol) in CH₂Cl₂ (5 mL) at 0 °C was added ethane thiol (1 mL) and BF₃•Et₂O (0.39 mL, 3.15 mmol). The reaction mixture was stirred for 18 h while warming to room temperature. A second aliquot of BF₃•Et₂O (0.39 mL, 3.15 mmol) was added and the reaction mixture was heated to 50 °C for 3 h. The mixture was then cooled to room temperature, diluted with EtOAc (10 mL), and washed with water (10 mL) and saturated aqueous NaCl (10 mL). The organic layer was dried over MgSO₄, filtered and concentrated. The resulting residue was purified by preparative HPLC to afford (119) (0.21 g, 72%). ¹H NMR (CD₃CN) δ 7.44 (d, *J* = 8.4 Hz, 1H), 7.27 (s, 1H), 7.17 (d, *J* = 2.5 Hz, 1H), 7.17 (s, 4H), 6.83 (dd, *J* = 8.4, 2.5 Hz, 1H), 6.38 (bs, 1H), 5.57 (q, *J* = 6.9 Hz, 1H), 1.21 (d, *J* = 6.9 Hz, 3H); ¹³C NMR (75 MHz, CD₃CN) δ 158.1, 139.5, 137.0, 134.8, 129.6, 129.2, 127.2, 124.6, 118.2, 117.8, 117.2, 116.0, 55.2, 50.9, 23.3; MS (ES) *m*/*z* 375.9 (M+H)⁺.

### Example 22

### 9-Fluoro-6-(4-fluorophenylsulfonyl)-5-methyl-5,6-dihydropyrimido[5,4-c]quinolin-2-amine (121).

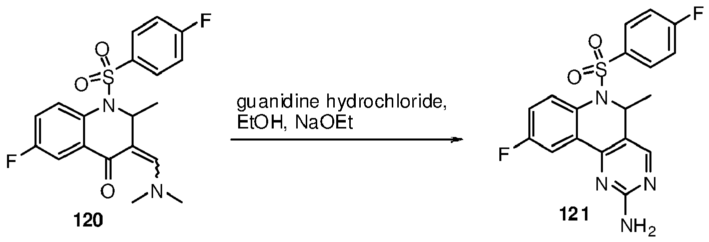

A solution of guanidine hydrochloride (0.49 mmol) and NaOEt (21% wt in EtOH, 1.34 mmol) was added to a reaction vial containing compound (120) (0.45 mmol) in EtOH (1.5 ml). The vial was heated for 5 minutes at 100 °C in a microwave and was subsequently filtered and purified by preparative HPLC to afford compound (121) (27%). ¹H NMR (CD₃OD) δ 8.13 (s, 1H), 7.85-7.79 (m, 2H), 7.51-7.45 (m, 1H), 7.34-7.29 (m, 2H), 7.17-7.12 (m, 1H), 7.02-6.96 (m, 2H), 6.76-6.69 (m, 1H), 5.57-5.50 (q, J = 6.9 Hz, 1H), 1.31-1.28 (m, 3H). MS *m*/*z* 388.9 (M+H)⁺.

### Example 23

### 5-(4-Chlorophenylsulfonyl)-3-(difluoromethyl)-4,5-dihydro-2H-pyrazolo[4,3-c]quinoline (123)

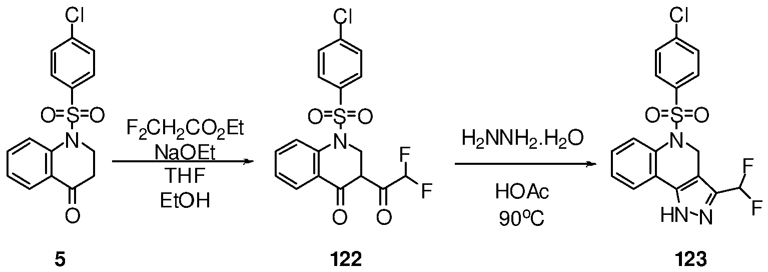

### 1-(4-Chlorophenylsulfonyl)-3-(2,2-difluoroacetyl)-2,3-dihydroquinolin-4(1H)-one (122).

Ketone (5) (481 mgs, 1.5 mmol) was dissolved in ethyl difluoroacetate (3.15 mL, 30 mmol), THF (1 mL) and dry ethanol (1 mL). To the reaction mixture was added 21% sodium ethoxide in ethanol (1.68 mL, 4.5 mmol). The solution was then stirred at ambient temperature. After 4h, LC-MS analysis showed partial conversion and the reaction continued at room temperature for an additional 14h. Evaporation of the amber reaction mixture under a stream of nitrogen resulted in intermediate (122), MS (ES) *m*/*z* 422.0 (M+Na)⁺.

### 5-(4-Chlorophenylsulfonyl)-3-(difluoromethyl)-4,5-dihydro-2H-pyrazolo[4,3-c]quinoline (123).

To a suspension of intermediate (122) (239 mgs, 0.6 mmol) and glacial acetic acid (3 mL) was added anhydrous hydrazine (30 µL, 0.6 mmol). The solution was stirred under nitrogen and heated at 90 °C for 3h, followed by cooling to ambient temperature. Upon complete consumption of (87) by LC-MS, the reaction mixture was evaporated by rotary evaporation. The resulting residue was dissolved in acetonitrile (5 mL) and purified by reverse-phase preparative HPLC, resulting in 172 mgs of compound (123). MS (ES) *m*/*z* 396.0 (M+H)⁺. ¹H NMR (CDCl₃) δ 7.90-7.83 (m, 2H), 7.52-7.43 (m, 1H), 7.42-7.35 (m, 2H), 7.27 (m, 1H), 7.19-7.04 (m, 3H), 5.05 (s, 2H). ¹³C NMR (CDCl₃) δ 139.2, 138.8, 135.8, 134.4, 129.5, 129.4, 128.4, 128.3, 128.0, 122.1, 121.7, 113.9, 110.8, 110.7, 107.7, 50.6, 42.8.

### Example 24

### Ethyl 5-(4-chlorophenylsulfonyl)-4,5-dihydro-2H-pyrazolo[4,3-c]quinoline-3-carboxylate (125)

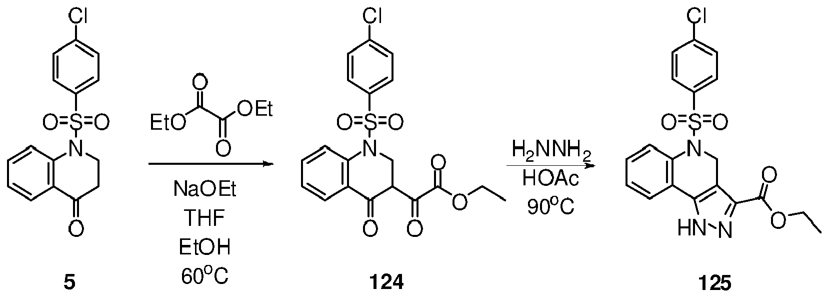

### Ethyl 2-(1-(4-chlorophenylsulfonyl)-4-oxo-1,2,3,4-tetrahydroquinolin-3-yl)-2-oxoacetate (124).

The ketone (5) (963 mgs, 3.0 mmol) was dissolved in diethyl oxalate (4.1 mL, 30 mmol), THF (2 mL) and dry ethanol (2 mL). To the reaction mixture was added 1% sodium ethoxide in ethanol (3.36 mL, 9.0 mmol). The solution was placed under nitrogen and allowed to stir at ambient temperature for 1h, followed by heating at 60 °C for 1h. The resulting mixture was concentrated by rotary evaporation, dissolved in MeOH (14 mL) and purified by reverse-phase preparative HPLC to afford 260 mgs of compound (124). MS (ES) *m*/*z* 421.9 (M+H)⁺.

### Ethyl 5-(4-chlorophenylsulfonyl)-4,5-dihydro-2H-pyrazolo[4,3-c]quinoline-3-carboxylate (125).

To a suspension of intermediate (124) (260 mgs, 0.62 mmol) and glacial acetic acid (2 mL) was added anhydrous hydrazine (35 µL, 0.62 mmol). The solution was stirred under nitrogen and heated at 90 °C for 6h, followed by cooling to ambient temperature. Upon complete consumption of (124) by LC-MS, the reaction mixture was evaporated by rotary evaporation. The resulting residue was dissolved in methanol (6 mL) and purified by reverse-phase preparative HPLC, resulting in 107 mgs of compound (125). MS (ES) *m*/*z* 417.9 (M+H)⁺. ¹H NMR (CDCl₃) δ 7.84-7.80 (dd, 2H), 7.74-7.74 (dd, 2H), 7.49-7.38 (m, 2H), 7.17-7.04 (m, 2H), 5.10 (s, 2H), 4.54-4.47 (q, 2H), 1.53-1.48 (t, 3H). ¹³C NMR (CDCl₃) δ 159.0, 145.2, 139.4, 136.0, 134.9, 129.9, 129.7, 129.3, 129.0, 128.4, 128.3, 128.2, 128.1, 124.3, 122.7, 115.6, 62.0, 43.1, 14.4.

### Example 25

### Ethyl 5-(4-chlorophenylsulfonyl)-8-fluoro-4,5-dihydro-2H-pyrazolo[4,3-c]quinoline-3-carboxylate (127).

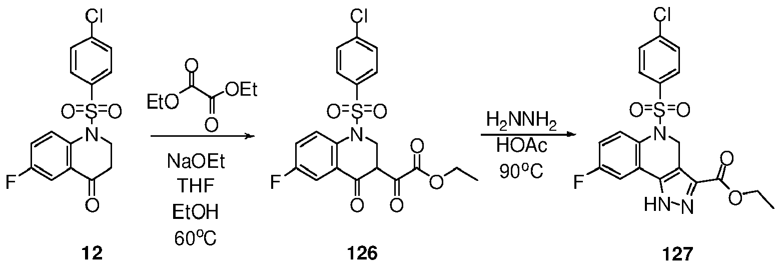

### Ethyl 2-(1-(4-chlorophenylsulfonyl)-6-fluoro-4-oxo-1,2,3,4-tetrahydroquinolin-3-yl)-2-oxoacetate (126).

The ketone (12) (1017 mgs, 3.0 mmol) was heated to dissolution in diethyl oxalate (4.1 mL, 30 mmol), THF (2 mL) and dry ethanol (2 mL). To the reaction mixture was added 21% sodium ethoxide in ethanol (3.36 mL, 9.0 mmol). The solution was stirred under nitrogen at ambient temperature for 1h, followed by heating at 60 °C for 1h. The resulting mixture was concentrated by rotary evaporation and carried forward crude. Compound (126) MS (ES) *m*/*z* 440.0 (M+H)⁺.

### Ethyl 5-(4-chlorophenylsulfonyl)-8-fluoro-4,5-dihydro-2H-pyrazolo[4,3-c]quinoline-3-carboxylate (127).

To a suspension of intermediate (126) (3.0 mmol) and glacial acetic acid (10 mL) was added anhydrous hydrazine (168 µL, 3.0 mmol). The solution was stirred under nitrogen and heated at 90 °C for 5h, followed by cooling to ambient temperature. Upon complete consumption of (126) by LC-MS, the reaction mixture was evaporated by rotary evaporation. The resulting residue was dissolved in methanol (12 mL) and purified by reverse-phase preparative HPLC, resulting in 379 mgs of compound (127). MS (ES) *m*/*z* 435.9 (M+H)⁺. ¹H NMR (CDCl₃) δ 7.81-7.76 (dd, 2H), 7.47-7.43 (dd, 2H), 7.16-7.06 (m, 3H), 5.07 (s, 2H), 4.54-4.46 (q, 2H), 1.53-1.48 (t, 3H). ¹³C NMR (CDCl₃) δ 163.5, 160.2, 159.0, 139.4, 135.8, 131.0, 130.9, 130.7, 128.4, 128.3, 116.0, 115.7, 115.6, 109.7, 109.4, 61.9, 50.8, 43.1, 14.4.

### Example 26

### 5-(4-Chlorophenylsulfonyl)-3-(methylsulfonyl)-4,5-dihydro-2H-pyrazolo[4,3-c]quinoline (131).

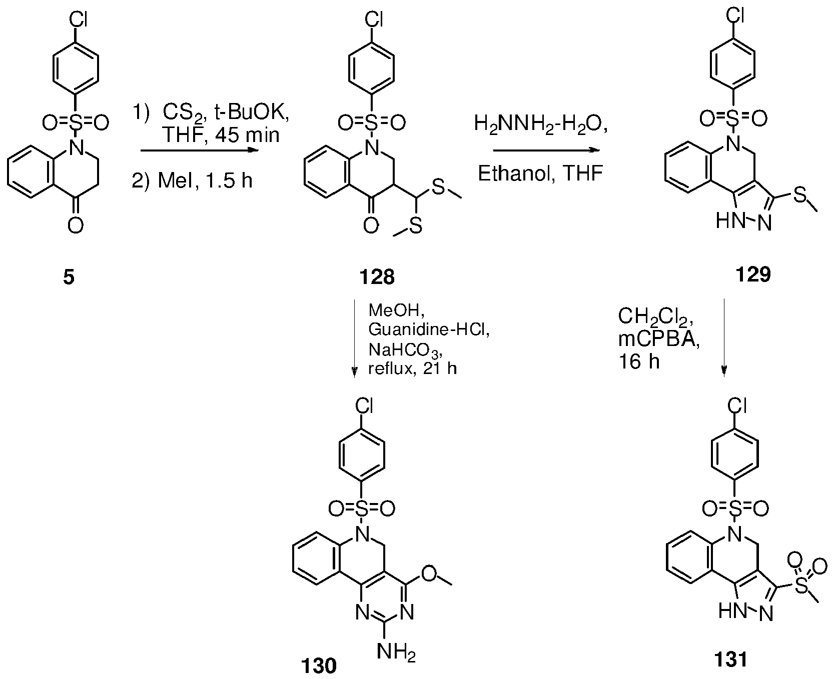

### 3-(Bis(methylthio)methyl)-1-(4-chlorophenylsulfonyl)-2,3-dihydroquinolin-4(1H)-one (128)

A solution of (5) (506 mg, 1.57 mmol) and carbon disulfide (239 mg, 0.189 mL, 3.15 mmol) in tetrahydrofuran (10 mL) was treated with potassium *tert*-butoxide (441 mg, 3.93 mmol) and stirred for 45 min. The mixture was then treated with iodomethane (1.12g, 0.49 mL), 7.9 mmol) and stirred at ambient temp for 1.5 h. The mixture was diluted with sat. aq sodium bicarbonate (25 mL) and extracted with ethyl acetate (2 x 25 mL). The combined organic extracts were washed with water (20 mL) and brine (10 mL), then dried (sodium sulfate), filtered and evaporated *in vacuo* to give (128) as a yellow oil (645 mg, 92% crude).

### 5-(4-Chlorophenylsulfonyl)-3-(methylthio)-4,5-dihydro-1H-pyrazolo[4,3-c]quinoline (129)

The ketene dithioacetal (128) (615 mg, 1.44 mmol) was taken up into ethanol (10 mL) and treated with hydrazine hydrate (87 mg, 0.084 mL, 1.73 mmol). Tetrahydrofuran (2 mL) was added to give a homogeneous solution, which was stirred for 4 h. The mixture was diluted with water (25 mL) and extracted with ethyl acetate (2 X 25 mL). The combined organic extracts were washed with water (25 mL) and brine (10 mL), then dried (sodium sulfate), filtered and evaporated *in vacuo* to give a crude residue which was purified by silica gel flash chromatography (eluted 2:1 hexanes/ethyl acetate) to give (129) (373 mg, 66%) as a tan solid. Mass spectrum: MS (ES) *m*/*z* 392.0 (M+H)⁺. ¹H NMR (CDCl₃) δ 7.81 (d, *J* = 7.7 Hz, 1H), 7.60 (m, 1H), 7.45-7.34 (m, 2H), 7.16 (d, *J* = 8.2 Hz, 2H), 7.08 (d, *J* = 8.2 Hz, 2H), 4.87 (s, 2H), 2.41 (s, 3H). ¹³C NMR (CDCl₃) δ 139.2, 136.3, 134.9, 129.1, 128.9, 128.4, 128.0, 122.4, 42.7.

### 5-(4-Chlorophenylsulfonyl)-3-(methylsulfonyl)-4,5-dihydro-2H-pyrazolo[4,3-c]quinoline (131)

A solution of (129) (307 mg, 0.78 mmol) in methylene chloride (10 mL) was treated with mCPBA (assay: 77%, 527 mg, 2.35 mmol) and stirred for 16 h. The mixture was diluted with ethyl acetate (50 mL) and washed with diluted aq sodium bicarbonate (2 x 25 mL). The volatiles were removed *in vacuo* and the residue was purified by silica gel flash chromatography (eluted 1:1 hexanes/ethyl acetate) to give (131) as a white solid (290 mg, 87%) Mass spectrum: MS (ES) *m*/*z* 424.0 (M+H)⁺. ¹H NMR (DMSO *d*₆) δ 7.75 (m, 1H), 7.65-7.54 (m, 3H), 7.35 (d, *J* = 8.8 Hz, 2H), 7.12 (d, *J* = 8.2 Hz, 2H), 5.11 (s, 2H), 3.33 (s, 3H). ¹³C NMR (DMSO *d*₆) δ 133.7,130.7, 128.7, 128.1, 124.8, 124.1, 124.1, 123.8, 123.7, 123.3, 117.9, 38.6, 37.6.

### 6-(4-Chlorophenylsulfonyl)-4-methoxy-5,6-dihydropyrimido[5,4-c]quinolin-2-amine (130)

The ketene dithioacetal (128) (0.58g, 1.36 mmol) was taken up into methanol (10 mL) and treated with guanidine hydrochloride (0.260 g, 2.77 mmol) the mixture was stirred for 16 h whereupon TLC analysis indicated no condensation had taken place. Sodium bicarbonate (300 mg) was added and the mixture was heated to reflux for 21 h. The mixture was cooled to ambient temp and diluted with water (15 mL); then extracted with ethyl acetate (3 x 25 mL). The extracts were dried (sodium sulfate), filtered and concentrated *in vacuo* to give a residue, which was purified by silica gel chromatography (eluted 1:1 hexanes/ethyl acetate) to give 190 mg of (130). MS *m*/*z* 403.0 (M+H)⁺. ¹H NMR (CDCl₃) δ 7.98 (dd, *J* = 1.1, 7.7 Hz, 1H), 7.75 (d, *J* = 8.2 Hz, 1H), 7.51 (m, 1H), 7.39 (m, 1H), 7.18 (d, *J* = 8.8 Hz, 2H), 7.10 (d, *J* = 8.8 Hz, 2H), 4.87 (br s, 2H), 4.76 (s,2H), 3.92 (s, 3H). ¹³C NMR (CDCl₃) δ 166.0, 162.9, 156.6, 139.5, 137.8, 136.5, 131.1, 128.9, 128.6, 128.2, 128.0, 127.7, 125.3, 99.9, 53, 7, 42.4.

### Example 27

### 5-(4-Chlorophenylsulfonyl)-4,5-dihydro-1H-pyrazolo[4,3-c]quinolin-3-ol.

Prepared by treatment of ethyl 1-(4-chlorophenylsulfonyl)-4-oxo-1,2,3,4-tetrahydroquinoline-3-carboxylate, which was prepared from compound (5) using triethyl phosphonoformate and NaH in THF, with hydrazine in AcOH. MS *m*/*z* 361.9 (M+H)⁺. ¹H NMR (CD₃OD) δ 7.74 (m, 1H), 7.42-7.37 (m, 3H), 7.25-7.16 (m, 4H), 4.82 (s, 4H).

### Example 28

### 8-Fluoro-4-methyl-5-(pyridin-3-ylsulfonyl)-4,5-dihydro-1H-pyrazolo[4,3-c]quinoline.

Prepared as described in Example 10 using 3-pyridinesulfonyl chloride and tert-butyl 3-(4-fluorophenylamino)butanoate, which was prepared from compound (25) and 4-fluoroaniline as described for compound (26) in Example 4. MS *m*/*z* 345.0 (M+H)⁺. ¹H NMR (CD₃OD) δ 8.52-8.50 (dd, J = 4.9, 1.3 Hz, 1H), 8.30-8.29 (d, J = 1.8 Hz, 1H), 7.81-7.77 (dd, J = 8.9, 4.9 Hz, 1H), 7.61-7.57 (m, 1H), 7.46 (s, 1H), 7.37-7.33 (dd, J = 8.7, 3.0 Hz, 1H), 7.27-7.17 (m, 2H), 5.76-5.73 (q, J = 6.9 Hz, 1H), 1.29-1.27 (d, J = 6.9 Hz, 3H).

### Example 29

### 5-(4-Chlorophenylsulfonyl)-8-fluoro-4-methyl-4,5-dihydro-2H-pyrazolo[4,3-c]quinoline (149).

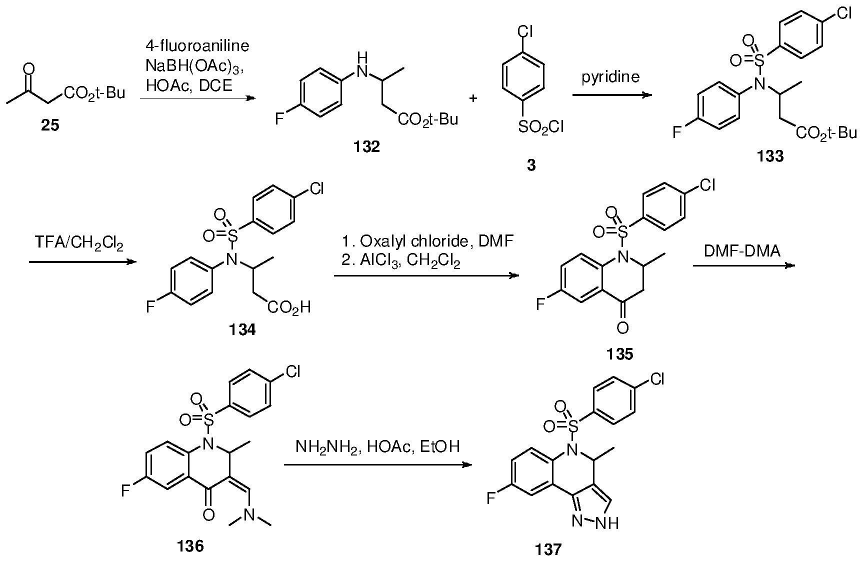

*tert*-Butyl 3-(4-fluorophenylamino)butanoate (132). To a 1,2-dichloroethane solution (146 mL) of 4-fluoroaniline (4.06 g, 36.5 mmol) was added *tert*-butyl acetoacetate (25) (6.05 mL, 36.5 mmol) and glacial acetic acid (3.0 mL, 47.5 mmol). Sodium triacetoxyborohydride (11.6 g, 54.8 mmol) was added in portions and the reaction was stirred overnight at room temperature. Saturated aqueous NaHCO₃ (250 mL) was slowly added and the two phase solution was well stirred. The separated organic portion was washed with sat. aq. NaHCO₃ (1 x 100 mL), water (1 x 100 mL), brine (1 x 20 mL), dried (MgSO₄), filtered and concentrated to yield 132 (9.03 g, 98%) as an oil. MS *m*/*z*: (M+H)⁺ = 254.1.

*tert*-Butyl 3-(4-chloro-*N*-(4-fluorophenyl)phenylsulfonamido)butanoate (133). To a solution of compound 132 (3.08 g, 12.2 mmol) in pyridine (35 mL) at 0 °C was added 4-chlorobenzenesulfonyl chloride (3) (8.98 g, 42.6 mmol) portionwise. The reaction was warmed to room temperature and then heated at 70 °C for 5.5 hours then cooled to room temperature. Dimethylaminopropylamine (~ 40 mL) was added to the reaction mixture which was then stirred overnight. The reaction mixture was then concentrated and the residue taken up in EtOAc (120 mL). The organic phase was washed with cold 1N HCl (6 x 100 mL), water (1 x 100 mL), sat. aq. NaHCO₃ (3 x 100 mL), brine (1 x 100 mL), dried (MgSO₄), filtered, and concentrated to yield ester 133 (5.2 g, 99%) as an oil. MS *m*/*z:* (M+Na)⁺ = 450.1.

3-(4-Chloro-*N*-(4-fluorophenyl)phenylsulfonamido)butanoic acid (134). To a solution of ester 133 (5.19 g, 12.13 mmol) in CH₂Cl₂ (10 mL) at 0 °C was added TFA (35 mL). The reaction mixture was allowed to warm to room temperature, stirred overnight and then concentrated to give 134 (4.7 g, 105%) as an oil. MS *m*/*z:* (M+H)⁺ = 372.1.

1-(4-Chlorophenylsulfonyl)-6-fluoro-2-methyl-2,3-dihydroquinolin-4(1H)-one (135). To an ice chilled solution of 134 (3.91 g, 10.52 mmol) in CH₂Cl₂ (45 mL) and DMF (4 drops) was added oxalyl chloride (3.67 mL, 42.10 mmol) dropwise. The resulting solution was stirred at - 5 °C for 15 minutes and then warmed to room temperature and stirred for 3 hours. The reaction mixture was then concentrated and the residue taken-up in diethyl ether, filtered through a plug of glass wool, and concentrated to give the acid chloride (4.09, 99%). A CH₂Cl₂ (20 mL) solution of the acid chloride was added dropwise over a 20 minute period to a chilled (-5 °C) suspension of AlCl₃ (1.81 g, 13.6 mmol) in CH₂Cl₂ (20 mL). After stirring for 0 °C for one hour the reaction was warmed to room temperature and stirred overnight. The reaction mixture was then chilled in an ice bath to which was added 10% HCl (15 mL) dropwise over 15 minutes. The organic portion was washed with 10% HCl (2 x 50 ml), water (1 x 20 mL), sat. aq. NaHCO₃ (3 x 50 mL), brine (1 x 20 mL), dried (MgSO₄), filtered, and concentrated. The crude product was then dissolved in THF (40 mL) to which was added sat. aq. NaHCO₃ (40 mL). This mixture was stirred for 7 hours and then concentrated. The residue was taken-up in water and EtOAc. The aqueous layer was extracted with EtOAc (4 x 30 mL) and the combined EtOAc extracts were washed with brine (1 x 20 mL), dried (MgSO₄) filtered, and concentrated to the give the crude product which was purified by flash chromatography eluting with 8:1 hexanes/EtOAc to give 135 (980 mg, 26%). MS *m*/*z*: (M+H)⁺ = 354.2.

5-(4-Chlorophenylsulfonyl)-8-fluoro-4-methyl-4,5-dihydro-2H-pyrazolo[4,3-c]quinoline (137). A dimethylformamide dimethyl acetal (5.2 mL) solution of 135 (922 mg, 2.61 mmol) was heated at 100 °C for 3 hours. HPLC/MS analysis of the reaction mixture determined that no starting material remained so the reaction was cooled to room temperature and poured into EtOAc (50 mL) and water (50 mL). The organic portion was washed with water (3 x 50 mL), brine (1 x 20 mL), dried (MgSO₄), filtered and concentrated to give 136 as a red foam which was used directly in the next reaction. To an EtOH/glacial HOAc solution (25:1 v/v, 12 mL) of 136 (982 mg, 2.40 mmol) was added hydrazine hydrate (582 mL, 12.0 mmol). The reaction mixture was stirred overnight at room temperature, concentrated, and the residue taken up in EtOAc (20 mL). The organic phase was washed with sat. aq. NaHCO₃ (3 x 25 mL), brine (1 x 20 mL), dried (MgSO₄), filtered and concentrated. The crude product was purified by flash chromatography eluting with 2:1 hexanes/EtOAc to give 137 as a yellow solid. ¹H NMR (CDCl₃) δ 7.81-7.76 (m, 1H), 7.37 (dd, *J* = 8.5, 2.7 Hz, 1H), 7.21 (s, 1H), 7.13-7.04 (m, 5H), 5.60 (q, *J* = 6.6 Hz), 1.28 (d, *J* = 7.1 Hz, 3H); MS: (M+H)⁺ = 378.0.

### Example 30

### 8-Fluoro-4-methyl-5-(pyridin-2-ylsulfonyl)-4,5-dihydro-1H-pyrazolo[4,3-c]quinoline.

Prepared as described in Example 10 using tert-butyl 3-(4-fluorophenylamino)butanoate, which was prepared from compound (25) and 4-fluoroaniline as described for compound (26) in Example 4. MS *m*/*z* 345 (M+H)⁺. ¹H NMR (CD₃OD) δ 8.25-8.24 (m, 1H), 7.78-7.68 (m, 2H), 7.44-7.32 (m, 4H), 7.22-7.10 (m, 2H), 5.75-5.68 (q, J = 6.9 Hz, 1H), 1.28-1.26 (d, J = 6.9 Hz, 3H).

### Example 31

### 5-(4-Chlorophenylsulfonyl)-8-fluoro-4-methyl-4,5-dihydro-1H-pyrazolo[4,3-c]quinoline.

**(Enantiomers A and B)** Prepared as described in Example 4 using 4-fluoroaniline followed by chromatographic separation using the specified column type and analyzed using Method 16. Enantiomer A Retention time = 9.8 min. Enantiomer B Retention time = 27.1 min. MS m/z 378.0 (M+H)⁺. ¹H-NMR (CDCl₃) δ 7.81-7.76 (m, 1H), 7.37 (dd, J = 8.5, 2.7 Hz, 1H), 7.21 (s, 1H), 7.13-7.04 (m, 5H), 5.60 (q, J = 6.6 Hz), 1.28 (t, J = 7.1 Hz, 3H).

### Example 32

**4-Methyl-5-(pyridin-3-ylsulfonyl)-4,5-dihydro-1*H*-pyrazolo[4,3-c]quinoline.** Prepared as described in Example 10 using 3-pyridinesulfonyl chloride in place of compound (50). MS *m*/*z* 327.0 (M+H)⁺. ¹H NMR (CDCl₃) δ 8.45-8.43 (m, 2H), 7.86-7.83 (dd, J = 7.8, 1.3 Hz, 1H), 7.66-7.63 (dd, J = 7.4, 1.6 Hz, 1H), 7.47-7.28 (m, 4H), 7.03-6.99 (m, 1H), 5.75-5.68 (q, J = 6.9 Hz, 1H), 1.35-1.33 (d, J = 6.9 Hz, 3H).

### Example 33

### 8-Fluoro-4-methyl-5-(pyridin-3-ylsulfonyl)-4,5-dihydro-1H-pyrazolo[4,3-c]quinoline.

**(Enantiomers A and B)** Prepared as described in Example 10 using 3-pyridinesulfonyl chloride and tert-butyl 3-(4-fluorophenylamino)butanoate, which was prepared from compound (25) and 4-fluoroaniline as described for compound (26) in Example 4 followed by chromatographic separation using the specified column type and analyzed using Method 15. Enantiomer A Retention time = 15.2 min. Enantiomer B Retention time = 17.5 min. MS *m*/*z* 345 (M+H)⁺. ¹H NMR (CDCl₃) δ 10.16 (s, 1H), 8.48-8.45 (d, J = 10.4 Hz, 2H), 7.83-7.79 (dd, J = 8.9, 5.0 Hz, 1H), 7.36-7.28 (m, 3H), 7.16-7.05 (m, 2H), 5.75-5.68 (q, J = 6.9 Hz, 1H), 1.34-1.31 (d, J = 6.9 Hz, 3H).

### Example 34

**5-(Pyridin-3-ylsulfonyl)-4,5-dihydro-1H-pyrazolo[4,3-c]quinoline.** Prepared as described in Example 1 using 3-pyridinesulfonyl chloride in place of compound (3). ¹H-NMR (CDCl₃) δ 8.47 (br s, 2H), 7.82 (d, J = 7.6 Hz, 1H), 7.66 (d, J = 6.0 Hz, 1H), 7.45-7.39 (m, 2H), 7.31-7.28 (m, 3H), 7.03-6.99 (m, 1H), 4.99 (s, 2H).

### Example 35

**8-Fluoro-5-(pyridin-3-ylsulfonyl)-4,5-dihydro-1*H*-pyrazolo[4,3-c]quinoline.** Prepared as described in Example 2 using 3-pyridinesulfonyl chloride in place of compound (3). ¹H NMR (CD₃OD) δ 8.50-8.49 (1H, d), 8.27 (1H, s), 7.82-7.77 (1H, dd), 7.56-7.53 (1H, d), 7.46 (1H, s), 7.35-7.32 (1H, d), 7.24-7.17 (2H, m), 5.00 (2H, s).

### Example 36

### 8-Fluoro-4-methyl-5-(pyridin-2-ylsulfonyl)-4,5-dihydro-1H-pyrazolo[4,3-c]quinoline.

**(Enantiomers A and B)** Prepared as described in Example 10 using *tert*-butyl 3-(4-fluorophenylamino)butanoate, which was prepared from compound (25) and 4-fluoroaniline as described for compound (26) in Example 4. Enantiomers were then separated using the specified column type and analyzed using Method 15. Enantiomer A Retention time = 16.3 min. Enantiomer B Retention time = 21.4 min. MS *m*/*z* 345.0 (M+H)⁺. ¹H NMR (CDCl₃) δ 8.31-8.30 (d, J = 4.6 Hz, 1H), 7.83-7.78 (dd, J = 8.9, 5.1 Hz, 1H), 7.60-7.55 (td, J = 7.8, 1.6 Hz, 1H), 7.39-7.33 (m, 2H), 7.23-7.19 (m, 1H), 7.10-7.04 (td, J = 8.7, 2.9 Hz, 1H), 5.83-5.76 (q, J = 6.9 Hz, 1H), 1.34-1.32 (d, J = 6.9 Hz, 3H).

### Example 37

### 4-Methyl-5-(pyridin-3-ylsulfonyl)-4,5-dihydro-1H-pyrazolo[4,3-c]quinoline.

**(Enantiomers A and B)** Prepared as described in Example 10 using 3-pyridinesulfonyl chloride in place of compound (50) followed by chromatographic separation using the specified column type and analyzed using Method 15. Enantiomer A Retention time = 13.5 min. Enantiomer B Retention time = 14.6 min. MS *m*/*z* 326.9 (M+H)⁺. ¹H NMR (CDCl₃) δ 9.44 (s, 1H) 8.48-8.43 (m, 2H), 7.86-7.83 (dd, J = 8.0, 1.3 Hz, 1H), 7.64-7.61 (dd, J = 7.4, 1.6 Hz, 1H), 7.47-7.28 (m, 4H), 7.04-7.00 (dd, J = 8.0, 4.9 Hz, 1H), 5.75-5.68 (q, J = 6.9 Hz, 1H), 1.35-1.33 (d, J = 6.9 Hz, 3H).

### Example 38

### 8-Fluoro-5-(4-fluorophenylsulfonyl)-4-methyl-4,5-dihydro-1H-pyrazolo[4,3-c]quinoline.

Prepared as described in Example 4 using 4-fluoroaniline and 4-fluorophenylsulfonyl chloride. MS *m*/*z* 361.9 (M+H)⁺. ¹H NMR (CDCl₃) δ 7.84-7.79 (dd, J = 5.1, 3.8 Hz, 1H), 7.38-7.34 (dd, J = 5.5, 2.9 Hz, 1H), 7.23 (s, 1H), 7.22-7.16 (m, 2H), 7.15-7.08 (dt, J = 5.7, 2.9 Hz, 1H), 6.80-6.75 (m, 2H), 5.67-5.60 (q, J = 6.9 Hz, 1H), 1.33-1.30 (d, J = 6.8, 3H).

### Example 39

**4-Methyl-5-(pyridin-2-ylsulfonyl)-4,5-dihydro-1*H*-pyrazolo[4,3-c]quinoline.** Prepared as described in Example 10 followed by chromatographic separation using the specified column type and analyzed using Method 21. Retention time = 7.9. MS *m*/*z* 373.9 (M+H)⁺. ¹H NMR (CDCl₃) δ 8.31-8.30 (d, J = 4.6 Hz, 1H), 7.84-7.81 (dd, J = 8.0, 1.0 Hz, 1H), 7.65-7.62 (dd, J = 7.4, 1.5 Hz, 2H), 7.57-7.51 (td, J = 7.8, 1.7 Hz, 1H), 7.41-7.30 (m, 3H), 7.20-7.16 (m, 1H), 5.85-5.78 (q, J = 6.9 Hz, 1H), 1.35-1.33 (d, J = 6.9 Hz, 3H).

### Example 40

### 5-(4-Chlorophenylsulfonyl)-4-cyclopropyl-4,5-dihydro-1H-pyrazolo[4,3-c]quinoline.

Prepared as described in Example 12 using 2-bromophenylboronic acid in place of compound (70). MS *m*/*z* 386 (M+H)⁺. ¹H NMR (CDCl₃) δ 8.88 (s, 1H), 7.91 (dd, J=7.8, 1.2 Hz, 1H), 7.62 (dd, J=7.8, 1.5 Hz, 1H), 7.49 (dt, J=7.8, 1.5 Hz, 1H), 7.39 (dt, J=7.8, 1.2 Hz, 1H), 7.34 (s, 1H), 7.06 (s, 4H), 4.95 (d, J=8.4 Hz, 1H), 1.02 (m, 1H), 0.44 (broad m, 3H), 0.14 (m, 1H).

### Example 41

### 5-(4-Chlorophenylsulfonyl)-4-isopropyl-4,5-dihydro-1H-pyrazolo[4,3-c]quinoline.

Prepared as described in Example 12 using 2-bromophenylboronic acid and isopropylmagnesium bromide. MS *m*/*z* 388.0 (M+H)⁺. ¹H NMR (CDCl₃) δ 7.86 (dd, J=7.5, 0.9 Hz, 1H), 7.58 (dd, J=7.5, 1.5 Hz, 1H), 7.48 (dt, J=7.5, 1.5 Hz, 1H), 7.39 (dt, J=7.5, 0.9 Hz, 1H), 7.33 (s, 1H), 7.05 (s, 4H), 4.94 (d, J=9.6 Hz, 1H), 1.51 (m, 1H) 1.09 (d, J=7.2 Hz, 3H), 0.83 (d, J=6.3 Hz, 3H).

### Example 42

### 8-Fluoro-5-(4-fluorophenylsulfonyl)-4-methyl-4,5-dihydro-1H-pyrazolo[4,3-c]quinoline.

**(Enantiomers A and B)** Prepared as described in Example 4 using 4-fluoroaniline and 4-fluorophenylsulfonyl chloride followed by chromatographic separation using the specified column type and analyzed using Method 17. Enantiomer A Retention time = 7.6 min. Enantiomer B Retention time = 10.1 min. MS *m*/*z* 361.9 (M+H)⁺. ¹H NMR (CDCl₃) δ 7.83-7.78 (dd, J = 5.1, 3.8 Hz, 1H), 7.34-7.30 (dd, J = 5.5, 2.9 Hz, 1H), 7.22 (s, 1H), 7.20-7.18 (m, 2H), 7.14-7.07 (m, 1H), 6.79-6.73 (m, 2H), 5.67-5.61 (q, J = 6.9 Hz, 1H), 1.32-1.29 (d, J = 6.9 Hz, 3H).

### Example.43

**5-(4-Chlorophenylsulfonyl)-7,8-difluoro-4-methyl-4,5-dihydro-1*H*-pyrazolo[4,3-c]quinoline.** Prepared as described in Example 12 using methylmagnesium bromide and 4-chlorophenylsulfonamide. MS *m*/*z* 396.0 (M+H)⁺. ¹H NMR (CDCl₃) δ 7.73 (dd, J=11.1, 7.5 Hz, 1H), 7.42 (dd, J=10.2, 8.4 Hz, 1H), 7.30 (s, 1H), 7.13 (broad m, 5H), 5.62 (q, J=6.9 Hz, 1H), 1.31 (q, J=6.9 Hz, 3H).

### Example 44

**7,8-Difluoro-4-methyl-5-(4-(trifluoromethyl)phenylsulfonyl)-4,5-dihydro-1*H-*pyrazolo[4,3-c]quinoline.** Prepared as described in Example 12 using methylmagnesium bromide. MS *m*/*z* 430.0 (M+H)⁺. ¹H NMR (CDCl₃) δ 8.84 (broad s, 1H), 7.74 (dd, J=11.1, 7.5 Hz, 1H), 7.41 (m, 3H), 7.32 (d, J=9.0 Hz, 2H), 7.29 (s, 1H), 5.63 (q, J=6.9 Hz, 1H), 1.32 (q, J=6.9 Hz, 3H).

### Example 45

**5-(5-Chlorothiophen-2-ylsulfonyl)-7,8-difluoro-4-methyl-4,5-dihydro-1*H*-pyrazolo[4,3-c]quinoline.** Prepared as described in Example 12 using 5-chlorothiophene-2-sulfonamide and methylmagnesium bromide. MS *m*/*z* 402.0 (M+H)⁺. ¹H NMR (CDCl₃) δ 7.66 (dd, J=11.4, 7.5 Hz, 1H), 7.55 (dd, J=10.5, 8.1 Hz, 1H), 7.33 (s, 1H), 6.73 (d, J=3.9 Hz, 1H), 6.57 (d, J=3.9 Hz, 1H), 6.14 (broad s, 1H), 5.63 (q, J=6.9 Hz, 1H), 1.32 (q, J=6.9 Hz, 3H).

### Example 46

**7,8-Difluoro-4-methyl-5-(4-(trifluoromethyl)phenylsulfonyl)-4,5-dihydro-1*H-*pyrazolo[4,3-c]quinoline. (Enantiomers A and B)** Prepared as described in Example 12 using methylmagnesium bromide followed by chromatographic separation using the specified column type and analyzed using Method 15. Enantiomer A Retention time = 7.0 min. Enantiomer B Retention time = 9.5 min. MS *m*/*z* 430.0 (M+H)⁺. ¹H NMR (CDCl₃) δ 8.84 (broad s, 1H), 7.74 (dd, J=11.1, 7.5 Hz, 1H), 7.41 (m, 3H), 7.32 (d, J=9.0 Hz, 2H), 7.29 (s, 1H), 5.63 (q, J=6.9 Hz, 1H), 1.32 (q, J=6.9 Hz, 3H).

### Example 47

**4-Cyclopropyl-7,8-difluoro-5-(4-(trifluoromethyl)phenylsulfonyl)-4,5-dihydro-1*H-*pyrazolo[4,3-c]quinoline. (Enantiomers A and B)** Prepared as described in Example 12 followed by chromatographic separation using the specified column type and analyzed using Method 16. Enantiomer A Retention time = 6.0 min. Enantiomer B Retention time = 9.3 min. MS *m*/*z* 456.0 (M+H)⁺. ¹H NMR (CDCl₃) δ 7.76 (dd, J = 11.1, 7.2 Hz, 1H), 7.47 (m, J = 10.2, 8.7 Hz, 1H), 7.39 (d, J = 8.7 Hz, 2H), 7.32 (d, J = 8.7 Hz, 2H), 7.20 (s, 1H), 4.95f (d, J = 7.8 Hz, 1H), 1.03 (m, 1H), 0.54 (m, 1H), 0.41 (m, 2H), 0.08 (m, 1H).

### Example 48

**4-(4-Cyclopropyl-1H-pyrazolo[4,3-c]quinolin-5(4*H*)-ylsulfonyl)benzonitrile.** Prepared as described in Example 12 using 2-bromophenylboronic acid and 4-cyanobenzenesulfonamide. MS m/z 377,2 (M+H)⁺. ¹H NMR (CDCl₃) δ 7.91-7.88 (m, 1H), 7.66-7.63 (m, 1H), 7.50-7.33 (m, 2H), 7.38 (d, J = 8.4 Hz, 2H), 7.27 (s, 1H), 7.25 (d, J = 8.4 Hz, 2H), 4.95 (d, J = 8.3 Hz, 1H), 1.07-0.98 (m, 1H), 0.57-0.54 (m, 1H), 0.51-0.39 (m, 2H), 0.17-0.09 (m, 1H).

### Example 49

4-Cyclopropyl-5-(4-(trifluoromethyl)phenylsulfonyl)-4,5-dihydro-1H-pyrazolo[4,3-c]quinoline. Prepared as described in Example 12 using 2-bromophenylboronic acid and 4-(trifluoromethyl)benzenesulfonamide. MS m/z 420.1 (M+H)⁺. ¹H NMR (CDCl₃) δ 7.90 (dd, J = 9.2, 2.0 Hz, 1H), 7.64 (dd, J = 7.0, 1.5 Hz, 1H), 7.48-7.32 (m, 4H), 7.28-7.25 (m, 2H), 7.21 (s, 1H), 4.95 (d, J = 7.8 Hz, 1H), 1.06-1.033 (m, 1H), 0.52-0.34 (m, 3H), 0.15-0.05 (m, 1H).

### Example 50

5-(5-Chlorothiophen-2-ylsulfonyl)-4-cyclopropyl-7,8-difluoro-4,5-dihydro-1H-pyrazolo[4,3-c]quinoline. Prepared as described in Example 12 using 5-chlorothiophene-2-sulfonamide. MS m/z 457.0 (M+H)⁺. ¹H NMR (CDCl₃) δ 9.97 (broad s, 1H), 7.70 (dd, J = 11.4, 7.2 Hz, 1H), 7.57 (dd, J = 10.2, 8.7 Hz, 1H), 7.31 (s, 1H), 6.70 (d, J = 4.8 Hz, 1H), 6.56(d, J = 4.8 Hz, 1H), 4.98 (d, J = 7.5 Hz, 1H), 1.03 (m, 1H), 0.53 (m, 1H), 0.40 (m, 2H), 0.10 (m, 1H).

### Example 51

4-Cyclopropyl-7,8-difluoro-5-(4-fluorophenylsulfonyl)-4,5-dihydro-1*H*-pyrazolo[4,3-c]quinoline. Prepared as described in Example 12 using 4-fluorophenylsulfonamide. MS m/z 406.0 (M+H)⁺. ¹H NMR (CDCl₃) δ 9.73 (broad s, 1H), 7.77 (dd, J = 11.1, 7.2 Hz, 1H), 7.43 (dd, J = 9.9, 8.4 Hz, 1H), 7.27 (s, 1H), 7.21 (dd, J = 8.7, 5.4 Hz, 1H), 6.80(t, J = 8.7 Hz, 1H), 4.96 (d, J = 7.8 Hz, 1H), 1.01 (m, 1H), 0.52 (m, 1H), 0.40 (m, 2H), 0.09 (m, 1H).

### Example 52

4-Cyclopropyl-5-(4-fluorophenylsulfonyl)-4,5-dihydro-1H-pyrazolo[4,3-c]quinoline. Prepared as described in Example 12 using 2-bromophenylboronic acid and 4-fluorophenylsulfonamide. MS m/z 371.0 (M+H)⁺. ¹H NMR (CDCl₃) δ 7.91 (d, J = 8.6 Hz, 1H), 7.62 (d, J = 7.6 Hz, 1H), 7.48-7.35 (m, 2H), 7.30-7.28 (m, 2H), 7.18-7.11 (m, 1H), 6.74 (dd, J = 9.1, 7.2 Hz, 2H), 4.96 (d, J = 7.6 Hz, 1H), 1.14-0.08 (m, 1H), 0.58-0.32 (m, 3H), 0.20-0.11, 1H).

### Example 53

5-(5-Chlorothiophen-2-ylsulfonyl)-4-cyclopropyl-7,8-difluoro-4,5-dihydro-1H-pyrazolo[4,3-c]quinoline. (Enantiomers A and B) Prepared as described in Example 12 using 5-chlorothiophene-2-sulfonamide followed by chromatographic separation using the specified column type and analyzed using Method 22. Enantiomer A Retention time = 10.3 min. Enantiomer B Retention time = 16.1 min. MS *m*/*z* 428.0 (M+H)⁺. ¹H NMR (CDCl₃) δ 9.97 (broad s, 1H), 7.70 (dd, J = 11.4, 7.2 Hz, 1H), 7.57 (dd, J = 10.2, 8.7 Hz, 1H), 7.31 (s, 1H), 6.70 (d, J = 4.8 Hz, 1H), 6.56(d, J = 4.8 Hz, 1H), 4.98 (d, J = 7.5 Hz, 1H), 1.03 (m, 1H), 0.53 (m, 1H), 0.40 (m, 2H), 0.10 (m, 1H).

### Example 54

**4-Cyclopropyl-7,8-difluoro-5-(4-fluorophenylsulfonyl)-4,5-dihydro-2*H*-pyrazolo[4,3-c]quinoline. (Enantiomers A and B)** Prepared as described in Example 12 using 4-fluorophenylsulfonamide followed by chromatographic separation using the specified column type and analyzed using Method 13. Enantiomer A Retention time = 16.9. Enantiomer B Retention time = 19.7. MS *m*/*z* 406.0 (M+H)⁺. ¹H NMR (CDCl₃) δ 9.73 (broad s, 1H), 7.77 (dd, J = 11.1, 7.2 Hz, 1H), 7.43 (dd, J = 9.9, 8.4 Hz, 1H), 7.27 (s, 1H), 7.21 (dd, J = 8.7, 5.4 Hz, 1H), 6.80(t, J = 8.7 Hz, 1H), 4.96 (d, J = 7.8 Hz, 1H), 1.01 (m, 1H), 0.52 (m, 1H), 0.40 (m, 2H), 0.09 (m, 1H).

### Example 55

**5-(5-Chloropyridin-2-ylsulfonyl)-4-cyclopropyl-8-fluoro-4,5-dihydro-1*H*-pyrazolo[4,3-c]quinoline.** Prepared as described in Example 12 using 5-chloro-2-pyridinesulfonamide. MS *m*/*z* 404.9 (M+H)⁺. ¹H NMR (DMSO-d₆) δ 8.36-8.35 (d, J = 2.2 Hz, 1H), 7.95-7.92 (dd, J = 8.4, 2.3 Hz, 3H), 7.71-7.67 (m, 1H), 7.50-7.44 (m, 1H), 7.38-7.34 (dd, J = 8.8, 2.9 Hz, 1H), 7.24-7.19 (dt, J = 8.7, 2.6 Hz, 1H), 4.90-4.88 (d, J = 7.7 Hz, 1H), 0.93-0.87 (m, 1H), 0.45-0.40 (m, 1H), 0.28-0.15 (m, 3H).

### Example 56

**5-(4-Chlorophenylsulfonyl)-4-ethyl-4,5-dihydro-1*H*-pyrazolo[4,3-c]quinoline.** Prepared by cyclization of 3-(4-chloro-N-phenylphenylsulfonamido)pentanoic acid, which was prepared as described for compound (27) using methyl 3-oxopentanoate followed by hydrolysis with NaOH, in the manner described for compound 55 followed by pyrazole formation as shown in Example 10. MS *m*/*z* 373.9 (M+H)⁺. ¹H NMR (CDCl₃) δ 7.90 - 7.87 (dd, J = 8.1, 0.9 Hz, 1H), 7.66-7.63 (dd, 7.7, 1.4 Hz, 1H), 7.56 - 7.39 (m, 3H), 7.14 - 7.11 (m, 4H), 5.39 - 5.34 (ABq, J = 8.5, 6.8 Hz, 1H), 1.67 - 1.43 (m, 2H), 1.02 - 0.95 (t, J = 7.3 Hz, 3H).

### Example 57

**5-(4-Chlorophenylsulfonyl)-4-methyl-4,5,5a,6,7,8,9,9a-octahydro-1*H*-pyrazolo[4,3-c]quinoline.** Prepared by sulfonylation of 2-methyloctahydroquinolin-4(1H)-one followed by pyrazole formation as described in Example 1. MS *m*/*z* 365.9 (M+H)⁺. ¹H NMR (CDCl₃) δ 7.80-7.76 (m, 2H), 7.55 (s, 1H), 7.49-7.45 (m, 2H), 4.21-4.15 (m, 1H), 3.15-3.06 (dt, J = 7.9, 2.4 Hz, 1H), 2.95-2.88 (dt, J = 7.9, 2.4 Hz, 1H), 1.95-1.92 (m, 1H), 1.80-1.70 (m, 3H), 1.56-1.55 (m, 1H), 1.51-1.48 (d, J = 7.0 Hz, 3H), 1.40-1.28 (m, 3H).

### Example 58

### 5-(4-Chlorophenylsulfonyl)-9-fluoro-4-methyl-4,5-dihydro-1H-pyrazolo[4,3-c]quinoline.

Prepared as described in Example 4 using 3-fluoroaniline. MS *m*/*z* 378.0 (M+H)⁺. ¹H NMR (CDCl₃) δ 7.70 (d, J = 8.10 Hz, 1H), 7.37 (m, 1H), 7.32 (s, 1H), 7.16-7.04 (m, 5H), 5.70 (q, J = 6.90 Hz, 1H), 1.33 (d, J = 6.93 Hz, 3H).

### Example 59

### 5-(4-Chlorophenylsulfonyl)-7-fluoro-4-methyl-4,5-dihydro-1H-pyrazolo[4,3-c]quinoline.

Prepared as described in Example 4 using 3-fluoroaniline. MS *m*/*z* 378.0 (M+H)⁺. ¹H NMR (CDCl₃) δ 10.40 (br s, 1H), 7.58-7.69 (m, 2H), 7.28-7.16 (m, 3H), 7.11-7.05 (m, 3H), 5.65 (q, J = 6.90 Hz, 1H), 1.33 (d, J = 6.90 Hz, 3H).

### Example 60

### 5-(4-Chlorophenylsulfonyl)-4-cyclopropyl-4,5-dihydro-1H-pyrazolo[4,3-c]quinoline.

Prepared as described in Example 12 using 2-bromophenylboronic acid in place of compound (70). MS *m*/*z* 386.0 (M+H)⁺. ¹H NMR (CDCl₃) δ 8.88 (s, 1H), 7.91 (dd, J=7.8, 1.2 Hz, 1H), 7.62 (dd, J=7.8, 1.5 Hz, 1H), 7.49 (dt, J=7.8, 1.5 Hz, 1H), 7.39 (dt, J=7.8, 1.2 Hz, 1H), 7.34 (s, 1H), 7.06 (s, 4H), 4.95 (d, J=8.4 Hz, 1H), 1.02 (m, 1H), 0.44 (broad m, 3H), 0.14 (m, 1H).

### Example 61

### 5-(4-Chlorophenylsulfonyl)-4-methyl-4,5-dihydro-1H-pyrazolo[4,3-c]quinolin-7-ol.

**(Enantiomers A and B)** Prepared as described in Example 21 followed by chromatographic separation using the specified column type and analyzed using Method 15. Enantiomer A Retention time = 13.4 min. Enantiomer B Retention time = 17.0 min. MS *m*/*z* 375.9(M+H)⁺. ¹H NMR (CDCl₃) δ 7.42 (d, J = 8.4 Hz, 1H), 7.23 (s, 1H), 7.18 (d, J = 2.4 Hz, 1H), 7.14 (m, 4H), 6.85 (dd, J = 8.4, 2.5 Hz, 1H), 5.59 (q, J = 6.9 Hz, 1H), 1.22 (d, J = 6.9 Hz, 3H).

### Example 62

### 5-(4-Chlorophenylsulfonyl)-4,5-dihydro-1H-imidazo[1,2-a]pyrazolo[4,3-e]pyrimidine.

Prepared by reductive amination of aldehyde (69) with 4-chlorobenzenesulfonamide as described for compound (26) in Example 4 to afford 4-chloro-N-((3-iodo-1-((2-(trimethylsilyl)ethoxy)methyl)-1*H*-pyrazol-4-yl)methyl)benzenesulfonamide. This was then coupled to 2-bromo-1*H*-imidazole and deproteced as described for compound (100) in Example 17. MS *m*/*z* 336.0(M+H)⁺. ¹H NMR (CD30D) δ 7.66 (d, J = 1.65 Hz, 1H), 7.61 (d, J = 8.79 Hz, 2H), 7.52 (s, 1H), 7.46 (d, J = 8.79 Hz, 2H), 7.35 (d, J = 1.65 Hz, 1H), 5.36 (s, 2H).

### Example 63

### 5-(4-Chlorophenylsulfonyl)-4-ethyl-4,5-dihydro-1H-pyrazolo[4,3-c]quinoline.

**(Enantiomers A and B)** Prepared by cyclization of 3-(4-chloro-N-phenylphenylsulfonamido)pentanoic acid, which was prepared as described for compound (27) using methyl 3-oxopentanoate followed by hydrolysis with NaOH, in the manner described for compound (53) followed by pyrazole formation as shown in Example 10. Enantiomers were then separated using the specified column type and analyzed using Method 17. Enantiomer A Retention time = 6.0 min. Enantiomer B Retention time = 9.3 min. MS *m*/*z* 373.9(M+H)⁺. ¹H NMR (CD₃OD) δ 7.77-7.74 (d, J = 7.3 Hz, 1H), 7.65 (s, 1H) 7.44-7.35 (m, 3H), 7.14 (s, 4H), 5.36-5.31 (t, J = 8.3 Hz, 1H), 1.51-1.44 (m, 2H), 1.02-0.98 (t, J = 7.2 Hz, 3H).

### Example 64

**5-(4-Chlorophenylsulfonyl)-7-methyl-4,5-dihydro-1*H*-imidazo[1,2-a]pyrazolo[4,3-e]pyrimidines and 5-(4-chlorophenylsulfonyl)-8-methyl-4,5-dihydro-1*H*-imidazo[1,2-a]pyrazolo[4,3-e]pyrimidine.** Prepared by reductive amination of aldehyde (69) with 4-chlorobenzenesulfonamide as described for compound 26 in Example 4 to afford 4-chloro-N-((3-iodo-1-((2-(trimethylsilyl)ethoxy)methyl)-1*H*-pyrazol-4-yl)methyl)benzenesulfonamide. This was then coupled to 2-bromo-4-methyl-1*H*-imidazole and deprotected as described for compound (100) in Example 18 to give a mixture of regioisomers. MS *m*/*z* 350.0 (M+H)⁺. ¹H NMR (CD₃OD) δ 7.39-7.31 (m, 10H), 7.13-7.11 (s, 1H), 6.75-6.73 (m, 1H), 4.98 (s, 2H), 4.97 (s, 2H), 2.41-2.40 (m, 3H), 2.67-2.50 (m, 3H).

### Example 65

**5-(4-Chlorophenylsulfonyl)-4-cyclopropyl-4,5-dihydro-1*H*-imidazo[1,2-a]pyrazolo[4,3-e]pyrimidine.** Prepared by imine formation using aldehyde 69 and 4-chlorobenzenesulfonamide as described for compound 73 in Example 12 to afford 4-chloro-*N*-((3-iodo-1-((2-(trimethylsilyl)ethoxy)methyl)-1*H*-pyrazol-4-yl)methylene)benzenesulfonamide. This was then reacted with isopropylmagnesium bromide as described for compound 74 in Example 12 to afford 4-chloro-*N*-(cyclopropyl(3-iodo-1-((2-(trimethylsilyl)ethoxy)methyl)-1*H*-pyrazol-4-yl)methyl)benzenesulfonamide which was then coupled to 2-bromo-1*H*-imidazole and deprotected as described in Example 17. MS *m*/*z* 376.0 (M+H)⁺. ¹H NMR (CDCl₃) δ 7.82 (d, J = 6.90 Hz, 2H), 7.71 (s, 1H), 7.40 (d, J = 6.6 Hz, 2H), 6.80 (s, 1H), 6.70 (s, 1H), 3.95 (d, J = 7.6 Hz, 1H), 1.10 - 1.04 (m, 1H), 0.59 - 0.51 (m, 2H), 0.48- 0.41 (m, 1H), 0.21 - 0.13 (m, 1H).

### Example 66

### 5-(4-Chlorophenylsulfonyl)-4-methyl-4,5-dihydro-1H-pyrazolo[4,3-c][1,7]naphthyridine.

Prepared as described in Example 18 using 3-bromopyridin-4-ylboronic acid, 4-chlorobenzenesulfonamide and methylmagnesium bromide. MS m/z 362.0 (M+H)⁺. 1H NMR (CDCl3) δ 8.99 (bs, 1H), 8.62 (bs, 1H), 8.03 (bs, 1H), 7.42 (s, 1H), 7.29 (m, 2H), 7.23 (m, 2H), 5.73 (q, J = 6.8 Hz, 1H), 1.28 (d, J = 6.9 Hz, 3H).

### Example 67

**4-Cyclopropyl-7,8-difluoro-5-(5-(trifluoromethyl)pyridin-2-ylsulfonyl)-4,5-dihydro-1*H-*pyrazolo[4,3-c]quinoline.** Prepared as described in Example 12 using aldehyde (103) and sulfonamide (117). MS *m*/*z* 457.0 (M+H)⁺. ¹H NMR (CDCl₃) δ 8.53 (s, 1H), 7.89 (dd, J = 10.6, 8.0 Hz, 1H), 7.82 (dd, J = 18.6, 11.3, 1H), 7.60 (d, J = 8.2 Hz, 1H), 7.45 (dd, J = 18.6, 10.0, 1H), 7.38 (s, 1H), 5.11 (d, J = 8.0 Hz, 1H), 1.06 (m, 1H), 0.58 (m, 1H), 0.47 (m, 2H), 0.20 (m, 1H).

### Example 68

**5-(5-Chlorothiophen-2-ylsulfonyl)-4-cyclopropyl-7,8-difluoro-4,5-dihydro-1*H-*pyrazolo[4,3-c]quinoline.** Prepared as described in Example 12 using 5-chlorothiophene-2-sulfonamide. MS *m*/*z* 428.0 (M+H)⁺. ¹H NMR (CDCl₃) δ 9.97 (broad s, 1H), 7.70 (dd, J = 11.4, 7.2 Hz, 1H), 7.57 (dd, J = 10.2, 8.7 Hz, 1H), 7.31 (s, 1H), 6.70 (d, J = 4.8 Hz, 1H), 6.56(d, J = 4.8 Hz, 1H), 4.98 (d, J = 7.5 Hz, 1H), 1.03 (m, 1H), 0.53 (m, 1H), 0.40 (m, 2H), 0.10 (m, 1H).

### Example 69

**4-Cyclopropyl-5-(4-fluorophenylsulfonyl)-4,5-dihydro-1*H*-pyrazolo[4,3-c][1,8]naphthyridine.** Prepared as described in Example 18 using 4-fluorobenzenesulfonamide. MS *m*/*z* 371.0 (M+H)⁺. ¹H NMR (CDCl₃) δ 8.42 (dd, J = 4.9, 1.6 Hz, 1H), 8.10 (dd, J = 7.6, 1.6 Hz, 1H), 7.98 (m, 2H), 7.58 (s, 1H), 7.22 (dd, J = 7.6, 4.9 Hz, 1H), 7.08 (m, 2H), 5.42 (d, J = 8.0 Hz, 1H), 1.14 (m, 1H), 0.46 (m, 3H), 0.21 (m, 1H).

### Example 70

**4-Cyclopropyl-5-(4-(trifluoromethyl)phenylsulfonyl)-4,5-dihydro-1*H*-imidazo[1,2-a]pyrazolo[4,3-e]pyrimidines.** Prepared by imine formation using aldehyde (69) and 4-(trifluoromethyl)benzenesulfonamide as described for compound (73) in Example 12 to afford 4-chloro-*N*-((3-iodo-1-((2-(trimethylsilyl)ethoxy)methyl)-1*H*-pyrazol-4-yl)methylene)benzenesulfonamide. This was then reacted with isopropylmagnesium bromide as described for compound (74) in Example 12 to afford 4-chloro-*N*-(cyclopropyl(3-iodo-1-((2-(trimethylsilyl)ethoxy)methyl)-1*H*-pyrazol-4-yl)methyl)benzenesulfonamide which was then coupled to 2-bromo-1*H*-imidazole and deprotected as described in Example 17.. MS *m*/*z* 410.0 (M+H)⁺. ¹H NMR (CD₃OD) δ 8.05 (d, J = 8.3 Hz, 2H), 7.78 (d, J = 9.4 Hz, 2H), 7.76 (s, 1H), 6.86 (dd, J = 13.0, 2.6 Hz, 2H), 3.80 (d, J = 8.2 Hz, 1H), 1.05-1.03 (m, 1H), 0.42-0.37 (m, 1H), 0.31-0.24 (m, 1H), 0.21-0.13 (m, 1H), 0.54-0.14 (m, 1H).

### Example 71

**4-Cyclopropyl-7,8-difluoro-5-(6-(trifluoromethyl)pyridin-3-ylsulfonyl)-4,5-dihydro-1*H-*pyrazolo[4,3-c]quinoline.** Prepared as described in Example 12 using aldehyde (103) and sulfonamide (83). MS *m*/*z* 457.0 (M+H)⁺. ¹H NMR (CDCl₃) δ 8.57 (s, 1H), 7.76 (m, J = 11.0, 7.6 Hz, 1H), 7.52 (m, 3H), 7.43 (d, J = 8.3 Hz, 1H), 7.33 (s, 1H), 5.03 (d, J = 7.6 Hz, 1H), 1.05 (m, 1H), 0.56 (m, 1H), 0.42 (m, 2H), 0.11 (m, 1H).

### Example 72

**5-(4-Fluorophenylsulfonyl)-4-(trifluoromethyl)-4,5-dihydro-1*H*-pyrazolo[4,3-c][1,8]naphthyridine.** Prepared as described in Example 18 using methyl trifluoromethanesulfonate in place of cyclopropymagnesium bromide. MS *m*/*z* 399.0 (M+H)⁺. ¹H NMR (CDCl₃) δ 8.30 (dd, J = 4.9, 1.9 Hz, 1H), 8.15 (dd, J = 7.6, 1.9 Hz, 1H), 8.05 (m, 2H), 7.82 (s, 1H), 7.22 (m, 3H), 6.58 (q, J = 7.4 Hz, 1H).

### Example 73

**7,8-Difluoro-4-(pyridin-3-yl)-5-(4-(trifluoromethyl)phenylsulfonyl)-4,5-dihydro-1*H-*pyrazolo[4,3-c]quinoline.** Prepared by addition of 3-lithiopyridine (Ota, T.; Terashima, M. J. Heterocycl. Chem. 1987, 24(2), 377) to compound (73) followed by pyrazole formation as described in Example 12. MS *m*/*z* 493.0 (M+H)⁺. ¹H NMR (CDCl₃) δ 8.52 (d, J = 3.8 Hz, 1H), 8.33 (s, 1H), 7.72-7.48 (m, 7H), 7.40-7.33 (m, 2H), 6.71 (s, 1H).

### Example 74

**4-Cyclopropyl-8-fluoro-5-(4-(trifluoromethyl)phenylsulfonyl)-4,5-dihydro-1*H-*pyrazolo[4,3-c]quinoline.** Prepared as described in Example 12 using 2-bromo-5-fluorophenylboronic acid in place of compound (70). MS *m*/*z* 437.9 (M+H)⁺. ¹H NMR (CD₃OD) δ 7.85-7.80 (dd, J = 8.9, 5.1 Hz, 1H), 7.50-7.46 (m, 2H) 7.40-7.34 (m, 4H), 7.22-7.16 (td, J = 8.7, 2.9 Hz, 1H), 5.07-5.04 (d, J = 7.5 Hz, 1H), 1.02-0.94 (m, 1H), 0.54-0.47 (m, 1H), 0.41-0.31 (m, 2H), 0.17-0.12 (m, 1H).

### Example 75

**4-Cyclopropyl-7,8-difluoro-5-(6-(trifluoromethyl)pyridin-3-ylsulfonyl)-4,5-dihydro-1*H-*pyrazolo[4,3-c]quinoline. (Enantiomers A and B)** Prepared as described in Example 13 using boronic acid (70) and sulfonyl chloride (83). Enantiomers were then separated using HPLC Method 19. Enantiomer A Retention time = 17.3 min. Enantiomer B Retention time = 13.0 min. MS *m*/*z* 457.0 (M+H)⁺. ¹H NMR (CDCl₃) δ 10.03 (br s, 1H), 8.58 (s, 1H), 7.76 (dd, J=11.0, 7.2 Hz, 1H), 7.49 (m, 2H), 7.44 (s, 1H), 5.03 (d, J=7.6 Hz), 1.04 (m, 1H), 0.56 (m, 1H), 0.41 (m, 2H), 0.11 (m, 1H).

### Example 76

**7,8-Difluoro-4-isopropyl-5-(4-(trifluoromethyl)phenylsulfonyl)-4,5-dihydro-2*H-*pyrazolo[4,3-c]quinoline.** Prepared as described in Example 12 using isopropylmagnesium bromide. The product was analyzed by HPLC using Method 7. Retention time = 8.77 min. MS *m*/*z* 458.0 (M+H)⁺. ¹H NMR (CDCl₃) δ 7.73 (dd, J = 11.1, 7.5 Hz, 1H), 7.35 (broad m, 5H), 7.25 (s, 1H), 4.92 (d, J = 9.9 Hz, 1H), 4.48 (broad s, 1H), 1.50 (m, 1H), 1.09 (d, J = 6.9 Hz, 3H), 0.83 (d, J = 6.9 Hz, 3H).

### Example 77

**4-Cyclopropyl-7,8-difluoro-5-(4-(trifluoromethoxy)phenylsulfonyl)-4,5-dihydro-1*H-*pyrazolo[4,3-c]quinoline.** Prepared as described in Example 12 using 4-trifluoromethoxybenzensulfonamide in place of compound (72). MS *m*/*z* 472.0 (M+H)⁺. ¹H NMR (CDCl₃) δ 7.78 (dd, J = 10.8, 6.8 Hz, 1H), 7.43 (dd, J = 9.8, 8.2 Hz, 1H), 7.26 (s, 1H), 7.23 (d, J = 9.3 Hz, 2H), 6.96 (d, J = 7.9 Hz, 2H), 4.93 (d, J = 7.6 Hz, 1H), 1.07-0.96 (m, 1H), 0.61-0.50 (m, 1H), 0.47-0.37 (m, 2H), 0.13-0.06 (m, 1H).

### Example 78

**4-Cyclopropyl-8-fluoro-5-(4-(trifluoromethyl)phenylsulfonyl)-4,5-dihydro-1*H-*pyrazolo[4,3-c]quinoline. (Enantiomers A and B)** Prepared as described in Example 12 using 2-bromo-5-fluorophenylboronic acid in place of compound (70). Enantiomers were then separated using the specified column type and analyzed using Method 15. Enantiomer A Retention time = 8.0 min. Enantiomer B Retention time = 12.6 min. MS *m*/*z* 437.9 (M+H)⁺. ¹H NMR (CD₃OD) δ 7.85-7.80 (dd, J = 8.9, 5.1 Hz, 1H), 7.50-7.46 (m, 2H) 7.40-7.34 (m, 4H), 7.22-7.16 (td, J = 8.7, 2.9 Hz, 1H), 5.07-5.04 (d, J = 7.5 Hz, 1H), 1.02-0.94 (m, 1H), 0.54-0.47 (m, 1H), 0.41-0.31 (m, 2H), 0.17-0.12 (m, 1H).

### Example 79

**5-(5-Chloropyridin-2-ylsulfonyl)-4-cyclopropyl-8-fluoro-4,5-dihydro-1*H*-pyrazolo[4,3-c]quinoline.** Prepared as described in Example 12 using 5-chloro-2-pyridinesulfonamide. MS *m*/*z* 405 (M+H)⁺. ¹H NMR (DMSO-D6) δ 8.36-8.35 (d, J = 2.2 Hz, 1H), 7.95-7.92 (dd, J = 8.4, 2.3 Hz, 3H), 7.71-7.67 (m, 1H), 7.50-7.44 (m, 1H), 7.38-7.34 (dd, J = 8.8, 2.9 Hz, 1H), 7.24-7.19 (dt, J = 8.7, 2.6 Hz, 1H), 4.90-4.88 (d, J = 7.7 Hz, 1H), 0.93-0.87 (m, 1H), 0.45-0.40 (m, 1H), 0.28-0.15 (m, 3H).

### Example 80

**4-Cyclopropyl-5-(6-(trifluoromethyl)pyridin-3-ylsulfonyl)-4,5-dihydro-1*H*-pyrazolo[4,3-c]quinoline.** Prepared as described in Example 13 using 2-bromoboronic acid and sulfonyl chloride (83). Enantiomers were then separated using the specified column type and analyzed using Method 17. MS *m*/*z* 421.1 (M+H)⁺. ¹H NMR (CDCl₃) δ 8.51 (s, 1H), 7.87 (d, J = 9.7, 1H), 7.68 (d, J = 7.7 Hz, 1H), 7.49-7.26 (m, 5H), 5.00 (d, J = 7.6 Hz, 1H), 1.07-1.00 (m, 1H), 0.56-0.31 (m, 3H), 0.17-0.08 (m, 1H).

### Example 81

### 5-(4-Chlorophenylsulfonyl)-4-methyl-4,5-dihydro-2H-pyrazolo[4,3-c]quinolin-8-ol.

Prepared from 8-(benzyloxy)-5-(4-chlorophenylsulfonyl)-4-methyl-4,5-dihydro-1*H-*pyrazolo[4,3-c]quinoline as described for compound 119 in Example 21. MS m/z 376 (M+H)⁺. 1H NMR (CD30D) δ 7.55 (d, J = 8.7 Hz, 1H), 7.45 (s, 1H), 7.11 (m, 4H), 6.84 (dd, J = 8.7, 2.8 Hz, 1H), 5.60 (q, J = 6.9 Hz, 1H), 1.25 (d, J = 7.0 Hz, 3H).

### Example 82

### (R)-4-(4-Cyclopropyl-7,8-difluoro-1H-pyrazolo[4,3-c]quinolin-5(4H)-ylsulfonyl)aniline.

Prepared as described in Example 13 using boronic acid (70) and 4-nitrobenzenesulfonyl chloride followed by reduction of the nitro group using hydrogen over Pt₂O in MeOH. MS *m*/*z* 403.1 (M+H)⁺. ¹H NMR (CDCl₃) δ 7.77 (dd, J = 11.2, 7.4 Hz, 1H), 7.41 (dd, J = 10.0, 8.0 Hz, 1H), 7.29 (s, 1H), 6.94 (d, J = 8.4 Hz, 2H), 6.27 (d, J = 9.0 Hz, 2H), 4.95 (d, J = 7.7 Hz, 1H), 1.05-0.96 (m, 1H), 0.54-0.48 (m, 1H), 0.44-0.33 (m, 2H), 0.13-0.04 (m, 1H).

### Example 83

**(*R*)-4-Cyclopropyl-7,8-difluoro-5-(4-nitrophenylsulfonyl)-4,5-dihydro-1*H*-pyrazolo[4,3-c]quinoline.** Prepared as described in Example 13 using boronic acid (70) and 4-nitrobenzenesulfonyl chloride. MS *m*/*z* 432.2 (M+H)⁺. ¹H NMR (CDCl₃) δ 7.78 (dd, J = 11.0, 7.0 Hz, 1H), 7.41 (dd, J = 9.7, 8.0 Hz, 1H), 7.28 (s, 1H), 6.95 (d, J = 8.9 Hz, 2H), 6.17 (d, J = 8.6 Hz, 2H) 4.97 (d, J = 7.1 Hz, 1H), 1.04-0.94 (m, 1H), 0.57-0.47 (m, 1H), 0.45-0.31 (m, 2H), 0.129-0.05 (m, 1H).

### Example 84

**5-Chloro-2-(4-cyclopropyl-7,8-difluoro-1*H*-pyrazolo[4,3-c]quinolin-5(4*H*)-ylsulfonyl)thiazole.** Prepared as described in Example 13 using boronic acid (70), aldehyde (103), and 5-chlorothiazole-2-sulfonyl chloride. MS *m*/*z* 429 (M+H)⁺. ¹H NMR (CDCl₃) δ 7.71 (dd, J = 11.2, 7.4 Hz, 1H), 7.58 (dd, J = 10.3, 8.8 Hz, 1H), 7.38 (s, 1H), 7.33 (s, 1H), 5.07 (d, J = 7.5 Hz, 1H), 1.09-1.02 (m, 1H), 0.56-0.32 (m, 2H), 0.21-0.08 (m, 1H).

### Example 85

**(*R*)-4-Cyclopropyl-5-(4-(difluoromethoxy)phenylsulfonyl)-7,8-difluoro-4,5-dihydro-1*H-*pyrazolo[4,3-c]quinoline.** Prepared as described in Example 13 using boronic acid (70) and 4-(difluoromethoxy)benzenesulfonyl chloride. MS *m*/*z* 454.1 (M+H)⁺. ¹H NMR (CDCl₃) δ 7.78 (dd, J = 7.9, 4.3 Hz, 1H), 7.42 (dd, J = 9.9, 8.1 Hz, 1H), 7.27 (s, 1H), 7.20 (d, J = 8.8 Hz, 2H), 6.85 (d, J = 8.9 Hz, 2H), 6.45 (t, J = 72.6 Hz, 1H), 4.94 (d, J = 7.7 Hz, 1H), 1.05-0.96 (m, 1H), 0.60-0.49 (m, 1H), 0.46 - 0.35 (m, 2H), 0.15-0.06 (m, 1H).

### Example 86

**4-Cyclopropyl-8-fluoro-5-(5-(trifluoromethyl)pyridin-2-ylsulfonyl)-4,5-dihydro-1*H-*pyrazolo[4,3-c]quinoline. ( Enantiomers A and B)** Prepared as described in Example 12 using boronic acid (77) and sulfonamide (117). Enantiomers were then separated using the specified column type and analyzed using Method 20.
Enantiomer A Retention time = 7.6 min.
Enantiomer B Retention time = 13.7 min.
MS *m*/*z* 439.1 (M+H)⁺. ¹H NMR (300 MHz, CDCl₃) δ 10.14 (br s, 1H), 8.53 (m, 1H), 7.86 (dd, J=9.3 Hz and 5.7 Hz, 1H), 7.81 (m, 1H), 7.46 (d, J=8.2 Hz, 1H), 7.38 (dd, J=8.5 Hz and 3.2 Hz, 1H), 7.10 (m, 1H), 5.10 (m, J=7.8 Hz, 1H), 1.06 (m, 1H), 48 (m, 3H), .16 (m, 1H).

### Example 87

**4-Cyclopropyl-5-(4-(difluoromethoxy)phenylsulfonyl)-8-fluoro-4,5-dihydro-1*H-*pyrazolo[4,3-c]quinoline.** Prepared as described in Example 13 using 4-(difluoromethoxy)benzenesulfonyl chloride in place of compound 83. MS *m*/*z* 436.1 (M+H)⁺. ¹H NMR (300 MHz, CDCl₃) δ 7.89 (dd, J = 8.5, 4.8 Hz, 1H), 7.29 (d, J = 8.5 Hz, 2H), 7.21-7.12 (m, 1H), 7.14 (d, J = 8.6 Hz, 2H), 6.83 (d, J = 8.7 Hz, 1H), 6.65 (s, 1H), 6.44 (t, J = 7.25 Hz, 1H), 4.93 (d, J = 8.2 Hz, 1H), 1.02-0.99 (m, 1H), 0.54-0.51 (m, 1H), 0.46-0.37 (m, 2H), 0.13-0.11 (m, 1H).

### Example 88

**7-(Benzyloxy)-5-(4-chlorophenylsulfonyl)-4-methyl-4,5-dihydro-1*H*-pyrazolo[4,3-c]quinoline.** Prepared as described in Example 4 using 3-benzyloxyaniline. MS *m*/*z* 467.8 (M+H)⁺. ¹H NMR (CDCl₃) δ 7.47 (m, 4H), 7.35 (m, 3H), 7.19 (s, 1H), 7.01 (m, 5H), 5.62 (q, J = 6.87 Hz, 1H), 5.16 (s, 2H), 1.30 (d, J = 6.93 Hz, 3H).

### Example 89

**5-(4-Fluorophenylsulfonyl)-4-methyl-4,5,5a,6,7,8,9,9a-octahydro-1*H*-pyrazolo[4,3-c]quinoline.** Prepared by sulfonylation of 2-methyloctahydroquinolin-4(1H)-one followed by pyrazole formation as described in Example 1. MS *m*/*z* 350 (M+H)⁺. ¹H NMR (CDCl₃) δ 7.88-7.83 (m, 2H), 7.56 (s, 1H), 7.20-7.15 (m, 2H), 5.16-5.09 (q, J = 6.8 Hz, 1H), 4.21-4.14 (m, 1H), 2.77 (m, 1H), 2.40-2.35 (m, 1H), 1.80-1.73 (m, 1H), 1.70-1.69 (m, 2H), 1.65-1.63 (d, J = 6.0 Hz, 3H), 1.55-1.52 (m, 1H), 1.44-1.27 (m, 2H), 1.08-0.99 (m, 1H).

The following compounds were prepared essentially according to the above methods and schemes described above.

### Example 90

**5'-(4-(Trifluoromethyl)-phenylsulfonyl)-1',5'-dihydrospiro[cyclopropane-1,4'-pyrazolo[4,3-c]quinoline].** MS *m*/*z* 439.1 (M+H)⁺. ¹H NMR (CDCl₃) δ 7.75-7.73 (m, 1H), 7.61-7.45 (m, 3H), 7.36 (d, J = 8.0 Hz, 2H), 7.26 (d, J = 8.0 Hz, 2H), 7.0 (s, 1H), 2.70-2.60 (m, 1H), 1.68-1.60 (m, 1H), 1.29-1.19 (m, 1H), 0.58-0.51 (m, 1H).

### Example 91

**5'-(4-Chlorophenylsulfonyl)-1',5'-dihydrospiro[cyclopropane-1,4'-pyrazolo[4,3-c]quinoline].** MS *m*/*z* 372.1 (M+H)⁺. ¹H NMR (CDCl₃) δ 7.72 - 7.69 (m, 1H), 7.64 - 7.61 (m, 1H), 7.52 - 7.42 (m, 2H), 7.28 - 7.25 (m, 2H), 7.08 - 7.04 (m, 2H), 7.0 (s, 1H), 2.64 - 2.56 (m, 1H), 1.64 - 1.56 (m, 1H), 1.29-1.19 (m, 1H), 0.56 - 0.52 (m, 1H).

### Example 92

**7,8-Difluoro-4-methyl-5-(thiophen-2-ylsulfonyl)-4,5-dihydro-1*H*-pyrazolo[4,3-c]quinoline.** MS *m*/*z* 368 (M+H)⁺. ¹H NMR (CDCl₃) δ 7.72 (dd, J=10.8, 7.2 Hz, 1H), 7.57 (dd, J=9.9, 8.1 Hz, 1H), 7.29 (m, 2H), 6.98 (dd, J=3.9, 1.2 Hz, 1H), 6.75 (d, J=4.8, 3.9 Hz, 1H), 5.84 (broad s, 1H), 5.69 (q, J=7.2 Hz, 1H), 1.32 (q, J=7.2 Hz, 3H).

### Example 93

### 5-(4-Chlorophenylsulfonyl)-4-methyl-4,5-dihydro-1H-pyrazolo[4,3-c][1,8]naphthyridine.

Prepared as described in Example 18 using methylmagnesium bromide in place of cyclopropylmagnesium bromide. MS *m*/*z* 368 (M+H)⁺. ¹H NMR (CDCl₃) δ 8.30 (dd, J = 4.9, 1.8 Hz, 1H), 8.07 (dd, J = 7.6, 1.8 Hz, 1H), 7.95 (m, 2H), 7.43 (s, 1H), 7.38 (m, 2H), 7.10 (dd, J = 7.6, 4.9 Hz, 1H).

### Example 94

**5-(4-Chlorophenylsulfonyl)-4-methyl-4,5-dihydro-1*H*-pyrazolo[4,3-c]quinolin-7-yl dimethylcarbamate.** MS *m*/*z* 447(M+H)⁺. ¹H NMR (CDCl₃) δ 7.65 (d, J=11.7 Hz, 1H), 7.64 (s, 1H), 7.22 (s, 1H), 7.18 (d, J=8.6 Hz, 1H) 7.17 (d, J=8.8 Hz, 2H), 7.06 (d, J=8.8 Hz, 2H), 5.64 (q, J=6.8 Hz, 1H), 3.17 (s, 3H), 3.07 (s, 3H), 1.32 (d, J=7.0 Hz, 3H).

### Example 95

**7-Chloro-4-methyl-5-(4-(trifluoromethyl)phenylsulfonyl)-4,5-dihydro-1*H*-pyrazolo[3,4-d]thieno[2,3-b]pyridine.** MS *m*/*z* 434.0(M+H)⁺. ¹H NMR (CDCl₃) δ 8.58 (s, 1H), 7.62 (d, J=9.0 Hz, 2H), 7.54 (dd, J=9.0 Hz, 2H), 7.36 (s, 1H), 7.03 (s, 1H), 5.66 (q, J=6.3 Hz, 1H), 1.43 (q, 6.3 Hz, 3H).

### Example 96

**4-Ethyl-7,8-difluoro-5-(5-(trifluoromethyl)pyridin-2-ylsulfonyl)-4,5-dihydro-1*H-*pyrazolo[4,3-c]quinoline.** MS *m*/*z* 445.0(M+H)⁺. ¹H NMR (CDCl₃) δ 8.53 (s, 1H), 7.89 (dd, J = 10.6, 8.0 Hz, 1H), 7.74 (dd, J = 18.6, 11.3, 1H), 7.60 (d, J = 8.2 Hz, 1H), 7.46 (dd, J = 18.6, 10.0, 1H), 7.34 (s, 1H), 5.45 (dd, J = 15.3, 8.5 Hz, 1H), 1.64 (m, 1H), 1.50 (m, 1H), 1.00 (t, J=7.3 Hz, 3H).

### Example 97

**4-Cyclopropyl-7,8-difluoro-5-(thiophen-2-ylsulfonyl)-4,5-dihydro-1*H*-pyrazolo[4,3-c]quinoline.** MS *m*/*z* 394.0 (M+H)⁺. ¹H NMR (CDCl₃) δ 7.75 (dd, J = 11.4, 7.5 Hz, 1H), 7.57 (dd, J = 10.5, 8.7 Hz, 1H), 7.29 (m, 2H), 7.08 (broad s, 1H), 6.96 (dd, J = 3.9, 1.2 Hz, 1H), 6.73 (d, J = 5.1, 3.9 Hz, 1H), 5.01 (d, J = 8.1 Hz, 1H), 1.03 (m, 1H), 0.53 (m, 1H), 0.40 (m, 2H), 0.11 (m, 1H).

### Example 98

**7,8-Difluoro-5-(4-(trifluoromethyl)-phenylsulfonyl)-4,5-dihydro-2*H*-pyrazolo[4,3-c]quinoline.** MS *m*/*z* 416 (M+H)⁺. ¹H NMR (CDCl₃) δ 7.69 (dd, J=11.1, 7.2 Hz, 1H), 7.48 (dd, J=10.2, 8.4 Hz, 1H), 7.38 (d, 11.4 Hz, 4H), 7.20 (s, 1H), 4.93 (s, 2H), 2.69 (broad s, 1H).

### Example 99

**4-Cyclopropyl-7-(trifluoromethoxy)-5-(6-(trifluoromethyl)pyridin-3-ylsulfonyl)-4,5-dihydro-2*H*-pyrazolo[4,3-c]quinoline.** Prepared as described in Example 13 using 2-bromo-4-(trifluoromethoxy)phenylboronic acid. MS *m*/*z* 506.1 (M+H)⁺. ¹H NMR (CD₃OD) δ 8.45 (s, 1H), 7.78-7.73 (m, 3H), 7.62-7.59 (m, 1H), 7.47 (s, 1H), 7.41-7.37 (m, 1H), 5.18-5.16 (d, J = 7.6 Hz, 1H), 1.06-0.98 (m, 1H), 0.60-0.52 (m, 1H), 0.45-0.34 (m, 2H), 0.23-0.14 (m, 1H).

### Example 100

**(*R*)-4-cyclopropyl-7-(trifluoromethyl)-5-(6-(trifluoromethyl)pyridin-3-ylsulfonyl)-4,5-dihydro-1*H*-pyrazolo[4,3-c]quinoline.** Prepared as described in Example 13 using 2-bromo-4-(trifluoromethyl)phenylboronic acid and sulfonyl chloride 83. MS *m*/*z* 488.9 (M+H)⁺. ¹H NMR (CDCl₃) δ 8.53 (s, 1H), 8.17 (s, 1H), 7.84-7.81 (m, 1H) 7.68-7.65 (m, 1H), 7.50-7.28 (m, 3H), 5.08-5.05 (d, J = 7.7 Hz, 1H), 1.09-0.93 (m, 1H), 0.64-0.55 (m, 1H), 0.50-0.36 (m, 2H), 0.18-0.13 (m, 1H).

### Example 101

**(*R*)-4-Cyclopropyl-7-(trifluoromethoxy)-5-(4-(trifluoromethoxy)phenylsulfonyl)-4,5-dihydro-2*H*-pyrazolo[4,3-c]quinoline.** Prepared as described in Example 13 using 2-bromo-4-(trifluoromethoxy)phenylboronic acid and 4-(trifluoromethoxy)benzenesulfonyl chloride. MS *m*/*z* 520.1 (M+H)⁺. ¹H NMR (CDCl₃) δ 7.85 (s, 1H), 7.71-7.68 (m, 1H), 7.43 (s, 1H), 7.31-7.24 (m, 3H) 6.99-6.96 (m, 2H), 5.02-4.99 (d, J = 8.0 Hz, 1H), 1.09-1.00 (m, 1H), 0.66-0.57 (m, 1H), 0.55-0.40 (m, 2H), 0.23-0.16 (m, 1H).

### Example 102

**(*R*)-4-cyclopropyl-8-(trifluoromethyl)-5-(6-(trifluoromethyl)pyridin-3-ylsulfonyl)-4,5-dihydro-1*H*-pyrazolo[4,3-c]quinoline.** Prepared as described in Example 13 using 2-bromo-5-(trifluoromethyl)phenylboronic acid. MS *m*/*z* 489.1 (M+H)⁺. ¹H NMR (CDCl₃) δ 8.59 (s, 1H), 8.11-8.08 (m, 1H), 7.91 (s, 1H) 7.82-7.79 (m, 1H), 7.61-7.49 (m, 3H), 5.08-5.05 (d, J = 8.0 Hz, 1H), 1.08-1.01 (m, 1H), 0.67-0.58 (m, 1H), 0.55-0.44 (m, 2H), 0.23-0.17 (m, 1H).

### Example 103

### 4-Cyclopropyl-7-methyl-5-(4-(trifluoromethyl)phenylsulfonyl)-4,5-dihydro-1H-pyrazolo[3,4-d]thiazolo[5,4-b]pyridine (149).

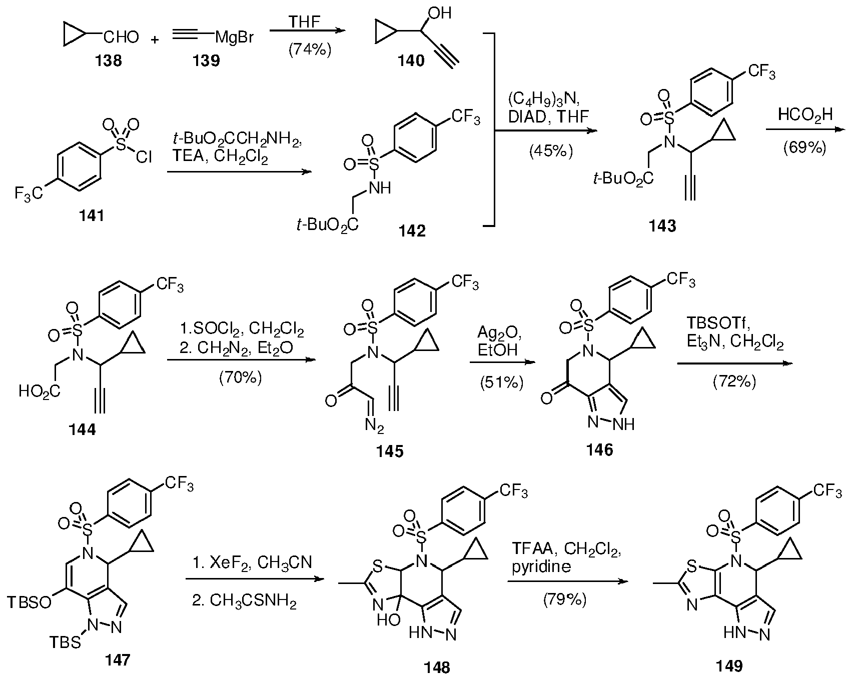

1-Cyclopropylprop-2-yn-1-01 (140). To a solution of aldehyde 138 (5.00 g, 71.3 mmol) in THF (50 mL) at 0 °C was added ethynylmagnesium bromide (139) (0.5M in THF, 178 mL, 89.2 mmol), dropwise via a pressure equalizing addition funnel. The reaction mixture was stirred for 3 h at 0 °C, and then quenched with saturated aqueous NH₄Cl (100 mL) and warmed to room temperature. The reaction mixture was filtered through a pad of Celite. The filtrate was diluted with EtOAc (100 mL) and the layers were separated. The organic layer was washed with saturated aqueous NaCl (100 mL), dried over MgSO₄, filtered and concentrated. The resulting residue was purified by flash chromatography (20% EtOAc in hexanes) to afford 6.15 g of alcohol 140 (90%). ¹H NMR (300 MHz, CDCl₃) δ 4.20 (bd, *J* = 5.6 Hz, 1H), 2.44 (d, *J* = 2.1 Hz, 1H), 1.90 (bs, 1H), 1.25 (m, 1H), 0.57 (m, 2H), 0.52 (m, 2H).

*tert*-Butyl 2-(4-(trifluoromethyl)phenylsulfonamido)acetate (142). To a solution of glycine *tert*-butyl ester (10.0 g, 76.2 mmol) in CH₂Cl₂ (150 mL) at 0 °C was added triethylamine (12.7 mL, 91.5 mmol) followed by sulfonyl chloride 141 (18.6 g, 76.2 mmol). The reaction mixture was stirred for 18 h while warming to room temperature. The reaction mixture was quenched with the addition of H₂O (100 mL), and the two layers were separated. The organic layer was dried over MgSO₄, filtered and concentrated to provide 25.9 g of 142 (100%). MS (ES) *m*/*z:* 340.0 (M+H)⁺.

*tert*-Butyl 2-(*N*-(1-cyclopropylprop-2-ynyl)-4-(trifluoromethyl)phenylsulfonamido)acetate (143). To a solution of 142 in THF (150 mL) at 0 °C was added tributylphosphine (29.1 g, 143.6 mmol) and alcohol 140 (5.06 g, 52.6 mmol). Diisopropyl azodicarboxylate (27.8 mL, 143.6 mmol) was then added in a drop wise manner. The reaction mixture was stirred at 0 °C for 2 h, and then concentrated. The resulting residue was purified by flash chromatography (10% EtOAc and 10% CH₂Cl₂ in hexanes) to afford 9.84 g of 143 (45%). MS (ES) *m*/*z:* 440.2 (M+Na)⁺.

2-(*N*-(1-Cyclopropylprop-2-ynyl)-4-(trifluoromethyl)phenylsulfonamido)acetic acid (144). Compound 143 (8.34 g, 20.0 mmol) was dissolved in formic acid and allowed to stand for 24 h at room temperature. A white precipitate formed, which was isolated by filtration. The solid was washed with cold EtOAc (10 mL) to provide 5.08 g of 144 (69%). MS (ES) *m*/*z*: 362.0 (M+H)⁺.

*N*-(1-Cyclopropylprop-2-ynyl)-*N*-(3-diazo-2-oxopropyl)-4-(trifluoromethyl)benzenesulfonamide (145). To thionyl chloride (2 mL) was added compound 144 (0.50 g, 1.38 mmol). The reaction mixture was heated to 70 °C for 2 h. The reaction mixture was cooled to room temperature and concentrated. The resulting residue was dissolved in Et₂O (5 mL) and added to a solution of freshly prepared diazomethane in Et₂O (30 mL) at 0 °C. The diazomethane was prepared in the following manner; to a solution of 40% KOH_{(aq)} (15 mL) and Et₂O (30 mL) at 0 °C was added 1-methyl-3-nitro-1-nitrosoguanadine (1.01 g, 6.91 mmol). The reaction mixture was allowed to stand at 0 °C for 45 minutes. The temperature was decreased to -40 °C, and the aqueous layer was frozen. The organic layer was decanted into a fresh vial at 0 °C containing solid KOH (2.50 g). The reaction mixture was allowed to stand at 0 °C for 15 minutes. The reaction mixture was then decanted into a clean vial at 0 °C, at which time the Et₂O solution of acid chloride was added.

The reaction mixture was allowed to stand for 30 minutes at 0 °C, and then quenched with acetic acid (2 mL). The reaction mixture was warmed to room temperature and diluted with EtOAc (25 mL) and H₂O (25 mL). The two layers were separated. The organic layer was washed with saturated NaHCO₃ (2x 30 mL) and saturated aqueous NaCl (30 mL). The organic layer was dried over MgSO₄, filtered and concentrated. The resulting residue was purified by flash chromatography (20% EtOAc in hexanes) to afford 0.37 g of 145 (70%). MS (ES) m/z: 408.1 (M+Na)⁺.

4-Cyclopropyl-5-(4-(trifluoromethyl)phenylsulfonyl)-5,6-dihydro-2*H*-pyrazolo[4,3-*c*]pyridin-7(4*H*)-one (146). To a solution of compound 145 (0.37 g, 0.96 mmol) in EtOH (5 mL) was added silver(I) oxide (0.02 g, 0.09 mmol). The reaction mixture was heated to 80 °C for 18h, and then cooled to room temperature. The reaction mixture was filtered through a pad of Celite and concentrated. The resulting residue was purified by flash chromatography (40% EtOAc in hexanes) to afford 0.19 g of 146 (51%). MS (ES) *m*/*z:* 386.1 (M+H)⁺.

1-(*tert*-Butyldimethylsilyl)-7-(*tert*-butyldimethylsilyloxy)-4-cyclopropyl-5-(4-(trifluoromethyl)phenylsulfonyl)-4,5-dihydro-1*H*-pyrazolo[4,3-*c*]pyridine (147). To a solution of compound 146 (0.33 g, 0.87 mmol) in CH₂Cl₂ (2.5 mL) at 0 °C was added triethylamine (0.14 mL, 1.04 mmol) followed by *tert*-butyldimethysilyl trifluoromethanesulfonate (0.21 mL, 0.87 mmol). The reaction mixture was stirred at 0 °C for 1.5 h, and then quenched with H₂O (1 mL). The reaction mixture was warmed to room temperature and the layers were separated. The organic layer was dried over MgSO₄, filtered and concentrated. The resulting residue was purified by flash chromatography (10% EtOAc in hexanes) to afford 0.38 g of 147 (72%). MS (ES) *m*/*z:* 500.1 (M-TBS)⁺.

.4-Cyclopropyl-7-methyl-5-(4-(trifluoromethyl)phenylsulfonyl)-4,5,5a,8a-tetrahydro-1*H-*pyrazolo[3,4-*d*]thiazolo[5,4-*b*]pyridin-8a-ol (148). To a solution of compound 147 (0.05 g, 0.08 mmol) in CH₃CN (1 mL) was added xenon difluoride (0.05 g, 0.28 mmol). The reaction mixture was stirred for 3 h at room temperature and then thioacetamide (0.03 g, 0.41 mmol) was added in bulk. The reaction mixture was stirred for 18 h at room temperature and then quenched with saturated aqueous NaHCO₃ (1 mL). The reaction mixture was diluted with EtOAc (5 mL) and the layers were separated. The organic layer was dried over MgSO₄, filtered and concentrated. The resulting residue was purified by flash chromatography (50% EtOAc in hexanes) to afford 0.03 g of 148 (80%). MS (ES) *m*/*z:* 459.0 (M+H)⁺.

4-Cyclopropyl-7-methyl-5-(4-(trifluoromethyl)phenylsulfonyl)-4,5-dihydro-1*H*-pyrazolo[3,4-dlthiazolo[5,4-*b*]pyridine (149). To a solution of compound 148 (0.03 g, 0.06 mmol) in CH₂Cl₂ (1 mL) at 0 °C was added trifluoroacetic anhydride (0.04 mL, 0.32 mmol) followed by pyridine (0.05 mL, 0.65 mmol). The reaction mixture was stirred for 1 h at 0 °C, and then quenched with saturated aqueous NaHCO₃ (1 mL) and diluted with CH₂Cl₂ (5 mL). The layers were separated. The organic layer was washed with H₂O (2 mL), dried over MgSO₄, filtered and concentrated. The resulting residue was purified by flash chromatography (50% EtOAc in hexanes) to afford 0.03 g of 149 (79%). ¹H NMR (300 MHz, CD₃Cl₃) δ 7.62 (d, *J* = 8.3 Hz, 2H), 7.50 (d, *J* = 8.4 Hz, 2H), 7.36 (s, 4H), 5.06 (d, *J* = 7.5 Hz, 1H), 2.78 (s, 3H), 1.24 (m, 2H), 0.46 (m, 3H), 0.22 (m, 1H); ¹³C NMR (75 MHz, CD₃Cl₃) δ 163.7, 140.9, 137.6, 136.8, 135.0, 134.6, 130.4, 127.4, 126.0, 121.8, 113.4, 61.0, 21.2, 17.6, 3.47, 2.92; MS (ES) *m*/*z:* 441.1 (M+H)⁺.

### Example 104

### 5-(4-chlorophenylsulfonyl)-4-methyl-4,5-dihydro-1H-pyrazolo[4,3-c]quinoline-7,8-diol.

MS m/z: 391.9 (M+H)⁺. ¹H NMR (CD₃CN) δ 7.28 (s, 1H), 7.18 (s, 1H), 7.13 (s 4H), 7.00 (s, 1H), 5.54 (q, *J* = 6.8Hz, 1H), 1.24 (d, *J* = 6.8 Hz, 1H)

### Example 105

### 5-(4-chlorophenylsulfonyl)-4-methyl-4,5-dihydro-2H-pyrazolo[4,3-c]quinolin-8-ol.

Prepared from 8-(benzyloxy)-5-(4-chlorophenylsulfonyl)-4-methyl-4,5-dihydro-1H-pyrazolo[4,3-c]quinoline as described for compound **119** in Example 21. MS m/z: 375.9 (M+H)⁺. ¹H NMR (CD₃OD) δ 7.55 (d, *J*= 8.7 Hz, 1H), 7.45 (s, 1H), 7.11 (m, 4H), 6.84 (dd, *J* = 8.7, 2.8 Hz, 1H), 5.60 (q, *J* = 6.9 Hz, 1H), 1.25 (d, *J* = 7.0 Hz, 3H).

### Example 106

**4-(8-fluoro-4-methyl-1*H*-pyrazolo[4,3-c]quinolin-5(4*H*)-ylsulfonyl)-3,5-dimethylisoxazole.** Prepared as described in Example 29 using 3,5-dimethylisoxazole-4-sulfonyl chloride. ¹H NMR (CDCl₃) δ 11-09 (s, 1H) 7.79-7.74 (dd, J = 8.9, 5.1 Hz, 1H), 7.53-7.49 (dd, J = 8.5, 3.0 Hz, 1H), 7.40 (s, 1H), 7.16-7.09 (dt, J = 8.4, 3.0 Hz, 1H), 5.64-5.57 (q, J = 6.9 Hz, 1H), 2.03 (s, 3H), 1.97 (s, 3H), 1.35-1.33 (d, J = 6.9 Hz, 3H).

### Example 107

**8-fluoro-4-methyl-5-(1-methyl-1*H*-pyrazol-4-ylsulfonyl)-4,5-dihydro-1*H*-pyrazolo[4,3-*c*]quinoline.** Prepared as described in Example 29 using 1-methyl-1H-pyrazole-4-sulfonyl chloride. ¹H NMR (CDCl₃) δ 7.79-7.74 (dd, J = 8.9, 5.1 Hz, 1H), 7.45-7.41 (dd, J = 8.4, 2.7 Hz, 1H), 7.36 (s, 1H), 7.15-7.07 (m, 2H), 6.98 (m, 1H), 5.70-5.63 (q, J = 6.9 Hz, 1H), 3.67 (s, 3H), 1.33-1.31 (d, J = 6.9 Hz, 3H).

### Example 108

**8-fluoro-4-methyl-5-(5-(pyridin-2-yl)thiophen-2-ylsulfonyl)-4,5-dihydro-1*H-*pyrazolo[4,3-*c*]quinoline.** Prepared as described in Example 29 using 5-pyrid-2-ylthiophene-2-sulfonyl chloride. ¹H NMR (CDCl₃) δ 10.25 (s, 1H) 8.51-8.49 (d, J = 4.0 Hz, 1H), 7.83-7.79 (dd, J = 8.8, 5.1 Hz, 1H), 7.69-7.65 (m, 1H), 7.49-7.41 (m, 2H), 7.28-7.11 (m, 5H), 6.82-6.80 (d, J = 3.8 Hz, 1H), 5.73-5.68 (q, J = 6.8 Hz, 1H), 1.36-1.34 (d, J = 6.8 Hz, 3H).

### Example 109

**8-fluoro-4-methyl-5-(1-methyl-1*H*-imidazol-4-ylsulfonyl)-4,5-dihydro-1*H*-pyrazolo[4,3-*c*]quinoline.** MS m/z: 348.1 (M+H)⁺. ¹H NMR (DMSO-d6) δ 12.83 (s, 1H), 7.67-7.63 (dd, J = 9.0, 5.4 Hz, 1H), 7.54-7.48 (m, 2H), 7.40-7.36 (dd, J = 9.0, 3.0 Hz, 1H), 7.17-7.10 (td, J = 8.7, 3.0 Hz, 1H), 5.58-5.51 (q, J = 7.2 Hz, 1H), 1.14-1.12 (d, J = 7.2 Hz, 3H).

### Example 110

**(*R*)-4-ethyl-7,8-difluoro-5-(4-(trifluoromethyl)phenylsulfonyl)-4,5-dihydro-1*H-*pyrazolo[4,3-*c*]quinoline.** Prepared as described in Example 13 using 2-bromo-4,5-difluorophenylboronic acid, ethylmagnesium bromide, and sulfonamide *72.* MS m/z: 444.1 (M+H)⁺. ¹H NMR (CDCl₃) δ 7.70 (dd, 1H, J=7.5, 11.1 Hz), 7.47 (t, J = 8.4 Hz, 1H), 7.40-7.34 (m, 4H), 7.19 (s, 1H), 5.25 (t, J = 8.4 Hz, 1H), 1.64-1.54(m, 1H), 1.47-1.38 (m, 1H), 0.97(t, J = 7.2Hz, 3H)

### Example 111

**(R)-4-cyclopropyl-8-fluoro-5-(4-(trifluoromethoxy)phenylsulfonyl)-4,5-dihydro-1H-pyrazolo[4,3-c]quinoline.** Prepared as described in Example 13 using 4-(trifluoromethoxy)benzenesulfonyl chloride. MS m/z: 454.0. ¹H NMR (CDCl₃) δ 7.69 (dd, *J* = 9.5 Hz, 2.2 Hz, 1H), 7.66 (dd, *J* = 8.4, 5.9 Hz, 1H), 7.27 (s, 1H), 7.20 (d, *J* = 8.7, 2H), 7.16-7.10 (m, 1H) 6.94 (d, *J* = 8.4 Hz, 2H), 4.95 (d, *J* = 8.1 Hz, 1H), 1.07-1.0 (m, 1H), 0.58-0.42 (m, 3H), 0.15-0.12 (m, 1H).

### Example 112

**(R)-4-cyclopropyl-8-fluoro-5-(4-methoxyphenylsulfonyl)-4,5-dihydro-1H-pyrazolo[4,3-c]quinoline.** Prepared as described in Example 13 using 4-methoxybenzenesulfonyl chloride. MS m/z: 400.1 (M+H)⁺. ¹H NMR (CDCl₃) δ 7.69 (dd, *J* = 4.0, 2.6 Hz, 1H), 7.60 (dd, *J* = 8.4, 5.9 Hz, 1H), 7.30 (s, 1H), 7.15-7.12 (m, 2H), 7.12-7.04 (m, 1H), 6.60-6.55 (m, 2H), 5.0 (d, *J* = 7.6 Hz, 1H), 3.71 (s, 3H), 1.20-0.92 (m, 1H), 0.60-0.35 (m, 3H), 0.19-0.14 (m, 1H).

Chiral chromatography was performed with an isocratic 10:90 ethanol:hexanes eluent at 1 mL/min on a CHIRALCEL #ODH0CE-EF074.

### BIOLOGICAL EXAMPLES

### Gamma-Secretase Assay

The gamma-secretase APP enzyme assay was designed to measure the specific proteolytic cleavage of an APP substrate (MBP-C125 Swe fusion protein) at the Aβ40 site. The assay used a partially purified extract of IMR-32 cell membranes as the gamma-secretase enzyme preparation and a recombinant fusion protein containing the C-terminal 125 amino acids of the Swedish variant of the APP (MBP-C125swe) as the substrate. This assay involved two steps beginning with the enzymatic reaction generating a cleavage product that was captured with an immobilized antibody specific for the neo-epitope Aβ40 site. The captured cleavage product was then detected in a sandwich ELISA assay with a biotinylated reporter antibody that is specific to Aβ (17-28). Streptavidin-linked alkaline phosphatase was then added that would generate a fluorescent signal proportional to the amount of cleavage product. This assay was used to discover small molecule inhibitors of gamma-secretase.

### Materials and Methods:

Briefly, a 149 mg/ml solution of BIGCHAP detergent was made with water at 42°C and then rotated for 30 minutes at the same temperature. This warmed solution of BigCHAPS (N,N-Bis(3-D-gluconamidopropyl)cholamide ) detergent was used to dissolve Brain Extract Type-V (lipid containing a minimum of 40% phosphatidylethanolamine) from Sigma (St. Louis, Mo.) to a concentration of 8 mg/ml. This solution containing BigCHAPS and lipid at 8mg/ml is then diluted to 0.53 mg/ml lipid with a pre-warmed solution of Hepes and sodium chloride. This final solution containing Hepes buffer, sodium chloride, BigCHAPS detergent and lipid is used to create working solutions of both gamma-secretase (25 Units) and the MBP-C125 substrate (0.05 mg/ml).

Gamma-secretase was then added to a 96-well micro-titre plate and then incubated with various concentrations of inhibitor for 30 minutes at 37° C. MBPC125 substrate was then added to initiate the reaction that would run for two hours at 37° C. The reaction was quenched with the addition of SDS to a final concentration of 0.1% and then 100µl of the reaction mixture was transferred to a capture ELISA plate and incubated overnight at 4° C. Detection of the cleavage product was performed using a standard sandwich ELISA assay and quantified using a six point standard curve.

### Results

The following compounds prepared essentially according to the above methods and schemes, when tested as described above exhibited inhibition in the gamma secretase assay with an IC₅₀ as described in the table below, where "A" refers to an IC₅₀ of 0.1 to 100 nm; "B" refers to an IC₅₀ of 101 to 500 nm, "C" refers to an IC₅₀ value of 501 to 1000 nm, and "D" refers to an IC₅₀ value of 1000 to 10,000.

**TABLE A**

| Name | IC₅₀ |
|---|---|
| 5-[(4-chlorophenyl)sulfonyl]-4,5-dihydro-1*H*-pyrazolo[4,3-*c*]quinoline | A |
| 5-[(4-chlorophenyl)sulfonyl]-8-fluoro-4,5-dihydro-2*H*-pyrazolo[4,3-*c*]quinoline | A |
| 5-[(4-chlorophenyl)sulfonyl]-7-fluoro-4,5-dihydro-2*H*-pyrazolo[4,3-*c*]quinoline | A |
| 5-[(4-chlorophenyl)sulfonyl]-9-fluoro-4,5-dihydro-2*H*-pyrazolo[4,3-*c*]quinoline | A |
| 5-[(4-chlorophenyl)sulfonyl]-4-methyl-4,5-dihydro-2*H*-pyrazolo[4,3-*c*]quinoline (racemic) | A |
| 5-[(4-chlorophenyl)sulfonyl]-7-methoxy-4-methyl-4,5-dihydro-1*H*-pyrazolo[4,3-*c*]quinoline | A |
| 5-[(4-chlorophenyl)sulfonyl]-9-methoxy-4-methyl-4,5-dihydro-1*H*-pyrazolo[4,3-*c*]quinoline | D |
| 5-[(4-chlorophenyl)sulfonyl]-3-methyl-4,5-dihydro-1*H*-pyrazolo[4,3-*c*]quinoline | D |
| 8-chloro-5-[(4-chlorophenyl)sulfonyl]-4,5-dihydro-2*H*-pyrazolo[4,3-*c*]quinoline | c |
| 4-methyl-5-(pyridin-2-ylsulfonyl)-4,5-dihydro-2*H*-pyrazolo[4,3-*c*]quinoline | B |
| 5-[(4-chlorophenyl)sulfonyl]-4,5-dihydro-1*H*-pyrazolo[4,3-*c*]quinolin-3-ol | D |
| 6-(4-chlorophenylsulfonyl)-5-ethyl-5,6-dihydropyrazolo[1,5-*c*]quinazoline | B |
| 5-[(4-chlorophenyl)sulfonyl]-4-methyl-4,5-dihydro-2*H*-pyrazolo[4,3-*c*]quinoline; (resolved enantiomer, with a retention time of about 9.3 min*.) | A |
| 5-[(4-chlorophenyl)sulfonyl]-4-methyl-4,5-dihydro-2*H*-pyrazolo[4,3-*c*]quinoline (resolved enantiomer, with a retention time of about 20.5 min*.) | D |
| 5-[(4-chlorophenyl)sulfonyl]-8-fluoro-4-methyl-4,5-dihydro-2*H*-pyrazolo[4,3-*c*]quinoline (racemic) | A |
| 5-[(4-chlorophenyl)sulfonyl]-8-fluoro-4-methyl-4,5-dihydro-2*H*-pyrazolo[4,3-*c*]quinoline (resolved enantiomer, with a retention time of about 9.8 min*.) | A |
| 8-fluoro-4-methyl-5-(pyridin-3-ylsulfonyl)-4,5-dihydro-1*H*-pyrazolo[4,3-*c*]quinoline (racemic) | B |
| 8-fluoro-4-methyl-5-(pyridin-2-ylsulfonyl)-4,5-dihydro-1*H*-pyrazolo[4,3-*c*]quinoline | B |
| 4-methyl-5-(pyridin-3-ylsulfonyl)-4,5-dihydro-1*H*-pyrazolo[4,3-*c*]quinoline | B |
| 8-fluoro-4-methyl-5-(pyridin-3-ylsulfonyl)-4,5-dihydro-1*H*-pyrazolo[4,3-*c*]quinoline (resolved enantiomer, with a retention time of about 15.2 min*.) | A |
| 8-fluoro-4-methyl-5-(pyridin-3-ylsulfonyl)-4,5-dihydro-1*H*-pyrazolo[4,3-*c*]quinoline (resolved enantiomer, with a retention time of about 17.2 min*.) | D |
| 5-[(4-chlorophenyl)sulfonyl]-1,5-dihydro-4*H*-pyrazolo[4,3-*c*]quinolin-4-one | B |
| 5-[(4-chlorophenyl)sulfonyl]-4-methyl-4,5-dihydro-1*H*-pyrazolo[4,3-*c*]-1,5-naphthyridine | A |
| 8-fluoro-4-methyl-5-(pyridin-2-ylsulfonyl)-4,5-dihydro-1*H*-pyrazolo[4,3-*c*]quinoline | A |
| 5-(4-chlorophenylsulfonyl)-4-ethyl-4,5-dihydro-1*H*-pyrazolo[4,3-*c*]quinoline | A |
| 5-(4-chlorophenylsulfonyl)-4-methyl-4,5-dihydro-1*H*-pyrazolo[4,3-c]quinolin-7-ol | A |
| 5-(4-chlorophenylsulfonyl)-8-fluoro-1*H*-pyrazolo[4,3-c]quinolin-4(5H)-one | D |
| 5-(4-chlorophenylsulfonyl)-4-methyl-4,5,5a,6,7,8,9,9a-octahydro-1*H*-pyrazolo[4,3-c]quinoline | |
| Racemate | A |
| Enantiomer A | B |
| Enantiomer B | A |
| 5-(4-chlorophenylsulfonyl)-9-fluoro-4-methyl-4,5-dihydro-2*H*-pyrazolo[4,3-c]quinoline | A |
| 5-(4-chlorophenylsulfonyl)-7-fluoro-4-methyl-4,5-dihydro-2*H*-pyrazolo[4,3-c]quinoline | A |
| 8-fluoro-5-(4-fluorophenylsulfonyl)-4-methyl-4,5-dihydro-1*H*-pyrazolo[4,3-c]quinoline | A |
| 4-methyl-5-(pyridin-2-ylsulfonyl)-4,5-dihydro-1*H*-pyrazolo[4,3-c]quinoline | |
| Enantiomer A (retention time of about 9.3 min*.) | B |
| Enantiomer B | D |
| 9-fluoro-6-(4-fluorophenylsulfonyl)-5-methyl-5,6-dihydropyrimido[5,4-c]quinolin-2-amine | |
| Enantiomer A | D |
| Enantiomer B | B |
| 5-(4-chlorophenylsulfonyl)-4-cyclopropyl-4,5-dihydro-2*H*-pyrazolo[4,3-c]quinoline | A |
| 5-(4-chlorophenylsulfonyl)-4-(trifluoromethyl)-4,5-dihydro-2*H*-pyrazolo[4,3-c]quinoline | A |
| 5-(4-chlorophenylsulfonyl)-4-methyl-4,5-dihydro-1*H*-pyrazolo[4,3-c]quinolin-7-ol | |
| Enantiomer A (retention time of about 13.4 min*.) | A |
| Enantiomer B (retention time of about 17.0 min*.) | A |
| 5-(4-chlorophenylsulfonyl)-4-isopropyl-4,5-dihydro-2*H*-pyrazolo[4,3-c]quinoline | A |
| 1,5-bis(4-chlorophenylsulfonyl)-1*H*-pyrazolo[4,3-c]quinolin-4(5H)-one | D |
| 5-(4-chlorophenylsulfonyl)-4,5-dihydro-1*H*-imidazo[1,2-a]pyrazolo[4,3-e]pyrimidine | B |
| 8-fluoro-5-(4-fluorophenylsulfonyl)-4-methyl-4,5-dihydro-1*H*-pyrazolo[4,3-c]quinoline | A |
| 5'-(4-chlorophenylsulfonyl)-2',5'-dihydrospiro[cyclopropane-1,4'-pyrazolo[4,3-c]quinoline] | A |
| 5-(4-chlorophenylsulfonyl)-7,8-difluoro-4-methyl-4,5-dihydro-2*H*-pyrazolo[4,3-c]quinoline | |
| Enantiomer A | A |
| Enantiomer B | A |
| 7,8-difluoro-4-methyl-5-(thiophen-2-ylsulfonyl)-4,5-dihydro-2*H*-pyrazolo[4,3-c]quinoline | A |
| 5-(4-chlorophenylsulfonyl)-4-methyl-4,5-dihydro-2*H*-pyrazolo[4,3-c][1,8]naphthyridine | A |
| 5-(4-chlorophenylsulfonyl)-4-ethyl-4,5-dihydro-1*H*-pyrazolo[4,3-c]quinoline | A |
| 5-(5-chlorothiophen-2-ylsulfonyl)-7,8-difluoro-4-methyl-4,5-dihydro-1*H*-pyrazolo[4,3-c]quinoline | A |
| 5-(4-chlorophenylsulfonyl)-4-methyl-4,5-dihydro-1*H*-pyrazolo[4,3-c]quinolin-7-yl dimethylcarbamate | A |
| 4-cyclopropyl-7,8-difluoro-5-(4-(trifluoromethyl)phenylsulfonyl)-4,5-dihydro-1*H*-pyrazolo[4,3-c]quinoline | |
| Racemate | A |
| Enantiomer A (retention time of about 6.0 min*.) | A |
| Enantiomer B (retention time of about 9.3 min*.) | A |
| 7-chloro-4-methyl-5-(4-(trifluoromethyl)phenylsulfonyl)-4,5-dihydro-1*H*-pyrazolo[3,4-d]thieno[2,3-b]pyridine | A |
| 5-(4-chlorophenylsulfonyl)-4-cyclopropyl-4,5-dihydro-1*H*-imidazo[1,2-a]pyrazolo[4,3-e]pyrimidine | B |
| 4-ethyl-7,8-difluoro-5-(5-(trifluoromethyl)pyridin-2-ylsulfonyl)-4,5-dihydro-1*H*-pyrazolo[4,3-c]quinoline | A |
| 7,8-difluoro-4-methyl-5-(4-(trifluoromethyl)phenylsulfonyl)-4,5-dihydro-1*H*-pyrazolo[4,3-c]quinoline | |
| Enantiomer A (retention time of about 7.0 min*.) | A |
| Enantiomer B (retention time of about 9.5 min*.) | B |
| 5-(4-chlorophenylsulfonyl)-4-methyl-4,5-dihydro-2*H*-pyrazolo[4,3-c] [1,7]naphthyridine | A |
| 5'-(4-(trifluoromethyl)phenylsulfonyl)-1',5'-dihydrospiro[cyclopropane-1,4'-pyrazolo[4,3-c]quinoline] | A |
| 4-cyclopropyl-7,8-difluoro-5-(5-(trifluoromethyl)pyridin-2-ylsulfonyl)-4,5-dihydro-1*H*-pyrazolo[4,3-c]quinoline | A |
| 5-(5-chlorothiophen-2-ylsulfonyl)-4-cyclopropyl-7,8-difluoro-4,5-dihydro-2*H*-pyrazolo[4,3-c]quinoline | A |
| 4-cyclopropyl-7,8-difluoro-5-(4-fluorophenylsulfonyl)-4,5-dihydro-2*H*-pyrazolo[4,3-c]quinoline | A |
| 4-cyclopropyl-5-(4-(trifluoromethyl)phenylsulfonyl)-4,5-dihydro-2*H*-pyrazolo[4,3-c][1,8]naphthyridine | A |
| 4-cyclopropyl-5-(4-fluorophenylsulfonyl)-4,5-dihydro-2*H*-pyrazolo[4,3-c][1,8]naphthyridine | A |
| 4-cyclopropyl-5-(4-fluorophenylsulfonyl)-4,5-dihydro-2*H*-pyrazolo[4,3-c]quinoline | A |
| 4-cyclopropyl-7,8-difluoro-5-(thiophen-2-ylsulfonyl)-4,5-dihydro-2*H*-pyrazolo[4,3-c]quinoline | A |
| 4-cyclopropyl-5-(4-(trifluoromethyl)phenylsulfonyl)-4,5-dihydro-1*H*-imidazo[1,2-a]pyrazolo[4,3-e]pyrimidine | C |
| 4-cyclopropyl-7,8-difluoro-5-(6-(trifluoromethyl)pyridin-3-ylsulfonyl)-4,5-dihydro-1*H*-pyrazolo[4,3-c]quinoline | A |
| 5-(4-fluorophenylsulfonyl)-4-(trifluoromethyl)-4,5-dihydro-2*H*-pyrazolo[4,3-c][1,8]naphthyridine | A |
| 7,8-difluoro-4-(pyridin-3-yl)-5-(4-(trifluoromethyl)phenylsulfonyl)-4,5-dihydro-1*H*-pyrazolo[4,3-c]quinoline | A |
| 4-cyclopropyl-8-fluoro-5-(4-(trifluoromethyl)phenylsulfonyl)-4,5-dihydro-1*H*-pyrazolo[4,3-c]quinoline | |
| Racemate | A |
| Enantiomer A (retention time of about 8.0 min*.) | A |
| Enantiomer B (retention time of about 12.6 min*.) | B |
| 5-(4-chlorophenylsulfonyl)-4-methyl-4,5-dihydro-2*H*-[1,3]dioxolo[4,5-g]pyrazolo[4,3-c]quinoline | A |
| 5-(5-chloropyridin-2-ylsulfonyl)-4-cyclopropyl-7,8-difluoro-4,5-dihydro-1*H*-pyrazolo[4,3-c]quinoline | A |
| 5-(5-chlorothiophen-2-ylsulfonyl)-4-cyclopropyl-7,8-difluoro-4,5-dihydro-2*H*-pyrazolo[4,3-c]quinoline | |
| Enantiomer A (retention time of about 10.3 min*.) | A |
| Enantiomer B (retention time of about 16.1 min*.) | A |
| 4-cyclopropyl-7,8-difluoro-5-(4-fluorophenylsulfonyl)-4,5-dihydro-2*H*-pyrazolo[4,3-c]quinoline | |
| Enantiomer A (retention time of about 16.9 min*.) | A |
| Enantiomer B (retention time of about 19.7 min*.) | A |
| 4-cyclopropyl-8-fluoro-5-(6-(trifluoromethyl)pyridin-3-ylsulfonyl)-4,5-dihydro-2*H*-pyrazolo[4,3-c]quinoline | |
| Enantiomer A | A |
| Enantiomer B | A |
| 4-cyclopropyl-7,8-difluoro-5-(6-(trifluoromethyl)pyridin-3-ylsulfonyl)-4,5-dihydro-1*H*-pyrazolo[4,3-c]quinoline | |
| Enantiomer A (retention time of about 17.3 min*.) | A |
| Enantiomer B (retention time of about 13.0 min*.) | A |
| 7,8-difluoro-4-isopropyl-5-(4-(trifluoromethyl)phenylsulfonyl)-4,5-dihydro-2*H*-pyrazolo[4,3-c]quinoline | A |
| 4-cyclopropyl-7,8-difluoro-5-(4-(trifluoromethoxy)phenylsulfonyl)-4,5-dihydro-1*H*-pyrazolo[4,3-c]quinoline | A |
| 7,8-difluoro-5-(4-(trifluoromethyl) phenylsulfonyl)-4,5-dihydro-2*H*-pyrazolo[4,3-c]quinoline | A |
| 5-(4-chlorophenylsulfonyl)-4-methyl-4,5-dihydro-1*H*-pyrazolo[4,3-c]quinoline-7,8-diol | A |
| 5-(5-chloropyridin-2-ylsulfonyl)-4-cyclopropyl-8-fluoro-4,5-dihydro-1*H*-pyrazolo[4,3-c]quinoline | A |
| 4-cyclopropyl-5-(6-(trifluoromethyl)pyridin-3-ylsulfonyl)-4,5-dihydro-1*H*-pyrazolo[4,3-c]quinoline | A |
| (R)-4-cyclopropyl-7-(trifluoromethoxy)-5-(6-(trifluoromethyl)pyridin-3-ylsulfonyl)-4,5-dihydro-2*H*-pyrazolo[4,3-c]quinoline | A |
| 4-cyclopropyl-5-(4-(trifluoromethylphenyl sulfonyl)-4,5-dihydro-2*H*-pyrazolo[4,3-c][1,7]naphthyridine 7-oxide | A |
| 5-(4-chlorophenylsulfonyl)-4-methyl-4,5-dihydro-2*H*-pyrazolo[4,3-c]quinolin-8-ol | A |
| 4-(8-fluoro-4-methyl-1*H*-pyrazolo[4,3-c]quinolin-5(4*H*)-ylsulfonyl)-3,5-dimethylisoxazole | c |
| 8-fluoro-4-methyl-5-(1-methyl-1*H*-pyrazol-4-ylsulfonyl)-4,5-dihydro-1*H*-pyrazolo[4,3-c]quinoline | B |
| 8-fluoro-4-methyl-5-(5-(pyridin-2-yl)thiophen-2-ylsulfonyl)-4,5-dihydro-1*H*-pyrazolo[4,3-c]quinoline | D |
| (*R*)-4-cyclopropyl-7-(trifluoromethyl)-5-(6-(trifluoromethyl)pyridin-3-ylsulfonyl)-4,5-dihydro-1*H*-pyrazolo[4,3-c]quinoline; | A |
| (*R*)-4-cyclopropyl-7-(trifluoromethoxy)-5-(4-(trifluoromethoxy)phenylsulfonyl)-4,5-dihydro-2*H*-pyrazolo[4,3-c]quinoline | A |
| (*R*)-4-(4-cyclopropyl-7,8-difluoro-1*H*-pyrazolo[4,3-c]quinolin-5(4*H*)-ylsulfonyl)aniline | A |
| (*R*)-4-cyclopropyl-7,8-difluoro-5-(4-nitrophenylsulfonyl)-4,5-dihydro-1*H*-pyrazolo[4,3-c]quinoline | A |
| (*R*)-4-cyclopropyl-8-(trifluoromethyl)-5-(6-(trifluoromethyl)pyridin-3-ylsulfonyl)-4,5-dihydro-1*H*-pyrazolo[4,3-c]quinoline | A |
| 5-chloro-2-(4-cyclopropyl-7,8-difluoro-1*H*-pyrazolo[4,3-c]quinolin-5(4*H*)-ylsulfonyl)thiazole | A |
| (*R*)-4-cyclopropyl-5-(4-(difluoromethoxy) phenylsulfonyl)-7,8-difluoro-4,5-dihydro-1*H*-pyrazolo[4,3-c]quinoline | A |
| 4-cyclopropyl-5-(4-(trifluoromethyl) phenylsulfonyl)-4,5-dihydro-2*H*-pyrazolo[4,3-c][1,5]naphthyridine | A |
| 4-cyclopropyl-8-fluoro-5-(5-(trifluoromethyl) pyridin-2-ylsulfonyl)-4,5-dihydro-1*H*-pyrazolo[4,3-c]quinoline | |
| Enantiomer A (retention time of about 13.7 min*.) | B |
| Enantiomer B (retention time of about 7.6 min*.) | A |
| 4-cyclopropyl-5-(4-(difluoromethoxy) phenylsulfonyl)-8-fluoro-4,5-dihydro-1*H*-pyrazolo[4,3-c]quinoline | A |
| 4-cyclopropyl-7,8-difluoro-5-(5-(trifluoromethyl)pyridin-2-ylsulfonyl)-4,5-dihydro-1*H*-pyrazolo[4,3-c]quinoline | A |
| 5-(4-chlorophenylsulfonyl)-4-methyl-4,5-dihydro-1*H*-pyrazolo[4,3-c]quinoline-7,8-diol | |
| Enantiomer A | A |
| Enantiomer B | B |
| 8-fluoro-4-methyl-5-(1-methyl-1*H*-imidazol-4-ylsulfonyl)-4,5-dihydro-1*H*-pyrazolo[4,3-c]quinoline | D |
| (*R*)-4-ethyl-7,8-difluoro-5-(4-(trifluoromethyl)phenylsulfonyl)-4,5-dihydro-1*H*-pyrazolo[4,3-c]quinoline | A |
| (R)-4-cyclopropyl-8-fluoro-5-(4-(trifluoromethoxy)phenylsulfonyl)-4,5-dihydro-1*H*-pyrazolo[4,3-c]quinoline | A |
| 4-cyclopropyl-7-methyl-5-(4-(trifluoromethyl)phenylsulfonyl)-4,5-dihydro-1*H*-pyrazolo[3,4-d]thiazolo[5,4-b]pyridine | A |
| (R)-4-cyclopropyl-8-fluoro-5-(4-methoxyphenylsulfonyl)-4,5-dihydro-1*H*-pyrazolo[4,3-c]quinoline | A |

### Notch signaling assay for selective inhibitors of gamma secretase.

A convergence of evidence indicates that the gamma secretase complex, comprised of the presenilin subunits, mediates the intra-membrane cleavage of Amyloid precursor protein (APP), and the Notch family of proteins (De Strooper, B., P. Saftig, K. Craessaerts, H. Vanderstichele, G. Guhde, W. Annaert, K. Von Figura and F. Van Leuven (1998). "Deficiency of presenilin-1 inhibits the normal cleavage of amyloid precursor protein." Nature 391 (6665): 387-90; De Strooper, B., W. Annaert, P. Cupers, P. Saftig, K. Craessaerts, J. S. Mumm, E. H. Schroeter, V. Schrijvers, M. S. Wolfe, W. J. Ray et al. (1999). "A presenilin-1-dependent gamma-secretase-like protease mediates release of Notch intracellular domain." Nature 398 (6727): 518-22; Mumm, J. S., E. H. Schroeter, M. T. Saxena, A. Griesemer, X. Tian, D. J. Pan, W. J. Ray and R. Kopan (2000). "A ligand-induced extracellular cleavage regulates gamma-secretase-like proteolytic activation of Notch1." Mol Cell 5(2): 197-206; Zhang, Z., P. Nadeau, W. Song, D. Donoviel, M. Yuan, A. Bernstein and B. A. Yankner (2000). "Presenilins are required for gamma-secretase cleavage of beta-APP and transmembrane cleavage of Notch-1." Nat Cell Biol 2(7): 463-5). Cleavage of APP by gamma secretase leads to beta-amyloid synthesis. Cleavage of Notch1 by gamma secretase results in release of the Notch intracellular domain (NICD), which translocates to the nucleus and activates gene expression (Jarriault, S., C. Brou, F. Logeat, E. H. Schroeter, R. Kopan and A. Israel (1995). "Signalling downstream of activated mammalian Notch." Nature 377(6547): 355-8; Kopan, R., E. H. Schroeter, H. Weintraub and J. S. Nye (1996). "Signal transduction by activated Notch: importance of proteolytic processing and its regulation by the extracellular domain." Proc Natl Acad Sci USA 93(4): 1683-8; Schroeter, E. H., J. A. Kisslinger and R. Kopan (1998). "Notch-1 signalling requires ligand-induced proteolytic release of intracellular domain." Nature 393(6683): 382-6). In particular, Notch signaling activates transcription of the mammalian homolog of the *Drosophila* transcription factor *hairy-enhancer of split* (*Hes*). Transcriptional activation of *Hes1* is mediated by de-repression of CBF1/RBPJk upon binding by NICD in the nucleus. These facts have been exploited to develop a reporter gene assay for Notch Signaling Hsieh, J. J., T. Henkel, P. Salmon, E. Robey, M. G. Peterson and S. D. Hayward (1996). "Truncated mammalian Notch1 activates CBF1/RBPJk-repressed genes by a mechanism resembling that of Epstein-Barr virus EBNA2." Mol Cell Biol 16(3): 952-9; Lu, F. M. and S. E. Lux (1996). "Constitutively active human Notch1 binds to the transcription factor CBF1 and stimulates transcription through a promoter containing a CBF1-responsive element." Proc Natl Acad Sci USA 93(11): 5663-7).

Gamma secretase inhibitors have been observed to block NICD formation, and inhibit Notch signaling (De Strooper, B., W. Annaert, P. Cupers, P. Saftig, K. Craessaerts, J. S. Mumm, E. H. Schroeter, V. Schrijvers, M. S. Wolfe, W. J. Ray et al. (1999). "A presenilin-1-dependent gamma-secretase-like protease mediates release of Notch intracellular domain." Nature 398(6727): 518-22). Due to the importance of Notch signaling in cell fate determination, and tissue differentiation during both development and in the adult, inhibition of Notch signaling by gamma secretase inhibitors is postulated to be a limiting factor in their therapeutic utility. In order to identify selective gamma secretase inhibitors, we have employed a reporter gene based Notch signaling assay using a constitutively active rat Notch1 construct (ZEDN1) provided by Dr Gerry Weinmaster, who is at the University of California at Los Angeles (UCLA) as described in Shawber, C., D. Nofziger, J. J. Hsieh, C. Lindsell, O. Bogler, D. Hayward and G. Weinmaster (1996). "Notch signaling inhibits muscle cell differentiation through a CBF1-independent pathway." Development 122(12): 3765-73 in combination with the CBF1 repressible Luciferase reporter gene 4xwtCBF1Luc (Hsieh, J. J., T. Henkel, P. Salmon, E. Robey, M. G. Peterson and S. D. Hayward (1996). "Truncated mammalian Notch1 activates CBF1/RBPJk-repressed genes by a mechanism resembling that of Epstein-Barr virus EBNA2." Mol Cell Biol 16(3): 952-9).

When 4xwtCBF1 Luciferase is co-transfected with NotchδE (ZEDN1), gamma-secretase cleavage of NotchδE releases the Notch intracellular domain (NICD), which translocates to the nucleus and de-represses CBF1mediated transcriptional repression, leading to transcription of the Luciferase reporter gene. Luciferase activity is easily assayed in cell extracts using commercially available kits. The activity of the reporter gene is directly correlated with gamma secretase cleavage of NotchδE, and as such, a reduction in Luciferase activity provides a convenient measure of inhibition of gamma secretase cleavage of NotchδE. A comparison of the IC₅₀ values of compounds for inhibition of Notch signaling versus inhibition of beta-amyloid production in 293sw cells is employed to guide in the selection of compounds that have the desired property of potent inhibition of beta-amyloid synthesis with minimal inhibition of Notch Signaling.

The invention and the manner and process of making and using it, are now described in such full, clear, concise and exact terms as to enable any person skilled in the art to which it pertains, to make and use the same. It is to be understood that the foregoing describes preferred embodiments of the invention and that modifications may be made therein without departing from the spirit or scope of the invention as set forth in the claims. To particularly point out and distinctly claim the subject matter regarded as invention, the following claims conclude this specification.

## Claims

1. A compound of the formula: 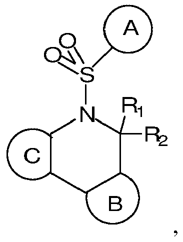
stereoisomers, tautomers, mixtures of stereoisomers and/or tautomers or pharmaceutically acceptable salts thereof, wherein
the C-ring is cycloalkyl, aryl, heterocycloalkyl, or heteroaryl, each of which is optionally substituted with 1, 2, 3, or 4 groups that are independently halogen, C₁-C₆ alkyl, C₁-C₆ alkoxy, aryloxy, aryl-(C₁-C₆alkoxy), C₁-C₆ haloalkyl, C₁-C₆ haloalkoxy, hydroxyl, hydroxy-(C₁-C₆ alkyl), -NR'R", -(C₁-C₆ alkyl)-NR'R", -NR'C(O)OR', -COR', -C(O)OR', -C(O)NR'R", -OC(O)NR'R", -NR'C(O)NR'R"; -(C₁-C₆ alkyl)-C(O)OR', -(C₁-C₆ alkyl)-C(O)NR'R", -NR'C(O)R", NO₂, CN, oxo, aryl-(C₁-C₆alkyl), aryl, heteroaryl, C₂-C₆ alkenyl, C₂-C₆ alkynyl, -NR'SO₂R", -SO₂-NR'R", -S(O)_{Z} aryl or -S(O)_{Z} (C₁-C₆ alkyl), where the alkyl, alkenyl and alkynyl portions of the above are unsubstituted or substituted with 1, 2 or 3 groups that are independently halogen, hydroxyl, -NR'R" or C₁-C₆ alkoxy, and where the aryl or heteroaryl portions of the above are optionally substituted with 1, 2, 3, or 4 groups that are independently C₁-C₆ alkyl, C₁-C₆ alkoxy, halogen, C₁-C₆ haloalkyl, C₁-C₆ haloalkoxy, hydroxyl, hydroxy-(C₁-C₆ alkyl), or -NR'R";
or where two adjacent substituents form -O-(CH₂)₁₋₃-O-;
the A-ring is aryl, cycloalkyl, heteroaryl or heterocycloalkyl, where each ring is optionally substituted at a substitutable position with halogen, C₁-C₆ alkyl, C₂-C₆ alkenyl, C₂-C₆ alkynyl, C₁-C₆ alkoxy, C₁-C₆ haloalkyl, C₁-C₆ haloalkoxy, hydroxyl, hydroxy-(C₁-C₆ alkyl), CN, N0₂, aryloxy, aryl-(C₁-C₆ alkoxy), -SO₂-(C₁-C₆ alkyl), -NR'R", C₁-C₆ alkanoyl, -C(O)OR', -(C₁-C₆ alkyl)-C(O)OR', heteroaryl, aryl, aryl-(C₁-C₆ alkyl), -SO₂-NR'R", -C(O)NR'R", -OC(O)NR'R", -NR'C(O)R", -NR'C(O)NR'R", or -NR'C(O)OR';
the B-ring is heteroaryl or heterocycloalkyl ring, each of which is optionally substituted at a substitutable position with a group that is independently C₁-C₆ alkyl, C₁-C₆ alkoxy, -NR'R", -SO₂-NR'R, -C(O)NR'R", -NR'SO₂R", -NR'SO₂N'R", -NR'SO₂-(C₁-C₆ alkyl), -NR'SO₂-phenyl, -NR'C(O)R", -NR'C(O)NR'R", -NR'C(O)OR', -(C₁-C₆ alkyl)-NR'R", -(C₁-C₆ alkyl)-C(O)OR', -(C₁-C₆ alkyl)-C(O)NR'R", NO₂, CN, -C(O)OR', hydroxyl, hydroxy-(C₁-C₆ alkyl), -S(O)_{Z} aryl, -S(O)_{Z} (C₁-C₆ alkyl), halogen, C₁-C₆ haloalkyl, C₁-C₆ haloalkoxy, phenyl, C₁-C₆ alkanoyl, aryl-C₁-C₆ alkanoyl, aryl-(C₁-C₆ alkyl), arylcarbonyl, heteroarylcarbonyl, or heteroaryl-C₁-C₆ alkanoyl, where the aryl or heteroaryl groups are optionally substituted with 1 to 5 groups that are independently halogen, hydroxyl, C₁-C₆ alkyl, C₁-C₆ alkoxy, C₁-C₆ alkanoyl, C₁-C₆ haloalkyl, C₁-C₆ haloalkoxy, CN or NO₂; and
where each z is independently 0, 1, or 2;
R₁ is hydrogen or C₁-C₆ alkyl;
R₂ is hydrogen, C₁-C₆ alkyl, C₁-C₄ haloalkyl, hydroxyl, hydroxy-(C₁-C₄ alkyl), -NO₂, -CN, C₂-C₆ alkenyl, C₂-C₆ alkynyl, C₁-C₆ alkanoyl, -C(O)OR', -NR'R", -X(CO)Y, - (C(R₃₀)₂)₁₋₄X(CO)Y, unsubstituted C₃-C₆ cycloalkyl, C₃-C₆ cycloalkyl substituted with one to four R₅₀ groups, unsubstituted aryl, aryl substituted with one to four R₅₀ groups, unsubstituted heteroaryl; or heteroaryl substituted with one to four R₅₀ groups, unsubstituted heterocycloalkyl; or heterocycloalkyl substituted with one to four R₅₀ groups; where
each R₃₀ is independently hydrogen or C₁-C₆ alkyl;
each R₅₀ is independently selected from: halogen, C₁-C₆ alkyl, C₁-C₆ haloalkyl, -OH, -O-(C₁-C₆ alkyl), -OCF₃, -CN, -NR₆₀R₇₀, -C(O)O-C₁-C₆ alkyl, -CONR₆₀R₇₀, -(C₁-C₆ alkyl)-NR₆₀R₇₀, -NR₆₀COalkyl, -NR₆₀COaryl, -NR₆₀COheteroaryl, -NR₆₀CONR₆₀R₇₀,
X is: -O-, -NH-, or -N(alkyl)-;
Y is selected from -O-(C₁-C₆ alkyl), -O- phenyl, -NR₆₀R₇₀, or -N(R₃₀)(CH₂)₂-₆NR₆₀R₇₀;
R₆₀ and R₇₀ are independently selected from: hydrogen, C₁-C₆ alkyl, cycloalkyl, arylalkyl; heteroarylalkyl; 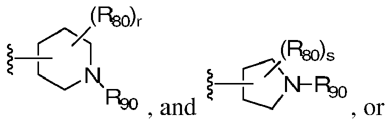
R₆₀ and R₇₀ taken together with the nitrogen atom to which they are bound form a heterocycloalkyl group selected from: 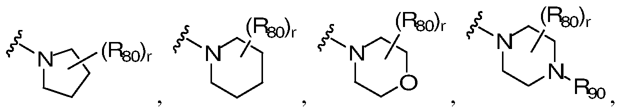 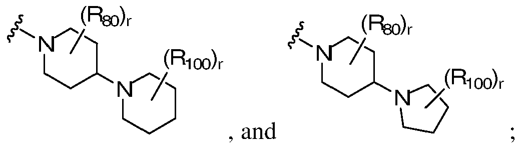 where
each R₈₀ is independently unsubstituted C₁-C₆ alkyl or C₁-C₆ alkyl substituted with hydroxyl or halogen;
each R₉₀ is independently H; unsubstituted C₁-C₆ alkyl; C₁-C₆ alkyl substituted with hydroxyl or halogen; unsubstituted cycloalkyl; cycloalkyl substituted with one to four R₅₀ groups; aryl-(C₁-C₆alkyl); heteroarylalkyl; -C(O)O-(C₁-C₆alkyl); -C(O)O-aryl; -SO₂-(C₁-C₆alkyl); -SO₂-aryl; unsubstituted aryl; aryl substituted with one to four R₅₀ groups; heteroaryl; or heteroaryl substituted with one to four R₅₀ groups;
each R₁₀₀ is independently hydrogen or C₁-C₆ alkyl;
each r is 0 to 4;
each s is 0 to 3;
or R₁ and R₂ combined are oxo or =N-OR where R is hydrogen, C₁-C₆ alkyl, aryl or arylalkyl;
or R₁ and R₂ together with the carbon to which they are attached form a C₃-C₆ cycloalkyl group wherein one of the carbons might be replaced with a heteroatom selected from N, O or S and wherein said C₃-C₆ cycloalkyl ring may be optionally substituted with C₁-C₆ alkyl; and
R' and R" are independently hydrogen, C₁-C₆ alkyl, or phenyl, where the phenyl is optionally substituted with 1 to 5 groups that are independently halogen, hydroxyl, C₁-C₆ alkyl, C₁-C₆ alkoxy, C₁-C₆ alkanoyl, C₁-C₄ haloalkyl, C₁-C₄ haloalkoxy, CN or NO₂, or
R' and R" taken together with the nitrogen atom to which they are bound form a 3 to 7 membered heterocycloalkyl group that may have an additional heteroatom selected from N, O or S and that may be optionally substituted with 1 to 3 R₈₀ or R₉₀ groups;
provided that 5-tosyl-4,5-dihydro-2*H*-pyrazolo[4,3-c]quinoline, 2-phenyl-5-tosyl-4,5-dihydro-2*H*-pyrazolo[4,3-c]quinoline, 7-methoxy-2-phenyl-5-tosyl-4,5-dihydro-2*H*-pyrazolo[4,3-c]quinoline, 8-methoxy-5-tosyl-4,5-dihydro-2*H*-pyrazolo[4,3-c]quinoline, 8-methoxy-2-phenyl-5-tosyl-4,5-dihydro-2*H*-pyrazolo[4,3-c]quinoline, 3-(methylthio)-5-tosyl-4,5-dihydro-2*H*-pyrazolo[4,3-c]quinoline, 7-methoxy-5-tosyl-4,5-dihydro-2*H*-pyrazolo[4,3-c]quinoline, 5-tosyl-4,5-dihydro-2*H*-pyrazolo[4,3-c]quinoline, ethyl 1-(4-sulfamoylphenyl)-5-tosyl-4,5-dihydro-1*H*-pyrazolo[4,3-c]quinoline-3-carboxylate, 1-(4-sulfamoylphenyl)-5-tosyl-4,5-dihydro-1*H*-pyrazolo[4,3-c]quinoline-3-carboxamide and ethyl 1-methyl-5-tosyl-4,5-dihydro-1*H*-pyrazolo[4,3-c]quinoline-3-carboxylate, are not encompassed by formula I.

2. Compounds or salts according to claim 1, wherein
the B-ring is pyrazolyl, imidazolyl, pyrrolyl, triazolyl, pyrrolidinyl, pyrazolidinyl, imidazolidinyl, pyridyl, pyrimidyl, or isoxazolyl, each of which is optionally substituted at a substitutable position with a group that is independently C₁-C₆ alkyl, C₁-C₆ alkoxy, -NR'R", -SO₂-NR'R", -C(O)NR'R", -NR'C(O)R", -NR'C(O)NR'R", -NR'C(O)O-(C₁-C₆ alkyl), -NR'C(O)O-phenyl, NO₂, CN, -C(O)OR', hydroxyl, hydroxy-(C₁-C₆ alkyl), halogen, -S(O)z (C₁-C₆ alkyl), -S(O)z aryl, halogen, C₁-C₂ haloalkyl, C₁-C₂ haloalkoxy, benzyl, or phenyl.

3. Compounds or salts according to claim 1, wherein
the C-ring is cyclohexyl, cyclopentyl, phenyl, naphthyl, thienyl, imidazolyl, pyrimidyl, pyrazinyl, furanyl, thiazolyl, or pyridyl, each of which is optionally substituted at each substitutable position with groups that are independently halogen, C₁-C₆ alkyl, C₁-C₆ alkoxy, aryloxy, aryl-(C₁-C₄alkoxy), C₁-C₆ haloalkyl, (such as CF₃), C₁-C₆ haloalkoxy (such as OCF₃), hydroxyl, hydroxy-(C₁-C₆ alkyl), -NR'R", -(C₁-C₄ alkyl)-NR'R", -NR'C(O)O-(C₁-C₆ alkyl), -NR'C(O)O-phenyl, -COR', -C(O)OR', -C(O)NR'R", -OC(O)NR'R", -NR'C(O)NR'R", -(C₁-C₆ alkyl)-C(O)OR', -(C₁-C₆ alkyl)-C(O)NR'R", -NR'C(O)R", NO₂, CN, oxo, benzyl, phenyl, oxazolyl, pyrazolyl, thiazolyl, pyridyl, furanyl, thienyl, C₂-C₆ alkenyl, C₂-C₆ alkynyl, -NR'SO₂R", -SO₂-NR'R", -S(O)_{Z} (C₁-C₆ alkyl), or -S(O)_{Z} aryl, where the alkyl, alkenyl and alkynyl portions of the above are unsubstituted or substituted with 1, 2 or 3 groups that are independently halogen, hydroxyl, -NR'R" or C₁-C₆ alkoxy, and where the aryl or heteroaryl portions of the above are optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R"; or where two adjacent substituents of the C-ring C-O-(CH₂)₁₋₃-O-.

4. Compounds or salts according to claim 3, wherein the B-ring has the formula: 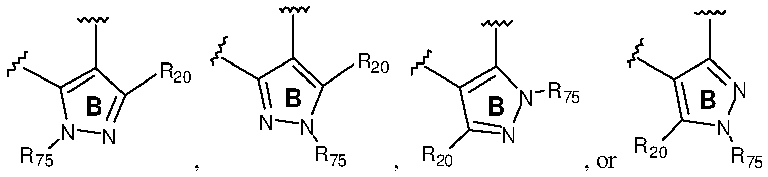 wherein
R₂₀ is hydrogen, C₁-C₆ alkyl, C₁-C₆ alkoxy, -NR'R", -(C₁-C₄ alkyl)-NR'R", -S(O)_{Z} (C₁-C₆ alkyl), hydroxyl, halogen, CN, NO₂, CH₂F, CHF₂, CF₃, -C(O)OR', -C(O)NR'R", -(C₁-C₆ alkyl)-C(O)OR', -(C₁-C₆ alkyl)-C(O)NR'R", or phenyl, and
R₇₅ is hydrogen, C₁-C₆ alkyl, C₁-C₆ alkanoyl, phenyl-(C₁-C₆ alkanoyl)-, -C(O)NR'R", -S(O)_{Z} (C₁-C₆ alkyl), -S(O)z -aryl, -SO₂NR'R", phenyl, phenyl-(C₁-C₆alkyl) (preferably phenethyl or benzyl, more preferably, benzyl), phenylcarbonyl, benzylcarbonyl, or heteroarylcarbonyl, where the heteroaryl group is pyridyl, pyrimidyl, thienyl or furanyl.

5. Compounds or salts according to claim 4, having the following formula: 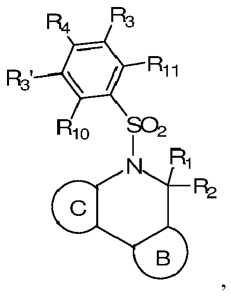 wherein
R₃, R_{3'}, R₄, R₁₀ or R₁₁ are independently hydrogen, halogen, C₁-C₆ alkyl, C₂-C₆ alkenyl, C₂-C₆ alkynyl, C₁-C₆ alkoxy, C₁-C₆ haloalkyl, C₁-C₆ haloalkoxy, hydroxyl, hydroxy-(C₁-C₄ alkyl), CN, NO₂, aryloxy (such as phenyloxy), aryl-(C₁-C₄alkoxy) such as benzyloxy, -SO₂-(C₁-C₆ alkyl), -NR'R", C₁-C₆ alkanoyl, -C(O)OR', -(C₁-C₄ alkyl)-C(O)OR', pyridyl, phenyl, phenyl-(C₁-C₄ alkyl)-, -SO₂-NR'R", -C(O)NR'R", -OC(O)NR'R", -NR'C(O)R", -NR'C(O)NR'R", or -NR'C(O)O-R', where each R' and R" is independently hydrogen, C₁-C₆ alkyl, or phenyl, where the phenyl is optionally substituted with 1 to 5 groups that are independently halogen, hydroxyl, C₁-C₆ alkyl, C₁-C₆ alkoxy, C₁-C₆ alkanoyl, C₁-C₄ haloalkyl, C₁-C₄ haloalkoxy, CN or NO₂; or
R₄ and R_{3'}, or R₁₀ and R₃, and the carbons to which they are attached form a benzo ring.

6. Compounds or salts according to claim 5, wherein the B-ring has the formula: 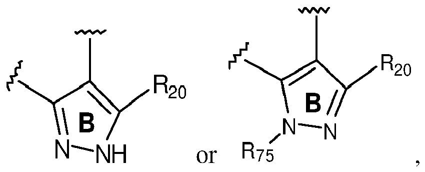 wherein R₂₀ and R₇₅ are both hydrogen.

7. Compounds or salts according to claim 6, wherein the C-ring is phenyl, which is optionally substituted with 1, 2, 3, or 4 groups that are independently halogen, C₁-C₄ alkyl, C₁-C₄ alkoxy, aryloxy, aryl-(C₁-C₄ alkoxy), C₁-C₄ haloalkyl (in another aspect, CF₃), C₁-C₄ haloalkoxy (in another aspect, OCF₃), hydroxyl, hydroxy-(C₁-C₄ alkyl), -NR'R", -(C₁-C₄ alkyl)-NR'R", -NR'C(O)O-(C₁-C₆ alkyl), -NR'C(O)O-phenyl, -C(O)OR', -C(O)NR'R", -OC(O)NR'R", -NR'C(O)NR'R"; -(C₁-C₆ alkyl)-C(O)OR', -(C₁-C₆ alkyl)-C(O)NR'R", -NR'C(O)R", NO₂, CN, oxo, benzyl, phenyl, oxazolyl, pyrazolyl, thiazolyl, pyridyl, furanyl, thienyl, C₂-C₆ alkenyl, C₂-C₆ alkynyl, -S(O)_{Z} phenyl, or -S(O)_{Z} (C₁-C₆ alkyl), where the alkyl, alkenyl and alkynyl portions of the above are unsubstituted or substituted with 1, 2 or 3 groups that are independently halogen, hydroxy, -NR'R" or C₁-C₆ alkoxy, and where the aryl or heteroaryl portions of the above are optionally substituted with 1, 2, 3, or 4 groups that are independently C₁-C₆ alkyl, C₁-C₆ alkoxy, halogen, C₁-C₆ haloalkyl (such as CF₃), C₁-C₆ haloalkoxy (such as OCF₃), hydroxyl, hydroxy-(C₁-C₆ alkyl), or -NR'R" or where two adjacent substituents from the C-ring form -O-(CH₂)₁₋₃-O-.

8. Compounds or salts according to claim 7, wherein R₄ is Cl, F, CH₂F, CHF₂, CF₃, OCH₂F, OCHF₂, or OCF₃; R₃, R_{3'}, R₁₀ and R₁₁ are independently Cl, F, methyl or hydrogen; R₂ is independently hydrogen, methyl, ethyl, propyl, isopropyl, or cyclopropyl; and R₁ is H or methyl.

9. Compounds or salts according to claim 8, wherein the C-ring is phenyl substituted with 1 or 2 groups that are Cl, F, methyl, ethyl, isopropyl, methoxy, CF₃, OCF₃, OH, -OC(O)N(CH₃)₂ or with two adjacent substituents forming -O-(CH₂)₁₋₃-O-, where the C-ring is substituted at least at the 8-position.

10. Compounds or salts according to claim 6, wherein
R₄ is halogen or C₁-C₄ alkyl; R₁ is H; R₂ is H, C₁-C₄ alkyl, or C₃-C₆ cycloalkyl; and the C-ring is phenyl substituted with one or two halogens.

11. Compounds or salts according to claim 6, wherein
R₄ is F, Cl, or CF₃; R₂ is H, methyl, ethyl, isopropyl, or cyclopropyl; and the C-ring is phenyl substituted with one or two halogens.

12. Compounds or salts according to claim 4, wherein
the A-ring is heteroaryl, which is pyridyl, pyrimidyl, quinolinyl, isoquinolinyl, thienyl, thiazolyl, imidazolyl, pyrazolyl, isoxazolyl, or oxazolyl, each of which is optionally substituted at a substitutable position with halogen, C₁-C₆ alkyl, C₂-C₆ alkenyl, C₂-C₆ alkynyl, C₁-C₆ alkoxy, C₁-C₆ haloalkyl, C₁-C₆ haloalkoxy, hydroxyl, hydroxy-(C₁-C₄ alkyl), CN, NO₂, aryloxy, aryl-(C₁-C₄ alkoxy) (such as benzyloxy),-SO₂-(C₁-C₆ alkyl), -NR'R", C₁-C₆ alkanoyl, -C(O)OR', -(C₁-C₄ alkyl)-C(O)OR', heteroaryl, aryl, aryl-(C₁-C₄ alkyl), -SO₂-NR'R", - C(O)NR'R", -OC(O)NR'R", -NR'C(O)R", -NR'C(O)NR'R", -NR'C(O)O-(C₁-C₆ alkyl) or -NR'C(O)O-phenyl, where each R' and R" is independently hydrogen or C₁-C₆ alkyl.

13. Compounds or salts according to claim 12, wherein the B-ring has the formula: 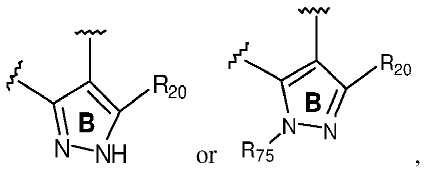 wherein R₂₀ and R₇₅ are both hydrogen.

14. Compounds or salts according to claim 13, wherein C-ring is phenyl, which is optionally substituted with 1, 2, 3, or 4 groups that are independently halogen, C₁-C₄ alkyl, C₁-C₄ alkoxy, aryloxy, aryl-(C₁-C₄ alkoxy), C₁-C₄ haloalkyl, C₁-C₄ haloalkoxy, hydroxyl, hydroxy-(C₁-C₄ alkyl), -NR'R", -(C₁-C₄ alkyl)-NR'R", -NR'C(O)O-(C₁-C₆ alkyl), -NR'C(O)O-phenyl, -COR', -C(O)OR', -C(O)NR'R", -OC(O)NR'R", -NR'C(O)NR'R"; -(C₁-C₆ alkyl)-C(O)OR', -(C₁-C₆ alkyl)-C(O)NR'R", -NR'C(O)R", NO₂, CN, phenyl, oxazolyl, pyrazolyl, thiazolyl, pyridyl, furanyl, thienyl, C₂-C₆ alkenyl, C₂-C₆ alkynyl, -NR'SO₂R", -SO₂-NR'R", -S(O)_{z} phenyl, or -S(O)_{z} (C₁-C₆ alkyl), where the alkyl, alkenyl and alkynyl portions of the above are unsubstituted or substituted with 1 or 2 groups that are independently halogen, hydroxyl, -NR'R" or C₁-C₆ alkoxy, and where the aryl or heteroaryl portions of the above are optionally substituted with 1, 2, 3, or 4 groups that are independently C₁-C₆ alkyl, C₁-C₆ alkoxy, halogen, C₁-C₆ haloalkyl, C₁-C₆ haloalkoxy, hydroxyl, hydroxy-(C₁-C₆ alkyl), or -NR'R".

15. Compounds or salts according to claim 14, wherein R₂ is independently hydrogen, methyl, ethyl, propyl, isopropyl, or cyclopropyl; and R₁ is H or methyl.

16. Compounds or salts according to claim 15, wherein the C-ring is phenyl substituted with 1 or 2 groups that are Cl, F, methyl, ethyl, isopropyl, methoxy, CF₃, OCF₃, OH, or -OC(O)N(CH₃)₂, where the C-ring is substituted at least at the 8-position.

17. Compounds or salts according to claim 13, wherein
R₄ is halogen or C₁-C₄ alkyl; R₁ is H; R₂ is H, C₁-C₄ alkyl, or C₃-C₆ cycloalkyl; and the C-ring is phenyl substituted with one or two halogens.

18. Compounds or salts according to claim 13, wherein
R₄ is F, Cl, or CF₃; R₂ is H, methyl, ethyl, isopropyl, or cyclopropyl; and the C-ring is phenyl substituted with one or two halogens.

19. Compounds or salts according to claim 13, wherein
the A-ring is heteroaryl, which is pyridyl, or thienyl, each of which is optionally substituted with 1 or 2 groups that are independently halo, methyl, methoxy, CF₃, or OCF₃.

20. Compounds or salts according to claim 12, wherein
the A-ring is a heteroaryl group, which has the following structures: 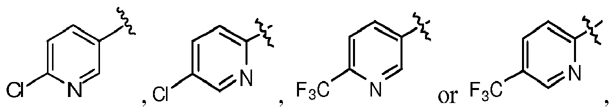 R₁ is hydrogen or methyl; R₂ is H, C₁-C₄ alkyl, or C₃-C₆ cycloalkyl; the C-ring is phenyl substituted with 1 or 2 groups that are independently Cl, F, methyl, ethyl, isopropyl, methoxy, ethoxy, CF₃, OCF₃, OH, CH₂OH, NH₂, NH(CH₃), or N(CH₃)₂; and the B-ring has the formula: 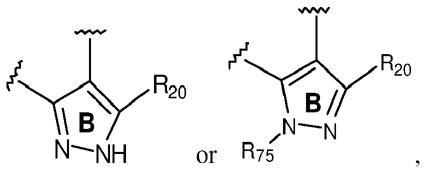 where R₂₀ and R₇₅ are both hydrogen.

21. Compounds, stereoisomers, tautomers, mixtures of stereoisomers and/or tautomers or pharmaceutically acceptable salts thereof, of claim 1 that are
5-[(4-chlorophenyl)sulfonyl]-4,5-dihydro-1*H*-pyrazolo[4,3-*c*]quinoline;
5-[(4-chlorophenyl)sulfonyl]-8-fluoro-4,5-dihydro-2*H*-pyrazolo[4,3-*c*]quinoline;
5-[(4-chlorophenyl)sulfonyl]-7-fluoro-4,5-dihydro-2*H*-pyrazolo[4,3-*c*]quinoline;
5-[(4-chlorophenyl)sulfonyl]-9-fluoro-4,5-dihydro-2*H*-pyrazolo[4,3-*c*]quinoline;
5-[(4-chlorophenyl)sulfonyl]-4-methyl-4,5-dihydro-2*H*-pyrazolo[4,3-*c*]quinoline (racemic);
5-[(4-chlorophenyl)sulfonyl]-7-methoxy-4-methyl-4,5-dihydro-1*H*-pyrazolo[4,3-*c*]quinoline;
5-[(4-chlorophenyl)sulfonyl]-9-methoxy-4-methyl-4,5-dihydro-1*H*-pyrazolo[4,3-*c*]quinoline;
5-[(4-chlorophenyl)sulfonyl]-3-methyl-4,5-dihydro-1*H*-pyrazolo[4,3-*c*]quinoline;
8-chloro-5-[(4-chlorophenyl)sulfonyl]-4,5-dihydro-2*H*-pyrazolo[4,3-*c*]quinoline;
4-methyl-5-(pyridin-2-ylsulfonyl)-4,5-dihydro-2*H*-pyrazolo[4,3-*c*]quinoline;
5-[(4-chlorophenyl)sulfonyl]-4,5-dihydro-1*H*-pyrazolo[4,3-*c*]quinolin-3-ol;
5-[(4-chlorophenyl)sulfonyl]-4-methyl-4,5-dihydro-2*H*-pyrazolo[4,3-*c*]quinofine; (resolved enantiomer, with a retention time of about 9.3 min.*
5-[(4-chlorophenyl)sulfonyl]-4-methyl-4,5-dihydro-2*H*-pyrazolo[4,3-*c*]quinoline (resolved enantiomer, with a retention time of about 20.5 min.*);
5-[(4-chlorophenyl)sulfonyl]-8-fluoro-4-methyl-4,5-dihydro-2*H*-pyrazolo[4,3-*c*]quinoline (racemic);
5-[(4-chlorophenyl)sulfonyl]-8-fluoro-4-methyl-4,5-dihydro-2*H*-pyrazolo[4,3-*c*]quinoline resolved enantiomer, with a retention time of about 9.8 min.*);
8-fluoro-4-methyl-5-(pyridin-3-ylsulfonyl)-4,5-dihydro-1*H*-pyrazolo[4,3-*c*]quinoline (racemic);
8-fluoro-4-methyl-5-(pyridin-2-ylsulfonyl)-4,5-dihydro-1*H*-pyrazolo[4,3-*c*]quinoline;
4-(4-cyclopropyl-1*H*-pyrazolo[4,3-*c*]quinolin-5(4*H*)-ylsulfonyl)benzonitrile;
4-methyl-5-(pyridin-3-ylsulfonyl)-4,5-dihydro-1*H*-pyrazolo[4,3-*c*]quinoline;
8-fluoro-4-methyl-5-(pyridin-3-ylsulfonyl)-4,5-dihydro-1*H*-pyrazolo[4,3-*c*]quinoline (resolved enantiomer, with a retention time of about 15.2 min.*);
8-fluoro-4-methyl-5-(pyridin-3-ylsulfonyl)-4,5-dihydro-1*H*-pyrazolo[4,3-*c*]quinoline (resolved enantiomer, with a retention time of about 17.2 min.*);
5-[(4-chlorophenyl)sulfonyl]-1,5-dihydro-4*H*-pyrazolo[4,3-*c*]quinolin-4-one;
5-[(4-chlorophenyl)sulfonyl]-4-methyl-4,5-dihydro-1*H*-pyrazolo[4,3-*c*]-1,5-naphthyridine;
8-fluoro-4-methyl-5-(pyridin-2-ylsulfonyl)-4,5-dihydro-1*H*-pyrazolo[4,3-c]quinoline;
5-(4-chlorophenylsulfonyl)-4-ethyl-4,5-dihydro-1*H*-pyrazolo[4,3-c]quinoline;
5-(4-chlorophenylsulfonyl)-4-methyl-4,5-dihydro-1*H*-pyrazolo[4,3-c]quinolin-7-ol;
5-(4-chlorophenylsulfonyl)-8-fluoro-1*H*-pyrazolo[4,3-c]quinolin-4(5*H*)-one;
5-(4-chlorophenylsulfonyl)-4-methyl-4,5,5a,6,7,8,9,9a-octahydro-1*H*-pyrazolo[4,3-c]quinoline;
5-(4-chlorophenylsulfonyl)-9-fluoro-4-methyl-4,5-dihydro-2*H*-pyrazolo[4,3-c]quinoline;
5-(4-chlorophenylsulfonyl)-7-fluoro-4-methyl-4,5-dihydro-2*H*-pyrazolo[4,3-c]quinoline;
8-fluoro-5-(4-fluorophenylsulfonyl)-4-methyl-4,5-dihydro-1*H*-pyrazolo[4,3-c]quinoline;
4-methyl-5-(pyridin-2-ylsulfonyl)-4,5-dihydro-1*H*-pyrazolo[4,3-c]quinoline;
5-(4-chlorophenylsulfonyl)-4-cyclopropyl-4,5-dihydro-2*H*-pyrazolo[4,3-c]quinoline;
5-(4-chlorophenylsulfonyl)-4-(trifluoromethyl)-4,5-dihydro-2*H*-pyrazolo[4,3-c]quinoline;
5-(4-chlorophenylsulfonyl)-4-methyl-4,5-dihydro-1*H*-pyrazolo[4,3-c]quinolin-7-ol;
5-(4-chlorophenylsulfonyl)-4-isopropyl-4,5-dihydro-2*H*-pyrazolo[4,3-c]quinoline;
1,5-bis(4-chlorophenylsulfonyl)-1*H*-pyrazolo[4,3-c]quinolin-4(5*H*)-one;
8-fluoro-5-(4-fluorophenylsulfonyl)-4-methyl-4,5-dihydro-1*H*-pyrazolo[4,3-c]quinoline;
5'-(4-chlorophenylsulfonyl)-2',5'-dihydrospiro[cyclopropane-1,4'-pyrazolo[4,3-c] quinoline] ;
5-(4-chlorophenylsulfonyl)-7,8-difluoro-4-methyl-4,5-dihydro-2*H*-pyrazolo[4,3-c]quinoline;
7,8-difluoro-4-methyl-5-(thiophen-2-ylsulfonyl)-4,5-dihydro-2*H*-pyrazolo[4,3-c]quinoline;
5-(4-chlorophenylsulfonyl)-4-methyl-4,5-dihydro-2*H*-pyrazolo[4,3-c][1,8]naphthyridine;
5-(4-chlorophenylsulfonyl)-4-ethyl-4,5-dihydro-1*H*-pyrazolo[4,3-c]quinoline;
5-(5-chlorothiophen-2-ylsulfonyl)-7,8-difluoro-4-methyl-4,5-dihydro-1*H-*pyrazolo[4,3-c]quinoline;
5-(4-chlorophenylsulfonyl)-4-methyl-4,5-dihydro-1*H*-pyrazolo[4,3-c]quinolin-7-yl dimethylcarbamate;
4-cyclopropyl-7,8-difluoro-5-(4-(trifluoromethyl)phenylsulfonyl)-4,5-dihydro-1*H-*pyrazolo[4,3-c]quinoline;
7-chloro-4-methyl-5-(4-(trifluoromethyl)phenylsulfonyl)-4,5-dihydro-1*H-*pyrazolo[3,4-d]thieno[2,3-b]pyridine;
4-ethyl-7,8-difluoro-5-(5-(trifluoromethyl)pyridin-2-ylsulfonyl)-4,5-dihydro-1*H-*pyrazolo[4,3-c]quinoline;
7,8-difluoro-4-methyl-5-(4-(trifluoromethyl)phenylsulfonyl)-4,5-dihydro-1*H-*pyrazolo[4,3-c]quinoline;
5-(4-chlorophenylsulfonyl)-4-methyl-4,5-dihydro-2*H*-pyrazolo[4,3-c][1,7]naphthyridine;
5'-(4-(trifluoromethyl)phenylsulfonyl)- ',5'-dihydrospiro[cyclopropane-1,4'-pyrazolo[4,3-c]quinoline];
5-(4-chlorophenylsulfonyl)-4-methyl-4,5-dihydro-1*H*-imidazo[1,2-a]pyrazolo[4,3-e]pyrimidine;
4-cyclopropyl-7,8-difluoro-5-(5-(trifluoromethyl)pyridin-2-ylsulfonyl)-4,5-dihydro-1*H*-pyrazolo[4,3-*c*]quinoline;
5-(5-chlorothiophen-2-ylsulfonyl)-4-cyclopropyl-7,8-difluoro-4,5-dihydro-2*H-*pyrazolo[4,3-c]quinoline;
4-cyclopropyl-7,8-difluoro-5-(4-fluorophenylsulfonyl)-4,5-dihydro-2*H*-pyrazolo[4,3-c]quinoline;
4-cyclopropyl-5-(4-(trifluoromethyl)phenylsulfonyl)-4,5-dihydro-2*H*-pyrazolo[4,3-c][1,8]naphthyridine;
4-cyclopropyl-5-(4-fluorophenylsulfonyl)-4,5-dihydro-2*H*-pyrazolo[4,3-c][1,8]naphthyridine;
4-cyclopropyl-5-(4-fluorophenylsulfonyl)-4,5-dihydro-2*H*-pyrazolo[4,3-c]quinoline;
4-cyclopropyl-7,8-difluoro-1-(methoxymethyl)-5-(6-(trifluoromethyl)pyridin-3-ylsulfonyl)-4,5-dihydro-1*H*-pyrazolo[4,3-c]quinoline;
4-cyclopropyl-7,8-difluoro-5-(thiophen-2-ylsulfonyl)-4,5-dihydro-2*H*-pyrazolo[4,3-c]quinoline;
4-cyclopropyl-5-(4-(trifluoromethyl)phenylsulfonyl)-4,5-dihydro-1*H*-imidazo[1,2-a]pyrazolo[4,3-e]pyrimidine;
4-cyclopropyl-7,8-difluoro-5-(6-(trifluoromethyl)pyridin-3-ylsulfonyl)-4,5-dihydro-1*H*-pyrazolo[4,3-*c*]quinoline;
5-(4-fluorophenylsulfonyl)-4-(trifluoromethyl)-4,5-dihydro-2*H*-pyrazolo[4,3-c][1,8]naphthyridine;
7,8-difluoro-4-(pyridin-3-yl)-5-(4-(trifluoromethyl)phenylsulfonyl)-4,5-dihydro-1*H-*pyrazolo[4,3-c]quinoline;
4-cyclopropyl-8-fluoro-5-(4-(trifluoromethyl)phenylsulfonyl)-4,5-dihydro-1*H-*pyrazolo[4,3-c]quinoline;
5-(4-chlorophenylsulfonyl)-4-methyl-4,5-dihydro-2*H-*[1,3]dioxolo[4,5-g]pyrazolo[4,3-c]quinoline;
5-(5-chloropyridin-2-ylsulfonyl)-4-cyclopropyl-7,8-difluoro-4,5-dihydro-1*H-*pyrazolo[4,3-c]quinoline;
5-(5-chlorothiophen-2-ylsulfonyl)-4-cyclopropyl-7,8-difluoro-4,5-dihydro-2*H-*pyrazolo[4,3-c]quinoline;
4-cyclopropyl-7,8-difluoro-5-(4-fluorophenylsulfonyl)-4,5-dihydro-2*H*-pyrazolo[4,3-c]quinoline;
4-cyclopropyl-8-fluoro-5-(6-(trifluoromethyl)pyridin-3-ylsulfonyl)-4,5-dihydro-2*H-*pyrazolo[4,3-c]quinoline;
4-cyclopropyl-7,8-difluoro-5-(6-(trifluoromethyl)pyridin-3-ylsulfonyl)-4,5-dihydro-1*H*-pyrazolo[4,3-*c*]quinoline;
7,8-difluoro-4-isopropyl-5-(4-(trifluoromethyl)phenylsulfonyl)-4,5-dihydro-2*H-*pyrazolo[4,3-c]quinoline;
4-cyclopropyl-7,8-difluoro-5-(4-(trifluoromethoxy)phenylsulfonyl)-4,5-dihydro-1*H-*pyrazolo[4,3-c]quinoline;
7,8-difluoro-5-(4-(trifluoromethyl)phenylsulfonyl)-4,5-dihydro-2*H*-pyrazolo[4,3-c]quinoline;
5-(4-chlorophenylsulfonyl)-4-methyl-4,5-dihydro-1*H*-pyrazolo[4,3-c]quinoline-7,8-diol;
5-(5-chloropyridin-2-ylsulfonyl)-4-cyclopropyl-8-fluoro-4,5-dihydro-1*H-*pyrazolo[4,3-c]quinoline;
4-cyclopropyl-5-(6-(trifluoromethyl)pyridin-3-ylsulfonyl)-4,5-dihydro-1*H-*pyrazolo[4,3-c]quinoline;
4-cyclopropyl-7-(trifluoromethoxy)-5-(6-(trifluoromethyl)pyridin-3-ylsulfonyl)-4,5-dihydro-2*H*-pyrazolo[4,3-c]quinoline;
4-cyclopropyl-5-(4-(trifluoromethyl)phenylsulfonyl)-4,5-dihydro-2*H*-pyrazolo[4,3-c][1,7]naphthyridine 7-oxide;
5-(4-chlorophenylsulfonyl)-4-methyl-4,5-dihydro-2*H*-pyrazolo[4,3-c]quinolin-8-ol;
4-(8-fluoro-4-methyl-1*H*-pyrazolo[4,3-*c*]quinolin-5(4*H*)-ylsulfonyl)-3,5-dimethylisoxazole;
8-fluoro-4-methyl-5-(1-methyl-1*H*-pyrazol-4-ylsulfonyl)-4,5-dihydro-1*H-*pyrazolo[4,3-c]quinoline;
8-fluoro-4-methyl-5-(5-(pyridin-2-yl)thiophen-2-ylsulfonyl)-4,5-dihydro-1*H-*pyrazolo[4,3-c]quinoline;
4-cyclopropyl-7-(trifluoromethyl)-5-(6-(trifluoromethyl)pyridin-3-ylsulfonyl)-4,5-dihydro-1*H*-pyrazolo[4,3-*c*]quinoline;
4-cyclopropyl-7-(trifluoromethoxy)-5-(4-(trifluoromethoxy)phenylsulfonyl)-4,5-dihydro-2*H*-pyrazolo[4,3-c]quinoline;
4-(4-cyclopropyl-7,8-difluoro-1*H*-pyrazolo[4,3-*c*]quinolin-5(4*H*)-ylsulfonyl)aniline;
4-cyclopropyl-7,8-difluoro-5-(4-nitrophenylsulfonyl)-4,5-dihydro-1*H*-pyrazolo[4,3-c]quinoline;
4-cyclopropyl-8-(trifluoromethyl)-5-(6-(trifluoromethyl)pyridin-3-ylsulfonyl)-4,5-dihydro-1*H*-pyrazolo[4,3-c]quinoline;
5-chloro-2-(4-cyclopropyl-7,8-difluoro-1H-pyrazolo[4,3-c]quinolin-5(4H)-ylsulfonyl)thiazole;
4-cyclopropyl-5-(4-(difluoromethoxy)phenylsulfonyl)-7, 8-difluoro-4,5-dihydro-1*H-*pyrazolo[4,3-*c*]quinoline;
4-cyclopropyl-5-(4-(trifluoromethyl)phenylsulfonyl)-4,5-dihydro-2*H*-pyrazolo[4,3-c][1,5]naphthyridine;
4-cyclopropyl-8-fluoro-5-(5-(trifluoromethyl)pyridin-2-ylsulfonyl)-4,5-dihydro-1*H-*pyrazolo[4,3-c]quinoline;
4-cyclopropyl-8-fluoro-5-(5-(trifluoromethyl)pyridin-2-ylsulfonyl)-4,5-dihydro-1*H-*pyrazolo[4,3-c]quinoline;
4-cyclopropyl-5-(4-(difluoromethoxy)phenylsulfonyl)-8-fluoro-4,5-dihydro-1*H-*pyrazolo[4,3-c]quinoline;
4-cyclopropyl-7,8-difluoro-5-(5-(trifluoromethyl)pyridin-2-ylsulfonyl)-4,5-dihydro-1*H*-pyrazolo[4,3-c]quinoline;
5-(4-chlorophenylsulfonyl)-4-methyl-4,5-dihydro-1*H*-pyrazolo[4,3-c]quinoline-7,8-diol;
8-fluoro-4-methyl-5-(1-methyl-1H-imidazol-4-ylsulfonyl)-4,5-dihydro-1*H-*pyrazolo[4,3-c]quinoline;
(*R*)-4-ethyl-7,8-difluoro-5-(4-(trifluoromethyl)phenylsulfonyl)-4,5-dihydro-1*H-*pyrazolo[4,3-c]quinoline;
(R)-4-cyclopropyl-8-fluoro-5-(4-(trifluoromethoxy)phenylsulfonyl)-4,5-dihydro-1H-pyrazolo[4,3-c]quinoline;
4-cyclopropyl-7-methyl-5-(4-(trifluoromethyl)phenylsulfonyl)-4,5-dihydro-1H-pyrazolo[3,4-d]thiazolo[5,4-b]pyridine; or
(R)-4-cyclopropyl-8-fluoro-5-(4-methoxyphenylsulfonyl)-4,5-dihydro-1H-pyrazolo[4,3-c]quinoline.

22. A pharmaceutical composition comprising a compound of claim 1, a pharmaceutically acceptable salt of claim 1, or a combination of a compound and a pharmaceutically acceptable salt of claim 1, further comprising at least one solvent, excipient, adjuvant, or a mixture thereof.

23. A method of treating an A beta-related disease comprising administering a therapeutically effective amount of a compound or salt of claim 1 to a patient in need of such treatment.

24. A method of treating Alzheimer's disease, mild cognitive impairment, dementia, or Down's syndrome, comprising administering a therapeutically effective amount of a compound or salt of claim 1 to a patient in need of such treatment.

25. A method of treating Alzheimer's disease, mild cognitive impairment, dementia, or Down's syndrome, comprising administering a therapeutically effective amount of a compound or salt of claim 1, where claim 1 further comprises 5-tosyl-4,5-dihydro-2*H-*pyrazolo[4,3-c]quinoline, 2-phenyl-5-tosyl-4,5-dihydro-2*H*-pyrazolo[4,3-c]quinoline, 7-methoxy-2-phenyl-5-tosyl-4,5-dihydro-2*H*-pyrazolo[4,3-c]quinoline, 8-methoxy-5-tosyl-4,5-dihydro-2*H*-pyrazolo[4,3-c]quinoline, 8-methoxy-2-phenyl-5-tosyl-4,5-dihydro-2*H-*pyrazolo[4,3-c]quinoline, 3-(methylthio)-5-tosyl-4,5-dihydro-2*H*-pyrazolo[4,3-c]quinoline, 7-methoxy-5-tosyl-4,5-dihydro-2*H*-pyrazolo[4,3-c]quinoline, 5-tosyl-4,5-dihydro-2*H-*pyrazolo[4,3-c]quinoline, ethyl 1-(4-sulfamoylphenyl)-5-tosyl-4,5-dihydro-1*H*-pyrazolo[4,3-c]quinoline-3-carboxylate, 1-(4-sulfamoylphenyl)-5-tosyl-4,5-dihydro-1*H*-pyrazolo[4,3-c]quinoline-3-carboxamide ethyl l-methyl-5-tosyl-4,5-dihydro-1*H*-pyrazolo[4,3-c]quinoline-3-carboxylate, and pharmaceutically acceptable salts thereof, or a combination of a compound and a pharmaceutically acceptable salt, to a patient in need of such treatment
